# EUROPEAN PATENT APPLICATION

(11) **EP 4 467 984 A2**
(43) Date of publication of application: **27.11.2024**
(21) Application number: 24200645.0
(22) Date of filing: 29.03.2010
(51) Int. Cl.: G01N 33/68

(54) **FIBROSIS BIOMARKER ASSAY**

(30) Priority: 30.03.2009 US 211467 P; 22.12.2009 US 289081 P
(62) Divisional of application: 19174151.1
(71) Applicant: Nordic Bioscience A/S, 2730 Herlev (DK); Novartis AG, 4056 Basel (CH)
(72) Inventor: VEIDAL, Sanne, 3650 Olstykke (DK); KARSDAL, Morten, 2100 København Ø (DK); LEEMING, Diana Julie, 3060 Espergaerde (DK); BARASCUK MICHAELSEN, Natasha, 2100 Copenhagen (DK); SKJOT-ARKIL, Helene, 2300 Copenhagen (DK); SEGOVIA-SILVESTRE, Antonio, 2200 Copenhagen (DK); VASSILIADIS, Efstathios, 2610 Rødovre (DK)
(74) Representative: Beck Greener LLP

(57) **Abstract**

Methods of diagnosis or of quantitation of fibrosis comprising conducting an immunoassay to measure neo-epitope containing protein fragments naturally present in a bioflui sample, and associating an elevation of said measure in said patient above a normal level with the presence or extent of fibrosis. The immunoassay is conducted by a method comprising:
contacting protein fragments naturally present in said sample with an immunological binding partner reactive with a neo-epitope formed by cleavage of a protein by a proteinase and measuring the extent of binding of peptide fragments to said immunological binding partner to measure therein protein fragments comprising said neo-epitope, and wherein said protein is collagen type III, collagen type I, collagen type IV, collagen type V, or collagen type VI, elastin, biglycan, decorin, lumican, versican, perlecan, neurocan, brevican, fibromodulin, serglycin, syndecan, betaglycan, vimentin, or C-reactive protein.

## Description

The present invention relates to assays for biomarkers useful in the diagnosis of fibrosis disease and prognosis of its development, including biomarkers indicative of the risk of developing fibrosis after a chronic injury.

In particular, according to the present invention, biomarkers relating to degradation fragments of Collagen type I, III, IV, V, and VI, elastin, C-reactive protein, and proteoglycans including Biglycan, Decorin, Versican, and Perlecan are found to be useful.

Fibrotic diseases (including those listed in Table 1) are a leading cause of morbidity and mortality, e.g. cirrhosis with 800,000 death per year worldwide¹.

**Table 1. Different fibrotic diseases²**

| Tissue | Examples of Causes |
|---|---|
| Liver | Viral hepatitis |
| | Schistosomiasis |
| | Steatohepatitis (Alcoholic or non-alcoholic) |
| Lung | Idiopathic pulmonary fibrosis (IPF) |
| | Systemic sclerosis (Scleroderma) |
| Kidney | Nephrogenic systemic fibrosis (NSF) |
| | Diabetes |
| | Untreated hypertension |
| Heart | Heart attack |
| | Hypertension |
| | Atherosclerosis |
| | Restenosis |
| Eye | Macular degeneration, retinal and vitreal retinopathy |
| Skin | Systemic sclerosis and scleroderma, keloids, hypertrophic scars, burns, genetic factors NFS |
| Pancreas | Autoimmune/hereditary causes |
| Intestine | Crohn's disease/inflammatory bowl disease |
| Brain | Alzheimer's disease, AIDS |
| Bone marrow | Cancer, ageing |
| Multi-organ fibrosis | Surgical complications, chemotherapeutic drug-induced fibrosis, radiation-induced fibrosis, mechanical injuries |

A 'fibrotic disease' is any disease giving rise to fibrosis, whether as a main or a secondary symptom.

Fibrosis is the end result of chronic inflammatory reactions induced by a variety of stimuli including persistent infections, autoimmune reactions, allergic responses, chemical insults, radiation, and tissue injury. Fibrosis is characterized by the accumulation and reorganization of the extracellular matrix (ECM). Despite having obvious etiological and clinical distinctions, most chronic fibrotic disorders have in common a persistent irritant that sustains the production of growth factors, proteolytic enzymes, angiogenic factors, and fibrogenic cytokines, which together stimulate the deposition of connective tissue elements, especially collagens and proteoglycans, which progressively remodel and destroy normal tissue architecture ^{3, 4}. Despite its enormous impact on human health, there are currently no approved treatments that directly target the mechanisms of fibrosis ⁵.

The key cellular mediator of fibrosis is the myofibroblast, which when activated serves as the primary collagen-producing cell.

### Extracellular Matrix (ECM)

Fibrogenesis is a dynamic process involving complex cellular and molecular mechanisms that usually originates from tissue injury ⁶. Fibrogenesis is the result of an imbalance in normal ECM regulation that alters the concentration of macromolecules leading to increased tissue size and density, with progressively impaired function. These macromolecules are mainly fibrous proteins with structural and adhesive functions, such as collagens and proteoglycans.

### Collagen

Collagens are widely distributed in the human body, i.e. ~ 30% of the protein mass in the human body is composed of collagens. Collagens are responsible for the structural integrity of the ECM of most connective tissues. The ECM content results from a fine balance between synthesis and degradation tightly controlled through regulation of gene expression and protein secretion, but also through endogenous protease inhibition and protein degradation by metalloproteinases and cysteine proteases ⁷⁻⁹. Table 2 lists the major collagen types with their major tissue distribution.

**Table 2. Major collagen types and their tissue distribution.**

| Collagen type | Tissue distribution |
|---|---|
| I | Most connective tissues |
| II | Cartilage, vitreous humor |
| III | Extensible connective tissues, e.g. liver, skin, lung, vascular system |
| IV | Basement membranes |
| V | Tissues containing collagen I |
| VI | Most connective tissues |
| VII | Skin, bladder, oral mucosa, umbilical cord, amnion |
| VIII | Many tissues, especially endothelium |
| XIII | Endothelial cells, skin, eye, heart, skeletal muscle |
| XIV | Vessel, bone, skin, cartilage, eye, nerve, tendon, uterus |
| XXI | Vessel, heart, stomach, kidney, skeletal muscle, placenta |

Type I collagen is the most abundant collagen and is found in most connective tissues. It is especially important for the structure of bone and skin where the major collagenous components are type I and III collagens ¹⁰.

Collagen type I and III are the major components of liver and lung in a 1:1 ratio in healthy tissue. In addition, collagen type IV and VI are found in the basement membranes in most tissues. The most common localization of type V collagen is within the characteristic collagen fibrils, in association with the collagen type I and III ¹⁰.

Some collagens have a restricted tissue distribution: for example, type II, which is found almost exclusively in cartilage ¹¹.

During fibrogenesis the net amount of collagens increases¹²⁻¹⁴. Table 3 shows by way of example the collagen increase during liver fibrosis.

**Table 3. Changes of the composition of collagen from normal to cirrhotic human liver ¹⁵.**

| Collagen type | Chains | Collagen normal liver (mg/g) | Collagen cirrhotic liver (mg/g) | Times increased | Distribut ion normal liver (%) | Distribut ion cirrhotic liver (%) |
|---|---|---|---|---|---|---|
| I | α₁ (I) | | 16 | 8 | 37 | 42 |
| | α₂ (I) | 2 | | | | |
| III | α₁ (III) | 2 | 8 | 4 | 37 | 21 |
| IV | α₁ (IV) | 0.5 | 7 | 14 | 9 | 18 |
| | α₂ (IV) | | | | | |
| V | α₁ (V) | 0.9 | 7 | 8 | 17 | 18 |
| | α₂ (V) | | | | | |
| | α₃ (V) | | | | | |
| VI | α₁ (VI) | 0.01 | 0.1 | 10 | 0.2 | 0.3 |
| | α₂ (VI) | | | | | |

### Elastin

Elastin is a protein present in many connective tissues, primarily those that are elastic. It has a very high content of the amino acids glycine, valine, alanine, and proline, and has a molecular weight of 64 to 66 kDa. It is organised in an irregular or random coil conformation made up of 830 amino acids. Elastin is made by linking many soluble tropoelastin protein molecules, in a reaction catalyzed by lysyl oxidase, to make a massive insoluble, durable cross-linked array.

Elastin serves an important function in arteries as a medium for pressure wave propagation to help blood flow and is particularly abundant in large elastic blood vessels such as the aorta. Elastin is also very important in the lungs, elastic ligaments and the skin. Despite much efforts devoted to the understanding of elastin synthesis and turnover, neo-epitopes originating from the proteolytic cleavage of this matrix molecules have until now not been associated with disease development in fibrosis.

### Vimentin

Vimentin is a member of the intermediate filament family of proteins. Intermediate filaments are an important structural feature of eukaryotic cells. They, along with microtubules and actin microfilaments, make up the cytoskeleton. Although most intermediate filaments are stable structures, in fibroblasts, vimentin exists as a dynamic structure. This filament is used as a marker for mesodermally derived tissues, and as such has been used used as an immunohistochemical marker for sarcomas.

Hertig and coworkers (Hertig et al., J Am Soc Nephrol. 2008 Aug;19(8):1584-91) investigated if epithelial-to-mesenchymal transition in renal tubular epithelial cells of subjects with chronic allograft nephropathy could predict the progression of fibrosis in the allograft and measured vimentin expression in 83 biopsies from these. They did find an association between elevated vimentin expression and the intestinal fibrosis score at 1 year after surgery.

In another study of hepatic fibrosis, Meriden and colleagues (Meriden et al., Clin Gastro & Hepatol 2010; 8:289-296) found a significant association between vimentin expression (in biopsies obtained at F0 stage) and fibrosis progression, with elevated levels predicting rapid progression of the hepatic fibrosis.

Accordingly, we wanted to investigate if circulating fragments of vimentin could serve as sensitive and specific biomarkers of fibrosis.

### Proteoglycans

Proteoglycans are a diverse group of macromolecules, which covalently link a variable number of glycosaminoglycan (GAG) side chains to a core protein ¹⁶. These GAGs are polymers of disaccharide repeats (e.g. N-acetyl glucosamine or N-acetyl galactosamine), which are acidic (negatively charged) due to hydroxyl, carboxylated and sulfated side groups on the disaccharide units. This makes them highly hydrophilic, thus aiding the diffusion of water and positive ions (e.g. sodium from extracellular fluids) ¹⁷. Furthermore, GAGs have the ability to form non-covalent links with for example hyaluronic acid chains to form even larger molecular complexes ¹⁶. Table 4 lists the most studied proteoglycans associated with connective tissue.

**Table 4. Proteoglycans of the extracellular matrix of connective tissue**

| Group | Proteoglycans | Origin | Function |
|---|---|---|---|
| Large extracellular proteoglycans (*aggregating and hyaluronan-binding*) | Aggrecan ¹⁸ | Articular cartilage chondrocytes, intervertebral disc, nasal cartilage | Extracellular matrix stability (hyaluronan binding) |
| | Versican ^{19, 20} | Connective tissue: fibroblast, keratinocytes, smooth muscle cells, mesangial cells | Cell-cell and cell-matrix interactions |
| | | | Binding of sugars in Ca-dependent manner |
| | Neurocan ²¹ | Nervous tissue | Binds to neural cell adhesion molecules |
| | Brevican ²² | Nervous tissue | Extracellular matrix stability |
| Small Leucine-rich proteoglycans (*collagen-binding*) | Decorin ²³ | Connective tissue, cartilage, bone | Binds to and connect collagen molecules (matrix stabilization and thickness) Organogenesis Binding of TGFβ |
| | Biglycans ²⁴ | Capillary endothelium, skin (keratinocytes), epithelium of kidney | Cell differentiation Binds and connect collagen fibrils |
| | Fibromodulin ¹⁷ | Connective tissue, bone, cartilage | Regulate orientation of collagen fibers |
| | Lumican ²³ | Cornea, muscle, cartilage, kidney, lung, intestine | Controls spacing and thickness of collagen fibers |
| Cell-associated proteoglycans | Serglycins ²⁵ | Widely distributed to endothelium - intercellular compartments | Hemopoietic cell differentiation Adhesion and activation of lymphoid cells |
| | Syndecans ²⁶ | Widely distributed - often cell membrane bound | Binds collagens, fibronectin, thrombospondin, tenascin and bFGF |
| | Betaglycan ²⁷ | Widely distributed | TGFβ receptor and signaling Possible reservoir of TGFβ |
| Basement membrane proteoglycans | Perlecan ²⁸ | All basement membranes | Selective barrier for macromolecules Cell-adhesion |

### C-REACTIVE PROTEIN

C-reactive protein (CRP) is an acute phase serum protein produced by the liver in response to different clinical conditions such as, inflammation, infection, or trauma²⁹. The production of CRP is induced by cytokines such as IL-6, released from the affected or damaged tissues. The physiological role of CRP is yet unknown and discussions on its pro- or anti-inflammatory actions are ongoing.

### Proteases

The imbalance between synthesis and degradation of ECM during fibrogenesis, results from conversion of the low-density subendothelial matrix into matrix rich in interstitial collagens. The increase in collagen and proteoglycans may be due to one or both of (1) a decrease in protein production and (2) impaired protein degradation, and hence less matrix degradation. The decreased protein degradation has recently received increased attention. In the regulation of this process matrix metalloproteinases (MMPs) and their tissue inhibitors (TIMPs) play important roles, as well as other proteases and their inhibitors, such as cystein proteases and the cystatins.

### MMPs

MMPs are a large group of endopeptidases, capable of degrading most if not all components of the ECM. Presently, more than 25 MMPs have been found. MMPs are characterized by an active site containing a metal atom, typically zinc, and are secreted as zymogens. Different MMPs are expressed in different tissues. In Table 5 MMPs in the liver are shown.

**Table 5. MMPs in the liver³⁰⁻³²**

| Family | Protease | Source | Substrate |
|---|---|---|---|
| Collagenases | MMP-1 | HSC | I, II, III, VII, VIII, X, gelatin |
| | MMP-8 | Neutrophil | I, II, III, V, VII, X, gelatin |
| | MMP-13 | HSC, MFB, KC | I, II, III, VII, X, gelatin |
| Stromelysins | MMP-3 | HSC | III, IV, V, IX, X, XI, gelatin, laminin, fibronectin, proteoglycans, glycoproteins, elastin, pro-MMP-1/13 |
| | MMP-10 | HSC | III, IV, V, gelatin, elastin, aggrecan |
| | MMP-11 | HC | PAI-1, week activity against matrix proteins |
| Gelatinases | MMP-2 | HSC, MBF | I, II, III, IV, V, VII, X, XI, gelagin, elastin, laminin |
| | MMP-9 | KC, HSC, HC | I, II, III, IV, V, VII, X, XI, gelagin, elastin, laminin |
| | MMP-7 | HSC | Entactin, gelatin, elastin, fibronectin, vitronectin, laminin, fibrinogen |
| Metalloelastase | MMP-12 | Macrophages | Elastin, gelatins, IV, laminin, fibronectin, entactin, vitronectin, proteoglycan, myelin basic protein, α1-antitripsin |
| MT-MMPs | MMP-14 | HSC, MFB, KC | I, II, III, gelatin, fibronectin, vitronectin, laminin, fibrinogen, pro-MMP-2, pro-MMP-13 |
| | MMP-15 | HC, BDEC | Pro-MMP-2, fibronectin, tenascin, laminin, aggrecan, perlecan |

TIMPs block MMPs' proteolytic activity by binding in a substrate- and tissue- specific manner to MMP and membrane-type 1 metalloproteinase in a trimolecular complex (Table 6). During fibrosis TIMP levels increase dramatically, and MMP levels increase modestly or remain relatively static (except MMP-2) which in all gives a decrease in degradation of collagens.

**Table 6. TIMPs in the liver³¹**

| Name | Sources | Metalloproteinase inhibited |
|---|---|---|
| TIMP-1 | HSC, MFB, KC, HC | Pro-MMP-9, MMP-1, MMP-2, MMP-3, MMP-13 |
| TIMP-2 | KC, HSC | MT-MMP-1, MT-MMP-2, proMMP-2, MMP-3, MMP-13, MMP-7 |
| TIMP-3 | HC | MT-MMP-1, MT-MMP-2, TACE, MMP-13 |

### Fibroblast activation protein

Fibroblast Activation Protein alpha subunit (FAPa or FAP, alpha) is an integral membrane gelatinase belonging to the serine protease family. FAPa is the alpha subunit and DPP4 (CD26) the beta subunit of a heterodimeric membrane-bound proteinase complex also known as 170 kDa Melanoma Membrane Gelatinase, Integral Membrane Serine Proteinase and Seprase. Some cells make only FAPa homodimers, some only DPP4 homodimers. The monomer is inactive. FAP, alpha is selectively expressed in reactive stromal fibroblasts of epithelial cancers, granulation tissue of healing wounds, and malignant cells of bone and soft tissue sarcomas³³. This protein is thought to be involved in the control of fibroblast growth or epithelial-mesenchymal interactions during development, tissue repair, and epithelial carcinogenesis. It has been shown that expression of FAP increase with the stage of fibrosis^{34, 35}.

### Fibrosis biomarkers

A number of biochemical markers have been suggested for fibrotic diseases, although not specific product of the disease. In Table 7 is an example of biochemical markers of liver fibrosis used in clinical trial. In addition there are a lot of examples of biomarkers of other fibrotic diseases^{12, 36-42}.

**Table 7 summarizes some of the known markers of liver fibrosis.**

| **Biomarker** | **Parameters** | **Chronic liver disease** | **Reference** |
|---|---|---|---|
| **One parameter** | | | |
| CRP | | NASH | ⁴³ |
| Hyaluronan | | HCV | ⁴⁴⁻⁴⁷ |
| IGF-I | | HCV | ⁴⁸ |
| Leptin | | HCV | ⁴⁹ |
| PIIIP | | HCV | ⁵⁰ |

| **Several parameters** | **Parameters** | **Chronic liver disease** | **References** |
|---|---|---|---|
| MP3 | PIIINP, MMP1 | HCV | ^{51, 52} |
| Zheng et al index | HA, PIIICP, PIIINP, Laminin, C-IV | Chronic hepatitis | ⁵³ |
| Lebensztjen et al index | Laminin-2, C-IV, MMP2, MMP9-TIMP1 index | HBV | ⁵⁴ |
| | Tenascin, hyaluronana, Colalegn VI, TIMP-1 | HBV | ⁵⁵ |
| Tsochatzis et al index | Leptin, adiponectin, resistin | HCV, HBC, NASH | ⁵⁶ |
| Patel et al index | Hyaluronan, TIMP-1, α₂-macroglobulin | HCV | ⁵⁷ |
| | TIMP-1, tenascin, collagen IV, PIIINP, MMP2, laminin, Hyaluronan | NASH | ⁵⁸ |
| Forns-index (76, 77) | Age, platelet count, γGT, cholesterol | HCV HIV/HCV | ^{51, 59-62} |
| FibroTest (76, 78) | Haptoglobin, α₂-macroglobulin, apolipoprotein A1, γGT, bilirubin | HCV | ^{45, 51, 60, 61, 63-75} |
| | | HIV/HCV | |
| | | NAFLD | |
| | | NAFLD in diabetes patients | |
| Actitest | FibroTest + ALT | HCV | ^{65, 76-78} |
| APRI (Wai-index) | AST, platelet count | HIV/HCV | ^{45, 51, 60, 61, 64, 66, 79-87} |
| | | HCV | |
| | | NAFLD | |
| Hepascore | Bilirubin, γGT, hyaluronan, α₂-macroglobulin, age, gender | HCV | ^{51, 61, 64, 66, 88} |
| | | HIV/HCV | |
| FIB-4 | Platelet count, AST, ALT, age | HIV/HCV | ^{61, 83} |
| SHASTA | Hyaluronan, albumin, AST | HIV/HCV | ⁶¹ |
| Fibroindex | FORN+APRI | HCV | ⁸⁹ |
| Fibrometer test | Platelet count, prothrombin index, AST, α₂-macroglobulin, hyaluronan, urea, age | HIV/HCV | ^{51, 61, 64, 66, 81} |
| | | HCV | |
| | | NAFLD | |
| NFSA | Age, hyperglycaemia, body mass index, platelets, albumin, AST/ALT | NAFLD | ⁸¹ |
| Ultrasound + APRI | | HCV | ⁸² |
| Metwally et al index | Platelet count, albumin, AST, history of blood transfusion, HBV core antibody | HCV | ⁹⁰ |
| Mohamadnejad et al index | Age, HBV DNA levels, alkaline phosphatase, albumin, platelet counts, AST | HCV | ⁹¹ |
| Fibro*Spect* II | Hyaluronan, TIMP-1, α₂-macroglobulin | HCV | ^{85, 92, 93} |
| Stepwise combination algorithms | Combination of APRI and Fibrotest | HCV | ⁹⁴ |
| Imbert-Bismut index | α₂ macroglobulin, AST, ALT γGT, total bilirubin, albumin, α₁ globulin, α₂ globulin, β globulin, γ globulin, apolipoprotein A₁ | HCV | ⁹⁵ |
| Nunes et al | Age, Platelets, INR, CD4, AST/ALT, Hyaluronan, YKL-40, PIIINP | HCV/HIV | ⁹⁶ |
| | | HCV | |
| Fibroscan +++ | Fibroscan, Fibrotest, APRI, | HCV | ⁹⁷ |

US5387504 describes the neo-epitope VDIPEN released by the action of stromelysin at the aggrecan site N₃₄₁ -F₃₄₂ and an RIA assay employing a monoclonal antibody specific for this neo-epitope. More generally the use of monospecific antibodies specific for fragments of aggrecan, generated by specific stromelysin cleavage are described. Elevations of stromelysin occur in osteoarthritis, rheumatoid arthritis, atherosclerotic lesions, gout, inflammatory bowel disease (IBD), idiopathic pulmonary fibrosis (IPF), certain cancers, joint injuries, and numerous inflammatory diseases. Stromelysin is reported to be elevated in idiopathic pulmonary fibrosis, and it is alleged that the assay can be conducted on blood or other biological fluids to detect stromelysin cleavage products of aggrecan and that quantitation of such fragments can be used diagnostically in respect of IPF as well as other conditions. However, no evidence for this is provided and there have to our knowledge been no subsequent publications validating this prediction. Such RIA assays have been commercially available for many years and no reports of their successful use in diagnosing or monitoring any fibrotic disease have appeared.

United States Patent 7,225,080 discloses a method for diagnosis of an inflammatory, a fibrotic or a cancerous disease in a patient by measuring the values of at least four biochemical markers selected from the group consisting of α2-macroglobulin, AST (aspartate aminotransferase), ALT (alanine aminotransferase), GGT (gammaglutamyl transpeptidase), γ-globulin, total bilirubin, albumin, α1-globulin, α2-globulin, haptoglobin, β-globulin, apoA1, IL-10, TGF-β1, apoA2, and apoB in the serum or plasma of said patient, and subsequently combining said values in order to determine the presence of liver fibrosis and/or liver necroinflammatory lesions in said patient. The patent does not teach the quantitative measurement of peptide fragment carrying neo-epitopes generated during fibrotic disease.

United States Patent 6,060,255 describes a method for diagnosing the degree of liver fibrosis, comprising the steps of measuring the concentration of type IV collagen high molecular weight form in a sample using an antibody that specifically binds to type IV collagen, and relating the measurement to the degree of liver fibrosis. Again, no use is made of neo-epitopes produced by proteolytic enzymes acting in the body. The sample is actually digested with pepsin, which may obscure the natural pattern of collagen cleavage in the sample.

United States Patent 4,628,027 (Gay) discloses the production of antibodies specific for connective tissue proteins and, more particularly, the production of monoclonal antibodies by fused cell hybrids against human collagens and enzymes involved in collagen degradation. The use of monoclonal antibodies against connective tissue proteins to establish the collagen profile of histological, cytological and biological fluid samples is described. However, the patent does not describe the measurement of connective tissue proteins based on the binding of antibodies to neo-epitopes on said connective tissue proteins.

Guañabens N et al, J Bone Miner Res, 1998 ⁹⁸ evaluated the bone turnover markers N-telopeptide of type I collagen (NTX) , C-telopeptide of type I collagen (CTX) and N-terminal pro-peptide of collagen type I (PINP) in patients with primary biliary cirrhosis, a disease with increased hepatic fibrosis. The level of NTX, CTX and PINP were elevated in patients compared to controls and correlated with the histological stage of the disease. The antibodies employed in the NTX were raised against a cathepsin K cleaved site in the N-terminal of collagen type I and are dependent on the neoepitope JYDGKGVG>k. The antibodies employed in the CTX were raised against a cathepsin K cleaved site in the C-terminal of collagen type I and are dependent on the neoepitope EKAHDGGR>k. These markers are located in telopeptides of collagen type I and not in the internal part (the triple helical part) of collagen type I. The monoclonal antibodies employed for the PINP assay were raised against an internal epitope in the PINP sequence which is not a neo-epitope.

Moller S et al, Gut., 1999 ⁹⁹ demonstrated that the C-terminal cross linked telopeptide of type I collagen (ICTP) was elevated in alcoholic cirrhosis patients compared to controls. The study described showed that a biochemical marker can reflect hepatic fibrosis. The ICTP polyclonal antibody has been raised against trypsin and collagenase cleaved collagen type I. However, the antibodies are not binding to a neo-epitope.

Rosen HN et al, Calcif Tissue Int, 2004 ¹⁰⁰ assessed the bone turnover markers N-telopeptide of type I collagen (NTX) and C-telopeptide of type I collagen (CTX) in women receiving hormone replacement treatment (HRT). In the study it was observed that the bone turnover markers decreased with treatment. The antibodies employed in the NTX were raised against a cathepsin K cleaved site in the N-terminal of collagen type I and are dependent on the neoepitope JYDGKGVG>k. The antibodies employed in the CTX were raised against a cathepsin K cleaved site in the C-terminal of collagen type I and are dependent on the neoepitope EKAHDGGR>k. In contrast to the present invention, these antibodies were used for evaluation of bone metabolism and not fibrosis.

Lein M et al, Eur Urol, 2007 ¹⁰¹ evaluated the use of the neo-epitope specific bone turnover markers N-telopeptide of type I collagen (NTX) and C-telopeptide of type I collagen (CTX) in prostate cancer patients receiving zoledronic acid. In the study it was observed that the bone turnover markers decreased with treatment. The antibodies employed in the NTX were raised against a cathepsin K cleaved site in the N-terminal of collagen type I and are dependent on the neoepitope JYDGKGVG>k. The antibodies employed in the CTX were raised against a cathepsin K cleaved site in the C-terminal of collagen type I and are dependent on the neoepitope EKAHDGGR>k. In contrast to the present invention, these antibodies were used for evaluation of the bone metabolism during invasion of bone metastases and not fibrosis.

PIIINP has been used in a number of studies to assess the severity of fibrotic disease ¹⁰², in patients with skin fibrosis following severe burn trauma ¹⁰³, for disease progression in noncirrhotic primary biliary cirrhosis ¹⁰⁴, in primary biliary cirrhosis and chronic viral hepatitis C ¹⁰⁵.

PIIINP and ICTP were measured in patients with fibrosis of the myocardium ¹⁰⁶.

Many reports combine a set of biochemical markers to improve the predictive value of the biochemical index. Eleven different serum markers were measured in 205 patients with fibrotic staging from F0 to F4, and the most informative markers were alpha2 macroglobulin, alpha2 globulin (or haptoglobin), gamma globulin, apolipoprotein A1, gamma glutamyltranspeptidase, and total bilirubin ¹⁰⁷. An index of these markers had a negative predictive value (100% certainty of absence of F2, F3, or F4) was obtained for scores ranging from zero to 0.10 (12% [41] of all patients), and high positive predictive value (>90% certainty of presence of F2, F3, or F4) for scores ranging from 0.60 to 1.00 (34% [115] of all patients).

However, in none of the above mentioned reports is it suggested that measurements of peptide fragments based on antibodies binding to neo-epitopes as now claimed might be useful for the assessment of patients with fibrotic disease.

The present invention now provides a method of diagnosis of fibrosis comprising, conducting an immunoassay to measure neo-epitope containing protein fragments naturally present in a patient biofluid sample, and associating an elevation of said measure in said patient above a normal level with the presence of fibrosis, wherein said immunoassay is conducted by a method comprising: contacting protein fragments naturally present in said sample with an immunological binding partner reactive with a neo-epitope formed by cleavage of a protein by a proteinase and measuring the extent of binding of peptide fragments to said immunological binding partner to measure therein protein fragments comprising said neo-epitope, and wherein said protein is collagen type I, collagen type III, collagen type IV, collagen type V or collagen type VI, biglycan, decorin, lumican, versican, perlecan, neurocan, brevican, fibromodulin, serglycin, syndecan, betaglycan, CRP, or vimentin subject to the proviso that when the neo-epitopes are formed by cleavage of type I collagen, the cleavage is not at a site at which collagen type I is cleaved by cathepsin K. WO2009/059972 published on 14th May 2009 (after the priority date hereof) discloses assays for neo-epitopes of collagen III, but does not disclose that an elevated level of such a measure is to be associated with the presence or extent of fibrosis. Optionally, an assay according to this invention is based on one of the proteins named above other than collagen Type III or if based on collagen Type III utilises an immunological binding partner against one of the neoepitopes formed at the cleavage sites PGIPGRNGDP* SEQ ID NO1, *ESCPTGPQNY SEQ ID NO2, or PKGDTGPRGP* SEQ ID NO3 (where * marks the cleavage site).

For these purposes, cardiovascular disease may not be regarded as fibrosis, or the fibrosis detected according to the invention may be other than fibrosis accompanying cardiovascular disease. Optionally, an elevated result in an immunoassay according to this invention is associated with skin fibrosis, lung fibrosis, or liver fibrosis.

The method may comprise the preliminary step of obtaining a patient biofluid sample.

The invention includes a method of immunoassay to measure neo-epitope containing protein fragments naturally present in body fluid sample, wherein said immunoassay is conducted by a method comprising:
contacting protein fragments naturally present in said sample with an immunological binding partner reactive with a neo-epitope formed by cleavage of a protein by a proteinase and measuring the extent of binding of peptide fragments to said immunological binding partner to measure therein protein fragments comprising said neo-epitope, and wherein said protein is neurocan, brevican, fibromodulin, serglycin, syndecan, betaglycan, collagen type I,
collagen type IV, collagen type V, collagen type VI, CRP, or vimentin subject to the proviso that when the neo-epitopes are formed by cleavage of type I collagen, the cleavage is not at a site at which collagen type I is cleaved by cathepsin K.
Optionally, an assay according to this invention is based on one of the proteins named above other than collagen Type III or if based on collagen Type III utilises an immunological binding partner against one of the neoepitopes formed at the cleavage sites
PGIPGRNGDP* SEQ ID NO1, *ESCPTGPQNY SEQ ID NO2, or PKGDTGPRGP* SEQ ID NO3 (where * marks the cleavage site).

Said immunological binding partner may have specific binding affinity for peptide fragments comprising a C-terminal neoepitope or an N-terminal neoepitope.

Specific reactivity with or immunological affinity for a neo-epitope will imply that the relevant immunological binding partner is not reactive with intact protein from which the neo-epitope derives. Preferably, said immunological binding partner is not reactive with a neo-epitope sequence, such as a sequence listed below, if the sequence is prolonged past the respective cleavage site.

The term `immunological binding partner' as used herein includes polyclonal and monoclonal antibodies and also specific binding fragments of antibodies such as Fab or F(ab')₂. Thus, said immunological binding partner may be a monoclonal antibody or a fragment of a monoclonal antibody having specific binding affinity.

Preferably, said peptide fragments are fragments of Type I, III, IV, V, or VI collagen, elastin, C-reactive protein, or one of the proteoglycans Biglycan, Decorin, Versican, and Perlecan. The connective tissue proteins are preferred. Preferably, the neo-epitope sequence to which the immunological binding partner binds is not found in any other protein or is not found in any of the other proteins to which the method of the invention relates.

Several candidate proteases may be responsible for the digestion of proteins in the fibrotic tissues. Most likely, this is the result of the large range of complicated processes resulting in different neo-epitope profiles dependent on the levels of disease.

### COLLAGEN ASSAYS

### Collagen type I

We have determined that the enzymes listed in the following table cleave type I collagen at least the following cleavage sites (marked "."):

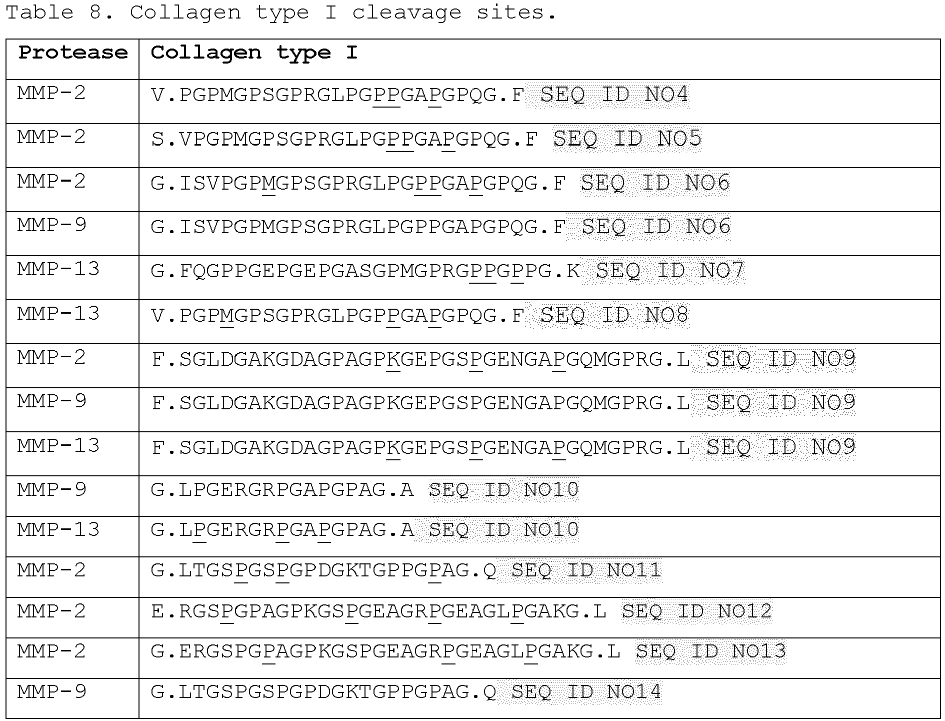

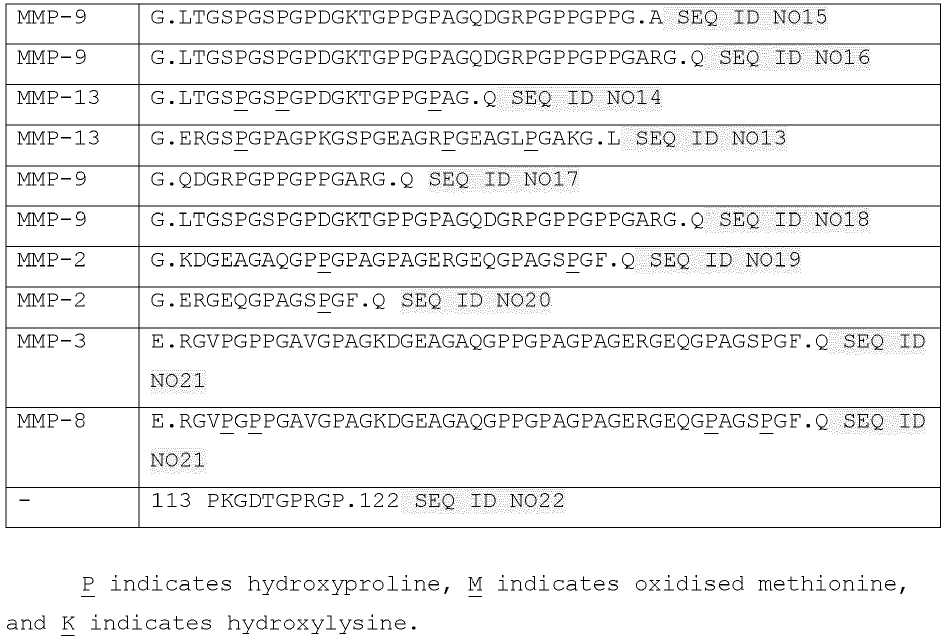

The immunological binding partner may be one specifically reactive with a C-terminal or N-terminal neoepitope formed by cleavage of type I collagen, excluding cleavage at a cathepsin K type I collagen site.

Suitable immunological binding partners may therefore be specifically reactive with any of the following sequences at the N terminal of a peptide:

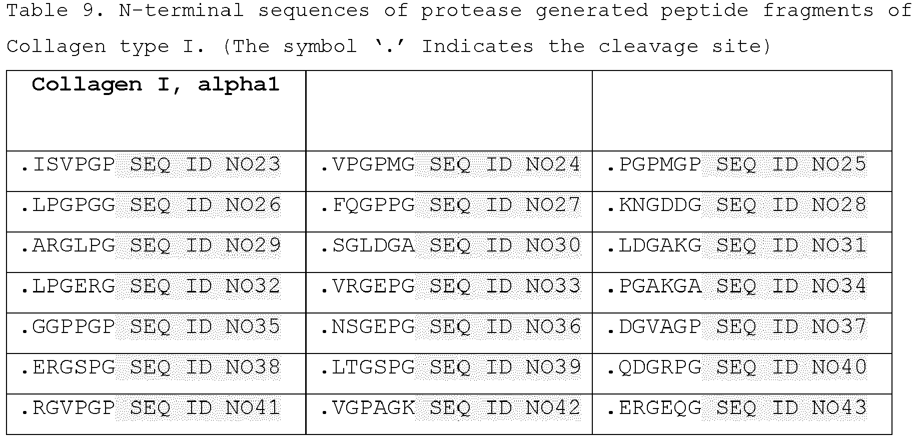

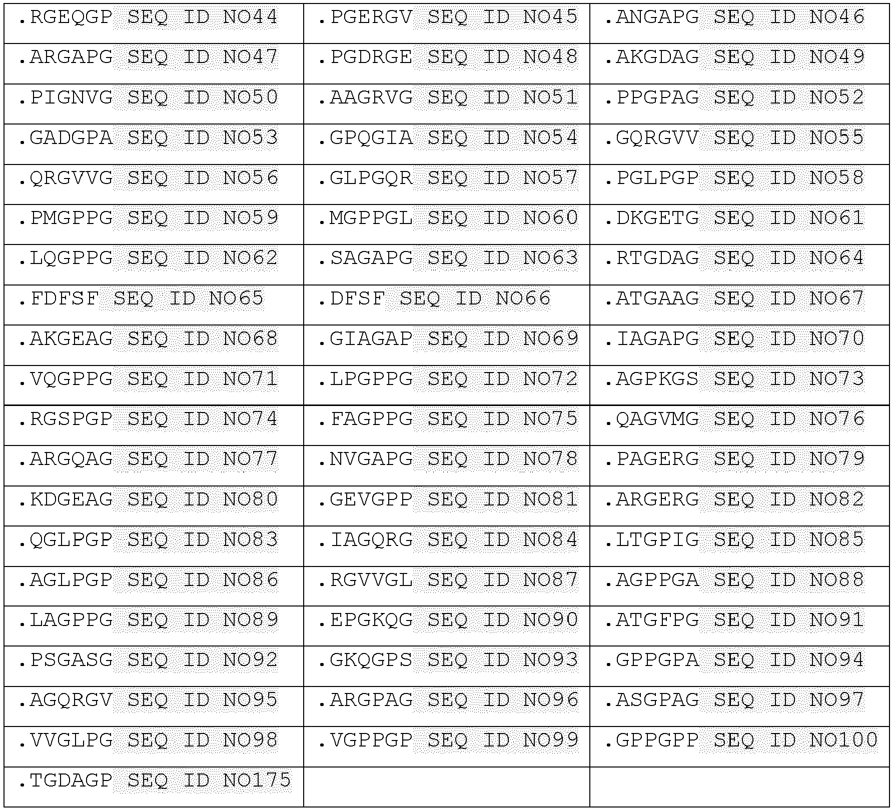

Alternatively, suitable immunological binding partners may be specifically reactive with any of the following sequences at the C terminal of a peptide:

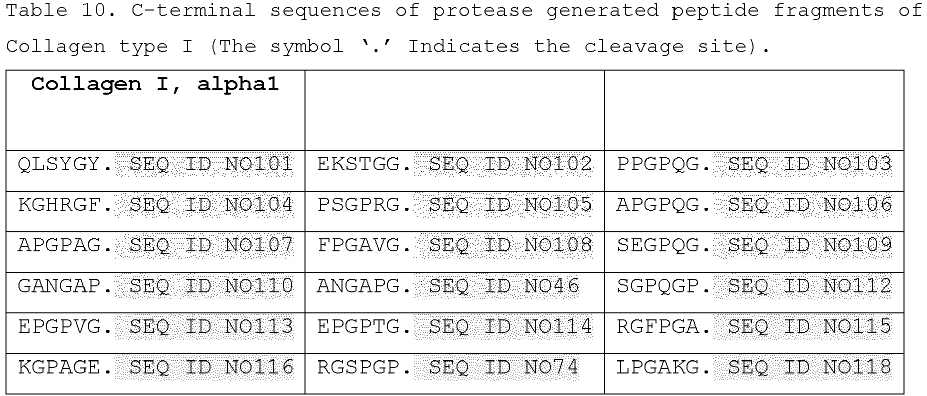

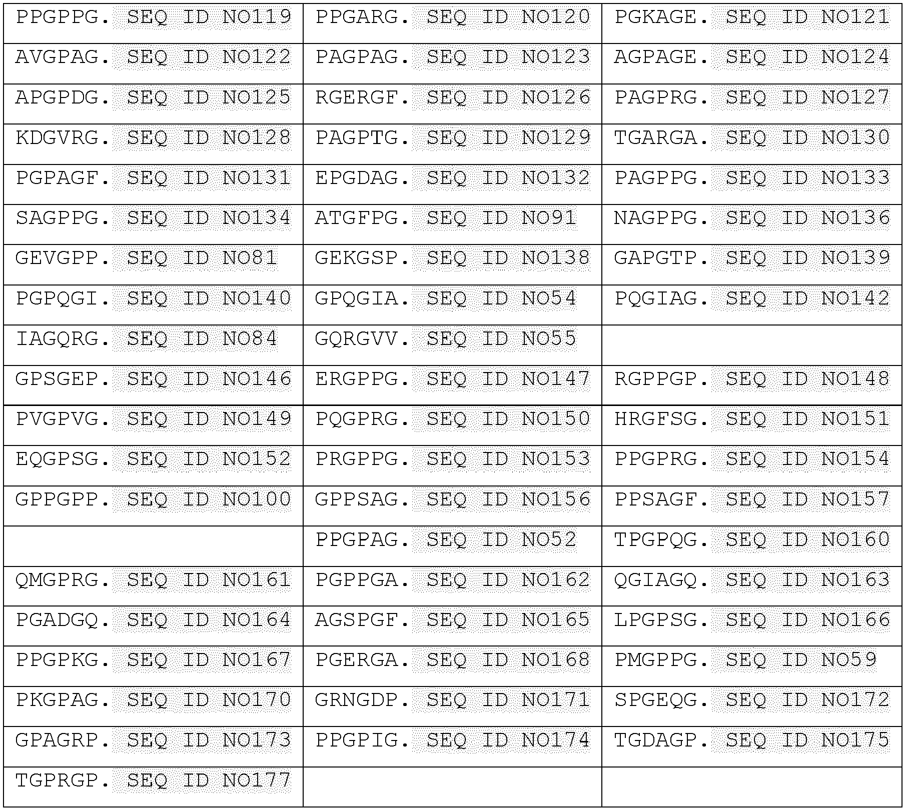

### Collagen type III

We have determined that the enzymes listed in the following table cleave type III collagen at least the following cleavage sites (marked *):

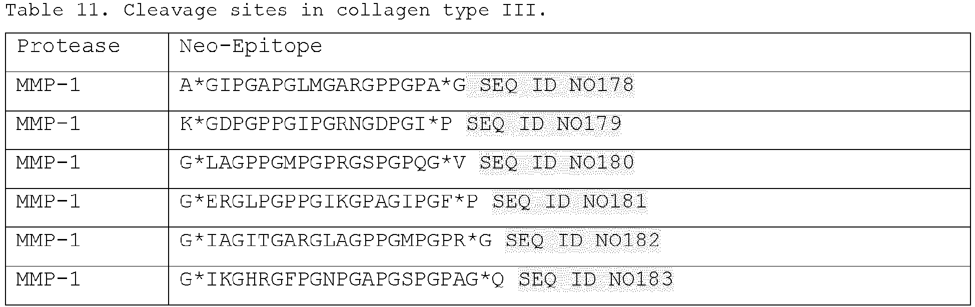

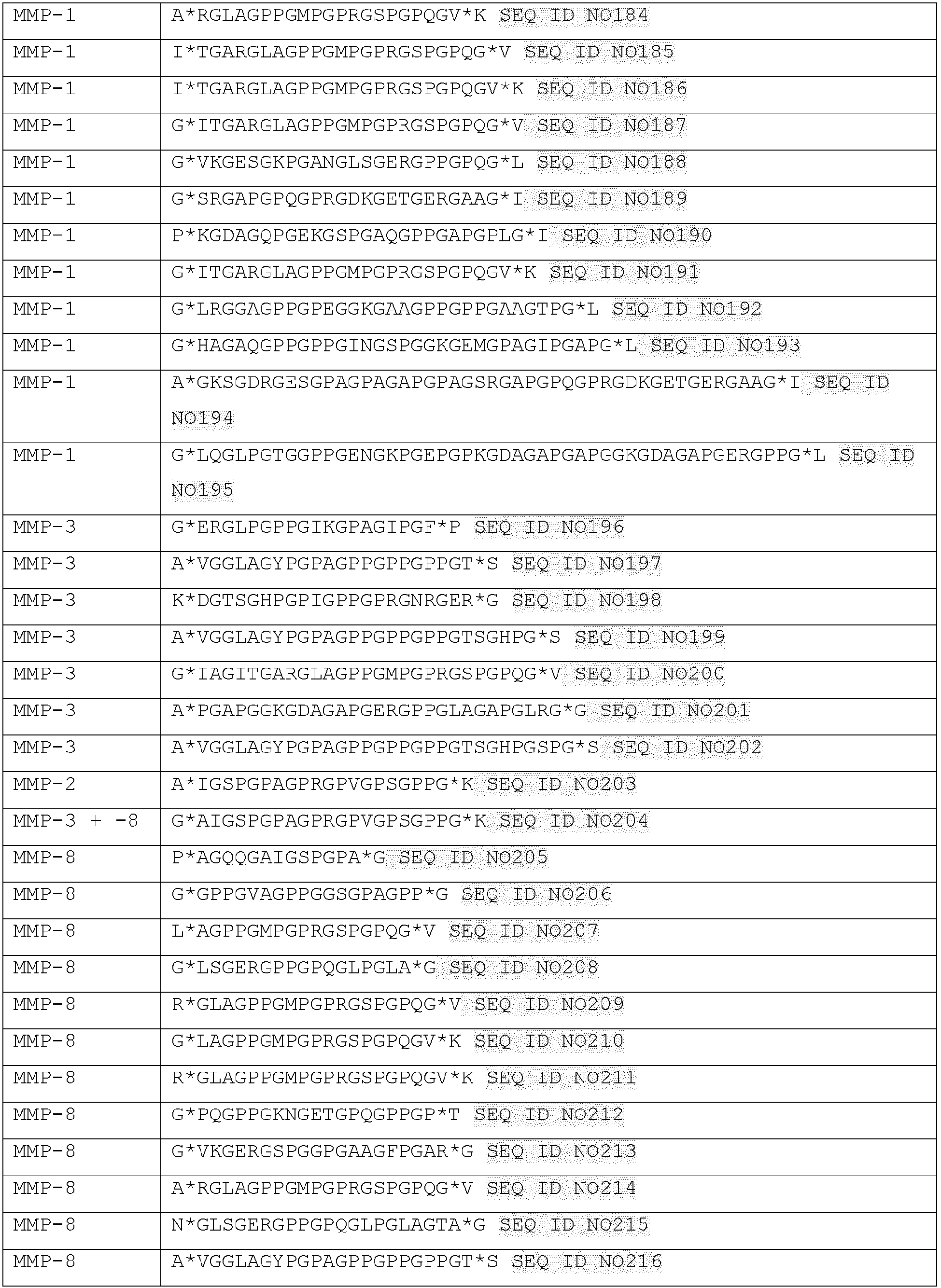

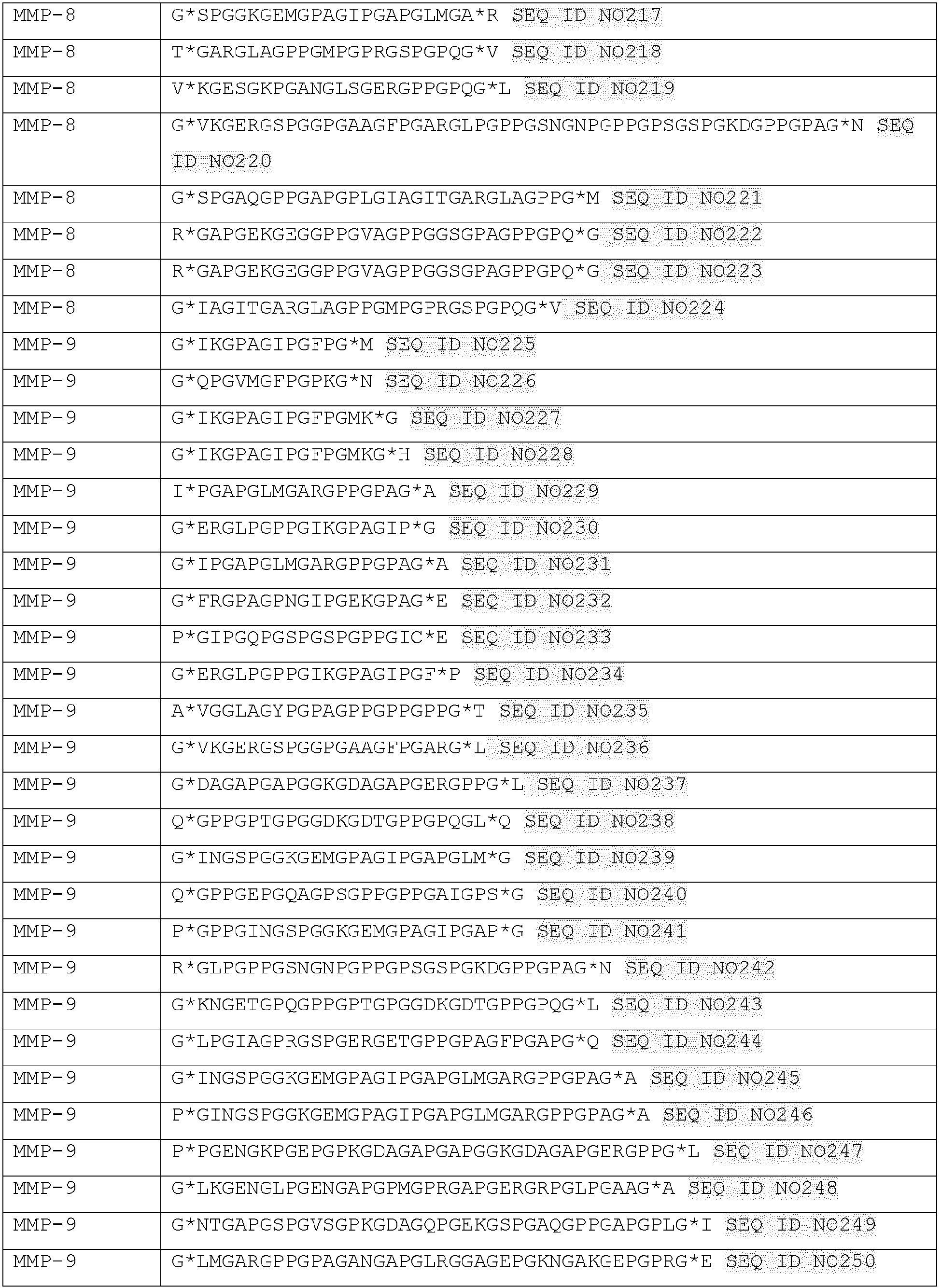

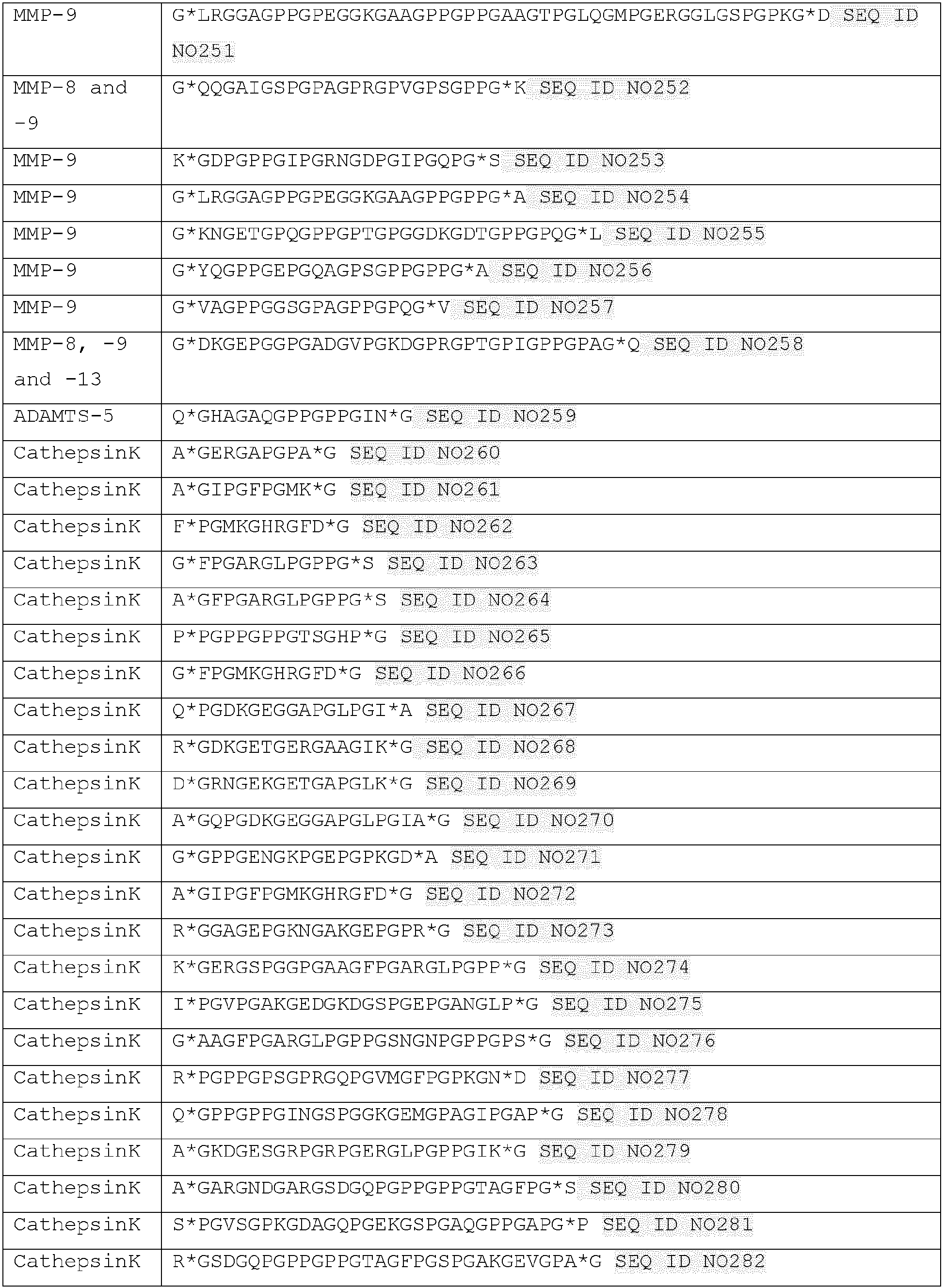

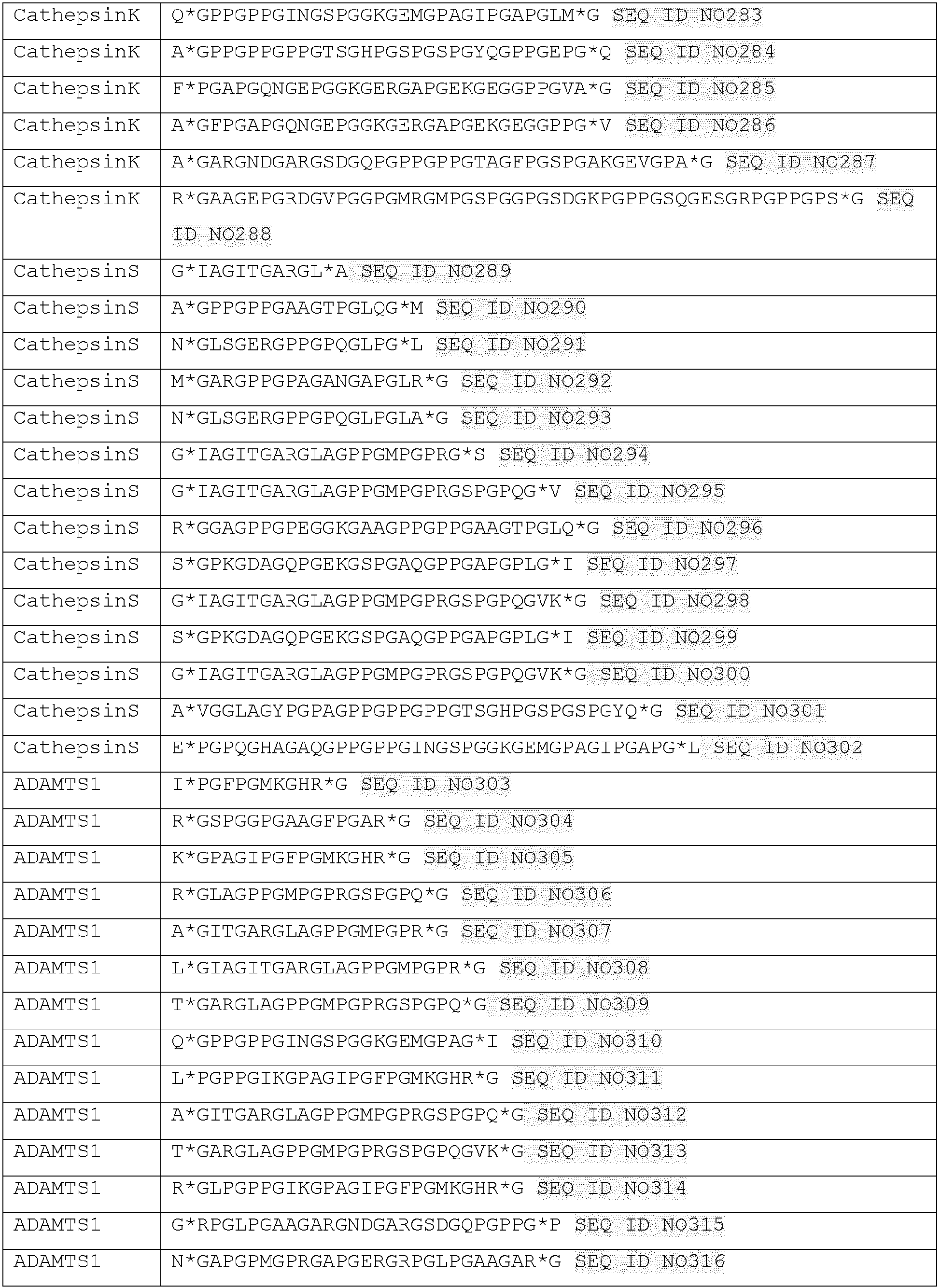

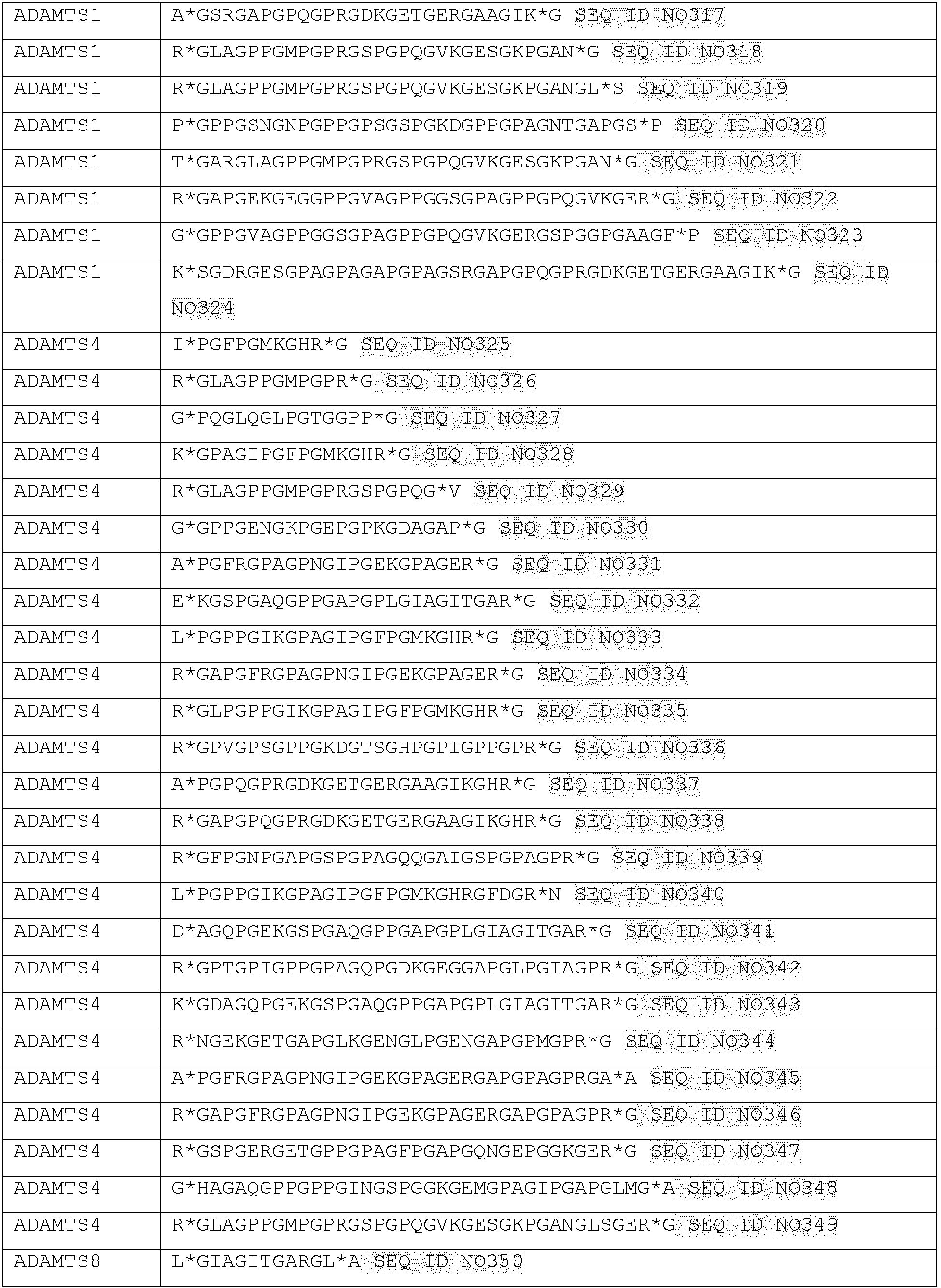

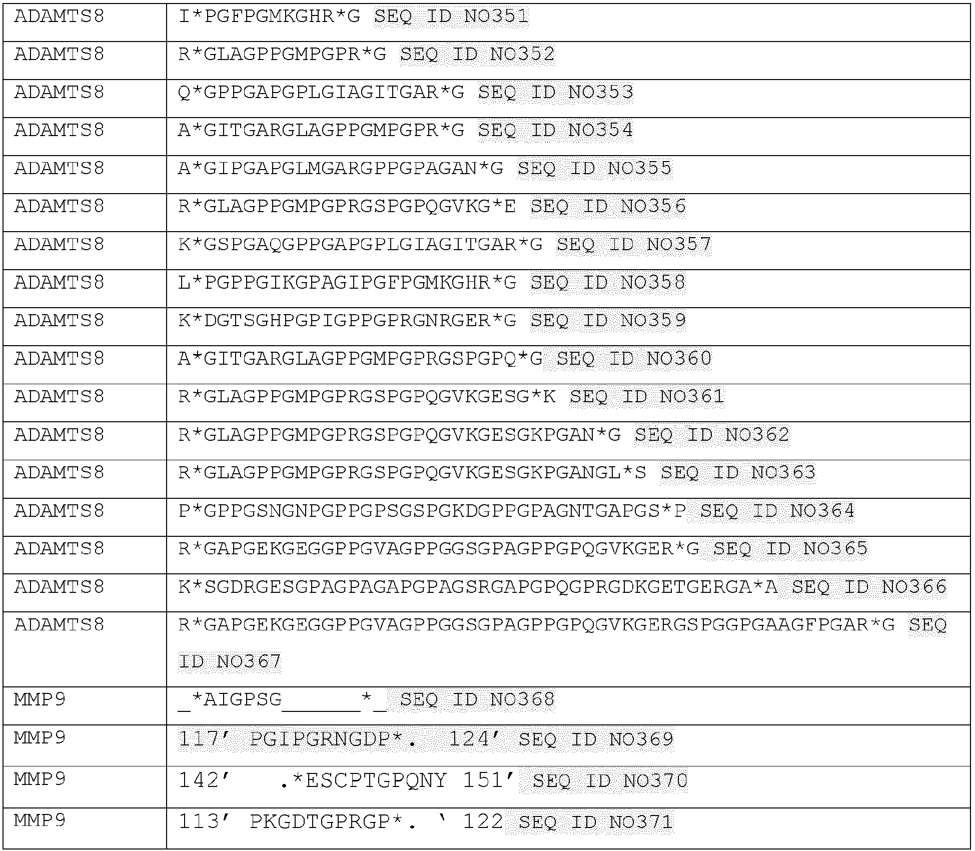

The immunological binding partner may be one specifically reactive with a C-terminal or N-terminal neoepitope formed by cleavage of type III collagen.

Suitable immunological binding partners may therefore be specifically reactive with any of the following sequences at the N terminal of a peptide:

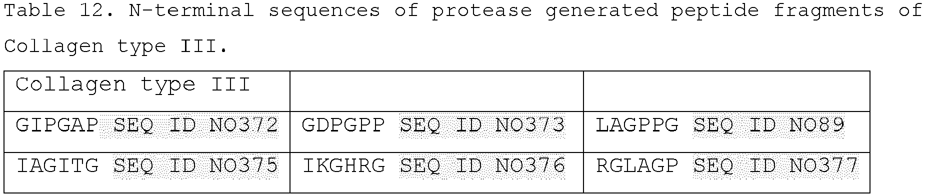

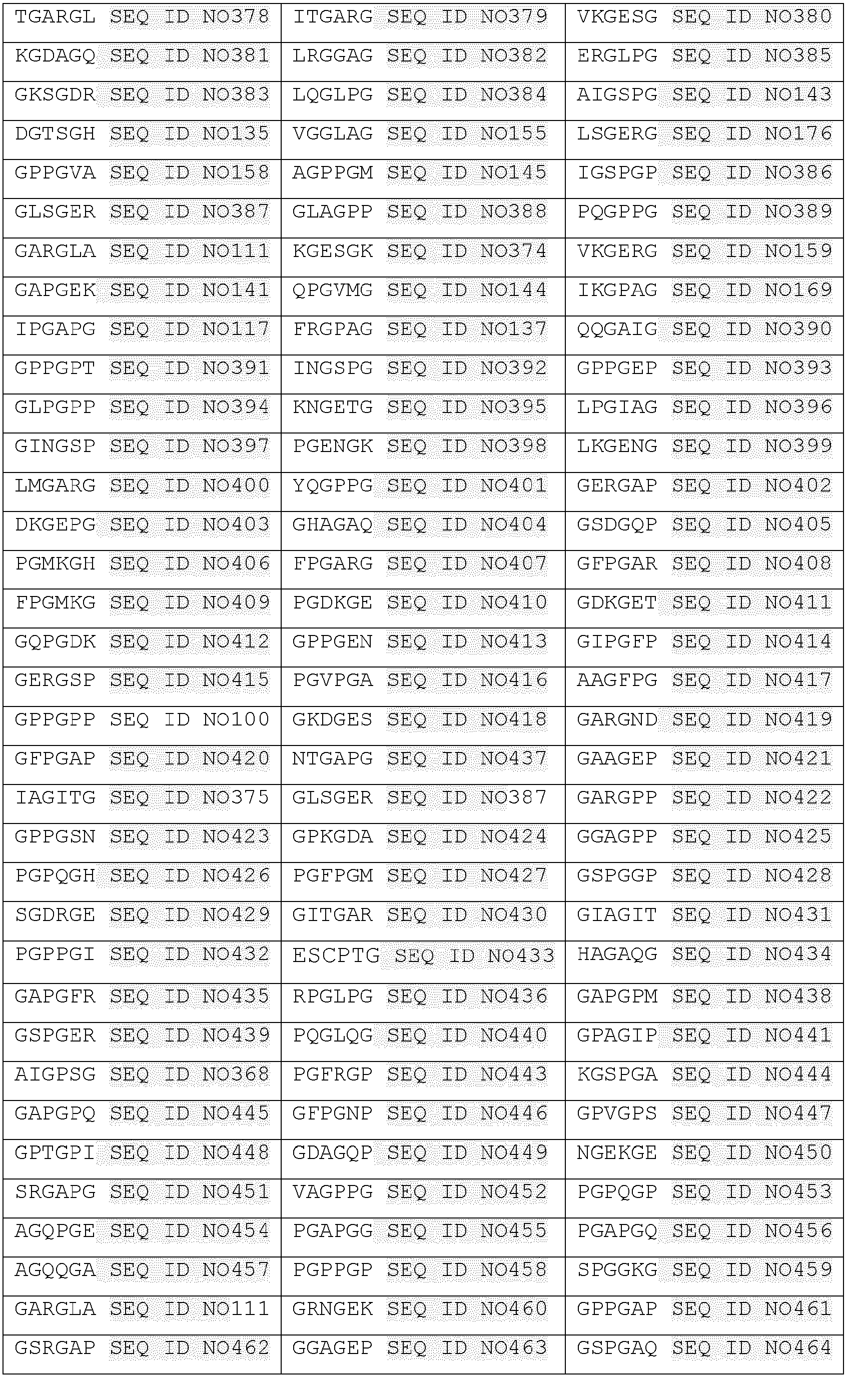

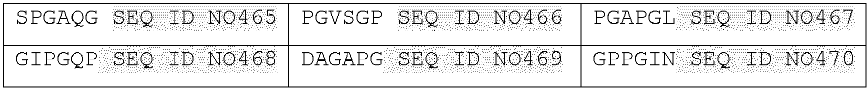

or with any of the following sequences at the C-terminal of a peptide:

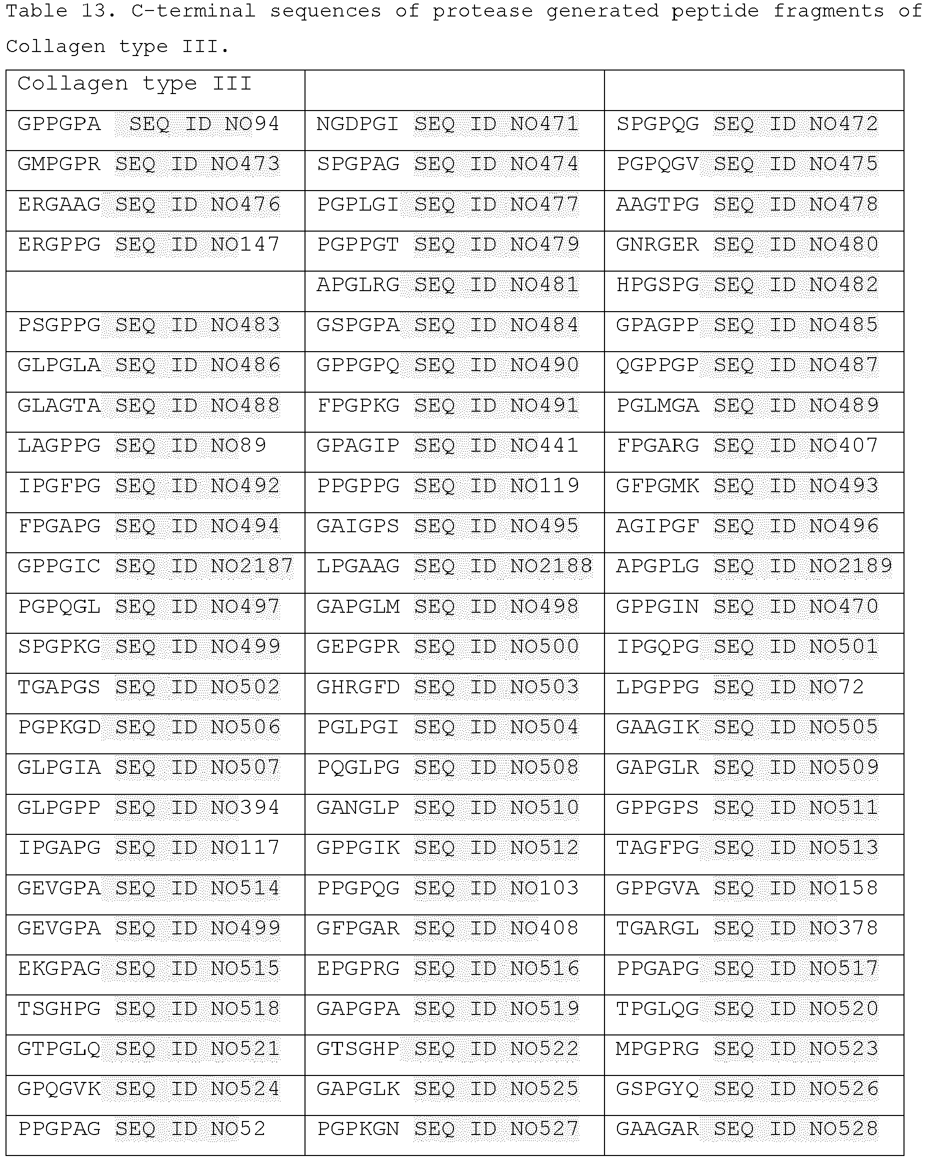

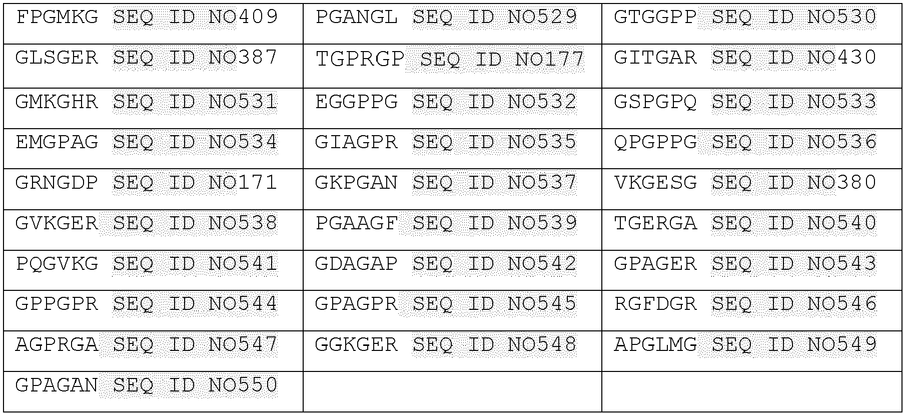

### Collagen IV

We have determined that the enzymes listed in the following table cleave type IV collagen at least the following cleavage sites (marked "."):

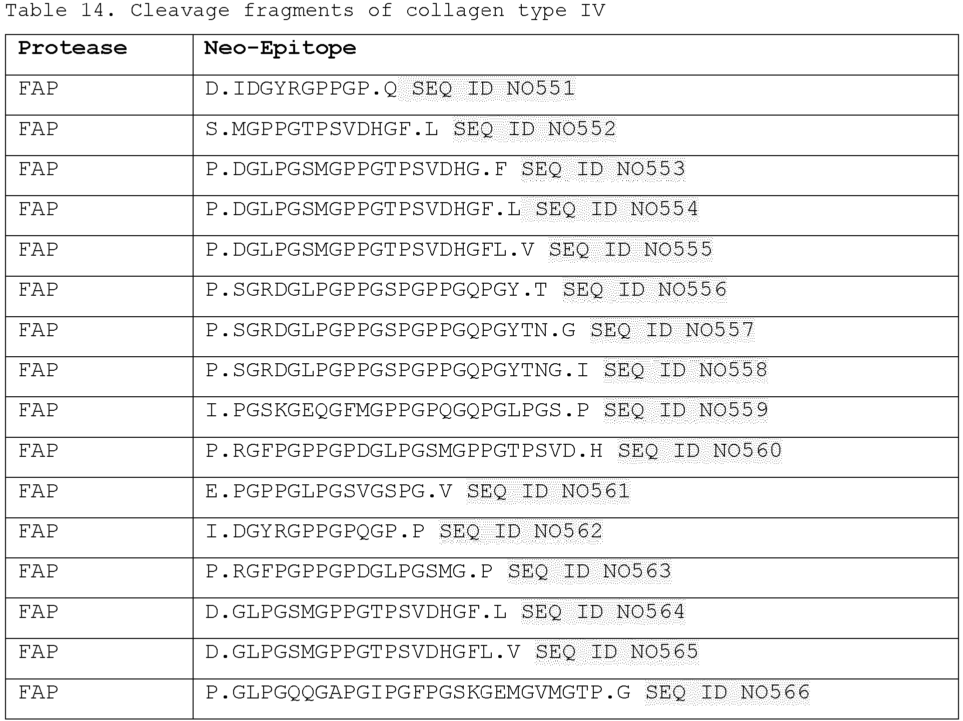

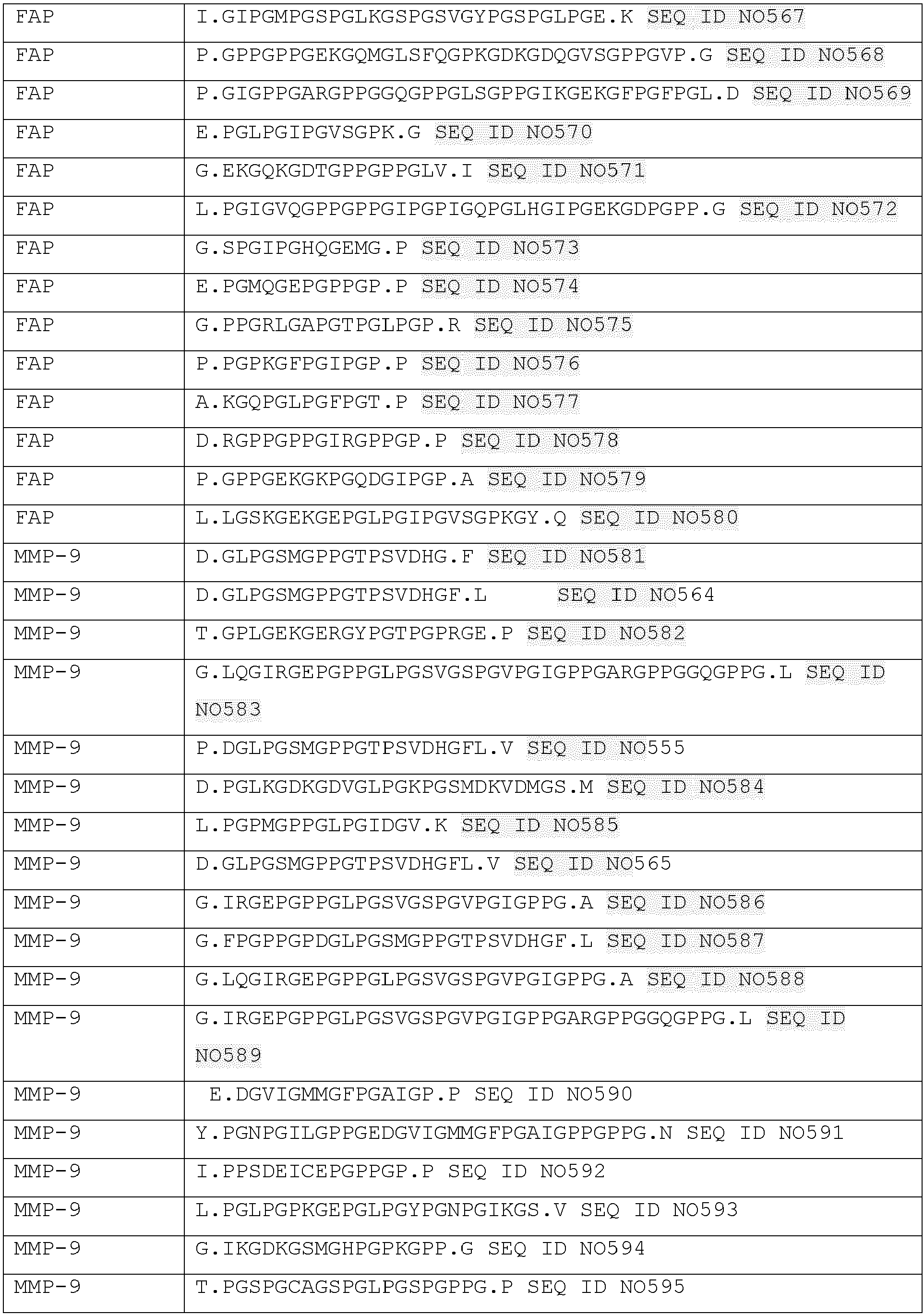

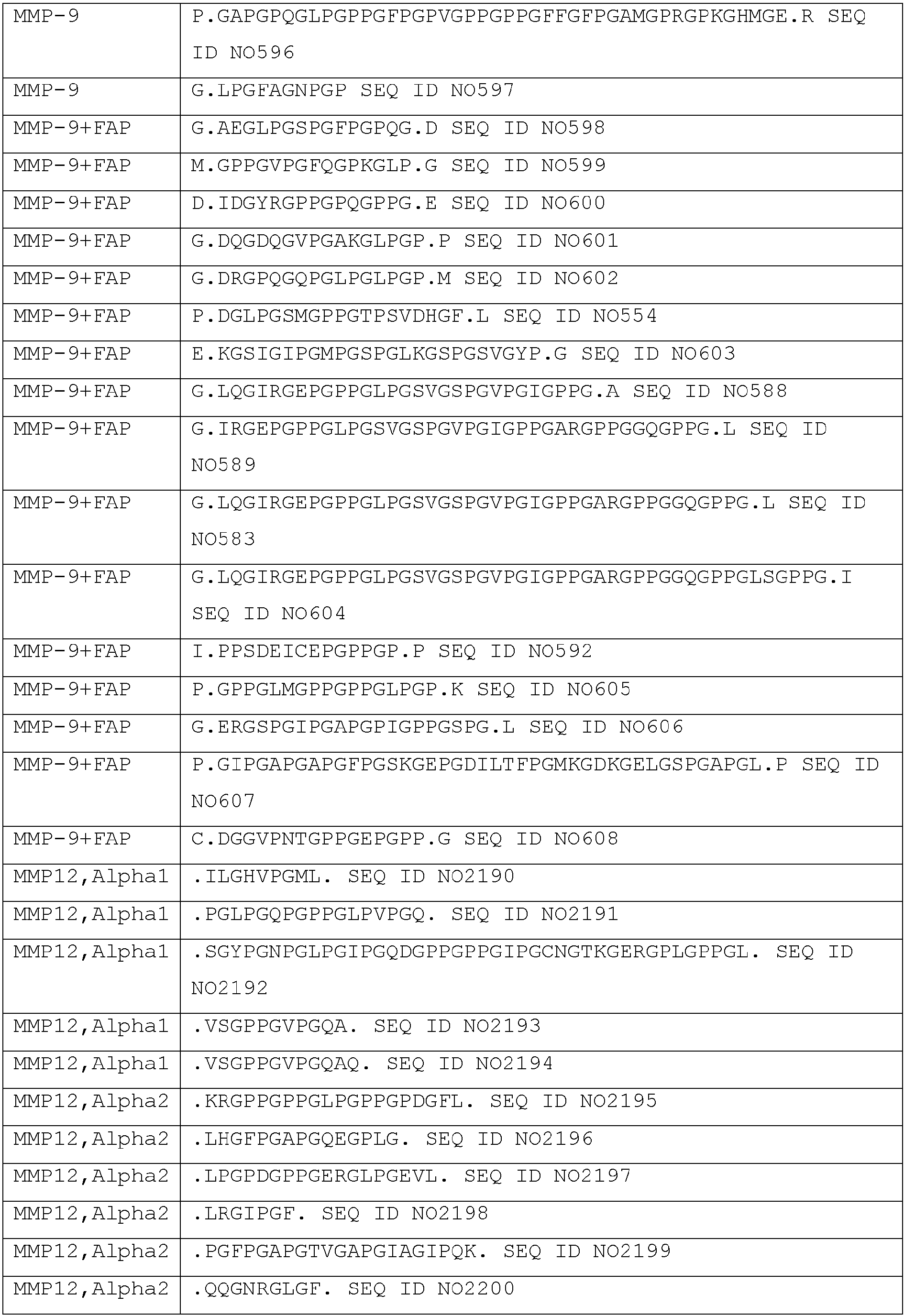

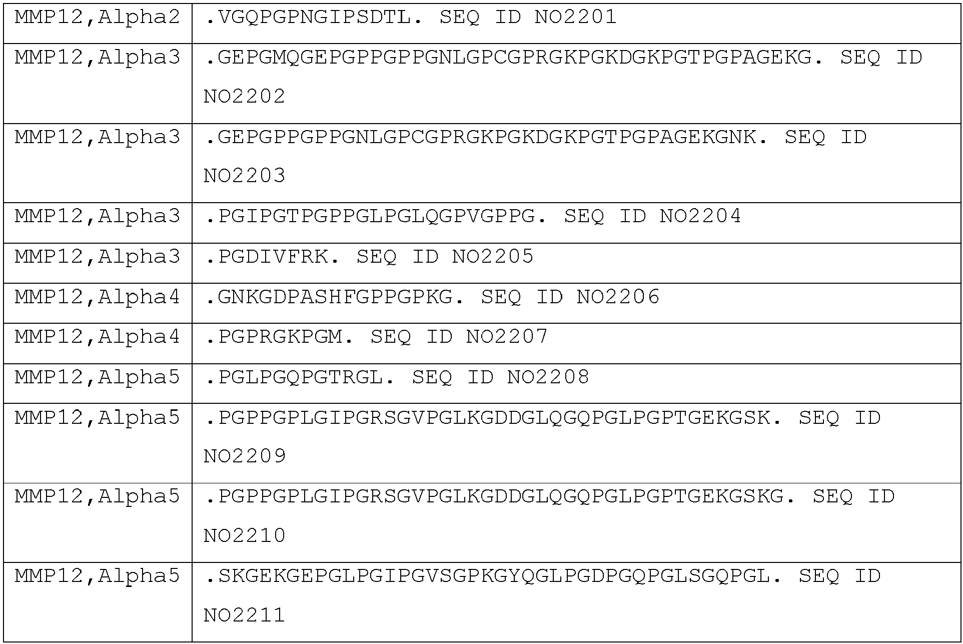

The immunological binding partner may be one specifically reactive with a C-terminal or N-terminal neoepitope formed by cleavage of type IV collagen.

Suitable immunological binding partners may therefore be specifically reactive with any of the following sequences at the N terminal of a peptide:

**Table 15. N-terminal sequences of protease generated peptide fragments of Collagen type IV.**

| Collagen type IV | | |
|---|---|---|
| IDGYRG SEQ ID NO609 | MGPPGT SEQ ID NO610 | DGLPGS SEQ ID NO611 |
| PGSKGE SEQ ID NO612 | RGFPGP SEQ ID NO613 | PGPPGL SEQ ID NO614 |
| GPPGPP SEQ ID NO100 | GLPGSM SEQ ID NO615 | GLPGQQ SEQ ID NO616 |
| LGSKGE SEQ ID NO617 | GIGPPG SEQ ID NO611 | PGLPGI SEQ ID NO504 |
| PGIGVQ SEQ ID NO618 | SPGIPG SEQ ID NO619 | PGMQGE SEQ ID NO620 |
| PGPKGF SEQ ID NO621 | KGQPGL SEQ ID NO622 | RGPPGP SEQ ID NO148 |
| PPSDEI SEQ ID NO623 | PGLKGD SEQ ID NO624 | GPLGEK SEQ ID NO625 |
| IRGEPG SEQ ID NO626 | FPGPPG SEQ ID NO627 | LQGIRG SEQ ID NO628 |
| DGVIGM SEQ ID NO629 | PGNPGI SEQ ID NO630 | PGLPGP SEQ ID NO58 |
| IKGDKG SEQ ID NO631 | PGSPGC SEQ ID NO632 | GAPGPQ SEQ ID NO445 |
| GPPGVP SEQ ID NO633 | DQGDQG SEQ ID NO634 | DRGPQG SEQ ID NO442 |
| KGSIGI SEQ ID NO635 | PPGRLG SEQ ID NO636 | EKGQKG SEQ ID NO637 |
| ERGSPG SEQ ID NO38 | GIPGAP SEQ ID NO372 | DGGVPN SEQ ID NO638 |
| SGRDGL SEQ ID NO639 | GPPGEK SEQ ID NO640 | AEGLPG SEQ ID NO641 |
| DGYRGP SEQ ID NO642 | GPPGLM SEQ ID NO643 | LPGFAG SEQ ID NO644 |
| GIPGMP SEQ ID NO645 | PGPMGP SEQ ID NO25 | .PGIPGT SEQ ID NO2227 |
| .ILGHVP SEQ ID NO2212 | .LPGPDG SEQ ID NO2214 | .PGDIVF SEQ ID NO2215 |
| .PGLPGQ SEQ ID NO2213 | .LRGIPG SEQ ID NO760 | .GNKGDP SEQ ID NO2216 |
| .SGYPGN SEQ ID NO2217 | .PGFPGA SEQ ID NO2218 | .PGPRGK SEQ ID NO2219 |
| .VSGPPG SEQ ID NO2220 | .QQGNRG SEQ ID NO2221 | .PGPPGP SEQ ID NO458 |
| .KRGPPG SEQ ID NO2223 | .VGQPGP SEQ ID NO2222 | .SKGEKG SEQ ID NO2226 |
| .LHGFPG SEQ ID NO2225 | .GEPGMQ SEQ ID NO2224 | .GEPGPP SEQ ID NO675 |
| | | |

or with any of the following sequences at the C-terminal of a peptide:

**Table 16. C-terminal sequences of protease generated peptide fragments of Collagen type IV.**

| Collagen type IV | | |
|---|---|---|
| RGPPGP SEQ ID NO148 | SVDHGF SEQ ID NO646 | PSVDHG SEQ ID NO647 |
| VDHGFL SEQ ID NO648 | PGQPGY SEQ ID NO649 | QPGYTN SEQ ID NO650 |
| PGLPGS SEQ ID NO651 | GTPSVD SEQ ID NO652 | SVGSPG SEQ ID NO653 |
| LPGSMG SEQ ID NO654 | GPPGVP SEQ ID NO633 | PGFPGL SEQ ID NO655 |
| PGLPGE SEQ ID NO656 | GDPGPP SEQ ID NO373 | HQGEMG SEQ ID NO657 |
| GPPGLV SEQ ID NO658 | PGIPGP SEQ ID NO659 | PGFPGT SEQ ID NO660 |
| PGLPGP SEQ ID NO58 | DGIPGP SEQ ID NO661 | SGPKGY SEQ ID NO662 |
| PGPRGE SEQ ID NO663 | GQGPPG SEQ ID NO664 | KVDMGS SEQ ID NO665 |
| GIGPPG SEQ ID NO611 | PGIDGV SEQ ID NO666 | KGHMGE SEQ ID NO667 |
| PGAIGP SEQ ID NO668 | PPGPPG SEQ ID NO 119 | KGLPGP SEQ ID NO669 |
| GPKGPP SEQ ID NO671 | SPGPPG SEQ ID NO672 | GPKGLP SEQ ID NO670 |
| GSVGYP SEQ ID NO673 | PQGPPG SEQ ID NO389 | PPGSPG SEQ ID NO674 |
| GEPGPP SEQ ID NO675 | AGNPGP SEQ ID NO676 | PGIKGS SEQ ID NO677 |
| PGYTNG SEQ ID NO678 | FPGPQG SEQ ID NO679 | PGPQGP SEQ ID NO453 |
| LSGPPG SEQ ID NO680 | PGAPGL SEQ ID NO467 | GVMGTP SEQ ID NO681 |
| GVSGPK SEQ ID NO682 | PGPPGP SEQ ID NO458 | |
| HVPGML. SEQ ID NO2228 | LPVPGQ. SEQ ID NO2229 | LGPPGL. SEQ ID NO2230 |
| GVPGQA. SEQ ID NO2231 | VPGQAQ. SEQ ID NO2232 | GPDGFL. SEQ ID NO2233 |
| QEGPLG. SEQ ID NO2234 | LPGEVL. SEQ ID NO2235 | RGIPGF. SEQ ID NO2236 |
| NRGLGF. SEQ ID NO2238 | IPSDTL. SEQ ID NO2239 | PAGEKG. SEQ ID NO2240 |
| GEKGNK. SEQ ID NO2241 | PVGPPG. SEQ ID NO2242 | DIVFRK. SEQ ID NO2243 |
| PPGPKG. SEQ ID NO167 | RGKPGM. SEQ ID NO2244 | PGTRGL. SEQ ID NO2245 |
| GEKGSK. SEQ ID NO2246 | EKGSKG. SEQ ID NO2247 | SGQPGL. SEQ ID NO2248 |
| AGIPQK. SEQ ID NO2237 | | |

### Collagen V

We have determined that the enzymes listed in the following table cleave type v collagen at least the following cleavage sites (marked "." or in the ablsence of a '.', at the end of the sequence):

**Table 14A. Cleavage fragments of collagen type V**

| Protease | Neo-epitope (COV) |
|---|---|
| MMP2,Alpha3 | K.GDPGPPGPIGSLG.H SEQ ID NO683 |
| MMP2,Alpha3 | G.LRGIPGPVGEPG.L SEQ ID NO684 |
| MMP2,Alpha3 | V.IGPPGLQGLPGPPGE.K SEQ ID NO685 |
| MMP2,Alpha3 | G.KDGIPGPLGPLGPPG.A SEQ ID NO686 |
| MMP2,Alpha3 | G.LRGIPGPVGEPGLL.G SEQ ID NO687 |
| MMP2,Alpha3 | G.VLGPQGKTGEVGPLG.E SEQ ID NO688 |
| MMP2,Alpha3 | K.DGIPGPLGPLGPPGAA.G SEQ ID NO689 |
| MMP2,Alpha3 | G.EDGERGAEGPPGPTG.Q SEQ ID NO690 |
| MMP2,Alpha3 | G.LQGPPGFPGPKGPPG.H SEQ ID NO691 |
| MMP2,Alpha3 | P.IGSLGHPGPPGVAGPLG.Q SEQ ID NO692 |
| MMP2,Alpha3 | G.IRGPPGTVIMMPFQ.F SEQ ID NO693 |
| MMP2,Alpha3 | G.QMGPPGPLGPSGLPGLK.G SEQ ID NO694 |
| MMP2,Alpha3 | G.LLGAPGQMGPPGPLGPSG.L SEQ ID NO695 |
| MMP2,Alpha3 | G.LRGIPGPVGEPGLLGAPG.Q SEQ ID NO696 |
| MMP2,Alpha3 | G.LLGPRGSPGPTGRPGVTG.I SEQ ID NO697 |
| MMP2,Alpha3 | G.IRGPPGTVIMMPFQF.A SEQ ID NO698 |
| MMP2,Alpha3 | G.KDGIPGPLGPLGPPGAAGP.S SEQ ID NO699 |
| MMP2,Alpha3 | G.KDGIPGPLGPLGPPGAAGPSG.E SEQ ID NO700 |
| MMP2,Alpha3 | Q.GLPGLEGREGAKGELGPPGPLG.K SEQ ID NO701 |
| MMP2,Alpha3 | L.GPIGEKGKSGKTGQPGLEGERGPPGSRG.E SEQ ID NO702 |
| MMP2,Alpha3 | G.LRGIPGPVGEPGLLGAPGQMGPPGPLGPSG.L SEQ ID NO703 |
| MMP2,Alpha3 | G.ANGSPGERGPLGPAGGIGLPGQSGSEGPVGPAG.K SEQ ID NO704 |
| MMP2,Alpha3 | G.LIGTPGEKGPPGNPGIPGLPGSDGPLGHPGHEGPTG.E SEQ ID NO705 |
| MMP2,Alpha1 | G.LPGEPGPRG.L SEQ ID NO706 |
| MMP2,Alpha1 | L.ALRGPAGPMG.L SEQ ID NO707 |
| MMP2,Alpha1 | R.LALRGPAGPMG.L SEQ ID NO708 |
| MMP2,Alpha1 | G.LTGRPGPVGPPGSGG.L SEQ ID NO709 |
| MMP2,Alpha1 | G.LLGPKGPPGPPGPPG.V SEQ ID NO710 |
| MMP2,Alpha1 | G.IPGRPGPQGPPGPAG.E SEQ ID NO711 |
| MMP2,Alpha1 | P.GPDGPPGPMGPPGLP.G SEQ ID NO712 |
| MMP2,Alpha1 | G.QPGPSGADGEPGPRG.Q SEQ ID NO713 |
| MMP2,Alpha1 | G.ETGFQGKTGPPGPPG.V SEQ ID NO714 |
| MMP2,Alpha1 | G.LRGFPGDRGLPGPV.G SEQ ID NO715 |
| MMP2,Alpha1 | G.LRGFPGDRGLPGPVG.A SEQ ID NO716 |
| MMP2,Alpha1 | G.KTGPIGPQGAPGKPGPDG.L SEQ ID NO717 |
| MMP2,Alpha1 | G.PPGRPGLPGADGLPGPPG.T SEQ ID NO718 |
| MMP2,Alpha1 | G.LKGNEGPPGPPGPAGSPGE.R SEQ ID NO719 |
| MMP2,Alpha1 | G.LRGFPGDRGLPGPVGALG.L SEQ ID NO720 |
| MMP2,Alpha1 | G.ERGHPGPPGPPGEQGLPG.L SEQ ID NO721 |
| MMP2,Alpha1 | I.GPPGEQGEKGDRGLPGPQG.S SEQ ID NO722 |
| MMP2,Alpha1 | G.EAGHPGPPGPPGPPGEVIQPLP.I SEQ ID NO723 |
| MMP2,Alpha1 | K.PGPKGNSGGDGPAGPPGERGPNGP.Q SEQ ID NO724 |
| MMP2,Alpha1 | G.EQGLPGSPGPDGPPGPMGPPGLPG.L SEQ ID NO725 |
| MMP2,Alpha1 | E.GPPGEKGGQGPPGPQGPIGYPGPRG.V SEQ ID NO726 |
| MMP2,Alpha1 | G.FPGPKGPPGPPGKDGLPGHPGQRG.E SEQ ID NO727 |
| MMP2 | L.PFRFGGGGDA SEQ ID NO728 |
| MMP2 and 9 | GSKGPMVSAQ.E SEQ ID NO729 |
| MMP2 and 9 | Q.ESQAQAILQQ SEQ ID NO730 |
| MMP9,Alpha1 | L.ALRGPAGPMG.L SEQ ID NO707 |
| MMP9,Alpha1 | G.AIGPPGEKGPLG.K SEQ ID NO731 |
| MMP9,Alpha1 | G.GPNGDPGPLGPPG.E SEQ ID NO732 |
| MMP9,Alpha1 | P.PGPPGEQGLPGL.A SEQ ID NO733 |
| MMP9,Alpha1 | G.LLGPKGPPGPPGPPG.V SEQ ID NO734 |
| MMP9,Alpha1 | G.IPGRPGPQGPPGPAG.E SEQ ID NO711 |
| MMP9,Alpha1 | G.QPGPSGADGEPGPRG.Q SEQ ID NO713 |
| MMP9,Alpha1 | G.QQGNPGAQGLPGPQG.A SEQ ID NO735 |
| MMP9,Alpha1 | G.KEGPPGEKGGQGPPG.P SEQ ID NO736 |
| MMP9,Alpha1 | G.ETGFQGKTGPPGPPG.V SEQ ID NO737 |
| MMP9,Alpha1 | G.EKGHPGLIGLIGPPG.E SEQ ID NO738 |
| MMP9,Alpha1 | G.LRGFPGDRGLPGPVG.A SEQ ID NO716 |
| MMP9,Alpha1 | G.KTGPIGPQGAPGKPGPDG.L SEQ ID NO739 |
| MMP9,Alpha1 | P.GPDGPPGPMGPPGLPGLK.G SEQ ID NO740 |
| MMP9,Alpha1 | G.ERGHPGPPGPPGEQGLPG.L SEQ ID NO721 |
| MMP9,Alpha1 | G.ERGPNGPQGPTGFPGPKGPPGPPG.K SEQ ID NO741 |
| MMP9,Alpha1 | L.IGLIGPPGEQGEKGDRGLPGPQGS.S SEQ ID NO742 |
| MMP9,Alpha1 | E.GPPGEKGGQGPPGPQGPIGYPGPRG.V SEQ ID NO726 |
| MMP9,Alpha1 | I.GPPGPPGLPGPPGPKGAKGSSGPTGPKGE.A SEQ ID NO743 |
| MMP9,Alpha1 | P.LGPPGEKGKLGVPGLPGYPGRQGPKGSI.G SEQ ID NO744 |
| MMP9,Alpha1 | Q.GPKGSIGFPGFPGANGEKGGRGTPGKPGPRG.Q SEQ ID NO745 |
| MMP9,Alpha3 | P.GPKGDPGPPGPIG.S SEQ ID NO746 |
| MMP9,Alpha3 | K.GDPGPPGPIGSLG.H SEQ ID NO683 |
| MMP9,Alpha3 | A.PGIPGEKGLPGL.Q SEQ ID NO747 |
| MMP9,Alpha3 | Q.GPPGPKGDPGPPGP.I SEQ ID NO748 |
| MMP9,Alpha3 | G.SLGHPGPPGVAGPLG.Q SEQ ID NO749 |
| MMP9,Alpha3 | G.KDGIPGPLGPLGPPG.A SEQ ID NO686 |
| MMP9,Alpha3 | G.VLGPQGKTGEVGPLG.E SEQ ID NO688 |
| MMP9,Alpha3 | G.ELGFQGQTGPPGPAG.V SEQ ID NO750 |
| MMP9,Alpha3 | G.EDGERGAEGPPGPTG.Q SEQ ID NO690 |
| MMP9,Alpha3 | G.LQGPPGFPGPKGPPG.H SEQ ID NO691 |
| MMP9,Alpha3 | G.EKGHIGLIGLIGPPG.E SEQ ID NO751 |
| MMP9,Alpha3 | G.QMGPPGPLGPSGLPGLK.G) SEQ ID NO694 |
| MMP9,Alpha3 | G.PVGEPGLLGAPGQMGPPG.P SEQ ID NO752 |
| MMP9,Alpha3 | G.LRGIPGPVGEPGLLGAPG.Q SEQ ID NO696 |
| MMP9,Alpha3 | G.LLGPRGSPGPTGRPGVTG.I SEQ ID NO697 |
| MMP9,Alpha3 | G.KDGIPGPLGPLGPPGAAGPSG.ESEQ ID NO700 |
| MMP9,Alpha3 | Q.GLPGLEGREGAKGELGPPGPLG.K SEQ ID NO701 |
| MMP9,Alpha3 | G.SRGERGPPGPTGKDGIPGPLGPLG.P SEQ ID NO753 |
| MMP9,Alpha3 | G.EKGKSGKTGQPGLEGERGPPGSRG.E SEQ ID NO754 |
| MMP9,Alpha3 | L.GPIGEKGKSGKTGQPGLEGERGPPGSRG.E SEQ ID NO702 |
| MMP9,Alpha3 | G.ANGSPGERGPLGPAGGIGLPGQSGSEGPVGPAG.K SEQ ID NO 704 |
| MMP9,Alpha3 | G.LIGTPGEKGPPGNPGIPGLPGSDGPLGHPGHEGPTG.E SEQ ID NO705 |
| MMP13,Alpha1 | L.PGEPGPRG.L SEQ ID NO755 |
| MMP13,Alpha1 | A.LRGPAGPMG.L SEQ ID NO756 |
| MMP13,Alpha1 | G.LPGEPGPRG.L SEQ ID NO706 |
| MMP13,Alpha1 | L.ALRGPAGPMG.L SEQ ID NO707 |
| MMP13,Alpha1 | R.LALRGPAGPMG.L SEQ ID NO708 |
| MMP13,Alpha1 | G.LRGFPGDRGLPGPVG.A SEQ ID NO716 |
| MMP13,Alpha1 | Q.ESQAQAILQQARLA.L SEQ ID NO730 |
| MMP13,Alpha1 | P.GPDGPPGPMGPPGLPGLK.G SEQ ID NO740 |
| MMP13,Alpha1 | G.PQGAIGPPGEKGPLGKPGLPGMPGADGPPGHPG.K SEQ ID NO757 |
| MMP13,Alpha1 | A.GPMGLTGRPGPVGPPGSGGLKGEPGDVGPQGPRG.V SEQ ID NO758 |
| MMP13,Alpha3 | G.VLGPQGKTGEVGPLG.E SEQ ID NO688 |
| MMP13,Alpha3 | G.LRGIPGPVGEPGLLGAPG.Q SEQ ID NO696 |
| MMP13,Alpha3 | G.LRGIPGPVGEPGLLGAPGQMGPPGPLGPSG.L SEQ ID NO703 |
| MMP13,Alpha3 | G.LRGIPGPVGEPGLLGAPGQMGPPGPLGPSGLPG.L SEQ ID NO759 |

| | |
|---|---|
| P is hydroxyproline, K indicates hydroxylysine, glycosylation, lipoxidation or cross linking. | |

The immunological binding partner may be one specifically reactive with a C-terminal or N-terminal neoepitope formed by cleavage of type v collagen.

Suitable immunological binding partners may therefore be specifically reactive with any of the following sequences at the N terminal of a peptide:

**Table 15a. N-terminal sequences of protease generated peptide fragments of Collagen type V.**

| Collagen type V | | |
|---|---|---|
| GDPGPP SEQ ID NO373 | LRGIPG SEQ ID NO760 | IGPPGI SEQ ID NO761 |
| LQGPPG SEQ ID NO62 | IGSLGH SEQ ID NO762 | IRGPPG SEQ ID NO763 |
| ANGSPG SEQ ID NO764 | LIGTPG SEQ ID NO765 | LPGEPG SEQ ID NO766 |
| IPGRPG SEQ ID NO767 | GPDGPP SEQ ID NO768 | QPGPSG SEQ ID NO769 |
| LKGNEG SEQ ID NO770 | ERGHPG SEQ ID NO771 | GPPGEQ SEQ ID NO772 |
| FPGPKG SEQ ID NO491 | PFRFGG SEQ ID NO773 | ESQAQA SEQ ID NO774 |
| LLGPKG SEQ ID NO775 | QQGNPG SEQ ID NO776 | KEGPPG SEQ ID NO777 |
| IGLIGP SEQ ID NO778 | GPPGPP SEQ ID NO100 | LGPPGE SEQ ID NO779 |
| GPPGPK SEQ ID NO780 | SLGHPG SEQ ID NO781 | KDGIPG SEQ ID NO782 |
| PVGEPG SEQ ID NO783 | LRGIPG SEQ ID NO760 | KDGIPG SEQ ID NO782 |
| ANGSPG SEQ ID NO764 | LIGTPG SEQ ID NO765 | PGEPGP SEQ ID NO784 |
| LLGAPG SEQ ID NO785 | GLPGLE SEQ ID NO786 | GIPGEK SEQ ID NO787 |
| LALRGP SEQ ID NO788 | LTGRPG SEQ ID NO789 | LLGPKG SEQ ID NO775 |
| LRGFPG SEQ ID NO790 | KTGPIG SEQ ID NO791 | PPGRPG SEQ ID NO792 |
| PGPKGN SEQ ID NO527 | EQGLPG SEQ ID NO793 | GPPGEK SEQ ID NO640 |
| AIGGPP SEQ ID NO794 | GPNGDP SEQ ID NO795 | PGPPGE SEQ ID NO796 |
| LLGPRG SEQ ID NO797 | GPDGPP SEQ ID NO768 | ERGPNG SEQ ID NO798 |
| GPKGDP SEQ ID NO799 | GDPGPP SEQ ID NO373 | PGIPGE SEQ ID NO800 |
| LQGPPG SEQ ID NO62 | EKGHIG SEQ ID NO801 | QMGPPG SEQ ID NO802 |
| SRGERG SEQ ID NO803 | EKGKSG SEQ ID NO804 | GPIGEK SEQ ID NO805 |
| LPGEPG SEQ ID NO766 | PQGAIG SEQ ID NO806 | GPMGLT SEQ ID NO807 |
| QMGPPG SEQ ID NO 802 | ETGFQG SEQ ID NO808 | GSKGPM SEQ ID NO809 |
| ALRGPA SEQ ID NO810 | EAGHPG SEQ ID NO811 | EKGHPG SEQ ID NO812 |
| KDGIP SEQ ID NO813 | VLGPQG SEQ ID NO814 | EDGERG SEQ ID NO815 |
| GPKGSI SEQ ID NO816 | ELGFQG SEQ ID NO817 | LRGPAG SEQ ID NO818 |

| | | |
|---|---|---|
| P is hydroxyproline, K indicates hydroxylysine, glycosylation, lipoxidation or cross linking. | | |

or with any of the following sequences at the C-terminal of a peptide:

**Table 16a. C-terminal sequences of protease generated peptide fragments of Collagen type V.**

| Collagen type V | | |
|---|---|---|
| PIGSLG SEQ ID NO 819 | PVGEPG SEQ ID NO783 | PGPPGE SEQ ID NO796 |
| PPGPTG SEQ ID NO820 | PKGPPG SEQ ID NO821 | VAGPLG SEQ ID NO 822 |
| RPGVTG SEQ ID NO823 | MMPFQF SEQ ID NO824 | PGAAGP SEQ ID NO825 |
| PVGPAG SEQ ID NO826 | HEGPTG SEQ ID NO827 | EPGPRG SEQ ID NO516 |
| GPPGLP SEQ ID NO828 | GQGPPG SEQ ID NO664 | PPGPPG SEQ ID NO119 |
| AGSPGE SEQ ID NO829 | PVGALG SEQ ID NO830 | EQGLPG SEQ ID NO793 |
| YPGPRG SEQ ID NO831 | HPGQRG SEQ ID NO832 | GGGGDA SEQ ID NO833 |
| LIGPPG SEQ ID NO834 | GLPGLK SEQ ID NO835 | PPGPPG SEQ ID NO 119 |
| PPGPIG SEQ ID NO 174 | KGLPGL SEQ ID NO836 | PGPPGP SEQ ID NO 458 |
| QMGPPG SEQ ID NO802 | LLGAPG SEQ ID NO 785 | RPGVTG SEQ ID NO 823 |
| QQARLA SEQ ID NO837 | PPGHPG SEQ ID NO838 | PQGPRG SEQ ID NO 150 |
| PLGPPG SEQ ID NO839 | GEPGLL SEQ ID NO840 | EVGPLG SEQ ID NO841 |
| IMMPFQ SEQ ID NO842 | GLPGLK SEQ ID NO835 | PLGPSG SEQ ID NO843 |
| AAGPSG SEQ ID NO844 | PPGPLG SEQ ID NO845 | PPGSRG SEQ ID NO846 |
| PAGPMG SEQ ID NO847 | PPGSGG SEQ ID NO848 | PPGPPG SEQ ID NO119 |
| GLPGPV SEQ ID NO849 | LPGPVG SEQ ID NO850 | KPGPDG SEQ ID NO851 |
| LPGPQG SEQ ID NO852 | VIQPLP SEQ ID NO853 | RGPNGP SEQ ID NO854 |
| PGPQGS SEQ ID NO855 | EKGPLG SEQ ID NO856 | PLGPPG SEQ ID NO839 |
| GPPGAA SEQ ID NO857 | TGPKGE SEQ ID NO858 | GPKGSI SEQ ID NO816 |
| PPGPAG SEQ ID NO52 | PPGPAG SEQ ID NO52 | PPGPTG SEQ ID NO820 |
| QGLPGL SEQ ID NO859 | PSGLPG SEQ ID NO860 | LLGAPG SEQ ID NO785 |
| PPGSRG SEQ ID NO 846 | LPGPPG SEQ ID NO72 | PPGLPG SEQ ID NO861 |
| PPGPLG SEQ ID NO 845 | KPGPRG SEQ ID NO862 | PKGPPG SEQ ID NO 821 |
| PLGPLG SEQ ID NO863 | PPGSRG SEQ ID NO846 | |

| | | |
|---|---|---|
| P is hydroxyproline, K indicates hydroxylysine, glycosylation, lipoxidation or cross linking. | | |

### Collagen VI

We have determined that the enzymes listed in the following table cleave type vi collagen at least the following cleavage sites (marked "." or in the ablsence of a '.', at the end of the sequence):

**Table 14B. Cleavage fragments of collagen type VI**

| Protease | Neoepitope |
|---|---|
| MMP2 | G.YRGPEGPQGPPG.H SEQ ID NO864 |
| MMP2 | G.PIGPKGYRGDEGPP.G SEQ ID NO865 |
| MMP2, (a3) | I.GIGIGNADIT.E SEQ ID NO866 |
| MMP2, (a3) | G.AQGPAGPAGPPG.L SEQ ID NO867 |
| MMP9 | G.LIGEQGISGPRG.S SEQ ID NO868 |
| MMP9 | P.PGLIGEQGISGPR.G SEQ ID NO869 |
| MMP9 | E.PGEPGPKGGIGNRG.P SEQ ID NO870 |
| MMP9 | G.ISGPRGSGGAAGAPGERGRTGPLG.R SEQ ID NO871 |
| MMP13 | PGPAGPPGDPGLMG SEQ ID NO872 |
| FAP-1 | VAAKPAAVRPPAAAAAKPVATKPEVPRP SEQ ID NO873 |
| FAP-1 | GEPGLNGTTGPKGI SEQ ID NO874 |
| FAP-1 | IGPKGIPGEDGYRGYPG SEQ ID NO875 |
| FAP-1 | VAVVQHAPSESVDNASMPPVKVEFSL SEQ ID NO876 |
| FAP-2 | LGPMGVPGRD SEQ ID NO877 |
| FAP-2 | GEPGPPGEKGEAGDEGNPGPDGAPGERG SEQ ID NO878 |
| FAP-2 | RGPIGSIGPKGIPGEDGYRGYPGDEGGP SEQ ID NO879 |
| FAP-2 | PPPPQPARSAS SEQ ID NO880 |
| FAP-2 | FGPSAATPAPPG SEQ ID NO881 |
| FAP-2 | GPKGETGDLGPMGVPGRDGVPGGPGETGK SEQ ID NO882 |

The immunological binding partner may be one specifically reactive with a C-terminal or N-terminal neoepitope formed by cleavage of type v collagen.

Suitable immunological binding partners may therefore be specifically reactive with any of the following sequences at the N terminal of a peptide:

**Table 15b. N-terminal sequences of protease generated peptide fragments of Collagen type VI.**

| Collagen type VI | | |
|---|---|---|
| YRGPEG SEQ ID NO883 | PIGPKG SEQ ID NO865 | GIGIGN SEQ ID NO885 |
| ISGPRG SEQ ID NO886 | PGPAGP SEQ ID NO887 | VAAKPA SEQ ID NO888 |
| GEPGPP SEQ ID NO675 | RGPIGS SEQ ID NO889 | PPPPQP SEQ ID NO890 |
| AQGPAG SEQ ID NO891 | LIGEQG SEQ ID NO892 | PGLIGE SEQ ID NO893 |
| GEPGLN SEQ ID NO894 | IGPKGI SEQ ID NO895 | VAVVQH SEQ ID NO896 |
| FGPSAA SEQ ID NO897 | GPKGET SEQ ID NO898 | PGEPGP SEQ ID NO784 |
| LGPMGV SEQ ID NO899 | | |

| | | |
|---|---|---|
| or with any of the following sequences at the C-terminal of a peptide: | | |

**Table 16b. C-terminal sequences of protease generated peptide fragments of Collagen type VI.**

| Collagen type VI | | |
|---|---|---|
| GDEGPP SEQ ID NO900 | GNADIT SEQ ID NO901 | PAGPPG SEQ ID NO133 |
| DPGLMG SEQ ID NO902 | PEVPRP SEQ ID NO903 | TGPKGISEQ ID NO904 |
| GDEGGP SEQ ID NO905 | PARSAS SEQ ID NO906 | TPAPPG SEQ ID NO915 |
| ISGPRG SEQ ID NO886 | GISGPRSEQ ID NO907 | GIGNRG SEQ ID NO908 |
| YRGYPGSEQ ID NO909 | KVEFSL SEQ ID NO910 | GVPGRD SEQ ID NO911 |
| PGETGKSEQ ID NO912 | RTGPLG SEQ ID NO913 | APGERG SEQ ID NO914 |

### Proteoglycans

In another aspect of the invention, said peptide fragments are fragments of proteoglycans versican, lumican, perlecan, biglycan and decorin, which are all identified in fibrotic tissue.

Several candidate proteases may be responsible for the digestion of proteoglycans in fibrotic lesions We have determined that the enzymes listed in table 17 generate lumican, versican, biglycan, perlecan and decorin resulting in at least following cleavage products:

**Table 17. Cleavage fragments of biglycan, decorin, versican, lumican, and perlecan.**

| **Protease** | **Biglycan** |
|---|---|
| MMP-3 | SVPKEISPDTTLLDLQNNDISE SEQ ID NO916 |
| MMP-3 | KSVPKEISPDTTLLDLQNNDISE SEQ ID NO917 |
| MMP-9 | NSGFEPGAFDGLKLNYLRISEAK SEQ ID NO918 |
| MMP-9 | LKSVPKEISPDTTLLDLQNNDISE SEQ ID NO919 |
| MMP-12 | LRISEAKLTGIPKDLPET SEQ ID NO920 |
| MMP-13 | LKSVPKEISPDTTLLDLQNNDISE SEQ ID NO919 |
| MMP-13 | LTGIPKDLPETLNELHLDHNKIQAIE SEQ ID NO921 |
| ADAMTS4 | RISEAKLTGIPKDLPETLNE SEQ ID NO922 |
| ADAMTS4 | AIELEDLLRYSK SEQ ID NO923 |
| ADAMTS4 | AIELEDLLRY SEQ ID NO924 |
| ADAMTS4 | EAKLTGIPKDLPETLNE SEQ ID NO925 |
| ADAMTS4 | LKAVPKEISPDTTLLDLQNNDISE SEQ ID NO926 |
| MMP-8 | LLDLQNNDISELRKDD SEQ ID NO927 |
| MMP-8 | IELEDLLRYS SEQ ID NO928 |
| CathepsinS | NSGFEPGAFDGLK SEQ ID NO929 |

| **Protease** | **Decorin** |
|---|---|
| MMP-12 | IVIELGTNPLK SEQ ID NO930 |
| MMP-3 | DEASGIGPEVPDDR SEQ ID NO931 |
| MMP-3 | LHLDGNKISRVDAAS SEQ ID NO932 |
| MMP-3 | VNNKISKVSPGAFTPL SEQ ID NO933 |
| MMP-3 | LILVNNKISKVSPGAFTPLVKLER SEQ ID NO934 |
| MMP-9 | SNPVQYWEIQPSTFR SEQ ID NO935 |
| CathepsinK | SSGIENGAFQGMK SEQ ID NO884 |
| CathepsinK | SSGIENGAFQGMKKLS SEQ ID NO946 |
| ADAMTS1 | KITEIKDGDFK SEQ ID NO936 |
| ADAMTS1 | GLPPSLTELHLDGNK SEQ ID NO937 |

| | **Versican** |
|---|---|
| Unknown | LLASDAGLYR SEQ ID NO938 |
| Unknown | LATVGELQAAWR SEQ ID NO939 |
| Unknown | ETTVLVAQNGNIK SEQ ID NO940 |

| | **Lumican** |
|---|---|
| Unknown | SLEDLQLTHNK SEQ ID NO941 |
| Unknown | LKEDAVSAAFK SEQ ID NO942 |

| | **Perlecan** |
|---|---|
| Unknown | SIEYSPQLEDAGSR SEQ ID NO943 |
| Unknown | LEGDTLIIPR SEQ ID NO944 |
| ADAMTS4 | VSEAVVEKLEPEYR SEQ ID NO945 |
| ADAMTS4 | EVSEAVVEKLEPEYR SEQ ID NO947 |
| ADAMTS4 | SIEYSPQLEDASAKEFR SEQ ID NO948 |

The immunological binding partner may be one specifically reactive with a C-terminal or N-terminal neo-epitope formed by cleavage of type versican, lumican, decorin, perlecan, and biglycan.

Suitable immunological binding partners may therefore be specifically reactive with any of the following at the N terminal of a peptide:

**Table 18. N-terminal sequences of protease generated peptide fragments of biglycan, decorin, lumican, versican, and perlecan.**

| Biglycan | | |
|---|---|---|
| SVPKEI SEQ ID NO949 | GLKLNY SEQ ID NO950 | RISEAK SEQ ID NO951 |
| NSGFEP SEQ ID NO952 | LKSVPK SEQ ID NO953 | AIELED SEQ ID NO954 |
| IELEDL SEQ ID NO957 | QCSDLG SEQ ID NO955 | EAKLTG SEQ ID NO956 |
| LRISEA SEQ ID NO958 | LTGIPK SEQ ID NO959 | LKAVPK SEQ ID NO960 |
| LLDLQN SEQ ID NO961 | | |

| Decorin | | |
|---|---|---|
| IVIELG SEQ ID NO962 | DEASGI SEQ ID NO 963 | VNNKIS SEQ ID NO964 |
| NGLNQM SEQ ID NO965 | LHLDGN SEQ ID NO966 | LILVNN SEQ ID NO967 |
| SSGIEN SEQ ID NO968 | KITEIK SEQ ID NO969 | GLPPSL SEQ ID NO970 |
| SNPVQY SEQ ID NO971 | | |

| Versican | | |
|---|---|---|
| LLASDA SEQ ID NO972 | LATVGE SEQ ID NO973 | ETTVLV SEQ ID NO974 |
| ENQDAR SEQ ID NO975 | NGFDQC SEQ ID NO976 | SLTVVK SEQ ID NO977 |
| | | |

| Lumican | | |
|---|---|---|
| SLEDLQ SEQ ID NO978 | LKEDAV SEQ ID NO979 | HLQHNR SEQ ID NO980 |
| LQHNRL SEQ ID NO985 | | |

| Perlecan | | |
|---|---|---|
| SIEYSP SEQ ID NO981 | LVNFTR SEQ ID NO982 | VSEAVV SEQ ID NO983 |
| EVSEAV SEQ ID NO984 | | |

or with any of the following sequences in table 19, at the C-terminal of a peptide:

**Table 19. C-terminal sequences of protease generated peptide fragments of biglycan, decorin, lumican, versican, and perlecan.**

| Biglycan | | |
|---|---|---|
| NNDISE SEQ ID NO986 | YWEVQP SEQ ID NO987 | EDLLRY SEQ ID NO988 |
| RISEAK SEQ ID NO951 | KIQAIE SEQ ID NO989 | PETLNE SEQ ID NO990 |
| LRKDDF SEQ ID NO991 | LLRYSK SEQ ID NO992 | ELRKDD SEQ ID NO993 |
| KDLPET SEQ ID NO994 | DLLRYS SEQ ID NO995 | AFDGLK SEQ ID NO996 |
| LNELHL SEQ ID NO997 | | |

| Decorin | | |
|---|---|---|
| GTNPLK SEQ ID NO998 | EVPDDR SEQ ID NO999 | GAFTPL SEQ ID NO1000 |
| SSGIEN SEQ ID NO968 | RVDAAS SEQ ID NO1001 | LVKLER SEQ ID NO1002 |
| GMKKLS SEQ ID NO1003 | KDGDFK SEQ ID NO1004 | HLDGNK SEQ ID NO1005 |
| QPSTFR SEQ ID NO1006 | AFQGMK SEQ ID NO1007 | |

| Versican | | |
|---|---|---|
| CDVMYG SEQ ID NO1008 | NGFDQC SEQ ID NO976 | QNGINK SEQ ID NO1009 |
| IGQDYK SEQ ID NO1010 | | |

| Lumican | | |
|---|---|---|
| QLTHNK SEQ ID NO1011 | VSAAFK SEQ ID NO1012 | GLKSLE SEQ ID NO1013 |
| | | |

| Perlecan | | |
|---|---|---|
| EDAGSR SEQ ID NO1014 | EFREVS SEQ ID NO1015 | VAQQDS SEQ ID NO1016 |
| SAKEFR SEQ ID NO1017 | LEPEYR SEQ ID NO1018 | |

### CRP

Several candidate proteases may be responsible for the digestion of CRP in fibrotic tissue the literature reports many different proteases in fibrotic tissue. Most likely, this is the result of the large range of complicated processes eventually leading to fibrosis. However, in our assessment, early phases may consist of a range of MMPs, whereas later stages may rely more on cathepsin K degradation of the matrix, resulting in different neo-epitope profiles dependent on the levels of disease. We have through a range of in vitro cleavages of pure native proteins determined that the enzymes listed in the following tables of cleaved CRP at least following cleavage sites (marked * in Table 20, but at the ends of each sequence in Table 21):

**Table 20. CRP fragments generated by specific proteases.**

| **Protease/Protein** | **Neo-epitope** |
|---|---|
| CRP + CatK | K*ESDTSYVSLKAPLT*K SEQ ID NO1019 |
| CRP + CatK | G*GNFEGSQSLVGDIG*N SEQ ID NO1020 |
| CRP + MMP9 | A*LKYEVQGEVFTKPQ*L SEQ ID NO1021 |
| CRP + MMP9 | G*IVEFWVDGKPRV*R SEQ ID NO1022 |
| CRP + MMP1/MMP3 | R*KAFVFPKE*S SEQ ID NO1023 |
| CRP + MMP3 | K*YEVQGEVFTKPQLWP*- SEQ ID NO1024 |
| CRP + MMP3 | D*SFGGNFEGSQS*L SEQ ID NO1025 |
| CRP + MMP3 | D*FVLSPDEINT*I SEQ ID NO1026 |
| CRP + MMP3 | S*LKKGYTVGAEA*S SEQ ID NO1027 |
| CRP + MMP3 | A*FGQTDMSRKA*F SEQ ID NO1028 |
| CRP + MMP3 | S*LKKGYTVGAEAS*I SEQ ID NO1029 |
| CRP + MMP3 | G*EVFTKPQLWP*- SEQ ID NO1030 |
| CRP + MMP3 | S*IILGQEQDSFGGN.F SEQ ID NO1031 |
| CRP + MMP3 | K*YEVQGEVFTKPQ.L SEQ ID NO1032 |

**Table 21 CRP fragments generated by specific proteases.**

| **Protease** | **Neoepitope** | **Aminoacid Nos*** |
|---|---|---|
| MMP9 | AFVFPK SEQ ID NO1033 | 026-031 |
| MMP9 | FGQTDMSR SEQ ID NO1034 | 017-024 |
| MMP9 | FGQTDMSRK SEQ ID NO1035 | 017-025 |
| MMP9 | FGQTDMSRKA SEQ ID NO1036 | 017-026 |
| MMP9 | FGQTDMSRKAF SEQ ID NO1037 | 017-027 |
| MMP9 | FGQTDMSRKAFVFPKE SEQ ID NO1038 | 017-032 |
| MMP9 | FGQTDMSRKAFVFPKESDTS SEQ ID NO1039 | 017-036 |
| MMP9 | FGQTDMSRKAFVFPKESDTSYV SEQ ID NO1040 | 017-038 |
| MMP9 | FGQTDMSRKAFVFPKESDTSYVS SEQ ID NO1041 | 017-039 |
| MMP9 | TDMSRKAFVFPKESDTSYV SEQ ID NO1042 | 020-038 |
| MMP9 | MSRKAFVFPKESDTS SEQ ID NO1043 | 022-036 |
| MMP9 | SRKAFVFPKESDTSYV SEQ ID NO1044 | 023-038 |
| MMP9 | RKAFVFPKE SEQ ID NO1045 | 024-032 |
| MMP9 | RKAFVFPKESDTSYV SEQ ID NO1046 | 024-038 |
| MMP9 | RKAFVFPKESDTSYVS SEQ ID NO1047 | 024-039 |
| MMP9 | KAFVFPKE SEQ ID NO1048 | 025-032 |
| MMP9 | KAFVFPKESD SEQ ID NO1049 | 025-034 |
| MMP9 | KAFVFPKESDT SEQ ID NO1050 | 025-035 |
| MMP9 | KAFVFPKESDTS SEQ ID NO1051 | 025-036 |
| MMP9 | KAFVFPKESDTSYV SEQ ID NO1052 | 025-038 |
| MMP9 | KAFVFPKESDTSYVS SEQ ID NO1053 | 025-039 |
| MMP9 | AFVFPKE SEQ ID NO1054 | 026-032 |
| MMP9 | AFVFPKESDT SEQ ID NO1055 | 026-035 |
| MMP9 | AFVFPKESDTSYV SEQ ID NO1056 | 026-038 |
| MMP9 | AFVFPKESDTSYVS SEQ ID NO1057 | 026-039 |
| MMP9 | AFVFPKESDTSYVSL SEQ ID NO1058 | 026-040 |
| MMP9 | FVFPK SEQ ID NO1059 | 027-031 |
| MMP9 | FVFPKE SEQ ID NO1060 | 027-032 |
| MMP9 | FVFPKESD SEQ ID NO1061 | 027-034 |
| MMP9 | FVFPKESDTS SEQ ID NO1062 | 027-036 |
| MMP9 | FVFPKESDTSY SEQ ID NO1063 | 027-037 |
| MMP9 | FVFPKESDTSYV SEQ ID NO1064 | 027-038 |
| MMP9 | FVFPKESDTSYVS SEQ ID NO1065 | 027-039 |
| MMP9 | FVFPKESDTSYVSL SEQ ID NO1066 | 027-040 |
| MMP9 | VFPKESDTS SEQ ID NO1067 | 028-036 |
| MMP9 | VFPKESDTSYV SEQ ID NO1068 | 028-038 |
| MMP9 | VFPKESDTSYVS SEQ ID NO1069 | 028-039 |
| MMP9 | VFPKESDTSYVSL SEQ ID NO1070 | 028-040 |
| MMP9 | FPKESDTSYVS SEQ ID NO1071 | 029-039 |
| MMP9 | KESDTSYVSLKAPLTKP SEQ ID NO1072 | 031-047 |
| MMP9 | SDTSYVSLKAPLTKP SEQ ID NO1073 | 033-047 |
| MMP9 | SLKAPLTKP SEQ ID NO1074 | 039-047 |
| MMP9 | SLKAPLTKPLK SEQ ID NO1075 | 039-049 |
| MMP9 | LKAPLTKPLK SEQ ID NO1076 | 040-049 |
| MMP9 | FYTELSSTRGYS SEQ ID NO1077 | 057-068 |
| MMP9 | LSSTRGYS SEQ ID NO1078 | 061-068 |
| MMP9 | SSTRGYS SEQ ID NO1079 | 062-068 |
| MMP9 | STRGYS SEQ ID NO1080 | 063-068 |
| MMP9 | IFSYATKRQ SEQ ID NO1081 | 069-077 |
| MMP9 | IFSYATKRQDNEILI SEQ ID NO1082 | 069-083 |
| MMP9 | SYATKRQDNEILI SEQ ID NO1083 | 071-083 |
| MMP9 | YATKRQDNEIL SEQ ID NO1084 | 072-082 |
| MMP9 | YATKRQDNEILI SEQ ID NO1085 | 072-083 |
| MMP9 | YATKRQDNEILIF SEQ ID NO1086 | 072-084 |
| MMP9 | TKRQDNEILI SEQ ID NO1087 | 074-083 |
| MMP9 | TKRQDNEILIF SEQ ID NO1088 | 074-084 |
| MMP9 | TKRQDNEILIFWSKDI SEQ ID NO1089 | 074-089 |
| MMP9 | KRQDNEILI SEQ ID NO1090 | 075-083 |
| MMP9 | KRQDNEILIF SEQ ID NO1091 | 075-084 |
| MMP9 | WSKDIGYS SEQ ID NO1092 | 085-092 |
| MMP9 | SKDIGYS SEQ ID NO1093 | 086-092 |
| MMP9 | IVEFWVDGKPRV SEQ ID NO1094 | 124-135 |
| MMP9 | EFWVDGKPR SEQ ID NO1095 | 126-134 |
| MMP9 | WVDGKPRV SEQ ID NO1096 | 128-135 |
| MMP9 | VDGKPRV SEQ ID NO1097 | 129-135 |
| MMP9 | SLKKGYTVGAE SEQ ID NO1098 | 138-148 |
| MMP9 | SLKKGYTVGAEA SEQ ID NO1099 | 138-149 |
| MMP9 | SLKKGYTVGAEAS SEQ ID NO1100 | 138-150 |
| MMP9 | LKKGYTV SEQ ID NO1101 | 139-145 |
| MMP9 | LKKGYTVG SEQ ID NO1102 | 139-146 |
| MMP9 | LKKGYTVGA SEQ ID NO1103 | 139-147 |
| MMP9 | LKKGYTVGAE SEQ ID NO1104 | 139-148 |
| MMP9 | LKKGYTVGAEA SEQ ID NO1105 | 139-149 |
| MMP9 | LKKGYTVGAEAS SEQ ID NO1106 | 139-150 |
| MMP9 | LKKGYTVGAEASI SEQ ID NO1107 | 139-151 |
| MMP9 | SIILGQEQDSFGGN SEQ ID NO1108 | 150-163 |
| MMP9 | SIILGQEQDSFGGNFEGSQ SEQ ID NO1109 | 150-168 |
| MMP9 | SIILGQEQDSFGGNFEGSQS SEQ ID NO1110 | 150-169 |
| MMP9 | IILGQEQDSFGGNFEGS SEQ ID NO1111 | 151-067 |
| MMP9 | IILGQEQDSFGGNFEGSQS SEQ ID NO1112 | 151-169 |
| MMP9 | ILGQEQDSFGGN SEQ ID NO1113 | 152-163 |
| MMP9 | ILGQEQDSFGGNFEGSQ SEQ ID NO1114 | 152-168 |
| MMP9 | ILGQEQDSFGGNFEGSQS SEQ ID NO1115 | 152-169 |
| MMP9 | LGQEQDSFGGNFEGSQ SEQ ID NO1116 | 153-168 |
| MMP9 | LGQEQDSFGGNFEGSQS SEQ ID NO1117 | 153-169 |
| MMP9 | GQEQDSFGGNFEGSQS SEQ ID NO1118 | 154-169 |
| MMP9 | SFGGNFEGSQS SEQ ID NO1119 | 159-169 |
| MMP9 | QSLVGDIGNVN SEQ ID NO1120 | 168-178 |
| MMP9 | INTIYLGGPFSPNV SEQ ID NO1121 | 189-202 |
| MMP9 | INTIYLGGPFSPNVLN SEQ ID NO1122 | 189-204 |
| MMP9 | IYLGGPFSPNVLN SEQ ID NO1123 | 192-204 |
| MMP9 | YLGGPFSPNVLN SEQ ID NO1124 | 193-204 |
| MMP9 | LGGPFSPN SEQ ID NO1125 | 194-201 |
| MMP9 | SPNVLNWRALKYEVQGEVFTKPQLWP SEQ ID NO1126 | 199-224 |
| MMP9 | LNWRA SEQ ID NO1127 | 203-207 |
| MMP9 | LNWRAL SEQ ID NO1128 | 203-208 |
| MMP9 | LNWRALK SEQ ID NO1129 | 203-209 |
| MMP9 | WRALKYE SEQ ID NO1130 | 205-211 |
| MMP9 | WRALKYEV SEQ ID NO1131 | 205-212 |
| MMP9 | WRALKYEVQGE SEQ ID NO1132 | 205-215 |
| MMP9 | ALKYEV SEQ ID NO1133 | 207-212 |
| MMP9 | LKYEVQ SEQ ID NO1134 | 208-213 |
| MMP9 | LKYEVQG SEQ ID NO1135 | 208-214 |
| MMP9 | LKYEVQGE SEQ ID NO1136 | 208-215 |
| MMP9 | LKYEVQGEVFTKP SEQ ID NO1137 | 208-220 |
| MMP9 | LKYEVQGEVFTKPQ SEQ ID NO1138 | 208-221 |
| MMP9 | LKYEVQGEVFTKPQLWP SEQ ID NO1139 | 208-224 |
| MMP9 | KYEVQGE SEQ ID NO1140 | 209-215 |
| MMP9 | KYEVQGEVFTKPQ SEQ ID NO1141 | 209-221 |
| MMP9 | KYEVQGEVFTKPQLWP SEQ ID NO1142 | 209-224 |
| MMP9 | YEVQGEVFTKP SEQ ID NO1143 | 210-220 |
| MMP9 | YEVQGEVFTKPQ SEQ ID NO1144 | 210-221 |
| MMP9 | YEVQGEVFTKPQLWP SEQ ID NO1145 | 210-224 |
| MMP9 | VQGEVFTKPQ SEQ ID NO1146 | 212-221 |
| MMP9 | VQGEVFTKPQLWP SEQ ID NO1147 | 212-224 |
| MMP9 | QGEVFTKPQ SEQ ID NO1148 | 213-221 |
| MMP9 | GEVFTKP SEQ ID NO1149 | 214-220 |
| MMP9 | GEVFTKPQ SEQ ID NO1150 | 214-221 |
| MMP9 | EVFTKPQ SEQ ID NO1151 | 215-221 |
| MMP9 | EVFTKPQLWP SEQ ID NO1152 | 215-224 |
| MMP9 | VFTKPQ SEQ ID NO1153 | 216-221 |
| MMP9 | FTKPQ SEQ ID NO1154 | 217-221 |
| MMP9 | FTKPQLWP SEQ ID NO1155 | 217-224 |
| MMP9 | TKPQLWP SEQ ID NO1156 | 218-224 |
| MMP9 | KPQLWP SEQ ID NO1157 | 219-224 |
| MMP12 | FGQTDMSRKA SEQ ID NO1036 | 017-026 |
| MMP12 | MSRKAFVFP SEQ ID NO1158 | 022-030 |
| MMP12 | MSRKAFVFPKE SEQ ID NO1159 | 022-032 |
| MMP12 | MSRKAFVFPKESD SEQ ID NO1160 | 022-034 |
| MMP12 | MSRKAFVFPKESDTS SEQ ID NO1043 | 022-036 |
| MMP12 | MSRKAFVFPKESDTSYVS SEQ ID NO1161 | 022-039 |
| MMP12 | SRKAFVFP SEQ ID NO1162 | 023-030 |
| MMP12 | SRKAFVFPKESD SEQ ID NO1163 | 023-034 |
| MMP12 | SRKAFVFPKESDTS SEQ ID NO1164 | 023-036 |
| MMP12 | RKAFVFP SEQ ID NO1165 | 024-030 |
| MMP12 | RKAFVFPKESD SEQ ID NO1166 | 024-034 |
| MMP12 | KAFVFP SEQ ID NO1167 | 025-030 |
| MMP12 | KAFVFPKE SEQ ID NO1048 | 025-032 |
| MMP12 | KAFVFPKESD SEQ ID NO1049 | 025-034 |
| MMP12 | AFVFPKE SEQ ID NO1054 | 026-032 |
| MMP12 | AFVFPKESDTS SEQ ID NO1168 | 026-036 |
| MMP12 | AFVFPKESDTSYVS SEQ ID NO1057 | 026-039 |
| MMP12 | FVFPKE SEQ ID NO1060 | 027-032 |
| MMP12 | FVFPKESD SEQ ID NO1061 | 027-034 |
| MMP12 | FVFPKESDTS SEQ ID NO1062 | 027-036 |
| MMP12 | FVFPKESDTSY SEQ ID NO1063 | 027-037 |
| MMP12 | FVFPKESDTSYVS SEQ ID NO1065 | 027-039 |
| MMP12 | VFPKESD SEQ ID NO1169 | 028-034 |
| MMP12 | KESDTSY SEQ ID NO1170 | 031-037 |
| MMP12 | KESDTSYVS SEQ ID NO1171 | 031-039 |
| MMP12 | VSLKAP SEQ ID NO1172 | 038-043 |
| MMP12 | LKAPLT SEQ ID NO1173 | 040-045 |
| MMP12 | LKAPLTKP SEQ ID NO1174 | 040-047 |
| MMP12 | YTELSSTRGYS SEQ ID NO1175 | 058-068 |
| MMP12 | LSSTRGYS SEQ ID NO1078 | 061-068 |
| MMP12 | STRGYS SEQ ID NO1080 | 063-068 |
| MMP12 | YATKRQDNE SEQ ID NO1176 | 072-080 |
| MMP12 | YATKRQDNEI SEQ ID NO1177 | 072-081 |
| MMP12 | YATKRQDNEIL SEQ ID NO1084 | 072-082 |
| MMP12 | TKRQDNEIL SEQ ID NO1178 | 074-082 |
| MMP12 | KRQDNEIL SEQ ID NO1179 | 075-082 |
| MMP12 | ILIFWSKD SEQ ID NO1180 | 081-088 |
| MMP12 | IFWSKD SEQ ID NO1181 | 083-088 |
| MMP12 | SKDIGYS SEQ ID NO1093 | 086-092 |
| MMP12 | WVDGKPRV SEQ ID NO1096 | 128-135 |
| MMP12 | WVDGKPRVR SEQ ID NO1182 | 128-136 |
| MMP12 | VRKSLKKGYTVGAEAS SEQ ID NO1183 | 135-150 |
| MMP12 | SLKKGYT SEQ ID NO1184 | 138-144 |
| MMP12 | SLKKGYTVG SEQ ID NO1185 | 138-146 |
| MMP12 | SLKKGYTVGA SEQ ID NO1186 | 138-147 |
| MMP12 | SLKKGYTVGAE SEQ ID NO1098 | 138-148 |
| MMP12 | SLKKGYTVGAEA SEQ ID NO1099 | 138-149 |
| MMP12 | SLKKGYTVGAEAS SEQ ID NO1100 | 138-150 |
| MMP12 | SLKKGYTVGAEASI SEQ ID NO1187 | 138-151 |
| MMP12 | LKKGYTV SEQ ID NO1101 | 139-145 |
| MMP12 | LKKGYTVG SEQ ID NO1102 | 139-146 |
| MMP12 | LKKGYTVGA SEQ ID NO1103 | 139-147 |
| MMP12 | LKKGYTVGAE SEQ ID NO1104 | 139-148 |
| MMP12 | LKKGYTVGAEA SEQ ID NO1105 | 139-149 |
| MMP12 | LKKGYTVGAEAS SEQ ID NO 1106 | 139-150 |
| MMP12 | LKKGYTVGAEASI SEQ ID NO 1107 | 139-151 |
| MMP12 | KKGYTVGAEAS SEQ ID NO1188 | 140-150 |
| MMP12 | KGYTVGAEAS SEQ ID NO1189 | 141-150 |
| MMP12 | KGYTVGAEASI SEQ ID NO1190 | 141-151 |
| MMP12 | SIILGQEQDSFGGN SEQ ID NO 1108 | 150-163 |
| MMP12 | IILGQEQD SEQ ID NO1191 | 151-158 |
| MMP12 | IILGQEQDSFGGN SEQ ID NO1192 | 151-163 |
| MMP12 | IILGQEQDSFGGNFEGSQS SEQ ID NO1112 | 151-169 |
| MMP12 | ILGQEQDSFGGN SEQ ID NO1113 | 152-163 |
| MMP12 | LVGDIGNVNMWD SEQ ID NO1193 | 170-181 |
| MMP12 | INTIYLGGPFSPNVLN SEQ ID NO1122 | 189-204 |
| MMP12 | IYLGGPFSPN SEQ ID NO1194 | 192-201 |
| MMP12 | IYLGGPFSPNV SEQ ID NO1195 | 192-202 |
| MMP12 | IYLGGPFSPNVLN SEQ ID NO1123 | 192-204 |
| MMP12 | LGGPFSPNVLN SEQ ID NO1196 | 194-204 |
| MMP12 | WRALKYE SEQ ID NO1130 | 205-210 |
| MMP12 | YEVQGEVFTKP SEQ ID NO1143 | 210-220 |
| MMP12 | YEVQGEVFTKPQ SEQ ID NO1144 | 210-221 |
| MMP12 | YEVQGEVFTKPQLWP SEQ ID NO1145 | 210-224 |
| MMP12 | EVQGEVFTKP SEQ ID NO1197 | 211-220 |
| MMP12 | EVQGEVFTKPQLWP SEQ ID NO1198 | 211-224 |
| MMP12 | VQGEVFTKP SEQ ID NO1199 | 212-220 |
| MMP12 | VQGEVFTKPQ SEQ ID NO1146 | 212-221 |
| MMP12 | VQGEVFTKPQLWP SEQ ID NO1147 | 212-224 |
| MMP12 | GEVFTKPQLWP SEQ ID NO1200 | 214-224 |
| MMP12 | EVFTKP SEQ ID NO1201 | 215-220 |
| MMP12 | EVFTKPQLWP SEQ ID NO1152 | 215-224 |
| MMP12 | VFTKPQ SEQ ID NO1153 | 216-221 |
| MMP12 | VFTKPQL SEQ ID NO1202 | 216-222 |
| MMP12 | VFTKPQLWP SEQ ID NO1203 | 216-224 |
| MMP12 | FTKPQLWP SEQ ID NO1155 | 217-224 |
| MMP12 | TKPQLWP SEQ ID NO1156 | 218-224 |
| MMP1 | AFVFPK SEQ ID NO1033 | 006-031 |
| MMP1 | KAFVFPK SEQ ID NO1204 | 025-031 |
| MMP1 | VRKSLK SEQ ID NO1205 | 135-140 |
| MMP1 | YEVQGEVFTKPQLWP SEQ ID NO1145 | 210-224 |
| MMP3 | FGQTDMSRKA SEQ ID NO 1036 | 017-026 |
| MMP3 | FGQTDMSRKAF SEQ ID NO 1037 | 017-027 |
| MMP3 | MSRKAFVFPKESDTSYV SEQ ID NO1206 | 022-038 |
| MMP3 | MSRKAFVFPKESDTSYVS SEQ ID NO1161 | 022-039 |
| MMP3 | SRKAFVFPKESDTSYV SEQ ID NO1044 | 023-038 |
| MMP3 | SRKAFVFPKESDTSYVS SEQ ID NO1207 | 023-039 |
| MMP3 | RKAFVFPKESDTSYV SEQ ID NO1046 | 024-038 |
| MMP3 | RKAFVFPKESDTSYVS SEQ ID NO1047 | 024-039 |
| MMP3 | KAFVFPKE SEQ ID NO1048 | 025-032 |
| MMP3 | KAFVFPKESDTS SEQ ID NO1051 | 025-036 |
| MMP3 | KAFVFPKESDTSYVS SEQ ID NO1053 | 025-039 |
| MMP3 | KAFVFPKESDTSYVSL SEQ ID NO1208 | 025-040 |
| MMP3 | KAFVFPKESDTSYVSLK SEQ ID NO1209 | 025-041 |
| MMP3 | AFVFPKESDTSYVS SEQ ID NO1057 | 026-039 |
| MMP3 | AFVFPKESDTSYVSL SEQ ID NO1058 | 026-040 |
| MMP3 | AFVFPKESDTSYVSLKAP SEQ ID NO1210 | 026-043 |
| MMP3 | FVFPKESDTSYV SEQ ID NO1064 | 027-038 |
| MMP3 | FVFPKESDTSYVSLK SEQ ID NO1211 | 027-041 |
| MMP3 | VFPKESDTSYVSLK SEQ ID NO1212 | 028-041 |
| MMP3 | KESDTSYVSLKAP SEQ ID NO1213 | 031-043 |
| MMP3 | TKRQDNEILIFW SEQ ID NO1214 | 074-085 |
| MMP3 | IVEFWVDGKPRVRKS SEQ ID NO1215 | 124-138 |
| MMP3 | SLKKGYTVGAEA SEQ ID NO1099 | 138-149 |
| MMP3 | SLKKGYTVGAEAS SEQ ID NO1100 | 138-150 |
| MMP3 | LKKGYTVGAEA SEQ ID NO1105 | 139-149 |
| MMP3 | LKKGYTVGAEAS SEQ ID NO1106 | 139-150 |
| MMP3 | LKKGYTVGAEASI SEQ ID NO1107 | 139-151 |
| MMP3 | LKKGYTVGAEASII SEQ ID NO1216 | 139-152 |
| MMP3 | SIILGQEQDSFGGNFEGSQS SEQ ID NO1110 | 150-169 |
| MMP3 | IILGQEQDSFGGN SEQ ID NO1192 | 151-163 |
| MMP3 | IILGQEQDSFGGNFEGSQS SEQ ID NO1112 | 151-169 |
| MMP3 | ILGQEQDSFGGNFEGSQS SEQ ID NO1115 | 152-169 |
| MMP3 | LGQEQDSFGGNFEGSQS SEQ ID NO1117 | 153-169 |
| MMP3 | QEQDSFGGNFEGSQS SEQ ID NO1217 | 155-169 |
| MMP3 | SFGGNFEGSQS SEQ ID NO1119 | 159-169 |
| MMP3 | LVGDIGNVNMWD SEQ ID NO1193 | 170-181 |
| MMP3 | FVLSPDEINT SEQ ID NO1218 | 182-191 |
| MMP3 | YLGGPFSPNVLN SEQ ID NO1124 | 193-204 |
| MMP3 | LKYEVQGEVFTKPQ SEQ ID NO1138 | 208-221 |
| MMP3 | KYEVQGEVFTKPQ SEQ ID NO1141 | 209-221 |
| MMP3 | KYEVQGEVFTKPQLWP SEQ ID NO1142 | 209-224 |
| MMP3 | YEVQGEVFTKPQ SEQ ID NO1144 | 210-221 |
| MMP3 | YEVQGEVFTKPQLWP SEQ ID NO1145 | 210-224 |
| MMP3 | EVQGEVFTKPQLWP SEQ ID NO1198 | 211-224 |
| MMP3 | VQGEVFTKPQLWP SEQ ID NO1147 | 212-224 |
| MMP3 | GEVFTKPQLWP SEQ ID NO1200 | 214-224 |
| MMP3 | EVFTKPQLWP SEQ ID NO1152 | 215-224 |
| MMP3 | SKDIGYSFTVGGSEI SEQ ID NO1219 | 86-100 |
| MMP8 | FGQTDMSR SEQ ID NO1034 | 017-024 |
| MMP8 | FGQTDMSRK SEQ ID NO1035 | 017-025 |
| MMP8 | FGQTDMSRKA SEQ ID NO1036 | 017-026 |
| MMP8 | FGQTDMSRKAF SEQ ID NO1037 | 017-027 |
| MMP8 | FGQTDMSRKAFV SEQ ID NO1220 | 017-028 |
| MMP8 | FGQTDMSRKAFVFPKESDTSYV SEQ ID NO1040 | 017-038 |
| MMP8 | MSRKAFVFPKESDTSYV SEQ ID NO1206 | 022-038 |
| MMP8 | SRKAFVFPKESDTSYV SEQ ID NO1044 | 023-038 |
| MMP8 | RKAFVFPKESDTSYV SEQ ID NO1046 | 024-038 |
| MMP8 | KAFVFPKESDT SEQ ID NO1050 | 025-035 |
| MMP8 | KAFVFPKESDTS SEQ ID NO1051 | 025-036 |
| MMP8 | KAFVFPKESDTSYV SEQ ID NO1052 | 025-038 |
| MMP8 | KAFVFPKESDTSYVS SEQ ID NO1053 | 025-039 |
| MMP8 | AFVFPKESDTSYV SEQ ID NO1056 | 026-038 |
| MMP8 | FVFPKESDTSYV SEQ ID NO1064 | 027-038 |
| MMP8 | VFPKESDTSYV SEQ ID NO1068 | 028-038 |
| MMP8 | FPKESDTSYV SEQ ID NO1221 | 029-038 |
| MMP8 | SLKAPL SEQ ID NO1222 | 039-044 |
| MMP8 | SLKAPLTKP SEQ ID NO1074 | 039-047 |
| MMP8 | SLKAPLTKPLKA SEQ ID NO1223 | 039-050 |
| MMP8 | RGYSIFSYA SEQ ID NO1224 | 065-073 |
| MMP8 | FSYATKRQDNEILI SEQ ID NO1225 | 070-083 |
| MMP8 | SYATKRQDNEILI SEQ ID NO1083 | 071-083 |
| MMP8 | YATKRQDNEILI SEQ ID NO1085 | 072-083 |
| MMP8 | ATKRQDNEILI SEQ ID NO1226 | 073-083 |
| MMP8 | TKRQDNEILI SEQ ID NO1087 | 074-083 |
| MMP8 | TKRQDNEILIF SEQ ID NO1088 | 074-084 |
| MMP8 | FWSKDIGYS SEQ ID NO1227 | 084-092 |
| MMP8 | FWSKDIGYSFT SEQ ID NO1228 | 084-094 |
| MMP8 | FWSKDIGYSFTV SEQ ID NO1229 | 084-095 |
| MMP8 | WSKDIGYSFTV SEQ ID NO1230 | 085-095 |
| MMP8 | KSLKKGYTVGAEA SEQ ID NO1231 | 137-149 |
| MMP8 | SLKKGYTVGAEA SEQ ID NO1099 | 138-149 |
| MMP8 | LKKGYTV SEQ ID NO1101 | 139-145 |
| MMP8 | LKKGYTVGAEA SEQ ID NO1105 | 139-149 |
| MMP8 | LKKGYTVGAEAS SEQ ID NO1106 | 139-150 |
| MMP8 | KKGYTVGAEA SEQ ID NO1232 | 140-149 |
| MMP8 | GAEASIILGQE SEQ ID NO1233 | 146-156 |
| MMP8 | GAEASIILGQEQD SEQ ID NO1234 | 146-158 |
| MMP8 | SIILGQEQD SEQ ID NO1235 | 150-158 |
| MMP8 | SIILGQEQDSFGGNFEGSQ SEQ ID NO1109 | 150-168 |
| MMP8 | SIILGQEQDSFGGNFEGSQS SEQ ID NO1110 | 150-169 |
| MMP8 | IILGQEQDSFGGN SEQ ID NO1192 | 151-163 |
| MMP8 | IILGQEQDSFGGNFEGSQ SEQ ID NO1236 | 151-168 |
| MMP8 | IILGQEQDSFGGNFEGSQS SEQ ID NO1112 | 151-169 |
| MMP8 | ILGQEQDSFGGN SEQ ID NO1113 | 152-163 |
| MMP8 | ILGQEQDSFGGNFEGS SEQ ID NO1237 | 152-167 |
| MMP8 | ILGQEQDSFGGNFEGSQ SEQ ID NO1114 | 152-168 |
| MMP8 | ILGQEQDSFGGNFEGSQS SEQ ID NO1115 | 152-169 |
| MMP8 | LGQEQDSFGGN SEQ ID NO1238 | 153-163 |
| MMP8 | LGQEQDSFGGNFEGS SEQ ID NO1239 | 153-167 |
| MMP8 | LGQEQDSFGGNFEGSQ SEQ ID NO1116 | 153-168 |
| MMP8 | LGQEQDSFGGNFEGSQS SEQ ID NO1117 | 153-169 |
| MMP8 | LGQEQDSFGGNFEGSQSL SEQ ID NO1240 | 153-170 |
| MMP8 | LGQEQDSFGGNFEGSQSLV SEQ ID NO1241 | 153-171 |
| MMP8 | QDSFGGNFEGSQS SEQ ID NO1242 | 157-169 |
| MMP8 | SFGGNFEGSQ SEQ ID NO1243 | 159-168 |
| MMP8 | SFGGNFEGSQS SEQ ID NO1119 | 159-169 |
| MMP8 | SFGGNFEGSQSLVSEQ ID NO1244 | 159-171 |
| MMP8 | LVGDIGNVNMWSEQ ID NO1245 | 170-180 |
| MMP8 | INTIYLGGPFSPNSEQ ID NO1246 | 189-201 |
| MMP8 | TIYLGGPFSPNSEQ ID NO1247 | 191-201 |
| MMP8 | IYLGGPFSPN SEQ ID NO1194 | 192-201 |
| MMP8 | YLGGPFSPNVSEQ ID NO1248 | 193-202 |
| MMP8 | YLGGPFSPNVLN SEQ ID NO1124 | 193-204 |
| MMP8 | LGGPFSPNVLN SEQ ID NO1196 | 194-204 |
| MMP8 | VLNWRASEQ ID NO1249 | 202-207 |
| MMP8 | VLNWRALSEQ ID NO1250 | 202-208 |
| MMP8 | VLNWRALK SEQ ID NO1251 | 202-209 |
| MMP8 | LNWRAL SEQ ID NO1128 | 203-208 |
| MMP8 | LNWRALK SEQ ID NO1129 | 203-209 |
| MMP8 | LNWRALKYEV SEQ ID NO1252 | 203-212 |
| MMP8 | NWRAL SEQ ID NO1253 | 204-208 |
| MMP8 | NWRALKY SEQ ID NO1254 | 204-210 |
| MMP8 | NWRALKYEV SEQ ID NO1255 | 204-212 |
| MMP8 | NWRALKYEVQ SEQ ID NO1256 | 204-213 |
| MMP8 | WRALKYE SEQ ID NO1130 | 205-211 |
| MMP8 | WRALKYEVQ SEQ ID NO1257 | 205-213 |
| MMP8 | WRALKYEVQGE SEQ ID NO1132 | 205-215 |
| MMP8 | RALKYEV SEQ ID NO1258 | 206-212 |
| MMP8 | RALKYEVQ SEQ ID NO1259 | 206-213 |
| MMP8 | RALKYEVQGE SEQ ID NO1260 | 206-215 |
| MMP8 | ALKYEV SEQ ID NO1133 | 207-212 |
| MMP8 | ALKYEVQGEVFTKPQ SEQ ID NO1261 | 207-221 |
| MMP8 | LKYEVQGE SEQ ID NO1136 | 208-215 |
| MMP8 | LKYEVQGEVFTKPQ SEQ ID NO1138 | 208-221 |
| MMP8 | KYEVQGEVFTKPQ SEQ ID NO1141 | 209-221 |
| MMP8 | KYEVQGEVFTKPQLWP SEQ ID NO1142 | 209-224 |
| MMP8 | YEVQGEVFTKPQ SEQ ID NO1144 | 210-221 |
| MMP8 | YEVQGEVFTKPQLWP SEQ ID NO1145 | 210-224 |
| MMP8 | EVQGEVFTKPQ SEQ ID NO1262 | 211-221 |
| MMP8 | EVQGEVFTKPQLWP SEQ ID NO1198 | 211-224 |
| MMP8 | VQGEVFTKPQ SEQ ID NO1146 | 212-221 |
| MMP8 | VQGEVFTKPQLWP SEQ ID NO1147 | 212-224 |
| MMP8 | QGEVFTKPQ SEQ ID NO1148 | 213-221 |
| MMP8 | QGEVFTKPQL SEQ ID NO1263 | 213-222 |
| MMP8 | QGEVFTKPQLWP SEQ ID NO1264 | 213-224 |
| MMP8 | GEVFTKPQ SEQ ID NO1150 | 214-221 |
| MMP8 | GEVFTKPQLWP SEQ ID NO1200 | 214-224 |
| MMP8 | VFTKPQ SEQ ID NO1153 | 216-221 |
| MMP8 | VFTKPQLWP SEQ ID NO1203 | 216-224 |
| MMP8 | FTKPQLWP SEQ ID NO1155 | 217-224 |
| MMP8 | TKPQLWP SEQ ID NO1156 | 218-224 |
| ADAMTS-1 | ESDTSYVSLK SEQ ID NO1265 | 032-041 |
| ADAMTS-1 | QEQDSFGGNFEGSQ SEQ ID NO1266 | 155-168 |
| ADAMTS-1 | QEQDSFGGNFEGSQSLVG SEQ ID NO1267 | 155-172 |
| ADAMTS-1 | GNFEGSQSLVG SEQ ID NO1268 | 162-172 |
| ADAMTS-1 | YEVQGEVFT SEQ ID NO1269 | 210-218 |
| ADAMTS-1 | YEVQGEVFTKPQ SEQ ID NO1144 | 210-221 |
| ADAMTS-1 | GEVFTKPQ SEQ ID NO1150 | 214-221 |
| ADAMTS-8 | VFPKESDTSYVS SEQ ID NO1069 | 028-039 |
| ADAMTS-8 | QEQDSFGGNFEGSQSLVG SEQ ID NO1267 | 155-172 |
| ADAMTS-8 | EINTIYL SEQ ID NO1270 | 188-194 |
| ADAMTS-8 | KYEVQ SEQ ID NO1271 | 209-213 |
| ADAMTS-8 | KYEVQGE SEQ ID N01140 | 209-215 |
| Cat K | FGQTDMSR SEQ ID NO1034 | 017-024 |
| Cat K | AFVFPK SEQ ID NO1033 | 026-031 |
| Cat K | FVFPK SEQ ID NO1059 | 027-031 |
| Cat K | ESDTSYVSLK SEQ ID NO1265 | 032-041 |
| Cat K | ESDTSYVSLKAPLT SEQ ID NO1272 | 032-045 |
| Cat K | SDTSYVSLK SEQ ID NO1273 | 033-041 |
| Cat K | DTSYVSLK SEQ ID NO1274 | 034-041 |
| Cat K | STRGYS SEQ ID NO1080 | 063-068 |
| Cat K | IFWSKDIG SEQ ID NO1275 | 083-090 |
| Cat K | KGYTVGAE SEQ ID NO1276 | 141-148 |
| Cat K | AEASIILGQEQDSFG SEQ ID NO1277 | 147-161 |
| Cat K | LGQEQDSFG SEQ ID NO1278 | 153-161 |
| Cat K | LGQEQDSFGGNFE SEQ ID NO1279 | 153-165 |
| Cat K | GQEQDSFG SEQ ID NO1280 | 154-161 |
| Cat K | GQEQDSFGGNFE SEQ ID NO1281 | 154-165 |
| Cat K | GQEQDSFGGNFEGSQ SEQ ID NO1282 | 154-168 |
| Cat K | GQEQDSFGGNFEGSQS SEQ ID NO1118 | 154-169 |
| Cat K | QEQDSFGGN SEQ ID NO1283 | 155-163 |
| Cat K | QEQDSFGGNFE SEQ ID NO1284 | 155-165 |
| Cat K | QEQDSFGGNFEG SEQ ID NO1285 | 155-166 |
| Cat K | QEQDSFGGNFEGS SEQ ID NO1286 | 155-167 |
| Cat K | QEQDSFGGNFEGSQ SEQ ID NO1266 | 155-168 |
| Cat K | QEQDSFGGNFEGSQS SEQ ID NO1217 | 155-169 |
| Cat K | GNFEGSQSLV SEQ ID NO1287 | 162-171 |
| Cat K | GNFEGSQSLVG SEQ ID NO1268 | 162-172 |
| Cat K | GNFEGSQSLVGDIG SEQ ID NO1288 | 162-175 |
| Cat K | GSQSLVGDIG SEQ ID NO1289 | 166-175 |
| Cat K | GSQSLVGDIGNVN SEQ ID NO1290 | 166-178 |
| Cat K | DFVLSPDEIN SEQ ID NO1291 | 181-190 |
| Cat K | FVLSPDEINT SEQ ID NO1218 | 182-191 |
| Cat K | VLSPDEINT SEQ ID NO1291 | 183-191 |
| Cat K | GPFSPNVLN SEQ ID NO1292 | 196-204 |
| Cat K | SPNVLNWR SEQ ID NO1293 | 199-206 |
| Cat K | KYEVQG SEQ ID NO1294 | 209-214 |
| Cat K | YEVQGEVFT SEQ ID NO1269 | 210-218 |
| Cat K | YEVQGEVFTKPQ SEQ ID NO1144 | 210-221 |
| Cat K | VQGEVFTKPQ SEQ ID NO1146 | 212-221 |
| Cat K | GEVFTKPQ SEQ ID NO1150 | 214-221 |
| Cat K | EVFTKPQ SEQ ID NO1151 | 215-221 |
| Cat S | FGQTDMSR SEQ ID NO1034 | 017-024 |
| Cat S | AFVFPKESDTSYVS SEQ ID NO1057 | 026-039 |
| Cat S | FVFPKESDTSYVS SEQ ID NO1065 | 027-039 |
| Cat S | VFPKESDTSYVS SEQ ID NO1069 | 028-039 |
| Cat S | FPKESDTSYVS SEQ ID NO1071 | 029-039 |
| Cat S | ESDTSYVSLK SEQ ID NO1265 | 032-041 |
| Cat S | TSWESASGIVE SEQ ID NO1295 | 116-126 |
| Cat S | KGYTVG SEQ ID NO1296 | 141-146 |
| Cat S | QEQDSFGGNFE SEQ ID NO1284 | 155-165 |
| Cat S | QEQDSFGGNFEG SEQ ID NO1285 | 155-166 |
| Cat S | QEQDSFGGNFEGSQ SEQ ID NO1266 | 155-168 |
| Cat S | QEQDSFGGNFEGSQS SEQ ID NO1217 | 155-169 |
| Cat S | QEQDSFGGNFEGSQSLV SEQ ID NO1297 | 155-171 |
| Cat S | QEQDSFGGNFEGSQSLVG SEQ ID NO1267 | 155-172 |
| Cat S | SFGGNFEGSQSLVG SEQ ID NO1298 | 159-172 |
| Cat S | GNFEGSQSLVG SEQ ID NO1268 | 162-172 |
| Cat S | GNFEGSQSLVGDIG SEQ ID NO1288 | 162-175 |
| Cat S | SPDEINTIYL SEQ ID NO1299 | 185-194 |
| Cat S | SPDEINTIYLG SEQ ID NO1300 | 185-195 |
| Cat S | LGGPFSPNVLN SEQ ID NO1196 | 194-204 |
| Cat S | GGPFSPNVLN SEQ ID NO1301 | 195-204 |
| Cat S | GPFSPNVLN SEQ ID NO1292 | 196-204 |
| Cat S | ALKYE SEQ ID NO1302 | 207-211 |
| Cat S | ALKYEVQ SEQ ID NO1303 | 207-213 |
| Cat S | YEVQGEVF SEQ ID NO1304 | 210-217 |
| Cat S | YEVQGEVFT SEQ ID NO1269 | 210-218 |
| Cat S | YEVQGEVFTKPQ SEQ ID NO1144 | 210-221 |
| Cat S | YEVQGEVFTKPQLWP SEQ ID NO1145 | 210-224 |
| Cat S | VQGEVFTKPQLWP SEQ ID NO1147 | 212-224 |
| Cat S | GEVFTKPQ SEQ ID NO1150 | 214-221 |
| Cat S | GEVFTKPQLWP SEQ ID NO1200 | 214-224 |
| Cat S | EVFTKPQLWP SEQ ID NO1152 | 215-224 |
| Cat S | TKPQLWP SEQ ID NO1156 | 218-224 |
| Cat S | KPQLWP SEQ ID NO1157 | 219-224 |

| | | |
|---|---|---|
| ***numbers in the sequence of CRP** | | |

Accordingly, in a method of the invention, said peptide fragments preferably comprise a neo-epitope formed by cleavage of CRP by a protease at a site marked by the sign * in any one of the above partial sequences of CRP in Table 20 or at either end of any partial sequence of CRP in Table 21.

The immunological binding partner may be one specifically reactive with a C-terminal or N-terminal neo-epitope formed by cleavage of CRP.

Suitable immunological binding partners may therefore be specifically reactive with any of the following sequences at the N terminal of a peptide:

**Table 22. N-terminal sequences of protease generated peptide fragments of CRP.**

| CRP | | |
|---|---|---|
| AFVFPK SEQ ID NO1033 | SFGGNF SEQ ID NO1305 | FGQTDM SEQ ID NO1306 |
| VSLKAP SEQ ID NO1172 | KAFVFP SEQ ID NO1167 | EVFTKP SEQ ID NO1201 |
| TDMSRK SEQ ID NO1307 | MSRKAF SEQ ID NO1308 | SRKAFV SEQ ID NO1309 |
| VFPKES SEQ ID NO1310 | FPKESD SEQ ID NO1311 | KESDTS SEQ ID NO1312 |
| LSSTRG SEQ ID NO1313 | SSTRGY SEQ ID NO1314 | STRGYS SEQ ID NO1080 |
| KRQDNE SEQ ID NO1315 | WSKDIG SEQ ID NO1316 | SKDIGY SEQ ID NO1317 |
| SIILGQ SEQ ID NO1318 | IILGQE SEQ ID NO1319 | ILGQEQ SEQ ID NO1320 |
| IYLGGP SEQ ID NO1321 | YLGGPF SEQ ID NO1322 | LGGPFS SEQ ID NO1323 |
| ALKYEV SEQ ID NO1133 | KYEVQG SEQ ID NO1294 | VQGEVF SEQ ID NO1324 |
| KPQLWP SEQ ID NO1157 | YTELSS SEQ ID NO1325 | ILIFWS SEQ ID NO1326 |
| LVGDIG SEQ ID NO1327 | QEQDSF SEQ ID NO1328 | RGYSIF SEQ ID NO1329 |
| GAEASI SEQ ID NO1330 | QDSFGG SEQ ID NO1331 | TIYLGG SEQ ID NO1332 |
| EINTIY SEQ ID NO1333 | DTSYVS SEQ ID NO1334 | AEASII SEQ ID NO1335 |
| TSWESA SEQ ID NO1336 | SPDEIN SEQ ID NO1337 | GGPFSP SEQ ID NO1338 |
| YEVQGE SEQ ID NO1339 | FVLSPD SEQ ID NO1340 | LKKGYT SEQ ID NO1341 |
| RKAFVF SEQ ID NO1342 | IVEFWV SEQ ID NO1343 | ESDTSY SEQ ID NO1344 |
| TKPQLW SEQ ID NO1345 | EVQGEV SEQ ID NO1346 | FVFPK SEQ ID NO1059 |
| SDTSYV SEQ ID NO1347 | SLKAPL SEQ ID NO1222 | LKAPLT SEQ ID NO1173 |
| IFSYAT SEQ ID NO1348 | SYATKR SEQ ID NO1349 | YATKRQ SEQ ID NO1350 |
| EFWVDG SEQ ID NO1351 | WVDGKP SEQ ID NO1352 | VDGKPR SEQ ID NO1353 |
| LGQEQD SEQ ID NO1354 | GQEQDS SEQ ID NO1355 | QSLVGD SEQ ID NO1356 |
| SPNVLN SEQ ID NO1357 | LNWRA SEQ ID NO1127 | LNWRAL SEQ ID NO1128 |
| QGEVFT SEQ ID NO1358 | GEVFTK SEQ ID NO1359 | VFTKPQ SEQ ID NO1153 |
| IFWSKD SEQ ID NO1181 | VRKSLK SEQ ID NO1205 | KKGYTV SEQ ID NO1360 |
| FSYATK SEQ ID NO1361 | ATKRQD SEQ ID NO1362 | FWSKDI SEQ ID NO1363 |
| VLNWRA SEQ ID NO1249 | NWRAL SEQ ID NO1253 | NWRALK SEQ ID NO1364 |
| GSQSLV SEQ ID NO1365 | DFVLSP SEQ ID NO1366 | VLSPDE SEQ ID NO1367 |
| LKYEVQ SEQ ID NO1134 | TKRQDN SEQ ID NO1368 | KGYTVG SEQ ID NO1296 |
| GNFEGS SEQ ID NO1369 | SLKKGY SEQ ID NO1370 | KSLKKG SEQ ID NO1371 |
| FVFPKE SEQ ID NO1060 | INTIYL SEQ ID NO1372 | RALKYE SEQ ID NO1373 |
| FYTELS SEQ ID NO1374 | WRALKY SEQ ID NO1375 | GPFSPN SEQ ID NO1376 |

or with any of the following sequences at the C-terminal of a peptide:

**Table 23. C-terminal sequences of protease generated peptide fragments of CRP.**

| **CRP** | | |
|---|---|---|
| AFVFPK SEQ ID NO1033 | KPQLWP SEQ ID NO1157 | PDEINT SEQ ID NO1377 |
| SPDEIN SEQ ID NO1337 | DSFGGN SEQ ID NO1378 | VFTKPQ SEQ ID NO1153 |
| KESDTS SEQ ID NO1312 | SDTSYV SEQ ID NO1347 | DTSYVS SEQ ID NO1334 |
| LTKPLK SEQ ID NO1379 | STRGYS SEQ ID NO1080 | YATKRQ SEQ ID NO1350 |
| KDIGYS SEQ ID NO1380 | DGKPRV SEQ ID NO1381 | VDGKPR SEQ ID NO1353 |
| GAEASI SEQ ID NO1330 | QGEVFT SEQ ID NO1358 | NFEGSQ SEQ ID NO1382 |
| SPNVLN SEQ ID NO1357 | GPFSPN SEQ ID NO1376 | RALKYE SEQ ID NO1373 |
| ALKYEV SEQ ID NO1133 | YEVQGE SEQ ID NO1339 | LKYEVQ SEQ ID NO1134 |
| KAFVFP SEQ ID NO1167 | VSLKAP SEQ ID NO1172 | LKAPLT SEQ IDNO 1173 |
| LKKGYT SEQ ID NO1341 | LGQEQD SEQ ID NO1354 | NVNMWD SEQ ID NO1383 |
| PRVRKS SEQ ID NO1384 | TVGSEI SEQ ID NO1385 | SRKAFV SEQ ID NO1309 |
| GYSFTV SEQ ID NO1386 | IILGQE SEQ ID NO1319 | EGSQSL SEQ ID NO1387 |
| INTIYL SEQ ID NO1372 | WSKDIG SEQ ID NO1316 | EQDSFG SEQ ID NO1388 |
| NVLNWR SEQ ID NO1389 | ASGIVE SEQ ID NO1390 | NTIYLG SEQ ID NO1391 |
| VGAEAS SEQ ID NO1392 | APLTKP SEQ ID NO1393 | FVFPKE SEQ ID NO1060 |
| QTDMSR SEQ ID NO1394 | TDMSRK SEQ ID NO1307 | MSRKAF SEQ ID NO1308 |
| PKESDT SEQ ID NO1395 | TSYVSL SEQ ID NO1396 | ESDTSY SEQ ID NO1344 |
| DNEILI SEQ ID NO1397 | QDNEIL SEQ ID NO1398 | NEILIF SEQ ID NO1399 |
| YTVGAE SEQ ID NO1400 | TVGAEA SEQ ID NO1401 | PFSPNV SEQ ID NO1402 |
| FEGSQS SEQ ID NO1403 | GNFEGS SEQ ID NO1369 | DIGNVN SEQ ID NO1404 |
| LNWRA SEQ ID NO1127 | LNWRAL SEQ ID NO1128 | NWRALK SEQ ID NO1364 |
| KYEVQG SEQ ID NO1294 | EVFTKP SEQ ID NO1201 | VFTKPQ SEQ ID NO1153 |
| KRQDNE SEQ ID NO1315 | RQDNEI SEQ ID NO1405 | IFWSKD SEQ ID NO1181 |
| FTKPQL SEQ ID NO1406 | VRKSLK SEQ ID NO1205 | SYVSLK SEQ ID NO1407 |
| SLKAPL SEQ ID NO1222 | TKPLKA SEQ ID NO1408 | SIFSYA SEQ ID NO1409 |
| GSQSLV SEQ ID NO1365 | GNVNMW SEQ ID NO1410 | SQSLVG SEQ ID NO1411 |
| FGGNFE SEQ ID NO1412 | GGNFEG SEQ ID NO1413 | LVGDIG SEQ ID NO1327 |
| VQGEVF SEQ ID NO1324 | GKPRVR SEQ ID NO1414 | FWSKDI 1363 SEQ ID NO |
| DMSRKA SEQ ID NO1415 | EILIFW SEQ ID NO1416 | KKGYTV SEQ ID NO1360 |
| FPKESD SEQ ID NO1311 | IGYSFT SEQ ID NO1417 | |

### Elastin

Several candidate proteases may be responsible for the digestion of elastin in fibrotic tissue We have through a range of in vitro cleavages of pure native proteins determined that the enzymes listed in the following table cleaved elastin at least at the cleavage sites at each end of the following sequences or at the cleavage sites marked '.' or where no '.' is shown, at the ends of the sequences:

**Table 24: Elastin fragments generated by specific proteases.**

| **Proteas e** | **Sequence between cleavage sites** | **Nos*** |
|---|---|---|
| MMP9+12 | GVPGAIPGGVPG SEQ ID NO1418 | 028-039 |
| MMP9+12 | AIPGGVPGGVFYPGAGLG SEQ ID NO1419 | 032-049 |
| MMP9+12 | AIPGGVPGGVFYPGAGLGA SEQ ID NO1420 | 032-050 |
| MMP9+12 | GVPGGVFYPGAGLGA SEQ ID NO1421 | 036-050 |
| MMP9+12 | GVPGGVFYPGAGLGALG SEQ ID NO1422 | 036-052 |
| MMP9+12 | VPGGVFYPGAGLGALGG SEQ ID NO1423 | 037-053 |
| MMP9+12 | GVFYPGAGLGALGGGALGPGG SEQ ID NO1424 | 040-060 |
| MMP9+12 | VFYPGAGLG SEQ ID NO1425 | 041-049 |
| MMP9+12 | VFYPGAGLGA SEQ ID NO1426 | 041-050 |
| MMP9+12 | VFYPGAGLGAL SEQ ID NO1427 | 041-051 |
| MMP9+12 | VFYPGAGLGALG SEQ ID NO1428 | 041-052 |
| MMP9+12 | VFYPGAGLGALGG SEQ ID NO1429 | 041-053 |
| MMP9+12 | VFYPGAGLGALGGG SEQ ID NO1430 | 041-054 |
| MMP9+12 | VFYPGAGLGALGGGAL SEQ ID NO1431 | 041-056 |
| MMP9+12 | VFYPGAGLGALGGGALG SEQ ID NO1432 | 041-057 |
| MMP9+12 | VFYPGAGLGALGGGALGPG SEQ ID NO1433 | 041-059 |
| MMP9+12 | VFYPGAGLGALGGGALGPGG SEQ ID NO1434 | 041-060 |
| MMP9+12 | VFYPGAGLGALGGGALGPGGKPLKPVPGG SEQ ID NO1435 | 041-069 |
| MMP9+12 | LGALGGGALGPGGKPLKPVPGG SEQ ID NO1436 | 048-069 |
| MMP9+12 | ALGGGALGPGGKPLKPVPGG SEQ ID NO1437 | 050-069 |
| MMP9+12 | LGGGALGPGGKPLKPVPG SEQ ID NO1438 | 051-068 |
| MMP9+12 | LGGGALGPGGKPLKPVPGG SEQ ID NO1439 | 051-069 |
| MMP9+12 | GGALGPGGKPLKPVPGG SEQ ID NO1440 | 053-069 |
| MMP9+12 | LGPGGKPLKPVPGG SEQ ID NO1441 | 056-069 |
| MMP9+12 | GPGGKPLKPVPGG SEQ ID NO1442 | 057-069 |
| MMP9+12 | PGGKPLKPVPGG SEQ ID NO1443 | 058-069 |
| MMP9+12 | GKPLKPVPGG SEQ ID NO1444 | 060-069 |
| MMP9+12 | PLKPVPGG SEQ ID NO1445 | 062-069 |
| MMP9+12 | LKPVPGG SEQ ID NO SEQ ID NO1446 | 063-069 |
| MMP9+12 | GLAGAGLGAGLGAFP SEQ ID NO1447 | 069-083 |
| MMP9+12 | GLAGAGLGAGLGAFPA SEQ ID NO1448 | 069-084 |
| MMP9+12 | LAGAGLGAGLG SEQ ID NO1449 | 070-080 |
| MMP9+12 | LAGAGLGAGLGAFP SEQ ID NO1450 | 070-083 |
| MMP9+12 | LAGAGLGAGLGAFPA SEQ ID NO1451 | 070-084 |
| MMP9+12 | LAGAGLGAGLGAFPAVT SEQ ID NO1452 | 070-086 |
| MMP9+12 | LAGAGLGAGLGAFPAVTFPG SEQ ID NO1453 | 070-089 |
| MMP9+12 | LAGAGLGAGLGAFPAVTFPGA SEQ ID NO1454 | 070-090 |
| MMP9+12 | LAGAGLGAGLGAFPAVTFPGALVPGG SEQ ID NO1455 | 070-095 |
| MMP9+12 | LAGAGLGAGLGAFPAVTFPGALVPGGVA SEQ ID NO1456 | 070-097 |
| MMP9+12 | LAGAGLGAGLGAFPAVTFPGALVPGGVADAAAA SEQ ID NO1457 | 070-102 |
| MMP9+12 | AGAGLGAGLGAFPAVTFPGALVPGG SEQ ID NO1458 | 071-095 |
| MMP9+12 | GAGLGAGLGAFPA SEQ ID NO1459 | 072-084 |
| MMP9+12 | GAGLGAGLGAFPAVTFPGA SEQ ID NO1460 | 072-090 |
| MMP9+12 | AGLGAGLGAFPA SEQ ID NO1461 | 073-084 |
| MMP9+12 | GLGAGLGAFPA SEQ ID NO1462 | 074-084 |
| MMP9+12 | LGAGLGAFPA SEQ ID NO1463 | 075-084 |
| MMP9+12 | LGAGLGAFPAVTFPGA SEQ ID NO1464 | 075-090 |
| MMP9+12 | LGAGLGAFPAVTFPGALVPGG SEQ ID NO1465 | 075-095 |
| MMP9+12 | LGAGLGAFPAVTFPGALVPGGVADAAAA SEQ ID NO1466 | 075-102 |
| MMP9+12 | AGLGAFPAVTFPG SEQ ID NO1467 | 077-089 |
| MMP9+12 | LGAFPAVTFPGA SEQ ID NO1468 | 079-090 |
| MMP9+12 | LGAFPAVTFPGALVPGGVA SEQ ID NO1469 | 079-097 |
| MMP9+12 | LGAFPAVTFPGALVPGGVADAAAA SEQ ID NO1470 | 079-102 |
| MMP9+12 | AFPAVTFPGALVPGG SEQ ID NO1471 | 081-095 |
| MMP9+12 | AVTFPGALVPGG SEQ ID NO1472 | 084-095 |
| MMP9+12 | AVTFPGALVPGGVADAAAA SEQ ID NO1473 | 084-102 |
| MMP9+12 | VTFPGALVPGG SEQ ID NO1474 | 085-095 |
| MMP9+12 | VTFPGALVPGGVADAAAA SEQ ID NO1475 | 085-102 |
| MMP9+12 | LVPGGVADAAAA SEQ ID NO1476 | 091-102 |
| MMP9+12 | LVPGGVADAAAAYK SEQ ID NO1477 | 091-104 |
| MMP9+12 | VADAAAAYK SEQ ID NO1478 | 096-104 |
| MMP9+12 | KAAKAGA SEQ ID NO1479 | 104-110 |
| MMP9+12 | LGVSAGAVVPQPGA SEQ ID NO1480 | 121-134 |
| MMP9+12 | VPGVGLPGVYPGGVLPGAR SEQ ID NO1481 | 141-159 |
| MMP9+12 | PGVGLPGVYPGGVLPGAR SEQ ID NO1482 | 142-159 |
| MMP9+12 | GLPGVYPGGVLPGAR SEQ ID NO1483 | 145-159 |
| MMP9+12 | PGVYPGGVLPGAR SEQ ID NO1484 | 147-159 |
| MMP9+12 | ARFPGVG SEQ ID NO1485 | 158-164 |
| MMP9+12 | ARFPGVGVLPG SEQ ID NO1486 | 158-168 |
| MMP9+12 | RFPGVGVLPGVPTGAG SEQ ID NO1487 | 159-174 |
| MMP9+12 | FPGVGVLPGVPTG SEQ ID NO1488 | 160-172 |
| MMP9+12 | FPGVGVLPGVPTGA SEQ ID NO1489 | 160-173 |
| MMP9+12 | FPGVGVLPGVPTGAGV SEQ ID NO1490 | 160-175 |
| MMP9+12 | FPGVGVLPGVPTGAGVKPK SEQ ID NO1491 | 160-178 |
| MMP9+12 | KPKAPGV SEQ ID NO1492 | 176-182 |
| MMP9+12 | PKAPGV SEQ ID NO1493 | 177-182 |
| MMP9+12 | GAFAGIPGVGPFG SEQ ID NO1494 | 184-196 |
| MMP9+12 | VGPFGGPQPGVPLGYP SEQ ID NO1495 | 192-207 |
| MMP9+12 | GPQPGVPLGYP SEQ ID NO1496 | 197-207 |
| MMP9+12 | PQPGVPLGYP SEQ ID NO1497 | 198-207 |
| MMP9+12 | PGVPLGYP SEQ ID NO1498 | 200-207 |
| MMP9+12 | GYPIKAPK SEQ ID NO1499 | 205-212 |
| MMP9+12 | PKLPGGY SEQ ID NO1500 | 211-217 |
| MMP9+12 | YTTGKLPYGYGPG SEQ ID NO1501 | 221-233 |
| MMP9+12 | YTTGKLPYGYGPGGVAGAAGK SEQ ID NO1502 | 221-241 |
| MMP9+12 | TTGKLPYGYG SEQ ID NO1503 | 222-231 |
| MMP9+12 | TTGKLPYGYGPGGVAGAAGK SEQ ID NO1504 | 222-241 |
| MMP9+12 | LPYGYGPGGVAGAAGK SEQ ID NO1505 | 226-241 |
| MMP9+12 | GYGPGGVAGAAGK SEQ ID NO1506 | 229-241 |
| MMP9+12 | YGPGGVAGAAGK SEQ ID NO1507 | 230-241 |
| MMP9+12 | AGYPTGTGVGPQAAAAAAAK SEQ ID NO1508 | 242-261 |
| MMP9+12 | TGVGPQAAAAAAAK SEQ ID NO1509 | 248-261 |
| MMP9+12 | PQAAAAAAAK SEQ ID NO1510 | 252-261 |
| MMP9+12 | FGAGAAGVLPGVGGAGVPGVPGAIPGIGG SEQ ID NO1511 | 266-294 |
| MMP9+12 | FGAGAAGVLPGVGGAGVPGVPGAIPGIGGIAGVGTPAA SEQ ID NO1512 | 266-303 |
| MMP9+12 | GVLPGVGGAGVPGVPG SEQ ID NO1513 | 272-287 |
| MMP9+12 | VLPGVGGAGVPGVPGAIPGIGG SEQ ID NO1514 | 273-294 |
| MMP9+12 | VLPGVGGAGVPGVPGAIPGIGGIAGVGTPA SEQ ID NO1515 | 273-302 |
| MMP9+12 | VLPGVGGAGVPGVPGAIPGIGGIAGVGTPAA SEQ ID NO1516 | 273-303 |
| MMP9+12 | GAGVPGVPGAIPG SEQ ID NO1517 | 279-291 |
| MMP9+12 | GAGVPGVPGAIPGIGGIAGVG SEQ ID NO1518 | 279-299 |
| MMP9+12 | AGVPGVPGAIPGIG SEQ ID NO1519 | 280-293 |
| MMP9+12 | AGVPGVPGAIPGIGG SEQ ID NO1520 | 280-294 |
| MMP9+12 | AGVPGVPGAIPGIGGIAG SEQ ID NO1521 | 280-297 |
| MMP9+12 | AGVPGVPGAIPGIGGIAGVGTPA SEQ ID NO1522 | 280-302 |
| MMP9+12 | GVPGVPGAIPGIGG SEQ ID NO1523 | 281-294 |
| MMP9+12 | GVPGVPGAIPGIGGIA SEQ ID NO1524 | 281-296 |
| MMP9+12 | GVPGVPGAIPGIGGIAGVG SEQ ID NO1525 | 281-299 |
| MMP9+12 | VPGVPGAIPGIGG SEQ ID NO1526 | 282-294 |
| MMP9+12 | GVPGAIPGIGGIAGVGTPA SEQ ID NO1527 | 284-302 |
| MMP9+12 | VPGAIPGIGGIAGVG SEQ ID NO1528 | 285-299 |
| MMP9+12 | VPGAIPGIGGIAGVGTPA SEQ ID NO1529 | 285-302 |
| MMP9+12 | VPGAIPGIGGIAGVGTPAAA SEQ ID NO1530 | 285-304 |
| MMP9+12 | VPGAIPGIGGIAGVGTPAAAAAAAAAAK SEQ ID NO1531 | 285-312 |
| MMP9+12 | AIPGIGGIAGVG SEQ ID NO1532 | 288-299 |
| MMP9+12 | AIPGIGGIAGVGTPA SEQ ID NO1533 | 288-302 |
| MMP9+12 | AIPGIGGIAGVGTPAA SEQ ID NO1534 | 288-303 |
| MMP9+12 | AIPGIGGIAGVGTPAAA SEQ ID NO1535 | 288-304 |
| MMP9+12 | AIPGIGGIAGVGTPAAAAAA SEQ ID NO1536 | 288-307 |
| MMP9+12 | AIPGIGGIAGVGTPAAAAAAAAAAK SEQ ID NO1537 | 288-312 |
| MMP9+12 | IPGIGGIAGVGTPAAA SEQ ID NO1538 | 289-304 |
| MMP9+12 | IGGIAGVGTPAAAA SEQ ID NO1539 | 292-305 |
| MMP9+12 | GIAGVGTPAAAA SEQ ID NO1540 | 294-305 |
| MMP9+12 | GIAGVGTPAAAAAAAA SEQ ID NO1541 | 294-309 |
| MMP9+12 | GIAGVGTPAAAAAAAAAAK SEQ ID NO1542 | 294-312 |
| MMP9+12 | IAGVGTPAAAAAAAA SEQ ID NO1543 | 295-309 |
| MMP9+12 | IAGVGTPAAAAAAAAA SEQ ID NO1544 | 295-310 |
| MMP9+12 | IAGVGTPAAAAAAAAAAK SEQ ID NO1545 | 295-312 |
| MMP9+12 | TPAAAAAAAAAAK SEQ ID NO1546 | 300-312 |
| MMP9+12 | PAAAAAAAAAAK SEQ ID NO1547 | 301-312 |
| MMP9+12 | AAAAAAAAAAK SEQ ID NO1548 | 302-312 |
| MMP9+12 | AAAAAAAAAK SEQ ID NO1549 | 303-312 |
| MMP9+12 | AAAAAAAAK SEQ ID NO1550 | 304-312 |
| MMP9+12 | AAAAAAAAKA SEQ ID NO1551 | 304-313 |
| MMP9+12 | AAAAAAAK SEQ ID NO1552 | 305-312 |
| MMP9+12 | LVPGGPGFGPGVVGVPGA SEQ ID NO1553 | 322-339 |
| MMP9+12 | GPGFGPGWGVPG SEQ ID NO1554 | 326-338 |
| MMP9+12 | GPGFGPGVVGVPGAGVPGVG SEQ ID NO1555 | 326-345 |
| MMP9+12 | GPGFGPGVVGVPGAGVPGVGVPGAGIPVVPG SEQ ID NO1556 | 326-356 |
| MMP9+12 | PGFGPGWGVPG SEQ ID NO1557 | 327-338 |
| MMP9+12 | PGFGPGWGVPGA SEQ ID NO1558 | 327-339 |
| MMP9+12 | PGFGPGWGVPGAG SEQ ID NO1559 | 327-340 |
| MMP9+12 | PGVVGVPGAGVPG SEQ ID NO1560 | 331-343 |
| MMP9+12 | PGVVGVPGAGVPGVGVPG SEQ ID NO1561 | 331-348 |
| MMP9+12 | PGVVGVPGAGVPGVGVPGAGIPVVPGA SEQ ID NO1562 | 331-357 |
| MMP9+12 | VVGVPGAGVPGVGVPGA SEQ ID NO1563 | 333-349 |
| MMP9+12 | VGVPGAGVPGVGVPGAGIPVVPGAGIPGAAVPGVVSPEA SEQ ID NO1564 | 334-372 |
| MMP9+12 | AGVPGVGVPGAGIPVVPG SEQ ID NO1565 | 339-356 |
| MMP9+12 | GVPGVGVPGAGIPVVPG SEQ ID NO1566 | 340-356 |
| MMP9+12 | GVPGVGVPGAGIPVVPGA SEQ ID NO1567 | 340-357 |
| MMP9+12 | VPGVGVPGAGIPVVPG SEQ ID NO1568 | 341-356 |
| MMP9+12 | VGVPGAGIPVVPG SEQ ID NO1569 | 344-356 |
| MMP9+12 | VGVPGAGIPVVPGAGIPG SEQ ID NO1570 | 344-361 |
| MMP9+12 | VPGAGIPVVPG SEQ ID NO1571 | 346-356 |
| MMP9+12 | AGIPVVPGAGIPG SEQ ID NO1572 | 349-361 |
| MMP9+12 | AGIPVVPGAGIPGAAVPGVVSPEAAAK SEQ ID NO1573 | 349-375 |
| MMP9+12 | GIPVVPGAGIPG SEQ ID NO1574 | 350-361 |
| MMP9+12 | IPGAAVPGWSPEAAAK SEQ ID NO1575 | 359-375 |
| MMP9+12 | GAAVPGWSPEAAAK SEQ ID NO1576 | 361-375 |
| MMP9+12 | AVPGWSPEAAAK SEQ ID NO1577 | 363-375 |
| MMP9+12 | VPGWSPEAAAK SEQ ID NO1578 | 364-375 |
| MMP9+12 | YGARPGVG SEQ ID NO1579 | 383-390 |
| MMP9+12 | YGARPGVGVG SEQ ID NO1580 | 383-392 |
| MMP9+12 | YGARPGVGVGGIPT SEQ ID NO1581 | 383-396 |
| MMP9+12 | YGARPGVGVGGIPTY SEQ ID NO1582 | 383-397 |
| MMP9+12 | YGARPGVGVGGIPTYG SEQ ID NO1583 | 383-398 |
| MMP9+12 | YGARPGVGVGGIPTYGVG SEQ ID NO1584 | 383-400 |
| MMP9+12 | YGARPGVGVGGIPTYGVGA SEQ ID NO1585 | 383-401 |
| MMP9+12 | YGARPGVGVGGIPTYGVGAG SEQ ID NO1586 | 383-402 |
| MMP9+12 | GARPGVGV SEQ ID NO1587 | 384-391 |
| MMP9+12 | GARPGVGVGG SEQ ID NO1588 | 384-393 |
| MMP9+12 | GARPGVGVGGIP SEQ ID NO1589 | 384-395 |
| MMP9+12 | GARPGVGVGGIPTY SEQ ID NO1590 | 384-397 |
| MMP9+12 | GARPGVGVGGIPTYGV SEQ ID NO1591 | 384-399 |
| MMP9+12 | GARPGVGVGGIPTYGVG SEQ ID NO1592 | 384-400 |
| MMP9+12 | GARPGVGVGGIPTYGVGAGGF SEQ ID NO1593 | 384-404 |
| MMP9+12 | GARPGVGVGGIPTYGVGAGGFPGF SEQ ID NO1594 | 384-407 |
| MMP9+12 | GARPGVGVGGIPTYGVGAGGFPGFG SEQ ID NO1595 | 384-408 |
| MMP9+12 | GARPGVGVGGIPTYGVGAGGFPGFGVGVG SEQ ID NO1596 | 384-412 |
| MMP9+12 | ARPGVGVGG SEQ ID NO1597 | 385-393 |
| MMP9+12 | ARPGVGVGGIP SEQ ID NO1598 | 385-395 |
| MMP9+12 | ARPGVGVGGIPTY SEQ ID NO1599 | 385-397 |
| MMP9+12 | ARPGVGVGGIPTYGVGA SEQ ID NO1600 | 385-401 |
| MMP9+12 | ARPGVGVGGIPTYGVGAGG SEQ ID NO1601 | 385-403 |
| MMP9+12 | ARPGVGVGGIPTYGVGAGGFPG SEQ ID NO1602 | 385-406 |
| MMP9+12 | ARPGVGVGGIPTYGVGAGGFPGF SEQ ID NO1603 | 385-407 |
| MMP9+12 | RPGVGVG SEQ ID NO1604 | 386-392 |
| MMP9+12 | RPGVGVGG SEQ ID NO1605 | 386-393 |
| MMP9+12 | PGVGVGGIPTY SEQ ID NO1606 | 387-397 |
| MMP9+12 | PGVGVGGIPTYG SEQ ID NO1607 | 387-398 |
| MMP9+12 | PGVGVGGIPTYGVGAG SEQ ID NO1608 | 387-412 |
| MMP9+12 | VGGIPTYGVGAG SEQ ID NO1609 | 391-402 |
| MMP9+12 | GVGAGGFPGFGVGVGGIPGVA SEQ ID NO1610 | 398-418 |
| MMP9+12 | VGAGGFPGFGVGVG SEQ ID NO1611 | 399-412 |
| MMP9+12 | VGVGGIPGVAGVPSVGGVPGVGGVPGVGISPEA SEQ ID NO1612 | 409-441 |
| MMP9+12 | VAGVPSVGGVPGVGGVPG SEQ ID NO1613 | 417-434 |
| MMP9+12 | VAGVPSVGGVPGVGGVPGVGISPEA SEQ ID NO1614 | 417-441 |
| MMP9+12 | SVGGVPGVGGVPGVGISPEA SEQ ID NO1615 | 422-441 |
| MMP9+12 | VGGVPGVGGVPGVGISPEA SEQ ID NO1616 | 423-441 |
| MMP9+12 | GVPGVGGVPGVGIS SEQ ID NO1617 | 425-438 |
| MMP9+12 | GVPGVGGVPGVGISPEA SEQ ID NO1618 | 425-441 |
| MMP9+12 | GVPGVGGVPGVGISPEAQA SEQ ID NO1619 | 425-443 |
| MMP9+12 | GVPGVGISPEAQAAAAAK SEQ ID NO1620 | 431-448 |
| MMP9+12 | GVGTPAAAAAK SEQ ID NO1621 | 482-492 |
| MMP9+12 | TPAAAAAK SEQ ID NO1622 | 485-492 |
| MMP9+12 | FGLVPGVGVAPGVG SEQ ID NO1623 | 500-513 |
| MMP9+12 | FGLVPGVGVAPGVGVAPG SEQ ID NO1624 | 500-517 |
| MMP9+12 | FGLVPGVGVAPGVGVAPGVGVAPG SEQ ID NO1625 | 500-523 |
| MMP9+12 | FGLVPGVGVAPGVGVAPGVGVAPGVG SEQ ID NO1626 | 500-525 |
| MMP9+12 | FGLVPGVGVAPGVGVAPGVGVAPGVGLAPG SEQ ID NO1627 | 500-529 |
| MMP9+12 | FGLVPGVGVAPGVGVAPGVGVAPGVGLAPGVGVAPG SEQ ID NO1628 | 500-535 |
| MMP9+12 | FGLVPGVGVAPGVGVAPGVGVAPGVGLAPGVGVAPGV SEQ ID NO1629 | 500-536 |
| MMP9+12 | FGLVPGVGVAPGVGVAPGVGVAPGVGLAPGVGVAPGVGVAPG SEQ ID NO1630 | 500-541 |
| MMP9+12 | GLVPGVGVAPG SEQ ID NO1631 | 501-511 |
| MMP9+12 | GLVPGVGVAPGV SEQ ID NO1632 | 501-512 |
| MMP9+12 | GLVPGVGVAPGVGVA SEQ ID NO1633 | 501-515 |
| MMP9+12 | GLVPGVGVAPGVGVAP SEQ ID NO1634 | 501-516 |
| MMP9+12 | GLVPGVGVAPGVGVAPG SEQ ID NO1635 | 501-517 |
| MMP9+12 | GLVPGVGVAPGVGVAPGVG SEQ ID NO1636 | 501-519 |
| MMP9+12 | GLVPGVGVAPGVGVAPGVGVAPG SEQ ID NO1637 | 501-523 |
| MMP9+12 | GLVPGVGVAPGVGVAPGVGVAPGVGL SEQ ID NO1638 | 501-524 |
| MMP9+12 | GLVPGVGVAPGVGVAPGVGVAPGVGLA SEQ ID NO1639 | 501-525 |
| MMP9+12 | GLVPGVGVAPGVGVAPGVGVAPGVGLAPG SEQ ID NO1640 | 501-527 |
| MMP9+12 | GLVPGVGVAPGVGVAPGVGVAPGVGLAPGVG SEQ ID NO1641 | 501-529 |
| MMP9+12 | GLVPGVGVAPGVGVAPGVGVAPGVGLAPGVGVA SEQ ID NO1642 | 501-531 |
| MMP9+12 | GLVPGVGVAPGVGVAPGVGVAPGVGLAPGVGVAPG SEQ ID NO1643 | 501-533 |
| MMP9+12 | LVPGVGVAPGVG SEQ ID NO1644 | 502-513 |
| MMP9+12 | LVPGVGVAPGVGVAPG SEQ ID NO1645 | 502-517 |
| MMP9+12 | LVPGVGVAPGVGVAPGVG SEQ ID NO1646 | 502-519 |
| MMP9+12 | LVPGVGVAPGVGVAPGVGVAPGVG SEQ ID NO1647 | 502-525 |
| MMP9+12 | LVPGVGVAPGVGVAPGVGVAPGVGLAPGVGVAPGVG SEQ ID NO1648 | 502-537 |
| MMP9+12 | PGVGVAPGVGVAPG SEQ ID NO1649 | 504-517 |
| MMP9+12 | VGVAPGVGVAPGVGV SEQ ID NO1650 | 506-520 |
| MMP9+12 | VGVAPGVGVAPGVGVAPGVG SEQ ID NO1651 | 506-525 |
| MMP9+12 | VGVAPGVGVAPGVGVAPGVGLAPGVGVAPG SEQ ID NO1652 | 506-535 |
| MMP9+12 | VAPGVGVAPGVGVAPG SEQ ID NO1653 | 508-523 |
| MMP9+12 | VAPGVGVAPGVGVAPGVG SEQ ID NO1654 | 508-525 |
| MMP9+12 | VAPGVGVAPGVGVAPGVGLAPGVG SEQ ID NO1655 | 508-531 |
| MMP9+12 | VAPGVGVAPGVGVAPGVGLAPGVGVAPG SEQ ID NO1656 | 508-535 |
| MMP9+12 | VGVAPGVGVAPGVGLA SEQ ID NO1657 | 512-527 |
| MMP9+12 | VGVAPGVGVAPGVGLAPGVGVAPG SEQ ID NO1658 | 512-535 |
| MMP9+12 | VGVAPGVGVAPGVGLAPGVGVAPGVGVAPGVGVAPGIGPGG SEQ ID NO1659 | 512-552 |
| MMP9+12 | VAPGVGVAPGVGLAPGVGVAPGVG SEQ ID NO1660 | 514-537 |
| MMP9+12 | VAPGVGVAPGVGLAPGVGVAPGVGVA SEQ ID NO1661 | 514-539 |
| MMP9+12 | VAPGVGVAPGVGLAPGVGVAPGVGVAPG SEQ ID NO1662 | 514-541 |
| MMP9+12 | VAPGVGVAPGVGLAPGVGVAPGVGVAPGVGVAPGIGP SEQ ID NO1663 | 514-550 |
| MMP9+12 | VAPGVGVAPGVGLAPGVGVAPGVGVAPGVGVAPGIGPGG SEQ ID NO1664 | 514-552 |
| MMP9+12 | PGVGVAPGVGLAPG SEQ ID NO1665 | 516-529 |
| MMP9+12 | PGVGVAPGVGLAPGVGVAP SEQ ID NO1666 | 516-534 |
| MMP9+12 | PGVGVAPGVGLAPGVGVAPGVG SEQ ID NO1667 | 516-537 |
| MMP9+12 | VGVAPGVGLAPGVGVA SEQ ID NO1668 | 518-533 |
| MMP9+12 | VGVAPGVGLAPGVGVAP SEQ ID NO1669 | 518-534 |
| MMP9+12 | VGVAPGVGLAPGVGVAPGVGVAPG SEQ ID NO1670 | 518-541 |
| MMP9+12 | VGVAPGVGLAPGVGVAPGVGVAPGVG SEQ ID NO1671 | 518-543 |
| MMP9+12 | VGVAPGVGLAPGVGVAPGVGVAPGVGVAPG SEQ ID NO1672 | 518-547 |
| MMP9+12 | VGVAPGVGLAPGVGVAPGVGVAPGVGVAPGIGPGG SEQ ID NO1673 | 518-552 |
| MMP9+12 | GVAPGVGLAPGVGVAPGVGVAPGVGVAPGIGPGG SEQ ID NO1674 | 519-552 |
| MMP9+12 | VAPGVGLAPGVGVA SEQ ID NO1675 | 520-533 |
| MMP9+12 | VAPGVGLAPGVGVAPG SEQ ID NO1676 | 520-535 |
| MMP9+12 | VAPGVGLAPGVGVAPGVG SEQ ID NO1677 | 520-537 |
| MMP9+12 | VAPGVGLAPGVGVAPGVGVA SEQ ID NO1678 | 520-539 |
| MMP9+12 | VAPGVGLAPGVGVAPGVGVAPG SEQ ID NO1679 | 520-541 |
| MMP9+12 | VAPGVGLAPGVGVAPGVGVAPGVGVA SEQ ID NO1680 | 520-545 |
| MMP9+12 | VAPGVGLAPGVGVAPGVGVAPGVGVAPGIGPGG SEQ ID NO1681 | 520-552 |
| MMP9+12 | PGVGLAPGVGVAPG SEQ ID NO1682 | 522-535 |
| MMP9+12 | GVGLAPGVGVAPGVGVAPG SEQ ID NO1683 | 523-541 |
| MMP9+12 | VGLAPGVGVAPGVG SEQ ID NO1684 | 524-537 |
| MMP9+12 | VGLAPGVGVAPGVGVAPG SEQ ID NO1685 | 524-541 |
| MMP9+12 | VGLAPGVGVAPGVGVAPGVGVAPGIG SEQ ID NO1686 | 524-549 |
| MMP9+12 | VGLAPGVGVAPGVGVAPGVGVAPGIGPGG SEQ ID NO1687 | 524-552 |
| MMP9+12 | VGLAPGVGVAPGVGVAPGVGVAPGIGPG SEQ ID NO1688 | 524-553 |
| MMP9+12 | VGLAPGVGVAPGVGVAPGVGVAPGIGPGGVAAA SEQ ID NO1689 | 524-556 |
| MMP9+12 | LAPGVGVAPGVGVAPGVG SEQ ID NO1690 | 526-543 |
| MMP9+12 | LAPGVGVAPGVGVAPGVGVA SEQ ID NO1691 | 526-545 |
| MMP9+12 | LAPGVGVAPGVGVAPGVGVAPGIGP SEQ ID NO1692 | 526-550 |
| MMP9+12 | LAPGVGVAPGVGVAPGVGVAPGIGPGG SEQ ID NO1693 | 526-552 |
| MMP9+12 | GVGVAPGVGVAPGVGVAPGIGPGG SEQ ID NO1694 | 529-552 |
| MMP9+12 | VGVAPGVGVAPGVGVA SEQ ID NO1695 | 530-545 |
| MMP9+12 | VGVAPGVGVAPGVGVAPG SEQ ID NO1696 | 530-547 |
| MMP9+12 | VGVAPGVGVAPGVGVAPGIGPG SEQ ID NO1697 | 530-551 |
| MMP9+12 | VGVAPGVGVAPGVGVAPGIGPGG SEQ ID NO1698 | 530-552 |
| MMP9+12 | VGVAPGVGVAPGVGVAPGIGPGGVAAA SEQ ID NO1699 | 530-556 |
| MMP9+12 | VAPGVGVAPGVGVAP SEQ ID NO1700 | 532-546 |
| MMP9+12 | VAPGVGVAPGVGVAPGIG SEQ ID NO1701 | 532-549 |
| MMP9+12 | VAPGVGVAPGVGVAPGIGPGG SEQ ID NO1702 | 532-552 |
| MMP9+12 | PGVGVAPGVGVAPGIGPG SEQ ID NO1703 | 534-551 |
| MMP9+12 | PGVGVAPGVGVAPGIGPGG SEQ ID NO1704 | 534-552 |
| MMP9+12 | VGVAPGVGVAPGIGPGG SEQ ID NO1705 | 536-552 |
| MMP9+12 | VGVAPGVGVAPGIGPGGVAA SEQ ID NO1706 | 536-555 |
| MMP9+12 | VAPGVGVAPGIGPG SEQ ID NO1707 | 538-551 |
| MMP9+12 | PGVGVAPGIGPG SEQ ID NO1708 | 540-551 |
| MMP9+12 | VGVAPGIGPGGVAA SEQ ID NO1709 | 542-555 |
| MMP9+12 | PGGVAAAAK SEQ ID NO1710 | 550-558 |
| MMP9+12 | LRAAAGL SEQ ID NO1711 | 569-575 |
| MMP9+12 | LRAAAGLG SEQ ID NO1712 | 569-576 |
| MMP9+12 | LRAAAGLGA SEQ ID NO1713 | 569-577 |
| MMP9+12 | AAAGLGAGIPGLGVG SEQ ID NO1714 | 571-585 |
| MMP9+12 | AAAGLGAGIPGLGVGVG SEQ ID NO1715 | 571-587 |
| MMP9+12 | LGAGIPGLGVG SEQ ID NO1716 | 575-585 |
| MMP9+12 | LGAGIPGLGVGVG SEQ ID NO1717 | 575-587 |
| MMP9+12 | LGAGIPGLGVGVGVPGLGVG SEQ ID NO1718 | 575-594 |
| MMP9+12 | LGAGIPGLGVGVGVPG SEQ ID NO1719 | 575-590 |
| MMP9+12 | LGAGIPGLGVGVGVPGLGVGA SEQ ID NO1720 | 575-595 |
| MMP9+12 | LGAGIPGLGVGVGVPGL SEQ ID NO1721 | 575-591 |
| MMP9+12 | LGAGIPGLGVGVGVPGLG SEQ ID NO1722 | 575-592 |
| MMP9+12 | LGAGIPGLGVGVGVPGLGVGAGVPG SEQ ID NO1723 | 575-599 |
| MMP9+12 | LGAGIPGLGVGVGVPGLGVGAGVPGLG SEQ ID NO1724 | 575-601 |
| MMP9+12 | LGAGIPGLGVGVGVPGLGVGAGVPGLGVG SEQ ID NO1725 | 575-603 |
| MMP9+12 | LGAGIPGLGVGVGVPGLGVGAGVPGLGVGAGVPGFG SEQ ID NO1726 | 575-610 |
| MMP9+12 | GAGIPGLGVGVGVPGLG SEQ ID NO1727 | 576-592 |
| MMP9+12 | AGIPGLGVGVGVPG SEQ ID NO1728 | 577-590 |
| MMP9+12 | GIPGLGVGVGVPGLGVGA SEQ ID NO1729 | 578-595 |
| MMP9+12 | LGVGVGVPGLGVGA SEQ ID NO1730 | 582-595 |
| MMP9+12 | VGVPGLGVGAGVPG SEQ ID NO1731 | 586-599 |
| MMP9+12 | VGVPGLGVGAGVPGL SEQ ID NO1732 | 586-600 |
| MMP9+12 | VGVPGLGVGAGVPGLG SEQ ID NO1733 | 586-601 |
| MMP9+12 | VGVPGLGVGAGVPGLGVG SEQ ID NO1734 | 586-603 |
| MMP9+12 | VGVPGLGVGAGVPGLGVGA SEQ ID NO1735 | 586-604 |
| MMP9+12 | VGAGVPGLGVGAGVPGFG SEQ ID NO1736 | 593-610 |
| MMP9+12 | PGALAAAK SEQ ID NO1737 | 646-653 |
| MMP9+12 | AKYGAAVPGVLGGLGA SEQ ID NO1738 | 655-670 |
| MMP9+12 | YGAAVPGVLGG SEQ ID NO1739 | 657-667 |
| MMP9+12 | YGAAVPGVLGGLG SEQ ID NO1740 | 657-669 |
| MMP9+12 | YGAAVPGVLGGLGA SEQ ID NO1741 | 657-670 |
| MMP9+12 | YGAAVPGVLGGLGALG SEQ ID NO1742 | 657-672 |
| MMP9+12 | YGAAVPGVLGGLGALGGVGIPGG SEQ ID NO1743 | 657-679 |
| MMP9+12 | YGAAVPGVLGGLGALGGVGIPGGVVGAGPAA SEQ ID NO1744 | 657-687 |
| MMP9+12 | GAAVPGVLGGLG SEQ ID NO1745 | 658-669 |
| MMP9+12 | GAAVPGVLGGLGALGGVGIPGG SEQ ID NO1746 | 658-679 |
| MMP9+12 | AVPGVLGGLGA SEQ ID NO1747 | 660-670 |
| MMP9+12 | AVPGVLGGLGALGGVGIPGG SEQ ID NO1748 | 660-679 |
| MMP9+12 | VLGGLGALGGVGIPGG SEQ ID NO1749 | 664-679 |
| MMP9+12 | GGLGALGGVGIPGGVVGAGPA SEQ ID NO1750 | 666-686 |
| MMP9+12 | GGLGALGGVGIPGGVVGAGPAAA SEQ ID NO1751 | 666-688 |
| MMP9+12 | LGALGGVGIPGG SEQ ID NO1752 | 668-379 |
| MMP9+12 | LGALGGVGIPGGVVGAGPA SEQ ID NO1753 | 668-686 |
| MMP9+12 | LGALGGVGIPGGVVGAGPAA SEQ ID NO1754 | 668-687 |
| MMP9+12 | LGALGGVGIPGGVVGAGPAAA SEQ ID NO1755 | 668-688 |
| MMP9+12 | LGALGGVGIPGGVVGAGPAAAA SEQ ID NO1756 | 668-689 |
| MMP9+12 | ALGGVGIPGGVVGAGPAA SEQ ID NO1757 | 670-687 |
| MMP9+12 | ALGGVGIPGGVVGAGPAAA SEQ ID NO1758 | 670-688 |
| MMP9+12 | LGGVGIPGGV SEQ ID NO1759 | 671-680 |
| MMP9+12 | LGGVGIPGGVVGAGPA SEQ ID NO1760 | 671-686 |
| MMP9+12 | LGGVGIPGGVVGAGPAAA SEQ ID NO1761 | 671-688 |
| MMP9+12 | LGGVGIPGGVVGAGPAAAAA SEQ ID NO1762 | 671-690 |
| MMP9+12 | LGGVGIPGGVVGAGPAAAAAAAK SEQ ID NO1763 | 671-693 |
| MMP9+12 | GVGIPGGVVGAGPAAAA SEQ ID NO1764 | 673-689 |
| MMP9+12 | GVGIPGGVVGAGPAAAAAAAK SEQ ID NO1765 | 673-693 |
| MMP9+12 | VGIPGGVVGAGPAAA SEQ ID NO1766 | 674-688 |
| MMP9+12 | VGIPGGWGAGPAAAAAAAK SEQ ID NO1767 | 674-693 |
| MMP9+12 | IPGGWGAGPAAAA SEQ ID NO1768 | 676-689 |
| MMP9+12 | WGAGPAAAAAAAK SEQ ID NO1769 | 680-693 |
| MMP9+12 | VGAGPAAAAAAAK SEQ ID NO1770 | 681-693 |
| MMP9+12 | AGPAAAAAAAK SEQ ID NO1771 | 683-693 |
| MMP9+12 | GPAAAAAAAK SEQ ID NO1772 | 684-693 |
| MMP9+12 | PAAAAAAAK SEQ ID NO1773 | 685-693 |
| MMP9+12 | FGLVGAAGLGGLGVGGLGVPGVGG SEQ ID NO1774 | 701-724 |
| MMP9+12 | GLVGAAGLGGLG SEQ ID NO1775 | 702-713 |
| MMP9+12 | GLVGAAGLGGLGVGG SEQ ID NO1776 | 702-716 |
| MMP9+12 | GLVGAAGLGGLGVGGLGVPGVG SEQ ID NO1777 | 702-723 |
| MMP9+12 | GLVGAAGLGGLGVGGLGVPGVGG SEQ ID NO1778 | 702-724 |
| MMP9+12 | LVGAAGLGGLGVG SEQ ID NO1779 | 703-715 |
| MMP9+12 | LVGAAGLGGLGVGG SEQ ID NO1780 | 703-716 |
| MMP9+12 | LVGAAGLGGLGVGGL SEQ ID NO1781 | 703-717 |
| MMP9+12 | LVGAAGLGGLGVGGLGVPGVGGLG SEQ ID NO1782 | 703-726 |
| MMP9+12 | LVGAAGLGGLGVGGLGVPGVGGLGGIPPAAA SEQ ID NO1783 | 703-733 |
| MMP9+12 | VGAAGLGGLGVGG SEQ ID NO1784 | 704-716 |
| MMP9+12 | LGGLGVGGLGVPG SEQ ID NO1785 | 709-721 |
| MMP9+12 | LGGLGVGGLGVPGVG SEQ ID NO1786 | 709-723 |
| MMP9+12 | LGGLGVGGLGVPGVGGL SEQ ID NO1787 | 709-725 |
| MMP9+12 | LGGLGVGGLGVPGVGGLG SEQ ID NO1788 | 709-726 |
| MMP9+12 | LGVGGLGVPGVGGLG SEQ ID NO1789 | 712-726 |
| MMP9+12 | GLGVPGVGGLGGIPPAAAAK SEQ ID NO1790 | 716-735 |
| MMP9+12 | LGGIPPAAAAK SEQ ID NO1791 | 725-735 |
| MMP9+12 | LGGVLGGAGQFPL SEQ ID NO1792 | 744-756 |
| MMP9+12 | LGGVLGGAGQFPLGGVAAR SEQ ID NO1793 | 744-762 |
| MMP9+12 | LGGVLGGAGQFPLGGVAARPG SEQ ID NO1794 | 744-764 |
| MMP9+12 | LGGVLGGAGQFPLGGVAARPGFG SEQ ID NO1795 | 744-766 |
| MMP9+12 | GGVLGGAGQFPLGGVAARPG SEQ ID NO1796 | 745-764 |
| MMP9+12 | GAGQFPLGGVAAR SEQ ID NO1797 | 750-762 |
| MMP9+12 | GAGQFPLGGVAARPGFG SEQ ID NO1798 | 750-766 |
| MMP9+12 | AGQFPLGGVAARPGFG SEQ ID NO1799 | 751-766 |
| MMP9+12 | FPLGGVAARPG SEQ ID NO1800 | 754-764 |
| MMP9+12 | PLGGVAAR SEQ ID NO1801 | 755-762 |
| MMP9+12 | PLGGVAARPG SEQ ID NO1802 | 755-764 |
| MMP9+12 | PLGGVAARPGFG SEQ ID NO1803 | 755-766 |
| MMP9+12 | PLGGVAARPGFGL SEQ ID NO1804 | 755-767 |
| MMP9+12 | PLGGVAARPGFGLSPIFPG SEQ ID NO1805 | 755-773 |
| MMP9+12 | LGGVAAR SEQ ID NO1806 | 756-762 |
| MMP9+12 | LGGVAARP SEQ ID NO1807 | 756-763 |
| MMP9+12 | LGGVAARPG SEQ ID NO1808 | 756-764 |
| MMP9+12 | LGGVAARPGF SEQ ID NO1809 | 756-765 |
| MMP9+12 | LGGVAARPGFG SEQ ID NO1810 | 756-766 |
| MMP9+12 | LGGVAARPGFGL SEQ ID NO1811 | 756-767 |
| MMP9+12 | LGGVAARPGFGLSP SEQ ID NO1812 | 756-769 |
| MMP9+12 | LGGVAARPGFGLSPIFPG SEQ ID NO1813 | 756-773 |
| MMP9+12 | LGGVAARPGFGLSPIFPGG SEQ ID NO1814 | 756-774 |
| MMP9+12 | LGGVAARPGFGLSPIFPGGA SEQ ID NO1815 | 756-775 |
| MMP9+12 | GGVAARPGFG SEQ ID NO1816 | 757-766 |
| MMP9+12 | GGVAARPGFGL SEQ ID NO1817 | 757-767 |
| MMP9+12 | GGVAARPGFGLSPIFPGGA SEQ ID NO1818 | 757-775 |
| MMP9+12 | GVAARPGFGLSPIF SEQ ID NO1819 | 758-771 |
| MMP9+12 | GVAARPGFGLSPIFP SEQ ID NO1820 | 758-772 |
| MMP9+12 | VAARPGFG SEQ ID NO1821 | 759-766 |
| MMP9+12 | VAARPGFGLSPIFP SEQ ID NO1822 | 759-772 |
| MMP9+12 | VAARPGFGLSPIFPG SEQ ID NO1823 | 759-773 |
| MMP9+12 | RPGFGLSPIFPG SEQ ID NO1824 | 762-773 |
| MMP9+12 | PGFGLSPIFPGG SEQ ID NO1825 | 763-774 |
| MMP9+12 | PGFGLSPIFPGGA SEQ ID NO1826 | 763-775 |
| ADAMTS-1 | P.GVGLPGVYPGGVLPGAR.F SEQ ID NO1827 | 143-159 |
| ADAMTS-1 | G.VGLPGVYPGGVLPGAR.F SEQ ID NO1828 | 144-159 |
| ADAMTS-1 | G.LPGVYPGGVLPGAR.F SEQ ID NO1829 | 146-159 |
| ADAMTS-1 | P.GVYPGGVLPGAR.F SEQ ID NO1830 | 148-159 |
| ADAMTS-1 | K.AGYPTGTGVGPQAAAAAAAK.A SEQ ID NO1831 | 242-261 |
| ADAMTS-1 | G.GPGFGPGVVGVPGAGVPGVGVPGA.G SEQ ID NO1832 | 326-349 |
| ADAMTS-1 | G.FGPGVVGVPGAGVPGVGVPG.A SEQ ID NO1833 | 329-348 |
| ADAMTS-1 | F.GPGVVGVPGAGVPGVGVPG.A SEQ ID NO1834 | 330-348 |
| ADAMTS-1 | G.VPGVGVPGAGIPVVPG.A SEQ ID NO1835 | 341-356 |
| ADAMTS-1 | G.ARPGVGVGGIPTYGVG.A SEQ ID NO1836 | 385-400 |
| ADAMTS-1 | G.ARPGVGVGGIPTYGVGAGG.F SEQ ID NO1837 | 385-403 |
| ADAMTS-1 | A.RPGVGVGGIPTYGVGAG.G SEQ ID NO1838 | 386-402 |
| ADAMTS-1 | G.GVPGVGGVPGVGISPEAQAAAA.A SEQ ID NO1839 | 425-446 |
| ADAMTS-1 | G.VPGVGISPEAQAAAAAK.A SEQ ID NO1840 | 432-448 |
| ADAMTS-1 | G.VGISPEAQAAAAAK.A SEQ ID NO1841 | 435-448 |
| ADAMTS-1 | V.PGVGVAPGVGVAPGVGVAPGVGL.A SEQ ID NO1842 | 504-526 |
| ADAMTS-1 | G.VAPGVGVAPGVGVAPGVGLAPGVGVAPG.V SEQ ID NO1843 | 508-535 |
| ADAMTS-1 | G.VGVAPGVGVAPGVGLAPGVG.V SEQ ID NO1844 | 512-531 |
| ADAMTS-1 | G.VGVAPGVGVAPGVGLAPGVGVAPGVG.V SEQ ID NO1845 | 512-537 |
| ADAMTS-1 | A.PGVGVAPGVGVAPGVGVAPGIGPG.G SEQ ID NO1846 | 528-551 |
| ADAMTS-1 | G.VAPGVGVAPGVGVAPGIGPG.G SEQ ID NO1847 | 532-551 |
| ADAMTS-1 | G.AAVPGVLGGLGALGGVGIPG.G SEQ ID NO1848 | 659-678 |
| ADAMTS-1 | G.AAGLGGLGVGGLGVPGVGGLG.G SEQ ID NO1849 | 706-726 |
| ADAMTS-4 | P.GVGLPGVYPGGVLPGAR.F SEQ ID NO1827 | 143-159 |
| ADAMTS-4 | G.LPGVYPGGVLPGAR.F SEQ ID NO1829 | 146-159 |
| ADAMTS-4 | K.AGYPTGTGVGPQAAAAAAAK.A SEQ ID NO1831 | 242-261 |
| ADAMTS-4 | G.GAGVPGVPGAIPGIGGIAGVG.T SEQ ID NO1850 | 279-299 |
| ADAMTS-4 | G.AGVPGVPGAIPGIGGIAGVG.T SEQ ID NO1851 | 280-299 |
| ADAMTS-4 | A.GVGTPAAAAAAAAAAK.A SEQ ID NO1852 | 297-312 |
| ADAMTS-4 | G.VGTPAAAAAAAAAAK.A SEQ ID NO1853 | 298-312 |
| ADAMTS-4 | G.GPGFGPGVVGVPGAGVPGVGVPG.A SEQ ID NO1854 | 326-348 |
| ADAMTS-4 | G.ARPGVGVGGIPTYGVGA.G SEQ ID NO1855 | 385-401 |
| ADAMTS-4 | A.RPGVGVGGIPTYGVGAG.G SEQ ID NO1838 | 386-402 |
| ADAMTS-4 | A.RPGVGVGGIPTYGVGAGG.F SEQ ID NO1856 | 386-403 |
| ADAMTS-4 | G.VGISPEAQAAAAAK.A SEQ ID NO1841 | 435-448 |
| ADAMTS-4 | G.VGVAPGVGVAPGVGVAPGVGLAPGVG.V SEQ ID NO1857 | 506-531 |
| ADAMTS-4 | A.PGVGVAPGVGLAPGVGVAPGVGVA.P SEQ ID NO1858 | 516-539 |
| ADAMTS-4 | G.VGVAPGVGLAPGVGVAPGVG.V SEQ ID NO1859 | 518-537 |
| ADAMTS-4 | L.APGVGVAPGVGVAPGVGVAPGIGPG.G SEQ ID NO1860 | 527-551 |
| ADAMTS-4 | Y.GAAVPGVLGGLGALGGVGIPG.G SEQ ID NO1861 | 658-678 |
| ADAMTS-4 | G.AAVPGVLGGLGALGGVGIPG.G SEQ ID NO1848 | 659-678 |
| ADAMTS-4 | G.GAGQFPLGGVAARPGFGL.S SEQ ID NO1862 | 750-767 |
| ADAMTS-8 | L.VPGGVADAAAAYK.A SEQ ID NO1863 | 092-104 |
| ADAMTS-8 | G.VGLPGVYPGGVLPGAR.F SEQ ID NO1828 | 144-159 |
| ADAMTS-8 | G.LPGVYPGGVLPGAR.F SEQ ID NO1829 | 146-159 |
| ADAMTS-8 | P.GVYPGGVLPGAR.F SEQ ID NO1830 | 148-159 |
| ADAMTS-8 | V.YPGGVLPGAR.F SEQ ID NO1864 | 150-159 |
| ADAMTS-8 | F.GPGVVGVPGAGVPGVGVPG.A SEQ ID NO 1834 | 330-348 |
| ADAMTS-8 | G.ARPGVGVGGIPTYGVGA.G SEQ ID NO1855 | 385-401 |
| ADAMTS-8 | V.APGVGVAPGVGVAPGVGLAPGVGV.A SEQ ID NO1865 | 509-532 |
| ADAMTS-8 | L.APGVGVAPGVGVAPGVGV.A SEQ ID NO1866 | 527-544 |
| ADAMTS-8 | L.APGVGVAPGVGVAPGVGVAPG.I SEQ ID NO1867 | 527-547 |
| ADAMTS-8 | L.APGVGVAPGVGVAPGVGVAPGIG.P SEQ ID NO1868 | 527-549 |
| ADAMTS-8 | L.APGVGVAPGVGVAPGVGVAPGIGP.G SEQ ID NO1869 | 527-550 |
| ADAMTS-8 | L.APGVGVAPGVGVAPGVGVAPGIGPG.G SEQ ID NO1860 | 527-551 |
| ADAMTS-8 | L.APGVGVAPGVGVAPGVGVAPGIGPGGVAA.A SEQ ID NO1870 | 527-555 |
| ADAMTS-8 | G.VGVAPGVGVAPGVGVAPGIGPG.G SEQ ID NO1871 | 530-551 |
| ADAMTS-8 | G.VAPGVGVAPGVGVAPGIGPG.G SEQ ID NO1847 | 532-551 |
| ADAMTS-8 | G.AAVPGVLGGLGALGGVGIPG.G SEQ ID NO1848 | 659-678 |
| ADAMTS-8 | G.AAVPGVLGGLGALGGVGIPGG.V SEQ ID NO1872 | 659-679 |
| ADAMTS-8 | A.AVPGVLGGLGALGGVGIPG.G SEQ ID NO1873 | 660-678 |
| ADAMTS-8 | A.VPGVLGGLGALGGVGIPGG.V SEQ ID NO1874 | 661-679 |
| ADAMTS-8 | A.GQFPLGGVAARPGFGL.S SEQ ID NO1875 | 752-767 |
| | | |
| Cat K | G.ALVPGGVADAAAAYK.A SEQ ID NO1876 | 090-104 |
| Cat K | G.LPYTTGKLPYGYGPG.G SEQ ID NO1877 | 219-233 |
| Cat K | A.AAAAAAKAAAKFGA.G SEQ ID NO1878 | 255-268 |
| Cat K | A.GVGTPAAAAAAAAAAK.A SEQ ID NO1852 | 297-312 |
| Cat K | A.AAAAAAAAAKAAKYGA.A SEQ ID NO1879 | 303-318 |
| Cat K | G.FGPGVVGVPGAGVPGVGVPG.A SEQ ID NO1833 | 329-348 |
| Cat K | G.VGISPEAQAAAAAK.A SEQ ID NO1841 | 435-448 |
| Cat K | G.VAPGVGVAPGVGVAPGVGLAPGVG.V SEQ ID NO1880 | 508-531 |
| Cat K | G.VGVAPGVGVAPGVGVAPGIGPG.G SEQ ID NO1871 | 530-551 |
| Cat K | G.VAPGVGVAPGVGVAPGIGPG.G SEQ ID NO1847 | 532-551 |
| | | |
| Cat S | T.FPGALVPGGVADAAAAYK.A SEQ ID NO1881 | 087-104 |
| Cat S | G.VGLPGVYPGGVLPGAR.F SEQ ID NO1828 | 144-159 |
| Cat S | G.LPGVYPGGVLPGARFPGVG.V SEQ ID NO1882 | 146-164 |
| Cat S | G.YPTGTGVGPQAAAAAAAK.A SEQ ID NO1883 | 244-261 |
| Cat S | G.GAGVPGVPGAIPGIGGIAGVG.T SEQ ID NO1850 | 279-299 |
| Cat S | G.TPAAAAAAAAAAKAAK.Y SEQ ID NO1884 | 300-315 |
| Cat S | G.VPGAGVPGVGVPGAGIPVVP.G SEQ ID NO1885 | 336-355 |
| Cat S | G.VPGAGVPGVGVPGAGIPVVPGAGIPG.A SEQ ID NO1886 | 336-361 |
| Cat S | G.ISPEAQAAAAAKAAK.Y SEQ ID NO1887 | 437-451 |
| Cat S | V.PGVGVAPGVGVAPGVGVA.P SEQ ID NO1888 | 504-521 |
| Cat S | G.VAPGVGVAPGVGVAPGIGPGGVA.A SEQ ID NO1889 | 532-554 |
| Cat S | G.IPGGVVGAGPAAAAAAAK.A SEQ ID NO1890 | 676-693 |
| | | |
| MMP1 | G.GVLPGARFPGVGVLPGVPTGA.G SEQ ID NO1891 | 153-173 |
| MMP1 | G.GVPGVGGVPGVGISPEA.Q SEQ ID NO1892 | 425-441 |
| MMP1 | V.PGVGVAPGVGVAPGVGVA.P SEQ ID NO1888 | 504-521 |
| MMP1 | G.VGVAPGVGVAPGVGVAPGVG.L SEQ ID NO1893 | 506-525 |
| MMP1 | G.VAPGVGVAPGVGVAPGIGPG.G SEQ ID NO1847 | 532-551 |
| MMP1 | A.AVPGVLGGLGALGGVGIPG.G SEQ ID NO1873 | 660-678 |
| MMP1 | | |
| MMP3 | G.ALVPGGVADAAAAYK.A SEQ ID NO1876 | 090-104 |
| MMP3 | G.YPTGTGVGPQAAAAAAAK.A SEQ ID NO1883 | 244-261 |
| MMP3 | G.VPGVPGAIPGIGGIAGVG.T SEQ ID NO1894 | 282-299 |
| MMP3 | F.GPGVVGVPGAGVPGVGVPGA.G SEQ ID NO1895 | 330-349 |
| MMP3 | G.VGISPEAQAAAAAK.A SEQ ID NO1841 | 435-448 |
| MMP3 | G.VGVAPGVGVAPGVGLAPGVG.V SEQ ID NO1844 | 512-531 |
| MMP3 | G.VAPGVGVAPGVGVAPGIGPG.G SEQ ID NO1847 | 532-551 |
| | | |
| MMP8 | P.GVYPGGVLPGAR.F SEQ ID NO1830 | 148-159 |
| MMP8 | K.AGYPTGTGVGPQAAAAAAAK.A SEQ ID NO1831 | 242-261 |
| MMP8 | G.VPGVPGAIPGIGGIAGVG.T SEQ ID NO1894 | 282-299 |
| MMP8 | F.GPGVVGVPGAGVPGVGVPG.A SEQ ID NO1834 | 330-348 |
| MMP8 | G.VPGVGVPGAGIPVVPGA.G SEQ ID NO1896 | 341-357 |
| MMP8 | G.ARPGVGVGGIPTYGVG.A SEQ ID NO1836 | 385-400 |
| MMP8 | A.RPGVGVGGIPTYGVGAG.G SEQ ID NO1838 | 386-402 |
| MMP8 | G.VGVAPGVGVAPGVGVAP.G SEQ ID NO1897 | 506-522 |
| MMP8 | G.VGVAPGVGVAPGVGLAPGVG.V SEQ ID NO 1844 | 512-531 |
| MMP8 | G.VGVAPGVGVAPGVGVAP.G SEQ ID NO1897 | 530-546 |
| MMP8 | G.IPGGVVGAGPAAAAAAAK.A SEQ ID NO1890 | 676-693 |

| | | |
|---|---|---|
| * **Aminoacid residue numbers in the human elastin sequence** | | |

Accordingly, in a method of the invention, said peptide fragments preferably comprise a neo-epitope formed by cleavage of elastin by a protease at an N- or C-terminal site, or where indicated a site marked by the sign · in any one of the partial sequences of elastin in Table 24.

The immunological binding partner may be one specifically reactive with a C-terminal or N-terminal neo-epitope formed by cleavage of elastin.

Suitable immunological binding partners may therefore be specifically reactive with any of the following sequences at the N terminal of a peptide:

**Table 25. N-terminal sequences of protease generated peptide fragments of elastin. Elastin**

| | | |
|---|---|---|
| GVPGAI SEQ ID NO1898 | AIPGGV SEQ ID NO1899 | GVPGGV SEQ ID NO1900 |
| ALGGGA SEQ ID NO1901 | LGGGAL SEQ ID NO1902 | GGALGP SEQ ID NO1903 |
| PLKPVP SEQ ID NO1904 | LKPVPG SEQ ID NO1905 | GLAGAG SEQ ID NO1906 |
| GLGAGL SEQ ID NO1907 | LGAGLG SEQ ID NO1908 | AGLGAF SEQ ID NO1909 |
| LVPGGV SEQ ID NO1910 | VADAAA SEQ ID NO1911 | KAAKAG SEQ ID NO1912 |
| PVGYPG SEQ ID NO1913 | ARFPGV SEQ ID NO1914 | RFPGVG SEQ ID NO1915 |
| VGPFGG SEQ ID NO1916 | GPQPGV SEQ ID NO1917 | PQPGVP SEQ ID NO1918 |
| TTGKLP SEQ ID NO1919 | LPYGYG SEQ ID NO1920 | GYGPGG SEQ ID NO1921 |
| FGAGAA SEQ ID NO1922 | GVLPGV SEQ ID NO1923 | VLPGVG SEQ ID NO1924 |
| AGIPGL SEQ ID NO1925 | VPGAIP SEQ ID NO1926 | AIPGIG SEQ ID NO1927 |
| TPAAAA SEQ ID NO1928 | PAAAAA SEQ ID NO1929 | AAAAAA SEQ ID NO1930 |
| VGVPGA SEQ ID NO1931 | AVGPGV SEQ ID NO1932 | GVPGVG SEQ ID NO1933 |
| GIPVVP SEQ ID NO1934 | IPGAAV SEQ ID NO1935 | GAAVPG SEQ ID NO1936 |
| ARPGVG SEQ ID NO1937 | RPGVGV SEQ ID NO1938 | VGGIPT SEQ ID NO1939 |
| SVGGVP SEQ ID N01940 | VGGVPG SEQ ID NO1941 | GVGTPA SEQ ID NO1942 |
| VGVAPG SEQ ID NO1943 | VAPGVG SEQ ID NO1944 | GVAPGV SEQ ID NO1945 |
| GVPVAP SEQ ID NO1946 | GAGIPG SEQ ID NO1947 | PGGVAA SEQ ID NO1948 |
| LGAGIP SEQ ID NO1949 | PGFGPG SEQ ID NO1950 | PGVVGV SEQ ID NO1951 |
| PGALAA SEQ ID NO1952 | AKYGAA SEQ ID NO1953 | YGAAVP SEQ ID NO1954 |
| ALGGVG SEQ ID NO1955 | LGGVGI SEQ ID NO1956 | GVGIPG SEQ ID NO1957 |
| AGPAAA SEQ ID NO1958 | GPAAAA SEQ ID NO1959 | FGLVGA SEQ ID NO1960 |
| GLGVPG SEQ ID NO1961 | LGGIPP SEQ ID NO1962 | LGGVLG SEQ ID NO1963 |
| PLGGVA SEQ ID NO1964 | LGGVAA SEQ ID NO1965 | GGVAAR SEQ ID NO1966 |
| GVGLPG SEQ ID NO1967 | VGLPGV SEQ ID NO1968 | LPGVYP SEQ ID NO1969 |
| VPGVPV SEQ ID NO1970 | VPGVGI SEQ ID NO1971 | VGISPE SEQ ID NO1972 |
| APGVGV SEQ ID N01973 | VPGGVA SEQ ID NO1974 | YPGGVL SEQ ID NO1975 |
| GPGFGP SEQ ID NO1976 | YPTGTG SEQ ID NO1977 | VPGAGV SEQ ID NO1978 |
| VPGGVF SEQ ID NO1979 | GVFYPG SEQ ID NO1980 | VFYPGA SEQ ID NO1981 |
| LGPGGK SEQ ID NO1982 | GPGGKP SEQ ID NO1983 | PGGKPL SEQ ID NO1984 |
| LAGAGL SEQ ID NO1985 | AGAGLG SEQ ID NO1986 | GAGLGA SEQ ID NO1987 |
| LGAFPA SEQ ID N01988 | AFPAVT SEQ ID NO1989 | AVTFPG SEQ ID NO1990 |
| LGVSAG SEQ ID NO1991 | VPGVGL SEQ ID NO1992 | PGVGLP SEQ ID NO1993 |
| FPGVGV SEQ ID NO1994 | KPGAPG SEQ ID NO1995 | PKAPGV SEQ ID NO1493 |
| PGVPLG SEQ ID NO1996 | GYPIKA SEQ ID NO1997 | PKLPGG SEQ ID NO1998 |
| YGPGGV SEQ ID NO1999 | AGYPTG SEQ ID NO2000 | TGVGPQ SEQ ID NO2001 |
| GAGVPG SEQ ID NO2002 | AGVPGV SEQ ID NO2003 | GVPGVP SEQ ID NO2004 |
| IPGIGG SEQ ID NO2005 | IGGIAG SEQ ID NO2006 | GIAGVG SEQ ID NO2007 |
| VPGVGV SEQ ID NO2008 | VPVGVA SEQ ID NO2009 | VPGAGI SEQ ID NO2010 |
| AVPGVV SEQ ID NO2011 | VPGVVS SEQ ID NO2012 | YGARPG SEQ ID NO2013 |
| GVGAGG SEQ ID NO2014 | VGAGGF SEQ ID NO2015 | VGVGGI SEQ ID NO2016 |
| FGLVPG SEQ ID NO2017 | GLVPGV SEQ ID NO2018 | LVPGVG SEQ ID NO2019 |
| PGVGLA SEQ ID NO2020 | GVGLAP SEQ ID NO2021 | VGLAPG SEQ ID NO2022 |
| LRAAAG SEQ ID NO2023 | LVGAAG SEQ ID NO2024 | LVPGGP SEQ ID NO2025 |
| GIPGLG SEQ ID NO2026 | LGVGVG SEQ ID NO2027 | VGVPGL SEQ ID NO2028 |
| AAAGLG SEQ ID NO2029 | AVPGVL SEQ ID NO2030 | VLGGLG SEQ ID NO2031 |
| VGIPGG SEQ ID NO2032 | IPGGVV SEQ ID NO2033 | VVGAGP SEQ ID NO2034 |
| GLVGAA SEQ ID NO 2035 | VGAAGL SEQ ID NO2036 | LGGLGV SEQ ID NO2037 |
| GGVLGG SEQ ID NO2038 | GAGQFP SEQ ID NO2039 | AFQFPL SEQ ID NO2040 |
| GVAARP SEQ ID NO2041 | VAARPG SEQ ID NO2042 | RPGFGL SEQ ID NO2043 |
| GVYPGG SEQ ID NO2044 | LPYTTG SEQ ID NO2045 | FGPGVV SEQ ID NO2046 |
| AAVPGV SEQ ID NO2047 | AAGLGG SEQ ID NO2048 | FPGALV SEQ ID NO2049 |
| VPGVLG SEQ ID NO2050 | GQFPLG SEQ ID NO2051 | ALVPGG SEQ ID NO2052 |
| ISPEAQ SEQ ID NO2053 | GVLPGA SEQ ID NO2054 | VGAGVP SEQ ID NO2055 |
| LGALGG SEQ ID NO2056 | VPGVPG SEQ ID NO2057 | GGLGAL SEQ ID NO2058 |
| GKPLKP SEQ ID NO2059 | IAGVGT SEQ ID NO2060 | VGAGPA SEQ ID NO2061 |
| AGLGAG SEQ ID NO2062 | VVGVPG SEQ ID NO2063 | LGVGGL SEQ ID NO2064 |
| VTFPGA SEQ ID NO2065 | AGIPVV SEQ ID NO2066 | FPLGGV SEQ ID NO2067 |
| GLPGVY SEQ ID NO2068 | GARPGV SEQ ID NO2069 | PGFGLS SEQ ID NO2070 |
| GAFAGI SEQ ID NO2071 | VAGVPS SEQ ID NO2072 | GPGVVG SEQ ID NO2073 |
| YTTGKL SEQ ID NO2074 | PGVGVA SEQ ID NO2075 | VGTPAA SEQ ID NO2076 |
| PQAAAA SEQ ID NO2077 | LAPGVG SEQ ID NO2078 | |

or with any of the following sequences at the C-terminal of a peptide:

**Table 26. C-terminal sequences of protease generated peptide fragments of Elastin.**

| **Elastin** | | |
|---|---|---|
| PGGVPG SEQ ID NO2079 | PGAGLG SEQ ID NO2080 | GAGLGA SEQ ID NO1987 |
| GALGGG SEQ ID NO2081 | LGGGAL SEQ ID NO1902 | GGGALG SEQ ID NO2082 |
| GLGAFP SEQ ID NO2083 | LGAFPA SEQ ID NO1988 | LGAGLG SEQ ID NO1908 |
| VPGGVA SEQ ID NO1974 | ADAAAA SEQ ID NO2084 | PGVLGG SEQ ID NO2085 |
| RFPGVG SEQ ID NO1915 | VGVLPG SEQ ID NO2086 | VPTGAG SEQ ID NO2087 |
| PKAPGV SEQ ID NO1493 | GVGPFG SEQ ID NO2088 | VPLGYP SEQ ID NO2089 |
| LPYGYG SEQ ID NO1920 | AAAAAK SEQ ID NO2090 | IPGIGG SEQ ID NO2005 |
| GIAGVG SEQ ID NO2007 | AIPGIG SEQ ID NO1927 | IGGIAG SEQ ID NO2006 |
| AAAAKA SEQ ID NO2091 | VVGVPG SEQ ID NO2063 | GVPGVG SEQ ID NO1933 |
| GVGVPG SEQ ID NO2092 | PVVPGA SEQ ID NO2093 | VVSPEA SEQ ID NO2094 |
| VGGIPT SEQ ID NO1939 | GGIPTY SEQ ID NO2095 | GIPTYG SEQ ID NO2096 |
| GVGVGG SEQ ID NO2097 | GVGGIP SEQ ID NO2098 | VGVPGL SEQ ID NO2028 |
| GFPGFG SEQ ID NO2099 | FGVGVG SEQ ID NO2100 | GVGAGG SEQ ID NO2014 |
| PGVGIS SEQ ID NO2101 | SPEAQA SEQ ID NO2102 | VAPGVG SEQ ID NO1944 |
| PGVGVA SEQ ID NO2075 | GVGVAP SEQ ID NO2103 | APGVGL SEQ ID NO2104 |
| GIGPGG SEQ ID NO2105 | APGIGP SEQ ID NO2106 | VAPGIG SEQ ID NO2107 |
| RAAAGL SEQ ID NO2108 | AAAGLG SEQ ID NO2029 | AAGLGA SEQ ID NO2109 |
| GVPGLG SEQ ID NO2110 | GVPGFG SEQ ID NO2111 | AGVPGL SEQ ID NO2112 |
| VLGGLG SEQ ID NO2031 | VGIPGG SEQ ID NO2032 | GAGPAA SEQ ID NO2113 |
| PAAAAA SEQ ID NO1929 | VPGVGG SEQ ID NO2114 | GLGGLG SEQ ID NO2115 |
| GLGVPG SEQ ID NO1961 | PGVGGL SEQ ID NO2116 | PAAAAK SEQ ID NO2117 |
| RPGFGL SEQ ID NO2043 | GVAARP SEQ ID NO2041 | AARPGF SEQ ID NO2118 |
| LSPIFP SEQ ID NO2119 | AQAAAA SEQ ID NO2120 | GPGIPG SEQ ID NO2121 |
| GPGGVA SEQ ID NO2122 | TPAAAA SEQ ID NO1928 | PGGVAA SEQ ID NO1948 |
| GLGALG SEQ ID NO2123 | LGALGG SEQ ID NO2056 | ALGPGG SEQ ID NO2124 |
| GALGPG SEQ ID NO2125 | VAPVGV SEQ ID NO2126 | KPVPGG SEQ ID NO2127 |
| AFPAVT SEQ ID NO1989 | AVTFPG SEQ ID NO1990 | VTFPGA SEQ ID NO2065 |
| AAAAYK SEQ ID NO2128 | AAKAGA SEQ ID NO2129 | VPQPGA SEQ ID NO2130 |
| PGVPTG SEQ ID NO2131 | GVPTGA SEQ ID NO2132 | AGVKPK SEQ ID NO2133 |
| PIKAPK SEQ ID NO2134 | KLPGGY SEQ ID NO2135 | YGYGPG SEQ ID NO2136 |
| VGTPAA SEQ ID NO2076 | VPGVPG SEQ ID NO2057 | GVGTPA SEQ ID NO1942 |
| GIGGIA SEQ ID NO2137 | GTPAAA SEQ ID NO2138 | AAAAAA SEQ ID NO1930 |
| IPVVPG SEQ ID NO2139 | VGVPGA SEQ ID NO1931 | GVPGAG SEQ ID NO2140 |
| GAGIPG SEQ ID NO1947 | PEAAAK SEQ ID NO2141 | ARPGVG SEQ ID NO1937 |
| PTYGVG SEQ ID NO2142 | TYGVGA SEQ ID NO2143 | YGVGAG SEQ ID NO2144 |
| IPTYGV SEQ ID NO2145 | PGAIPG SEQ ID NO2146 | VGAGGF SEQ ID NO2015 |
| AGGFPG SEQ ID NO2147 | GIPGVA SEQ ID NO2148 | GISPEA SEQ ID NO2149 |
| VGVAPG SEQ ID NO1943 | VGLAPG SEQ ID NO2022 | VPGAPG SEQ ID NO2150 |
| PGVGLA SEQ ID NO2020 | LAPGVG SEQ ID NO2078 | APGVGV SEQ ID NO1973 |
| GVAPGV SEQ ID NO1945 | GGVAAA SEQ ID NO2151 | PGIGPG SEQ ID NO2152 |
| GLGVGG SEQ ID NO2153 | PGLGVG SEQ ID NO2154 | LGVGVG SEQ ID NO2027 |
| GLGVGA SEQ ID NO2155 | ALAAAK SEQ ID NO2156 | LGGLGA SEQ ID NO2157 |
| VGAGPA SEQ ID NO2061 | AGPAAA SEQ ID NO1958 | GPAAAA SEQ ID NO1959 |
| GGLGVG SEQ ID NO2158 | LGVGGL SEQ ID NO2064 | GVGGLG SEQ ID NO2159 |
| AGQFPL SEQ ID NO2160 | GGVAAR SEQ ID NO1966 | VAARPG SEQ ID NO2042 |
| GFGLSP SEQ ID NO2161 | PIFPGG SEQ ID NO2162 | IFPGGA SEQ ID NO2163 |
| AAKFGA SEQ ID NO2164 | AAKYGA SEQ ID NO2165 | AAKAAK SEQ ID NO2166 |
| AGLGAL SEQ ID NO2167 | GAGVPG SEQ ID NO2002 | GIPGGV SEQ ID NO2168 |
| LKPVPG SEQ ID NO1905 | PGVGVG SEQ ID NO2169 | IPPAAA SEQ ID NO2170 |
| ALVPGG SEQ ID NO2052 | RPGVGV SEQ ID NO1938 | ARPGFG SEQ ID NO2171 |
| VLPGAR SEQ ID NO2172 | GGFPGF SEQ ID NO2173 | GLSPIF SEQ ID NO2174 |
| PTGAGV SEQ ID NO2175 | VGGVPG SEQ ID NO1941 | GIPVVP SEQ ID NO1934 |
| AGAAGK SEQ ID NO2176 | | |

### Vimentin

Several candidate proteases may be responsible for the digestion of vimentin in fibrotic tissue We have through a range of in vitro cleavages of pure native proteins determined that the enzymes listed in the following table cleaved vimentin at least at the cleavage sites at each end of the following sequences or at the cleavage sites marked '.' or where no '.' is shown, at the ends of the sequences:

**Table 27: Vimentin fragments generated by specific proteases.**

| **Protease** | **Sequence between cleavage sites** | **Aminoacid residue numbers*** |
|---|---|---|
| MMP2, MMP8, Trypsin | RLRS**SVPGVR**. SEQ ID NO2177 | 69-78 |
| MMP2, MMP8, Trypsin | RLRS**SVPGVL**. SEQ ID NO2178 | 69-78 |
| MMP2, MMP8, Trypsin | .**LLQDSV**DFSL SEQ ID NO2179 | 79-89 |
| MMP2, MMP8, Trypsin | **.FADLSE**AANR SEQ ID NO2180 | 295-304 |
| MMP2 | **.ISLPLP**TFSS SEQ ID NO2181 | 410-420 |

| | | |
|---|---|---|
| * **in the human vimentin sequence** | | |

Accordingly, in a method of the invention, said peptide fragments preferably comprise a neo-epitope formed by cleavage of vimentin by a protease at an N- or C-terminal site, or where indicated a site marked by the sign · in any one of the partial sequences of vimentin in Table 24.

The immunological binding partner may be one specifically reactive with a C-terminal or N-terminal neo-epitope formed by cleavage of vimentin.

Suitable immunological binding partners may therefore be specifically reactive with any of the following sequences at the N terminal of a peptide:

**Table 28. N-terminal sequences of protease generated peptide fragments of vimentin.**

| **Vimentin** | | |
|---|---|---|
| LLQDSV SEQ ID NO2182 | FADLSE SEQ ID NO2183 | ISLPLP SEQ ID NO2184 |

or with any of the following sequences at the C-terminal of a peptide:

**Table 29. C-terminal sequences of protease generated peptide fragments of vimentin.**

| **Vimentin** | |
|---|---|
| SVPGVR SEQ ID NO2185 | SVPGVL SEQ ID NO2186 |

Further cleavage sites defining neo-epitopes that may be assayed in a similar manner can be identified by exposing collagens, elastin, CRP and proteoglycans or other fibrotic tissue proteins to any of the enzymes described herein and isolating and sequencing peptides thereby produced. Furthermore, assays may be based on the neo-epitopes generated adjacent the illustrated cleavage sites, i.e. in the C-terminal sequences that lead up to the N-terminal epitopes given above and the N-terminal sequences that connect to the C-terminal epitopes described.

Assays for more than one of the peptides described above may be conducted separately and their results combined or more than one of the peptides described above may be measured together.

The result of an assay according to the invention may be combined with one or more other measured biomarkers to form a composite index of diagnostic or prognostic value.

Generally, all previously known immunoassay formats can be used in accordance with this invention including heterogeneous and homogeneous formats, sandwich assays, competition assays, enzyme linked assays, radio-immune assays and the like. Thus, optionally, said method is conducted as a competition immunoassay in which said immunological binding partner and a competition agent are incubated in the presence of said sample and the competition agent competes with the peptide fragments in the sample to bind to the immunological binding partner.

Said competition agent may be (1) a synthetic peptide derived from the sequence of collagen type I, III, IV, V, or VI, or from CRP, or from any of the proteoglycans versican, lumican, perlecan, decorin and biglycan peptide, or a competition agent derived from (2) a purified native collagen type I, III, IV, V, or VI, or CRP, or any of the proteoglycans neurocan, brevican, fibromodulin, serglycins, syndecan, betaglycan, versican, lumican, perlecan, decorin and biglycan cleaved by proteases to reveal said neo-epitope.

One suitable method could be a competition immunoassay using monoclonal antibodies or antibody binding fragments binding to neo-epitopes of collagen type I, III, IV, V, VI, CRP, vimentin, or any of the proteoglycans neurocan, brevican, fibromodulin, serglycins, syndecan, betaglycan, versican, lumican, decorin, perlecan and biglycan fragments or neo-epitopes on peptide fragments from other proteins derived from fibrotic tissue. Appropriately selected synthetic peptides coated onto the solid surface of a microtitre plate could compete with the sample for binding to the monoclonal antibodies or binding fragments. Alternatively, purified, native collagen type I, III, IV, V, VI, CRP, neurocan, brevican, fibromodulin, serglycins, syndecan, betaglycan, versican, lumican, decorin, perlecan and biglycan fragments carrying the neo-epitope recognised by the monoclonal antibody or binding fragment could be used on the solid surface. Yet another alternative is to immobilise the monoclonal antibody or binding fragment on the solid surface and then co-incubate the sample with a synthetic peptide appropriately linked to a signal molecule, e.g. horseradish peroxidase or biotin.

The sample may be a sample of serum, blood, plasma or other, e.g. fibrotic tissue biopsy.

Assays may be conducted as sandwich assays using a first immunological binding partner specifically reactive with a said neo-epitope and a second immunological binding partner reactive with the relevant protein to which the neo-epitope belongs. Optionally, said second immunological binding partner is directed to a second neo-epitope of the same protein.

In certain preferred methods the method further comprises comparing the determined level of said binding of said peptide fragments with values characteristic of (a) comparable healthy individuals and/or (b) a pathological fibrotic condition and optionally associating a higher level of the measured peptide (normally indicated by a higher level of binding) with a more severe degree of a said condition.

An aspect of the present invention relates to the development of monoclonal antibodies recognising neo-epitopes as described above, especially for collagen types I and IV. This can be achieved by immunising mice with synthetic peptides originating from the amino acid sequence of collagen type I, III, IV, V, VI, CRP, vimentin, neurocan, brevican, fibromodulin, serglycins, syndecan, betaglycan, versican, lumican, decorin, perlecan and biglycan molecules (including the sequences listed above or sequences terminating therein), fusing the spleen-cells from selected mice to myeloma cells, and testing the monoclonal antibodies for binding to neo-epitopes on relevant synthetic peptides. Specificity for neo-epitopes can be ensured by requiring reactivity with a synthetic peptide and a lack of reactivity with either a C-prolongated form of the immunising peptide (for a C-terminal neo-epitope) or an N-terminal prolongated form of the immunising peptide (for an N-terminal neo-epitope). Antibodies for neo-epitopes may also be evaluated to establish a lack of binding capacity to native collagen type I, III, IV, V, VI, CRP, vimentin, neurocan, brevican, fibromodulin, serglycins, syndecan, betaglycan, versican, lumican, decorin, pelecan and biglycan. Alternatively, specificity for a neo-epitope can be ensured by requiring the reactivity of the antibody to be negatively dependent on the presence of biotin or other functional groups covalently linked to one of the terminal amino acids.

The invention includes an immunological binding partner which is specifically immunoreactive with a neo-epitope formed by cleavage of collagen type I, III, IV, V, VI, CRP, vimentin, neurocan, brevican, fibromodulin, serglycins, syndecan, betaglycan, versican, lumican, decorin, perlecan and biglycan by a protease at a end-site in any one of the partial sequences of collagen type I, III, IV, V, VI, CRP, vimentin, neurocan, brevican, fibromodulin, serglycins, syndecan, betaglycan, versican, lumican, decorin, perlecan and biglycan set out above, and may be for instance a monoclonal antibody or a binding fragment thereof.

The invention includes a cell line producing a monoclonal antibody against a C-terminal or N-terminal neo-epitope formed by cleavage of collagen type I, III, IV, V, VI, CRP, vimentin, neurocan, brevican, fibromodulin, serglycins, syndecan, betaglycan, versican, lumican, decorin, perlecan and biglycan at the end-sites of sequences in any one of the partial sequences of collagen type I, III, IV, V, VI, CRP, neurocan, brevican, fibromodulin, serglycins, syndecan, betaglycan, versican, lumican, decorin, perlecan and biglycan set out above.

The invention further provides a peptide comprising a C-terminal or N-terminal neo-epitope formed by cleavage of collagen type I, III, IV, V, VI, CRP, vimentin, neurocan, brevican, fibromodulin, serglycins, syndecan, betaglycan, versican, lumican, decorin, perlecan and biglycan in any one of the partial sequences of these proteins set out above. Such a peptide may be conjugated as a hapten to a carrier for producing an immune response to said peptide, or immobilised to a solid surface or conjugated to a detectable marker for use in an immunoassay.

The invention further comprises an isolated nucleic acid molecule coding for a peptide comprising a C-terminal or N-terminal neo-epitope formed by cleavage of collagen type I, III, IV, V, VI, CRP, vimentin, neurocan, brevican, fibromodulin, serglycins, syndecan, betaglycan, versican, lumican, decorin, perlecan and biglycan in any one of the partial sequences of collagen type I, III, IV, V, VI, CRP, vimentin, neurocan, brevican, fibromodulin, serglycins, syndecan, betaglycan, versican, lumican, decorin, perlecan and biglycan set out above.

The invention further comprises a vector comprising a nucleic acid sequence comprising an expression signal and a coding sequence which codes for the expression of a peptide comprising a C-terminal or N-terminal neo-epitope formed by cleavage of collagen type I, III, IV, V, VI, CRP, vimentin, versican, lumican, decorin, perlecan and biglycan in any one of the partial sequences of collagen type I, III, IV, V, VI, CRP, vimentin, neurocan, brevican, fibromodulin, serglycins, syndecan, betaglycan, versican, lumican, decorin, perlecan and biglycan set out above and further includes a host cell transformed with such a vector and expressing a said peptide.

Yet another aspect of the invention relates to kits, which can be used conveniently for carrying out the methods described above. Such kits may include (1) a microtitre plate coated with synthetic peptide; (2) a monoclonal antibody or antibody binding fragment of the invention reactive with said synthetic peptide; and (3) a labelled anti-mouse IgG immunoglobulin. Alternatively, such kits may include (1) a microtitre plate coated with purified native collagen type I, III, IV, V, VI, CRP, vimentin, versican, lumican, decorin, perlecan and biglycan fragments; (2) a monoclonal antibody recognising a neo-epitope on collagen type I, III, IV, V, VI, CRP, vimentin, neurocan, brevican, fibromodulin, serglycins, syndecan, betaglycan, versican, lumican, decorin, perlecan and biglycan fragments and reactive with said purified collagen type I, III, IV, V, VI, CRP, vimentin, neurocan, brevican, fibromodulin, serglycins, syndecan, betaglycan, versican, lumican, decorin, perlecan, and biglycan fragments; and (3) a labelled anti-mouse IgG immunoglobulin. Alternatively, such kits may include (1) a microtitre plate coated with streptavidin; (2) a synthetic peptide linked to biotin; (3) a monoclonal antibody recognising a neo-epitope on collagen type I, III, IV, V, VI, CRP, vimentin, neurocan, brevican, fibromodulin, serglycins, syndecan, betaglycan, versican, lumican, decorin, perlecan and biglycan fragments and reactive with said synthetic peptide; and (4) a labelled anti-mouse IgG immunoglobulin. Yet another alternative could be kits including (1) a microtitre plate coated with streptavidin; (2) a synthetic peptide linked to biotin; (3) a monoclonal antibody recognising a neo-epitope on collagen type I, III, IV, V, VI, CRP, vimentin, neurocan, brevican, fibromodulin, serglycins, syndecan, betaglycan, versican, lumican, decorin, perlecan and biglycan fragments (and reactive with said synthetic peptide) and conjugated to horseradish peroxidase.

Thus, the invention includes an immunoassay kit comprising an immunological binding partner as described herein, especially in respect of collagens types I and IV, and a competition agent which binds said immunological binding partner, and optionally one or more of a wash reagent, a buffer, a stopping reagent, an enzyme label, an enzyme label substrate, calibration standards, an anti-mouse antibody and instructions.

The assays described herein are useful in the diagnosis of fibrosis in patients. In addition, the tests are useful for the assessment of disease progression, and the monitoring of response to therapy. The immunological binding partners of the invention may also be used in immunostaining to show the presence or location of collagen type I, III, IV, V, VI, CRP, vimentin,neurocan, brevican, fibromodulin, serglycins, syndecan, betaglycan, versican, lumican, decorin, perlecan and biglycan cleavage products.

The invention will be further described and illustrated with reference to the following examples and the accompanying drawings, in which:
Figure 1 shows a graph showing CO3 ELISA results of different biological samples: Pooled human serum samples (Serum); Human amniotic fluid (AF); Human fibroblast culture media (Fibr. Cltr.);
Figure 2a shows a graph showing CO3 serum levels in sham operated (s) and bile duct ligated rats at baseline (b) and at termination (t);
Figure 2b shows the corresponding delta-values of CO3 in rat serum: Termination levels - Baseline levels;
Figure 3 shows a graph showing CO3 levels in different human serum samples. Normal serum: from healthy individuals. COPD: Chronic Obstructed Pulmonary Disease (leading to lung fibrosis).
   Scleroderma (leading to skin and lung fibrosis). HCV: Hepatitis virus C (leading to liver fibrosis);
Figure 4 shows Liver weights and Liver scores determined in Example 5;
Figure 5 shows levels of MMP-9 cleavage fragments of Type III collagen measured according to the invention in Example 5;
Figure 6 shows levels of Type III collagen gene expression in BDL or sham-operated rats determined in Example 5;
Figure 7 shows changes of expression levels of the MMP-9 cleavage fragment of Type III collagen reactive with the antibody used in Example 5 as determined by Western blot;
Figure 8 shows the results of histology staining of liver sections obtained in Example 5;
Figure 9 shows correlations between measurements of fragments of Type III collagen according to the invention with other liver biomarkers as determined in Example 5;
Figure 10 shows results obtained on human serum samples in Example 6; and
Figure 11 shows results obtained in testing the reactivity of a monoclonal antibody recognising an N-terminal neo-epitope from CRP.
Figure 12 shows collagen accumulation in rat liver measured in Example 8.
Figure 13 shows immunoassay results obtained in Example 8.
Figure 14 shows the correlation of the immunoassay results of Figure 13 with liver collagen content.
Figure 15 shows a comparison of the results of an immunoassay according to the invention with measurements of hyaluronic acid and of Sirius red staining in Example 8.
Figure 16 shows in the first panel the correlation of results from the immunoassay according to the invention with Sirius red staining and in the second panel the correlation between hyaluronic acid levels and Sirius red staining.
Figure 17 shows the lack of correlation between the results of the immunoassay of the invention and hyaluronic acid levels.
Figure 18 shows skin sections and skin thickness measurements described in Example 9.
Figure 19 shows results from an immunoassay according to the invention in Exmaple 9.
Figure 20 shows in panel A Western Blot images obtained in Example 9 and in panel B corresponding immunoassay results according to the invention.
Figure 21 shows a correlation between immunoassay results and skin thickness measurements.
Figure 22 shows a correlation between urine immunoassay results and Western blot measurements described in Example 9.

### Example 1: Collagen type III degraded with MMP-9

### Method

Cleavage: Collagen type III isolated from human placenta was dissolved in 10 mM acetic acid (1 mg/ml). The protein solution was then passed through a filter (Microcon Ultracel YM-10) to remove fragment contaminations. MMP-9 was preactivated with 4-aminophenylmercuric acetate (APMA, Sigma) at 37°C for 3 hours. After activations, collagen type III and MMP-9 were mixed 100:1 and incubated shaking for 3 days at 37°C.

The solution was analyzed by liquid chromatography/mass spectrometry (LC/MS) and the fragments were identified by performing Mascot Search. The peptide sequences were selected by homology search, ensuring no cross-reactivity to other or related proteins, as well as interspecies cross-reactivity.

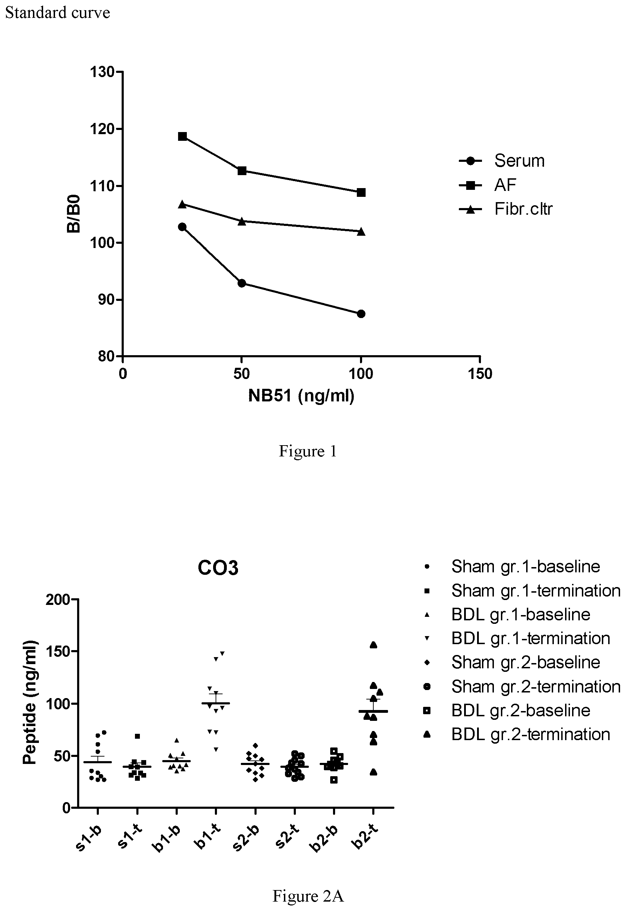

Antibody design: The peptide sequences were synthesized and conjugated to ovalbumin (OVA). Mice were immunized ever 2-3 weeks, up to five. Antibody titers were checked by screening peptides, both selection and de-selection. When sufficient antibody titers were achieved, positive mice were selected for fusion, euthanized, and the spleen was disintegrated and B-cells were removed for fusion with myeloma cells. Selections of antibody producing cells were done by culturing and re-seeding the surviving chimera cells in single cell clones. Clones are selected by selection and de-selection peptides followed by native reactivity testing (fig. 1), as neoepitopes are generated by synthetic small peptide sequences, which may not reflect the native proteins. An IgG subtype clone is selected for antibody production. Antibody purification is done by protein-G column.

Assay development: Optimal antibody concentrations are determined by checker-board analysis, with dilutions of antibody coating and screening peptide, in competitions ELISA. The different determination for the collagen degraded by MMP-9 (CO3) assay is shown in table 24.

**Table 24. Limit of Detection, Avarage Inter- and Intraassay variation of the CO3 assay.**

| Limit of Detection | 0.5 ng/ml |
|---|---|
| Average Interassay variation | 3.71 % |
| Average Intraassay variation | 5.48 % |

### Example 2: CO3 in biological relevant samples:

CO3 levels in bile duct ligated rats compared to sham operated rats.

Method: Forty female Sprague-Dawley rats (6 months old) were housed at the animal research facilities at Nordic Bioscience. The experiments were approved by the Experimental Animal Committee of the Danish Ministry of Justice, and were performed according to the European Standard for Good Clinical Practice (2008/561-1450). The rats were housed in standard type III-H cages at 18-22°C with bedding and nest material (Altromin 1324; Altromin, Lage, Germany) and purified water (Milli-Q system; Millipore, Glostrup, Denmark) ad libitum. Rats were kept under conditions of a 12-hour light/dark cycle.

Liver fibrosis was induced by common BDL. In short: The rat was anaesthetized, the bile duct found, two ligations were performed around the bile duct followed by dissection between the ligations, the abdomen was closed. In sham operated rats, the abdomen was closed without bile duct ligation.

The rats were divided into 2 groups: Group 1(10 BDL and 10 sham operated rats) were sacrificed after 2 weeks, and Group 2 (9 BDL and 10 sham operated rats) were sacrificed after 4 weeks. On completion of the study period (2, or 4 weeks), after at least 14 hours fasting, all surviving animals were asphyxiated by CO₂ and sacrificed by exsanguinations.

Blood samples were taken from the retro-orbital sinus of at least 14 hours fasting rats under light CO₂/O₂ anaesthesia at baseline and at termination. The blood were collected and left 30 minutes at room temperature to cloth, followed by centrifugation at 1500 *g* for 10 minutes. All clot-free liquid were transferred to new tubes and centrifuged again at 1500 *g* for 10 minutes. The serum were then transferred to clean tubes and stored at -80°C.

CO3 were measured in x 5 diluted serum samples from the rats. Sham and BDL levels were compared by Mann-Whitneys two-tailed nonparametric test (α=0.05) of statistical significance assuming normal distribution.

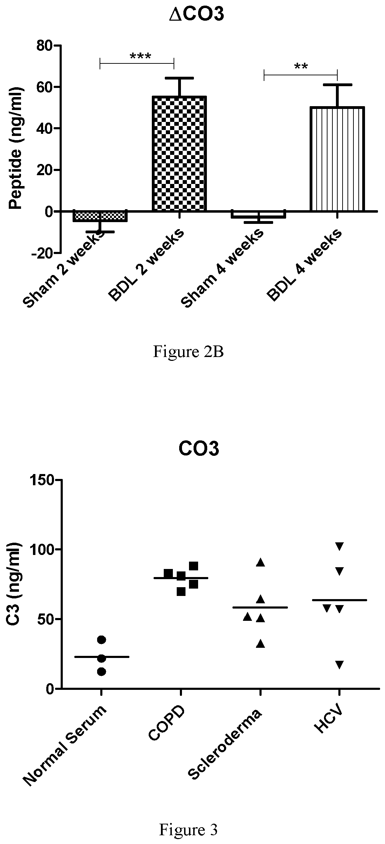

CO3 levels increased significantly in the BDL groups compared to the Sham-operated animals. The results are shown in Figure 2a and b.

### Example 3:

### CO3 in different fibrotic diseases (human serum)

CO3 levels were measured in serum from human with three different fibrotic diseases: Chronic obstructed pulmonary disease (COPD), Scleroderma, and Hepatitis virus C (HCV). The serum samples were retrieved from Sera Laboratories International Ltd (SLI Ltd), UK.

### CO3 levels were increased in the three different fibrotic diseases (Figure 3)

### Example 4:

### Antibody development - detection of marker C03-610C

Type III collagen (Abcam, Cambridge, UK) was degraded in vitro by activated MMP-9 (Merck KGaA, Darmstadt, Germany) for 2 days. Degradation fragments were sequenced by LS-MS/MS and identified by MASCOT search. A specific peptide sequence ⁶¹⁰KNGETGPQ was selected for antibody production. The N-terminal of this sequence is residue 610 of human collagen type III. The synthetic peptide was conjugated to ovalbumin prior to subcutaneous immunization of 4-6 week old Balb/C mice with about 200µE emulsified antigen and 50µg C03-610C (KNGETGPQGPGGC-OVA). Consecutive immunizations were performed at two week intervals until stable sera titer levels were reached in Freund's incomplete adjuvant. The mice were bled from the second immunization on. At each bleeding, the serum titer was measured and the mouse with highest anti-serum titer was selected for fusion. After the fourth immunization, this mouse was rested for one month and then boosted intravenously with 50µg C03-610C in 100µL 0.9% sodium chloride solution three days before isolation of the spleen for cell fusion.

Monoclonal antibody producing clones were selected using a) immunogenic peptide: KNGETGPQGP-GGC-Ovalbumine (OVA) (807678), b) screening peptide KNGETGPQGP-PG-K-Biotin (807971), c) de-selection peptides KDGETGAAGPPGK-Biotin (118318) representing a type II collagen alpha 1 chain, KDGEAGAQGPPGK-Biotin representing a type I collagen alpha 1 chain degradation product, purchased from the Chinese Peptide Company, Beijing, China. The ELISA coat plate was obtained from NUNC (Thermofisher, Copenhagen, Denmark). Peptide conjugation reagents and buffers were produced by Pierce (Thermofisher, Copenhagen, Denmark).

Buffer used for dissolving the coating peptide was composed of the following: 40 mM Na₂HPO₄, 12 H₂O, 7 mM KH₂PO₄, 137 mM NaCl, 2,7 mM KCl, 25 mM EDTA, 0,1 % Tween 20, 1 % BSA, 10 % sorbitol, pH 7. For a serum assay, buffer containing the following chemicals was used: 8 mM Na₂HPO₄, 12 H₂O, 1,5 mM KH₂PO₄, 13,7 mM NaCl, 2,7 mM KCl, 0,1 % Tween 20, 1 % BSA, 0,003 % phenol red, pH 7,4. A different buffer used for a urine assay contained 400 mM TRIZMA, 0,05 % Tween 20, 0,1 % BSA, 0,36 % Bronidox L5, pH 8,0. For both serum and urine assays we used a washing buffer composed of 25mM TRIZMA, 50 mM NaCl, 0,036 % Bronidox L5, 0,1 % Tween 20, and reaction-stopping buffer composed of 0,1 % H₂SO₄. ELISA-plates used for the assay development were Streptavidin-coated from Roche (Hvidovre, Denmark) cat.: 11940279. All ELISA plates were analyzed with the ELISA reader from Molecular Devices, SpectraMax M, (CA. USA).

In preliminary experiments, we optimized the reagents, their concentrations and the incubation periods by performing several checkerboard analyses. A 96-well ELISA plate coated with streptavidin was further coated with 5 ng/ml of the synthetic peptide KNGETGPQGP-Biotinylated dissolved in PBS-TBE buffer at 20°C for 30 minutes by constant shaking at 300 rpm. After washing with washing buffer, 20µl of sample was added, followed by 100µl of peroxidase conjugated anti-human mAb-NB51-32 C03-610C solution (23 pg/ml in incubation buffer). The plate was incubated for 1 hour at 20°C during which time it was shaken at 300 rpm. This was followed by washing and finally, 100µl tetramethylbenzinidine (TMB) (Kem-En-Tec cat.438OH) was dispensed and the plate incubated for 15 minutes in darkness and shaken at 300 rpm. In order to cease the reaction, 100µl of stopping solution was added and the plate analyzed in the ELISA reader at 450nm with 650nm as reference.

A standard curve was performed by serial dilution of biotinylated-NB51-32 C03-610C for a serum assay, and biotinylated-NB51-134 C03-610C for a urine assay. Standard concentrations were 0, 0.33, 1, 3, 9, 27, 81 and 162 ng/ml.

We designate fragments detected using the immunoassays so obtained as CO3-610C as the amino acid K at the N-terminal of the sequence KNGETGPQGP is amino acid 610 of the human collagen III sequence.

### Example 5

### Comparison of C03-610C and other biomarkers in induced liver fibrosis in rats

### Animals

40 female Sprague-Dawley rats aged 6 months were housed at the animal research facilities at Nordic Bioscience, Copenhagen, Denmark. The experiments were approved by the Experimental Animal Committee of the Danish Ministry of Justice and were performed according to the European Standard for Good Clinical Practice (2008/561-1450). The rats were housed in standard type III-H cages at 18-22°C with bedding and nest material (Altromin 1324; Altromin, Lage, Germany) and water ad libitum. Rats were kept under conditions of a 12-hour light/dark cycle.

### Study design

In 20 rats, liver fibrosis was induced by common BDL. The surgical procedure was performed under sterile conditions. The rat was anaesthetized, the bile duct localized and ligated in two places followed by dissection between the ligations, and the abdomen was closed. The other 20 rats were subjected to a sham operation, in which the abdomen was closed without bile duct ligation. The rats were then divided into 2 groups: Group 1 (10 BDL rats and 10 sham-operated rats) was sacrificed after 2 weeks and Group 2 (10 BDL and 10 sham-operated rats) was sacrificed after 4 weeks. On completion of the study period (2 or 4 weeks), after at least 14 hours fasting, all surviving animals were asphyxiated by CO₂ and sacrificed by exsanguinations.

### Blood sampling

Blood samples were taken from the retro-orbital sinus of rats after at least 14 hours fasting, under light CO₂/O₂ anaesthesia, at baseline and at termination. Blood was left 30 minutes at room temperature to clot, followed by centrifugation at 1500 g for 10 minutes. All clot-free liquid was transferred to fresh tubes and centrifuged again at 1500 g for 10 minutes. The serum was then transferred to clean tubes and stored at -80°C.

### Tissue handling

After the rats were put down, their livers were carefully dissected, weighed, fixed in 4% formaldehyde for a minimum of 24 hours, cut into appropriate slices and embedded in paraffin. Sections 5µm thick were cut, mounted on glass slides and stained with Sirius Red. The liver sections were evaluated histologically by assessment of the architecture, presence of inflammation, proliferation of bile ducts and fibrosis. The de novo bile duct formation in the parenchyma was evaluated semi-quantitatively using the following scoring system: normal = 0, mild changes (1/3 or less of the lobule affected) = 1, moderate changes (between 1/3 and 2/3 of the lobule affected) = 2, and severe changes (2/3 or more of the lobule affected) = 3. Digital photographs were captured using an Olympus BX60 microscope with x 40 and x 100 magnification and an Olympus 5050-zoom digital camera (Olympus, Tokyo, Japan).

### Determination of total collagen and serum CTX-II

The total collagen concentration was assayed using the commercial QuickZyme Collagen Assay (QuickZyme Bioscience, Leiden, The Netherlands). The concentration of CTX-II was assayed using the commercial Rat CTX-II kit (IDS Nordic, Herlev, Denmark). All samples were assayed in duplicate.

### Type III collagen mRNA quantification

The number of transcripts of type III collagen (Col3a1) in liver tissue samples was determined by quantitative real-time polymerase chain reaction (RT-PCR) using fluorescent reporter probes. The number of Col3a1 copies in the sample was extrapolated from a standard curve obtained using Col3a1 plasmid cDNA Image Clone 7097081 (Geneservice, Cambridge, UK) as dilution standard. Amounts of Col3a1 were normalized with those of housekeeping gene hypoxanthine phosphoribosyltransferase 1 (Hprt1). Primers and probes for Col3a1 and Hprt1 mRNAs were designed using NCBI Reference Sequences NM_032085.1 and NM_012583.2 as templates, respectively (TIB Molbiol GmbH, Berlin, Gemany). Total RNA was extracted from frozen liver samples using Absolutely RNA Miniprep kit (Stratagene, La Jolla, CA, USA) following the manufacturer's instructions and its quality assessed in RNA Nano chips using a 2100 Bioanalyzer instrument (Agilent Technologies, Santa Clara, CA, USA). Immediately after RNA isolation, complementary DNA (cDNA) was synthesised with Transcriptor First Strand cDNA Synthesis kit (Roche, Basel, Switzerland) using 1µg of RNA as the template. For each sample tested, a cDNA synthesis negative control, omitting reverse transcriptase enzyme from the reaction mix, was included. Separate PCR reactions for Col3a1 and Hprt1 were perfomed in a 20µL format using the Lightcycler Faststart DNA Master Plus Hybprobe kit (Roche) according to the manufacturer's instructions. Real time fluorescence data was collected in a Lightcycler 2.0 instrument (Roche).

### Extractions

Tissue was pulverized in excess of liquid nitrogen in a steel mortar. Samples were then transferred into a 1.5 ml eppendorf tube and left shaking overnight at 4 ⁰C in 0.5M Acetic Acid solution containing protease inhibitor cocktail (Roche). The samples were then sonicated with ultrasounds using 5 pulses at 60% amplitude (U50 control, IKA Labortechnik) and left for an additional 2 hours at 4 ⁰C after which they were centrifuged for 5 minutes at 13,000 rpm. Supernatant was carefully removed, transferred in a new eppendorf and stored at -80 ⁰C.

### Densitometry

Densitometry measurements were performed using UN-SCAN-IT Version 6.1 from Silk Scientific (give city, country).

### Histology image analysis

Histology sections stained with Sirius Red were analyzed using Visiopharm software Version 3.2.8.0 (give city, country). Images were acquired using Pixelink PL-A623C microscope digital camera.

### SDS PAGE and Western blots

20 µg of tissue extract was mixed with loading buffer (Invitrogen LDS 4x, NP0007) containing the reducing agent (NuPAGE, NP0004 from Invitrogen). Samples were then loaded into 4-12% Bis-Tris gradient gel (NP0332BOX from Invitrogen) and run for 52 minutes at 200V. Proteins were then transferred onto a nitrocellulose membrane using the i-Blot transfer system (Invitrogen), blocked with 5% milk in (? Need to spell out?) TTBS overnight at 4 degrees. Beta Actin antibody (AbCam ab8229, give company, city country?) was used as a loading control.

### Statistical analysis

Mean values and standard error of the mean (SEM) were calculated using GraphPad Prism (GraphPad Software, Inc., San Diego, CA, USA) and compared by Student's two-tailed paired t-test (α=0.05) of statistical significance assuming normal distribution, or by Mann-Whitney two-tailed non-parametric test (α=0.05). The coefficient of correlation (R²) and the corresponding p-value was determined by linear regression.

### Results

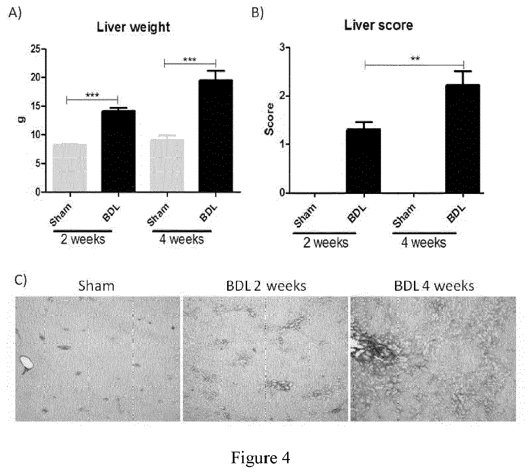

*Liver appearance:* At the time of sacrifice, livers of control animals showed normal gross morphology while livers of BDL animals were enlarged. The liver weights were significantly increased in BDL rats compared to the sham-operated controls (mean weights at sacrifice: 2 weeks post-surgery, sham 8.1 g; BDL 14.1 g; 4 weeks post-surgery, sham 9.0 g; BDL 19.4 g) (Figure 4 panel A). Semi-quantitative scoring of liver sections using the 0-3 scale showed significantly more structural changes of the liver at 4 weeks compared with 2 weeks (Figure 4 panel B). Figure 4, panel A shows liver weight in bile duct ligation (BDL)- or sham-operated rats. Data are shown as mean + SEM.[*** , P<0.0001. Panel B shows scoring of the structural changes in the liver of each group. Data are shown as mean + SEM. **, P=0.0094. Panel C shows Sirius Red photomicrographs showing the hepatic structure in sham-operated rats, and in BDL rats 2 and 4 weeks post-surgery. The hepatic structure around the portal tract is clearly disrupted in BDL rats compared with the sham-operated rats. Collagens are highlighted in red. Original magnification was x40.

Under histological examination, the livers of sham-operated animals showed no sign of fibrosis and were microscopically normal (Figure 4C). In the BDL livers, a marked ductal proliferation was observed. In the 2-week post-surgery group, the proliferation was located around the portal tract while in the 4-week group the proliferation had spread (Figure 4C). Collagen deposition was found around the ductular structures. Inflammation was minimal and confined to the portal tracts. No signs of cholestasis were seen, whether intracellular cholestasis, bile plugs, bile infarctions or hepatocytic rosette formation.

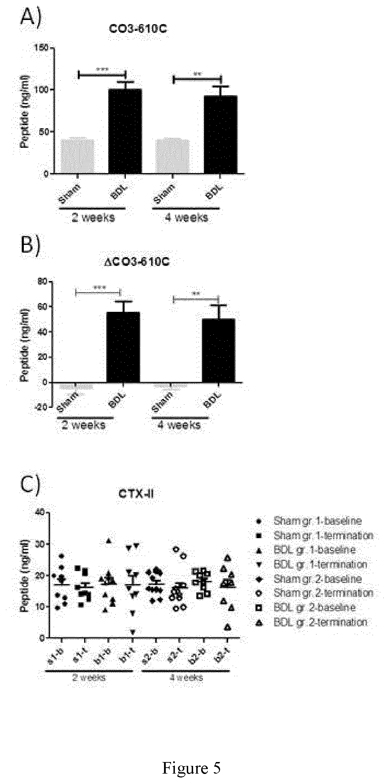

*Changes in CO3-610C levels:* Figure 5 shows in panel A MMP-9 mediated CO3 degradation serum levels in bile duct ligated (BDL)- or sham-operated rats. Data are shown as mean + standard error of mean. 2 weeks post-surgery *** P<0.0001 and 4 weeks post-surgery ** P=0.0014. In panel B are shown C03-610C delta values (termination-baseline paired), 2 weeks post-surgery P<0.0001 and 4 weeks post-surgery P=0.0016. In panel C are shown CTX-II levels in BDL- or sham-operated rats. Data are shown as mean + standard error of mean.

In the BDL groups C03-610C levels increased significantly compared to sham groups (mean values: 2 weeks,post-surgery sham 39.7 ng/ml, BDL 100.3 ng/ml; average increase between the groups was 1530; 4 weeks post-surgery, sham 39.7, BDL 92.6 ng/ml, average increase between the groups was 1330) (Figure 5 panels A and B). There were no changes in the sham groups. CTX-II levels indicating collagen type II degradation did not change in the sham or BDL groups (Figure 5 panel C).

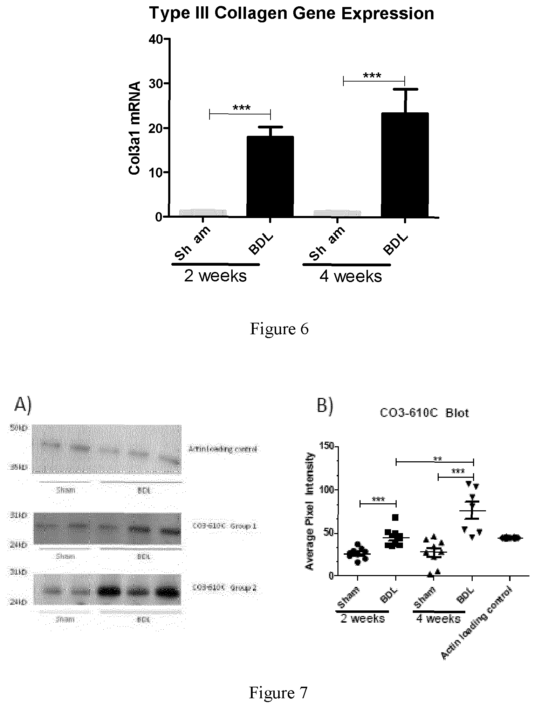

*Type III Collagen Gene Expression:* Figure 6 shows Type III collagen gene expression in BDL or sham-operated rats. Data are shown as mean + standard of mean; 2 weeks post-surgery P<0.0001 and 4 weeks post-surgery P=0.0006
Type III collagen a1 chain mRNA increased significantly in both BDL groups compared with sham-operated rats.

*Western Blot and Densitometry:* Figure 7 shows changes in the expression of C03-610C in the liver of rats in BDL- and sham-operated groups assessed by A) Western blot 2 and 4 weeks post-surgery and B) Bands from western blot quantified by densitometry.

Western blot analysis showed very low levels of C03-610C in sham-operated rats (Figure 7 panel A). At and after 2 weeks post-surgery C03-610C levels prominently increased (Figure 7 panel A). Results were quantified by densitometry analysis (Figure 7 panel B) .

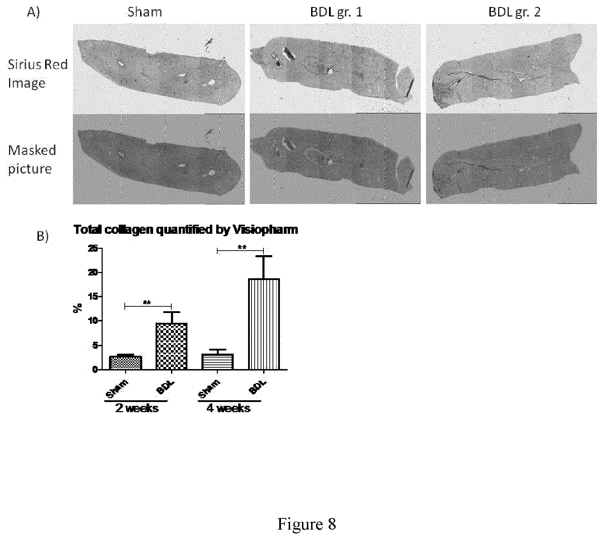

*Histology image analysis:* Figure 8 panel A shows in the top row histology sections from BDL- or sham- operated rats stained with Sirius Red. The bottom row shows masked histology sections for quantifying total collagen content (red colour) in the liver. Panel B shows total collagen quantified by Visiopharm software - 2 weeks post-surgery P=0.0081; 4 weeks post-surgery P=0.0047

Histology sections stained with Sirius Red and enhanced using Visiopharm software showed increasing collagen content over time in BDL-operated rats. (Figure 8 panel A). The red color in the mask representing collagen was quantified using the same software (Figure 8 panel B) and confirmed a significant increase in total collagen content in BDL-operated rats compared with sham-operated rats (2 weeks post-surgery P=0.0081; 4 weeks post-surgery P=0.0047).

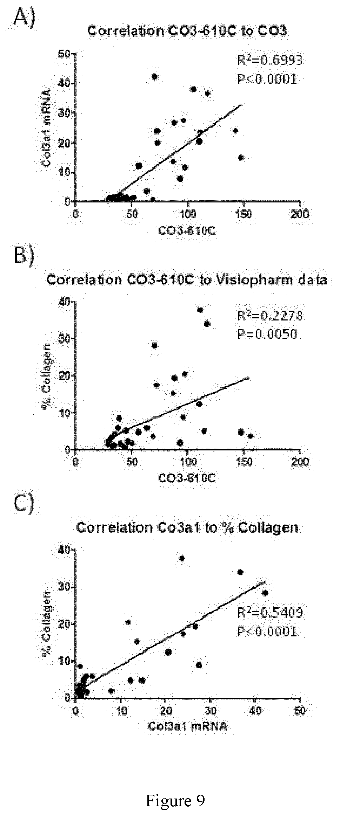

*Correlation:* Figure 9 panel A shows a correlation of Col3a1 to C03-610C was found with R²=0.6993, P<0.0001. In panel B, a correlation of C03-610C to % collagen was found with R²=0.2278 and P=0.0050. In panel C a correlation of Co13a1 to % collagen was found with R²=0.5409, P<0.0001.

Correlations were found of the following: Col3a1 mRNA to CO3-610C with R²=0.6993 and P<0.0001 (Figure 9A), and C03-610C to % collagen quantified by visiopharm with R²=0.2278 and P=0.0050 (Figure 9B), and Col3a1 mRNA to % collagen quantified by visiopharm with R²=0.5409 and P<0.0001 (Figure 9C).

ECM remodelling is an integrated process of tissue development, maintenance and pathogenesis. Proteolytic activity is essential in this process for cell migration, removal of damaged tissue, and sequestering of new proteins, for the correct and optimal tissue orientation and quality (108:109). The specific matrix degradation products, neo-epitopes, may be important for the identification of new biochemical markers of liver fibrosis matrix turnover and understanding fibrosis pathogenesis. At present there are no available measuring techniques, nor biochemical markers, that allow for assessment of ECM remodeling in the pathogenesis of fibrosis.

In this example, to investigate the CO3 - 610C marker under in vivo situations, 6 months BDL rats were chosen, as they previously have been shown to have a lower collagen remodelling compared to younger rats. The rats are skeletally mature, and the growth plate is almost dormant, thereby contributing to a much lower extent to the overall collagen turnover. This influences the sensitivity and specificity for biomarker. These rats clearly presented with hepatic fibrosis , as evaluated by both quantitative histological analysis, and enlargement with increased weight, thus the model was an appropriate one to look for evidence of ECM remodeling, in particular for evidence of collagen type III in serum.

The present data clearly demonstrate the neo-epitope CO3 - 610C from MMP-9 mediated collagen type III degradation is a diagnostic biochemical marker for liver fibrosis with an average increases in serum of up to 153% from sham to BDL- operated rats.

To further investigate the biological rationale for the increased CO3 - 610C marker, we did protein extractions from healthy and diseased livers. By western blotting, we identified a predominant band, suggesting this to be an abundant protein fragment in diseased but not healthy livers. This provides evidence for the pathological accuracy of this novel marker.

To further investigate the pathological turnover representation of the liver, we measured type III collagen mRNA.

We found an increase of mRNA in the BDL rats compared to those undergoing the sham operation, which correlates with previous findings. These data strongly suggest that liver fibrosis is not only an accumulation of ECM proteins, but also an accelerated turnover situation, in which both tissue formation and tissue degradation both are highly up regulated. Tissue formation outstrips tissue degradation, leading to an accumulation of scar tissue over time. Previous investigators have used other matrix turnover proteins to assess liver fibrosis, one being the type III collagen formation marker N-terminal type III pro-collagen. This marker represents collagen type III formation and has shown to be increased in liver fibrosis in previous studies.

To further understand and the dynamics of the biochemical makers CO3 - 610C, we did a range of correlations. Most importantly, there was a significant correlation of CO3 - 610C to the extent of fibrosis measured in the liver by quantitative histology. The level of liver fibrosis was correlated to the expression levels of the mRNA of collagen type III. Finally, the CO3 - 610C correlated to mRNA of collagen type III in the liver. Taken together, there was a significant correlation of the pathological processes in the liver with the levels of the systemic biochemical markers CO3 - 610C. In addition the tissue extractions provided evidence that the circulation levels were locally produced.

### Example 6: ELISA on human serum samples

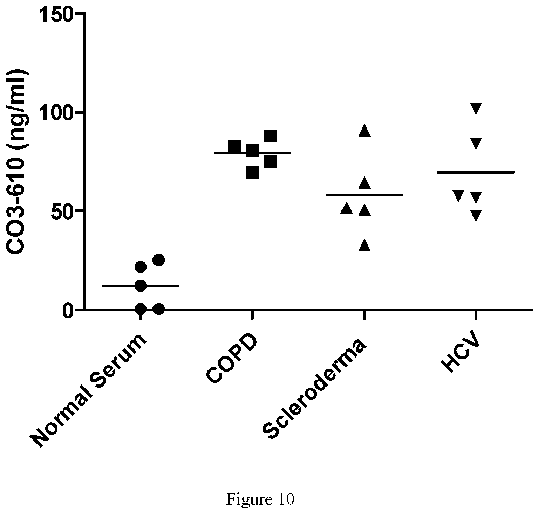

Human serum samples were obtained from patients with Chronic Obstructive Pulmonary Disease (COPD) (n=5), scleroderma (n=5), chronic hepatitis C virus infection (n=5), and healthy controls (n=5). The serum samples were tested in the CO3-610 ELISA (see Example 4 above) to determine the concentration of COS-610 fragments. Results are shown in Figure 10. While serum samples from the healthy subjects had concentration of CO3-610 fragments below 30 ng/ml, the diseased subjects were found to have elevated levels in circulation suggesting massive tissue remodelling in the affected fibrotic tissues.

### Example 7: Reactivity of clone nb94

Mice were immunized with synthetic peptide KAFVFP (SEQ ID NO1167) conjugated to ovalbumin (KAFVFPKESD-GGC-OVA (SEQ ID NO1049)), spleen cells were used for fusion, and monoclonal antibodies tested for reactivity to biotinylated KAFVFP (SEQ ID NO 1167), i.e. (KAFVFPKESD-biotin(SEQ ID NO1049)) immobilized in wells of microtitre plates precoated with streptavidin. Antibodies binding to biotinylated KAFVFPKESD(SEQ ID NO1049), which could be inhibited by co-incubation with KAFVFPKESD (SEQ ID NO1049) but not the elongated peptide RKAFVFPKESD (SEQ ID NO1166), were selected for further characterization. The preferred monoclonal antibody was designated NB94-37-1A7.
Using a competition ELISA, essentially as described above with biotinylated KAFVFPKESD (SEQ ID NO1049) (used at 0.15 ng/ml) immobilized in the wells of streptavidin-coated microtitre plates, an incubation step (90 minutes at 20°C) with sample and monoclonal antibody NB94-37-1A7 followed by a washing step, and then addition of peroxidase-conjugated anti-mouse immunoglobulins. For competition the following material was used in 2-fold dilutions; (1) the synthetic KAFVFP (SEQ ID NO1167) peptide; (2) a nonsense peptide (KNEGTG) unrelated to CRP; (3) a pool of human serum samples; (4) CRP proteolytically cleaved with MMP3 for 7 days, subsequently stopped by addition of EDTA to block protease activity, and stored at -80°C until testing; (5) same as (4) but using MMP8 instead of MMP3; (6) same as (4) except using Cathepsin K (for 2 days) instead of MMP3 (and E64 as inhibitor to block Cathepsin K activity).

The data demonstrate that monoclonal antibody NB94-37-1A7 binds strongly to the synthetic peptide KAFVFPKESD (SEQ ID NO1049), and with CPR cleaved with MMP3 and MMP8. Cleavage of CRP with Cathepsin K release less analyte recognized by monoclonal antibody NB94-37-1A7. Finally, the data shows that the antibody binds to peptide fragments in human serum confirming the presence of this sequence in circulating peptide fragments.

### Example 8: CO3 in biological relevant samples: CO3 levels in Carbon tetrachloride (CCl4)-induced cirrhosis in rats.

Animals and induction of cirrhosis:
This study included 52 male Wistar rats with fibrosis or cirrhosis and 35 male Wistar control rats. To cause them to develop fibrosis or cirrhosis three-month old animals were included in an induction program with carbon tetrachloride (CCl4) and Phenobarbital treatment. CCl₄ was administered by inhalation twice weekly and phenobarbital (0.3 g/l) added to the drinking water. Animals were allowed free access to water and food throughout the study.

Fibrosis Quantification:
Liver sections (4 µm) were stained in 0.1% Sirius red F3B (Sigma-Aldrich, St. Louis, MO) in saturated picric acid (Sigma-Aldrich). Relative fibrosis area (expressed as a percentage of total liver area) was assessed by analyzing 36 fields of Sirius red-stained liver sections per animal. Each field was acquired at 10X magnification [E600 microscope (Nikon) and RT-Slider SPOT digital camera (Diagnostic Instruments, Inc., Sterling Heights, MI). Results were analyzed using a computerized Bioquant Life Science morphometry system. To evaluate the relative fibrosis area, the measured collagen area was divided by the net field area and then multiplied by 100. Subtraction of vascular luminal area from the total field area yielded the final calculation of the net fibrosis area. From each animal analyzed, the amount of fibrosis as percentage was measured and the average value presented.

Classification of groups according to their fibrosis/cirrhosis stage:
Animals were classified into 4 different stages of fibrosis and cirrhosis (Group A: moderate fibrosis, group B: advanced fibrosis, Group C: moderate cirrhosis, and Group D: advanced cirrhosis) that were determined by the percentage of Sirius red positive liver area (Group A: <5%, Group B: 5 to10%, Group C: 10 to 15% and Group D: >15%). For this purpose, control and fibrotic/cirrhotic rats were studied considering four different time points during the CCl4 treatment: 8, 12, 16 and 20 weeks after starting the cirrhosis induction program.

Hyaluronic acid measurement:
Serum hyaluronan was measured using a sandwich ELISA kit (R&D Systems Inc., Minneapolis, MN, USA).

Statistics:
Statistical analysis of results was performed by unpaired Student's t tests when appropriate. Data were expressed as mean ±S.E.M., and they were considered significant at a p level of 0.05 or less.

Study design:
Animals included in this protocol were randomly assigned to one of the following groups: A/ eight weeks of CCl₄ treatment, B/ twelve weeks of CCl₄ treatment, C/ sixteen weeks of CCl₄ treatment and D/ twenty weeks of CCl₄ treatment. In parallel, four control groups were studied at the same time points. Thirteen fibrotic rats and seven control rats were included in each group. At the end of the study, rats were placed in standard metabolic cages (Tecniplast Deutschland, Hohenpeissenberg, Germany) during an adaptation period of 3 days before proceeding with the twenty-four-hour urine collection. Urinary volumes were determined gravimetrically. During the adaptation period, rats were allowed to get free access to tap water and food. Then, 24-hour urine samples were centrifuged for 5 min at 2,500 rpm and aliquoted into ten polypropylene tubes (400 µL each). Urine samples were stored at -80°C for subsequent analysis.

At scheduled necropsies, rats were weighed, anesthetized with pentobarbital (50 mg/kl) and decapitated. Blood were collected and allowed to stand at room temperature for 20 min to allow clotting and then centrifuged for 10 min at 2500 rpm. Serum were collected in polypropylene tubes aliquots (400 µl each) and transferred via dry ice to a -80°C freezer. Collection of baseline blood samples at the beginning of the CCl₄ treatment was not considered in order to avoid additional intervention that may increase the risk of infection and/or introduce modifications in the experimental model that may compromise the evolution of the induced pathophysiological process. For histology and Sirius red staining, half of the left lobe of the liver were placed in 10% neutral buffered formalin for 16 hours, embedded in paraffin and sectioned into 4-µm-thick slices. After liver fibrosis quantification, the unused paraffin block material was preserved for biomarker quantification. The other half of the left lobe was flash-frozen in liquid nitrogen and stored for Western blot, RT-PCR or immunohistochemical analysis. Measurements of liver fibrotic area, serum and urine osmolality, Na⁺ and K⁺, albumin, creatinine, alanine amino-transferase and lactate dehydrogenase were made according to the Material and Methods section.

### Results:

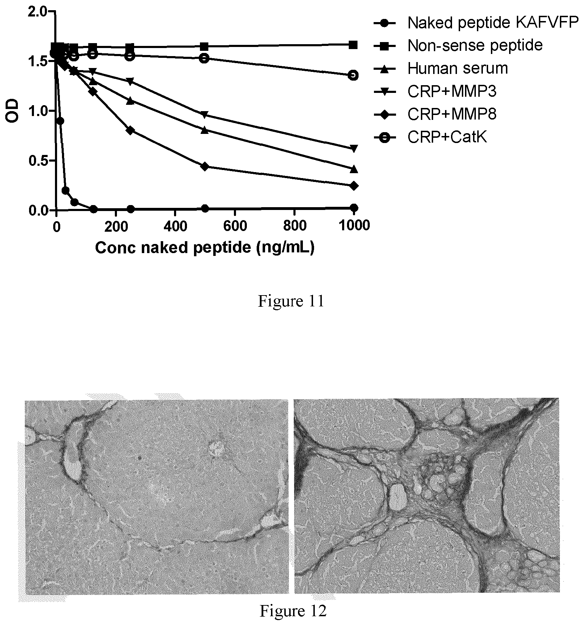

Histological validation of the model:
Liver collagen was quantified in all study animals by Sirius red staining of liver slices. The final data for each animal was taken as the average of red staining observed in 36 consecutive microscope fields (Figure 12).

Figure 12 shows representative pictures from two sets of 36 images used to quantify collagen accumulation in liver in rat #1 (left) and rat #43 (right) treated with carbon tetrachloride for eight and twenty weeks respectively.

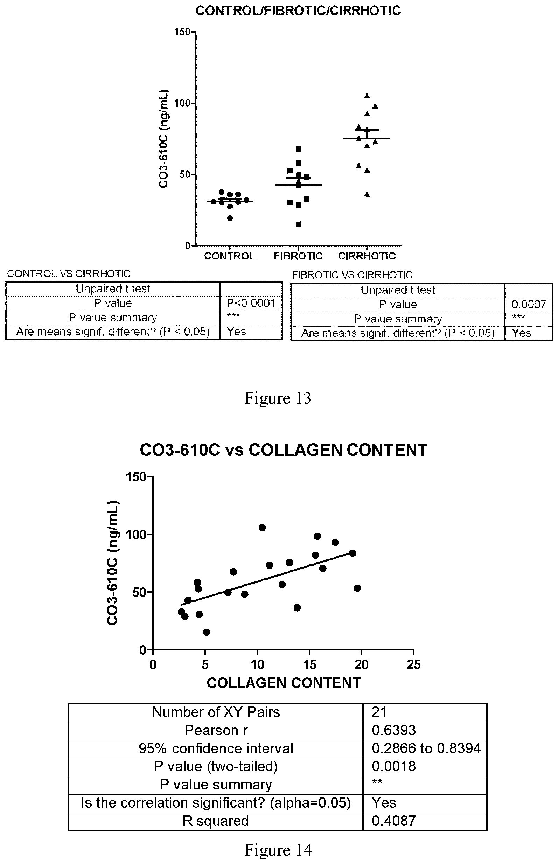

The serum CO3 marker shows statistically significant increases in both fibrotic and cirrhotic rats compared to control rats. Animals were classified according to a fully automated syrius red staining of the liver procedure used to quantify fibrosis (Figures 13 and 14).

Figure 13 shows serum CO3 levels in CCl₄ inhalation and control rats as performed in Hospital Clinic (Barcelona). Each point represents one animal. Rats were classified according a computerized image analysis method of syrius red staining of the liver used to quantify fibrosis.

When quantitative values of serum CO3 and syrius red staining of the liver were studied in each individual animal, we found a statistically significant correlation between the two variables (R2=0.4087; n=21) (Figure 14).

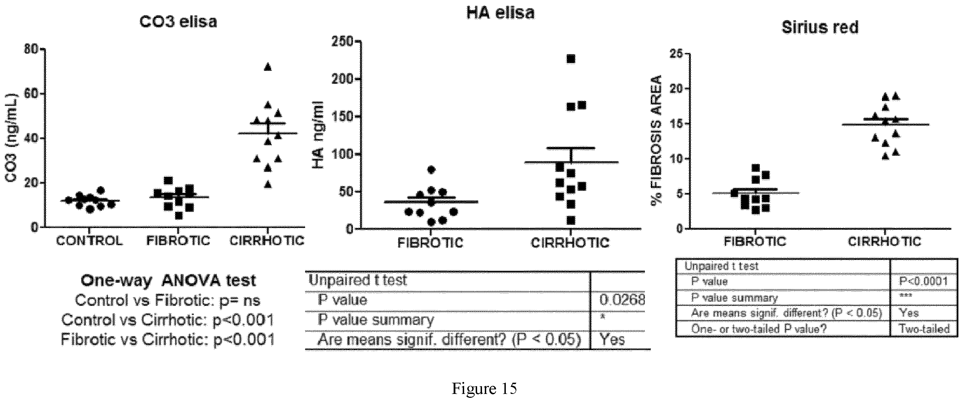

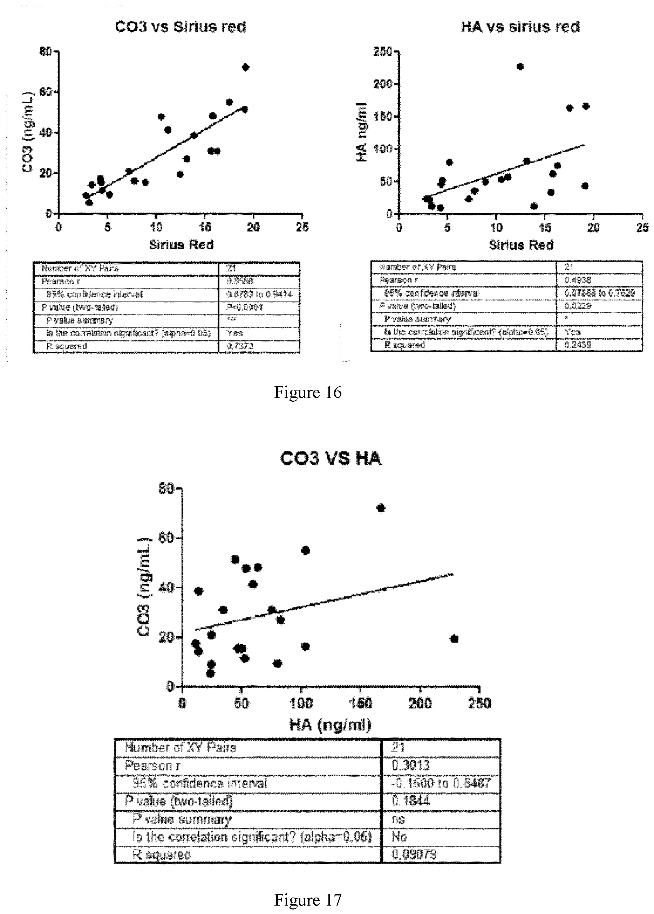

We have compared the levels of CO3-610C with the serological benchmark of liver fibrosis hyaluronic acid (HA). HA levels were quantified with a commercial ELISA kit and results show significant elevations of this ECM component in cirrhotic rats vs. fibrotic animals (Figures 15 and 16).

The correlation of CO3 to Sirius red outperformed that of HA. More than seventy percent of the variation in liver fibrosis histological quantification can be explained by the serological measurement of CO3. The remaining thirty percent is due to unknown variables or inherent variability. Instead only 25% of liver fibrosis can be explained by measuring hyaluronic acid (Figure 15).

As expected from the previous result no correlation could be found between CO3 and hyaluronic acid suggesting that they are the result of two independent pathophysiological processes in the development of liver fibrosis (Figure 17).

### Example 9: Bleomycin induced skin fibrosis in mice.

Mice were treated by application to the skin of PBS or bleomycin. Increasing levels in urine of the MMP-9 mediated collagen III (COS) degradation fragment CO3-610 were associated with skin fibrosis progression in mice.

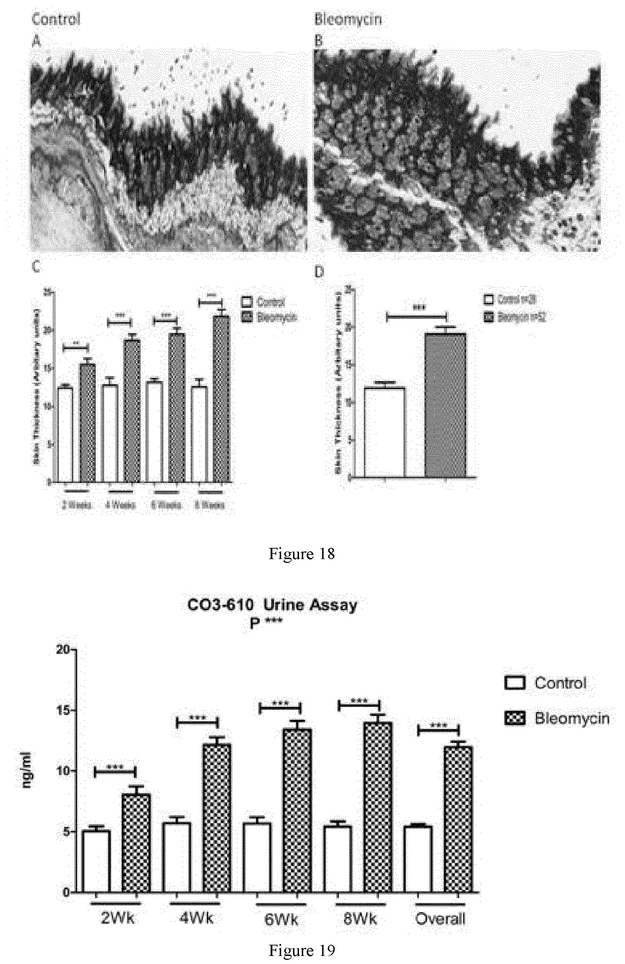

Figure 18 shows a skin section from a PBS treated mouse at 8 weeks of treatment (panel A) and a skin section from Bleomycin treated mouse at 8 weeks of treatment (panel B). Skin thickness increase between PBS (n=7/time point) and Bleomycin (n=13/time point) treated mice for 2 weeks (P = 0.0029), 4 weeks (P = 0.0004), 6 weeks (P < 0.0001) and 8 weeks (P < 0.0001) is plotted in panels C and D. Overall skin thickness increase between PBS (n=28) and Bleomycin (n=52) treated mice for the duration of the study (P < 0.0001). Skin width was calculated by Visiopharm software as an overall number per skin section instead of sampling pictures.

Figure 19 shows CO3-610 urine assay results which demonstrate a significant increase throughout the time points of the study. The figure shows result per time point (n=7 PBS, n=13 Bleomycin treated per termination point) and collective CO3-610 levels for all time points (n=28 PBS and n=52 Bleomycin treated mice). 2 weeks P = 0.0008, 4 weeks P < 0.0001, 6 weeks P < 0.0001, 8 weeks P < 0.0001 and overall P < 0.0001.

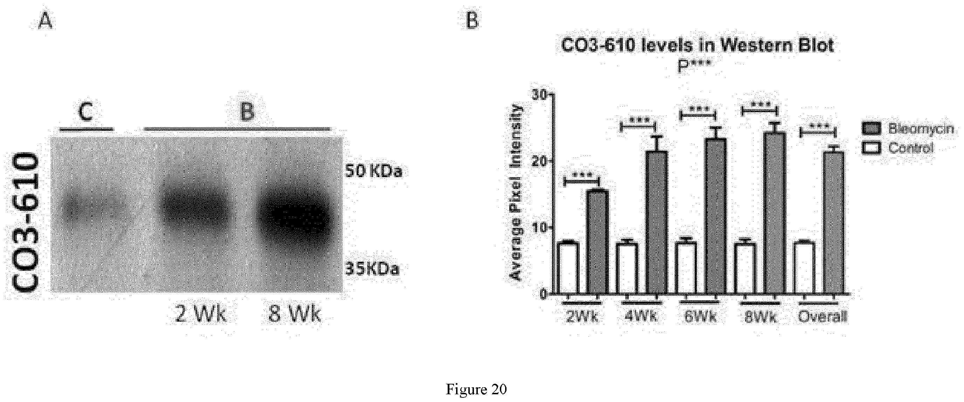

Figure 20 shows a CO3-610 Western blots image with control C and Bleomycin B after 2 and 8 weeks treatment (panel A). CO3-610 densitometry measurements for all time points (n=7 PBS and n=13 Bleomycin treated per termination point) and collective CO3-610 levels (n=28 PBS and n=52 Bleomycin treated mice) are shown in panel B, demonstating a statistically significant increase of CO3-610 levels (P < 0.0001).

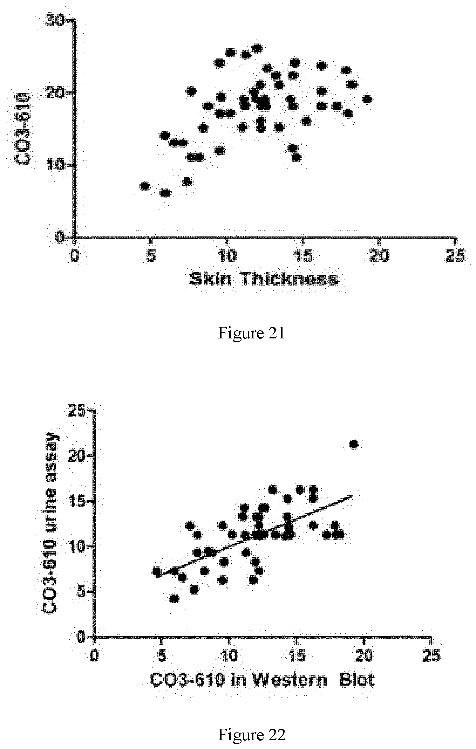

As seen in Figure 21, CO3-610 levels in urine assay were found to be correlated with skin thickness progression, and therefore total collagen deposition r=0.4883, R2=0.2384.

As seen in Figure 22,statistically significantcorrelation was found (r = 0.6528, P<0.0001) between results from the CO3-610 ELISA urine assay and Western blot densitometry measurements.

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than in to mean 'consisting of'. All prior teachings acknowledged above are hereby incorporated by reference. No acknowledgement of any prior published document herein should be taken to be an admission or representation that the teaching thereof was common general knowledge in Australia or elsewhere at the date hereof.

The invention provides the following aspects:
1. A method of diagnosis or of quantitation of fibrosis comprising obtaining a patient biofluid sample, conducting an immunoassay to measure neo-epitope containing protein fragments naturally present in said sample, and associating an elevation of said measure in said patient above a normal level with the presence or extent of fibrosis, wherein said immunoassay is conducted by a method comprising:
contacting protein fragments naturally present in said sample with an immunological binding partner reactive with a neo-epitope formed by cleavage of a protein by a proteinase and
measuring the extent of binding of peptide fragments to said immunological binding partner to measure therein protein fragments comprising said neo-epitope, and wherein said protein is collagen type III, collagen type I, collagen type IV,
collagen type V, or collagen type VI, elastin, biglycan, decorin, lumican, versican, perlecan, neurocan, brevican, fibromodulin, serglycin, syndecan, betaglycan, vimentin, or C-reactive protein, subject to the proviso that when the neo-epitopes are formed by cleavage of type I collagen, the cleavage is not at a site at which collagen type I is cleaved by cathepsin K.
2. A method as set out in aspect 1, wherein said immunological binding partner specifically binds fragments of collagen type III having an N-terminal sequence KNGETG...
3. A method as set out in aspect 1, wherein said immunological binding partner specifically binds fragments of CRP having an N-terminal sequence KAFVFP... (SEQ ID NO1167).
4. A method as set out in aspect 1, wherein said immunological binding partner specifically binds a neo-epitope constituted by an N-terminal amino acid sequence present in peptides produced by cleavage of collagen type III, said peptides comprising an N-terminal sequence selected from the group consisting of:

| Collagen type III | | |
|---|---|---|
| GIPGAP... SEQ ID NO372 | GDPGPP... SEQ ID NO373 | LAGPPG... SEQ ID NO89 |
| IAGITG... SEQ ID NO375 | IKGHRG... SEQ ID NO376 | RGLAGP... SEQ ID NO377 |
| KGDAGQ... SEQ ID NO 381 | ITGARG... SEQ ID NO379 | VKGESG... SEQ ID NO380 |
| GKSGDR... SEQ ID NO383 | LQGLPG... SEQ ID NO384 | LRGGAG... SEQ ID NO382 |
| DGTSGH... SEQ ID NO135 | IGSPGP... SEQ ID NO | AIGSPG... SEQ ID NO143 |
| GPPGVA... SEQ ID NO158 | AGPPGM... SEQ ID NO145 | LSGERG... SEQ ID NO176 |
| GARGLA... SEQ ID NO111 | GAPGEK... SEQ ID NO141 | PQGPPG... SEQ ID NO389 |
| KGESGK... SEQ ID NO374 | GLSGER... SEQ ID NO387 | YQGPPG... SEQ ID NO401 |
| QPGVMG... SEQ ID NO144 | IPGAPG... SEQ ID NO117 | FRGPAG... SEQ ID NO137 |
| GPPGPT... SEQ ID NO391 | INGSPG... SEQ ID NO392 | GPPGEP... SEQ ID NO393 |
| GLPGPP... SEQ ID NO394 | KNGETG... SEQ ID NO395 | LPGIAG... SEQ ID NO396 |
| GINGSP... SEQ ID NO397 | PGENGK... SEQ ID NO398 | LKGENG... SEQ ID NO399 |
| LMGARG... SEQ ID N0400 | GKDGES... SEQ ID NO418 | QQGAIG... SEQ ID NO390 |
| DKGEPG... SEQ ID NO403 | GHAGAQ... SEQ ID NO404 | GERGAP... SEQ ID NO402 |
| PGMKGH... SEQ ID NO406 | FPGARG... SEQ ID NO407 | GFPGAR... SEQ ID NO408 |
| FPGMKG... SEQ ID NO409 | PGDKGE... SEQ ID NO410 | GDKGET... SEQ ID NO411 |
| GQPGDK... SEQ ID NO412 | GPPGEN... SEQ ID NO413 | AAGFPG... SEQ ID NO417 |
| GERGSP... SEQ ID NO415 | PGVPGA... SEQ ID NO416 | GARGND... SEQ ID NO419 |
| GSDGQP... SEQ ID NO405 | GPPGPP... SEQ ID NO100 | GGAGPP... SEQ ID NO425 |
| GFPGAP... SEQ ID NO420 | GAAGEP... SEQ ID NO421 | GARGPP... SEQ ID NO422 |
| PGPQGH... SEQ ID NO426 | PGFPGM... SEQ ID NO427 | GSPGGP... SEQ ID NO428 |
| PGPPGI... SEQ ID NO432 | GITGAR... SEQ ID NO430 | GIAGIT... SEQ ID NO431 |
| GPPGSN... SEQ ID NO423 | RPGLPG... SEQ ID NO436 | GAPGPM... SEQ ID NO438 |
| PQGLQG... SEQ ID NO440 | GPPGVA... SEQ ID NO158 | SGDRGE... SEQ ID NO429 |
| GAPGFR... SEQ ID NO435 | PGFRGP... SEQ ID NO443 | GPVGPS... SEQ ID NO447 |
| GAPGPQ... SEQ ID NO445 | GFPGNP... SEQ ID NO446 | GPPGIN... SEQ ID NO470 |
| GPTGPI... SEQ ID NO448 | GDAGQP... SEQ ID NO449 | NGEKGE... SEQ ID NO450 |
| KGSPGA... SEQ ID NO444 | GSPGER... SEQ ID NO439 | AIGPSG... SEQ ID NO368 |
| GSRGAP... SEQ ID NO462 | TGARGL... SEQ ID NO378 | ERGLPG... SEQ ID NO385 |
| NTGAPG... SEQ ID NO437 | VGGLAG... SEQ ID NO155 | VAGPPG... SEQ ID NO452 |
| HAGAQG... SEQ ID NO434 | PGAPGG... SEQ ID NO455 | GIPGFP... SEQ ID NO414 |
| PGPQGP... SEQ ID NO453 | AGQQGA... SEQ ID NO457 | PGPPGP... SEQ ID NO458 |
| AGQPGE... SEQ ID NO454 | GLAGPP... SEQ ID NO388 | GRNGEK... SEQ ID NO460 |
| VKGERG... SEQ ID NO159 | GGAGEP... SEQ ID NO463 | SPGGKG... SEQ ID NO459 |
| GPPGAP... SEQ ID NO461 | SPGAQG... SEQ ID NO465 | PGVSGP... SEQ ID NO466 |
| GSPGAQ... SEQ ID NO464 | IKGPAG... SEQ ID NO169 | PGAPGQ... SEQ ID NO456 |
| PGAPGL... SEQ ID NO467 | GIPGQP... SEQ ID NO468 | SRGAPG... SEQ ID NO451 |
| ESCPTG... SEQ ID NO433 | DAGAPG... SEQ ID NO469 | GPKGDA... SEQ ID NO424 |
| GPAGIP... SEQ ID NO441 | | |

5. method as set out in aspect 1, wherein said immunological binding partner specifically binds a neo-epitope constituted by a C-terminal amino acid sequence present in peptides produced by cleavage of collagen type III, said peptides comprising an C-terminal sequence selected from the group consisting of:

| Collagen type III | | |
|---|---|---|
| ...GPPGPA SEQ ID NO94 | ...NGDPGI SEQ ID NO471 | ...SPGPQG SEQ ID NO472 |
| ...GMPGPR SEQ ID NO473 | ...SPGPAG SEQ ID NO474 | ...PGPQGV SEQ ID NO475 |
| ...ERGAAG SEQ ID NO476 | ...PGPLGI SEQ ID NO477 | ...AAGTPG SEQ ID NO478 |
| ...ERGPPG SEQ ID NO147 | ...PGPPGT SEQ ID NO479 | ...GNRGER SEQ ID NO480 |
| ...GLPGLA SEQ ID NO486 | ..APGLRG SEQ ID NO481 | ...HPGSPG SEQ ID NO482 |
| ...GLAGTA SEQ ID NO488 | ...GSPGPA SEQ ID NO484 | ...GPAGPP SEQ ID NO485 |
| ...LAGPPG SEQ ID NO89 | ...PGLMGA SEQ ID NO489 | ...QGPPGP SEQ ID NO487 |
| ...IPGFPG SEQ ID NO492 | ...GPPGPQ SEQ ID NO490 | ...GFPGMK SEQ ID NO493 |
| ...FPGPKG SEQ ID NO491 | ...GPAGIP SEQ ID NO441 | ...FPGAPG SEQ ID NO494 |
| ...GPPGIC SEQ ID NO2187 | ...PPGPPG SEQ ID NO119 | ...FPGARG SEQ ID NO407 |
| ...PGPQGL SEQ ID NO497 | ...GAPGLM SEQ ID NO498 | ...GAIGPS SEQ ID NO495 |
| ...SPGPKG SEQ ID NO499 | ...LPGAAG SEQ ID NO2188 | ...APGPLG SEQ ID NO2189 |
| ...LPGPPG SEQ ID NO72 | ...GPPGIN SEQ ID NO470 | ...IPGQPG SEQ ID NO501 |
| ...GHRGFD SEQ ID NO503 | ...PGLPGI SEQ ID NO504 | ...GAAGIK SEQ ID NO505 |
| ...GLPGIA SEQ ID NO507 | ...PGPKGD SEQ ID NO506 | ...GPPGVA SEQ ID NO158 |
| ...GLPGPP SEQ ID NO394 | ...GANGLP SEQ ID NO510 | ...GPPGPS SEQ ID NO511 |
| ...TGARGL SEQ ID NO378 | ...GPPGIK SEQ ID NO512 | ...TAGFPG SEQ ID NO513 |
| ...PQGLPG SEQ ID NO508 | ...GAPGLR SEQ ID NO509 | ...GPQGVK SEQ ID NO524 |
| ...GTPGLQ SEQ ID NO521 | ...GEVGPA SEQ ID NO514 | ...GSPGPQ SEQ ID NO533 |
| ...GMKGHR SEQ ID NO531 | ...GKPGAN SEQ ID NO537 | ...QPGPPG SEQ ID NO536 |
| ...EMGPAG SEQ ID NO534 | ...PGAAGF SEQ ID NO539 | ...PGANGL SEQ ID NO529 |
| ...GVKGER SEQ ID NO538 | ...GDAGAP SEQ ID NO542 | ...GPAGER SEQ ID NO543 |
| ...GPPGPR SEQ ID NO544 | ...GPAGPR SEQ ID NO545 | ...RGFDGR SEQ ID NO546 |
| AGPRGA SEQ ID NO547 | ...GGKGER SEQ ID NO548 | ..APGLMG SEQ ID NO549 |
| ...GRNGDP SEQ ID NO171 | ...GPAGAN SEQ ID NO550 | ...PQGVKG SEQ ID NO541 |
| ...AGIPGF SEQ ID NO496 | ...VKGESG SEQ ID NO380 | ...TGERGA SEQ ID NO540 |
| ...PPGPQG SEQ ID NO103 | ...TGPRGP SEQ ID NO177 | ...GSPGYQ SEQ ID NO526 |
| ...IPGAPG SEQ ID NO117 | ...EPGPRG SEQ ID NO516 | ...GAAGAR SEQ ID NO528 |
| ...TSGHPG SEQ ID NO518 | ...GAPGPA SEQ ID NO519 | ...TGAPGS SEQ ID NO502 |
| ...PSGPPG SEQ ID NO483 | ...GTSGHP SEQ ID NO522 | ...GTGGPP SEQ ID NO530 |
| ...PPGPAG SEQ ID NO52 | ...GAPGLK SEQ ID NO525 | ...GITGAR SEQ ID NO430 |
| ...FPGMKG SEQ ID NO409 | ...GEPGPR SEQ ID NO500 | ...GIAGPR SEQ ID NO535 |
| ...EKGPAG SEQ ID NO515 | ...PGPKGN SEQ ID NO527 | ...GLSGER SEQ ID NO387 |
| ...MPGPRG SEQ ID NO523 | ...PPGAPG SEQ ID NO517 | ...EGGPPG SEQ ID NO532 |
| ...GIPGAP SEQ ID NO372 | ...TPGLQG SEQ ID 520 | ...GFPGAR SEQ ID NO408 |
| | | |

6. A method as set out in aspect 1, wherein said immunological binding partner specifically binds a neo-epitope constituted by an N-terminal amino acid sequence present in peptides produced by cleavage of collagen type I, said peptides comprising an N-terminal sequence selected from the group consisting of:

| **Collagen I, alpha1** | | |
|---|---|---|
| ISVPGP... SEQ ID NO23 | VPGPMG... SEQ ID NO24 | PGPMGP... SEQ ID NO25 |
| FQGPPG... SEQ ID NO27 | KNGDDG... SEQ ID NO28 | ARGLPG... SEQ ID NO29 |
| LDGAKG... SEQ ID NO31 | LPGERG... SEQ ID NO32 | ATGAAG... SEQ ID NO67 |
| VRGEPG... SEQ ID NO33 | PGAKGA... SEQ ID NO34 | GIAGAP... SEQ ID NO69 |
| GGPPGP... SEQ ID NO35 | NSGEPG... SEQ ID NO36 | VQGPPG... SEQ ID NO71 |
| DGVAGP... SEQ ID NO37 | ERGSPG... SEQ ID NO38 | RGSPGP... SEQ ID NO74 |
| LTGSPG... SEQ ID NO39 | QDGRPG... SEQ ID NO40 | ARGQAG... SEQ ID NO77 |
| RGVPGP... SEQ ID NO41 | VGPAGK... SEQ ID NO42 | KDGEAG... SEQ ID NO80 |
| ERGEQG... SEQ ID NO43 | RGEQGP... SEQ ID NO44 | QGLPGP... SEQ ID NO83 |
| PGERGV... SEQ ID NO45 | ANGAPG... SEQ ID NO46 | AGLPGP... SEQ ID NO86 |
| ARGAPG... SEQ ID NO47 | PGDRGE... SEQ ID NO48 | FAGPPG... SEQ ID NO75 |
| AKGDAG... SEQ ID NO49 | PIGNVG... SEQ ID NO50 | NVGAPG... SEQ ID NO78 |
| AAGRVG... SEQ ID NO51 | PPGPAG... SEQ ID NO52 | GEVGPP... SEQ ID NO81 |
| GADGPA... SEQ ID NO53 | GPQGIA... SEQ ID NO54 | IAGQRG... SEQ ID NO84 |
| GQRGVV... SEQ ID NO55 | QRGVVG... SEQ ID NO56 | RGVVGL... SEQ ID NO87 |
| GLPGQR... SEQ ID NO57 | PGLPGP... SEQ ID NO58 | EPGKQG... SEQ ID NO90 |
| PMGPPG... SEQ ID NO59 | MGPPGL... SEQ ID NO60 | LAGPPG... SEQ ID NO89 |
| DKGETG... SEQ ID NO61 | LQGPPG... SEQ ID NO62 | PSGASG... SEQ ID NO92 |
| SAGAPG... SEQ ID NO63 | RTGDAG... SEQ ID NO64 | VGPPGP... SEQ ID NO99 |
| FDFSF... SEQ ID NO65 | DFSF... SEQ ID NO66 | AGQRGV... SEQ ID NO95 |
| LPGPGG... SEQ ID NO26 | QAGVMG... SEQ ID NO76 | VVGLPG... SEQ ID NO98 |
| SGLDGA... SEQ ID NO30 | PAGERG... SEQ ID NO79 | GKQGPS... SEQ ID NO93 |
| AKGEAG... SEQ ID NO68 | ARGERG... SEQ ID NO82 | ARGPAG... SEQ ID NO96 |
| IAGAPG... SEQ ID NO70 | LTGPIG... SEQ ID NO85 | ASGPAG... SEQ ID NO97 |
| LPGPPG... SEQ ID NO72 | AGPPGA... SEQ ID NO88 | GPPGPP... SEQ ID NO100 |
| AGPKGS... SEQ ID NO73 | ATGFPG... SEQ ID NO91 | |
| | GPPGPA... SEQ ID NO94 | |

7. A method as set out in aspect 1, wherein said immunological binding partner specifically binds a neo-epitope constituted by a C-terminal amino acid sequence present in peptides produced by cleavage of collagen type I, said peptides comprising an C-terminal sequence selected from the group consisting of:

| **Collagen I, alpha1** | | |
|---|---|---|
| ...QLSYGY SEQ ID NO101 | ...EKSTGG SEQ ID NO 102 | ...PPGPQG SEQ ID NO103 |
| ...KGHRGF SEQ ID NO104 | ...PSGPRG SEQ ID NO105 | ...APGPQG SEQ ID NO106 |
| ...APGPAG SEQ ID NO107 | ...FPGAVG SEQ ID NO108 | ...SEGPQG SEQ ID NO109 |
| ...GANGAP SEQ ID NO110 | ...ANGAPG SEQ ID NO46 | ...SGPQGP SEQ ID NO112 |
| ...EPGPVG SEQ ID NO113 | ...EPGPTG SEQ ID NO114 | ...RGFPGA SEQ ID NO115 |
| ...KGPAGE SEQ ID NO116 | ...RGSPGP SEQ ID NO74 | ...LPGAKG SEQ ID NO118 |
| ...AVGPAG SEQ ID NO122 | ...PPGARG SEQ ID NO120 | ...PGKAGE SEQ ID NO121 |
| ...APGPDG SEQ ID NO125 | ...PAGPAG SEQ ID NO123 | ...AGPAGE SEQ ID NO124 |
| ...KDGVRG SEQ ID NO128 | ...RGERGF SEQ ID NO126 | ...PAGPRG SEQ ID NO127 |
| ...PGPAGF SEQ ID NO131 | ...PAGPTG SEQ ID NO129 | ...TGARGA SEQ ID NO130 |
| ...SAGPPG SEQ ID NO134 | ...EPGDAG SEQ ID NO132 | ...PAGPPG SEQ ID NO133 |
| ...GEVGPP SEQ ID NO81 | ...ATGFPG SEQ ID NO91 | ...NAGPPG SEQ ID NO136 |
| ...PGPQGI SEQ ID NO140 | ...GEKGSP SEQ ID 138 | ...GAPGTP SEQ ID NO139 |
| ...IAGQRG SEQ ID NO84 | ...GPQGIA SEQ ID NO54 | ...PQGIAG SEQ ID NO142 |
| ...GPSGEP SEQ ID NO146 | ...GQRGVV SEQ ID NO55 | ...RGERGF SEQ ID NO126 |
| ...PVGPVG SEQ ID NO149 | ...ERGPPG SEQ ID NO147 | ...RGPPGP SEQ ID NO148 |
| ...EQGPSG SEQ ID NO152 | ...PQGPRG SEQ ID 150 | ...HRGFSG SEQ ID NO151 |
| ...GPPGPP SEQ ID NO100 | ...PRGPPG SEQ ID NO153 | ...PPGPRG SEQ ID NO154 |
| ...PPGPPG SEQ ID NO119 | ...GPPSAG SEQ ID NO156 | ...PPSAGF SEQ ID NO157 |
| ...QMGPRG SEQ ID NO161 | ...PPGPAG SEQ ID NO52 | ...TPGPQG SEQ ID NO160 |
| ...PGADGQ SEQ ID NO164 | ...PGPPGA SEQ ID 162 | ...QGIAGQ SEQ ID 163 |
| ...PPGPKG SEQ ID NO167 | ...AGSPGF SEQ ID NO165 | ...LPGPSG SEQ ID NO166 |
| ...PKGPAG SEQ ID NO170 | ...PGERGA SEQ ID NO168 | ...PMGPPG SEQ ID NO59 |
| ...GPAGRP SEQ ID NO173 | ...PPGPIG SEQ ID NO174 | ...SPGEQG SEQ ID NO172 |
| ...TGDAGP SEQ ID NO175 | | |
| | | |

8. A method as set out in aspect 1, wherein said immunological binding partner specifically binds a neo-epitope constituted by an N-terminal amino acid sequence present in peptides produced by cleavage of collagen type IV, said peptides comprising an N-terminal sequence selected from the group consisting of:

| Collagen type IV | | |
|---|---|---|
| IDGYRG... SEQ ID NO609 | MGPPGT... SEQ ID NO610 | DGLPGS... SEQ ID NO611 |
| PGSKGE... SEQ ID NO612 | RGFPGP... SEQ ID NO613 | PGPPGL... SEQ ID NO614 |
| GPPGPP... SEQ ID NO100 | GLPGSM... SEQ ID NO615 | GLPGQQ... SEQ ID NO616 |
| PGIGVQ... SEQ ID NO618 | GIGPPG... SEQ ID NO611 | PGLPGI... SEQ ID NO504 |
| PGPKGF... SEQ ID NO621 | SPGIPG... SEQ ID NO619 | PGMQGE... SEQ ID NO620 |
| LGSKGE... SEQ ID NO617 | KGQPGL... SEQ ID NO622 | RGPPGP... SEQ ID NO148 |
| IRGEPG... SEQ ID NO626 | FPGPPG... SEQ ID NO627 | GPLGEK... SEQ ID NO625 |
| DGVIGM... SEQ ID NO629 | PGNPGI... SEQ ID NO630 | PGLKGD... SEQ ID NO624 |
| IKGDKG... SEQ ID NO631 | PGSPGC... SEQ ID NO632 | PPSDEI... SEQ ID NO623 |
| GPPGVP... SEQ ID NO633 | DQGDQG... SEQ ID NO634 | GAPGPQ... SEQ ID NO445 |
| KGSIGI... SEQ ID NO635 | GIPGAP... SEQ ID NO372 | DRGPQG... SEQ ID NO442 |
| ERGSPG... SEQ ID NO38 | LQGIRG... SEQ ID NO628 | DGGVPN... SEQ ID NO638 |
| SGRDGL... SEQ ID NO639 | PGPMGP... SEQ ID NO25 | GPPGLM... SEQ ID NO643 |
| DGYRGP... SEQ ID NO642 | AEGLPG... SEQ ID NO641 | LPGFAG... SEQ ID NO644 |
| GIPGMP... SEQ ID NO645 | PPGRLG... SEQ ID NO636 | GPPGEK... SEQ ID NO640 |
| EKGQKG... SEQ ID NO637 | .LPGPDG SEQ ID NO2214 | .PGIPGT SEQ ID NO2227 |
| .ILGHVP SEQ ID NO2212 | .LRGIPG SEQ ID NO760 | .PGDIVF SEQ ID NO2215 |
| .PGLPGQ SEQ ID NO2213 | .PGFPGA SEQ ID NO2218 | .GNKGDP SEQ ID NO2216 |
| .SGYPGN SEQ ID NO2217 | .QQGNRG SEQ ID NO2221 | .PGPRGK SEQ ID NO2219 |
| .VSGPPG SEQ ID NO2220 | .VGQPGP SEQ ID NO2222 | .PGPPGP SEQ ID NO458 |
| .KRGPPG SEQ ID NO2223 | .GEPGMQ SEQ ID NO2224 | .SKGEKG SEQ ID NO2226 |
| .LHGFPG SEQ ID NO2225 | | .GEPGPP SEQ ID NO675 |
| | | |

9. A method as set out in aspect 1, wherein said immunological binding partner specifically binds a neo-epitope constituted by a C-terminal amino acid sequence present in peptides produced by cleavage of collagen type IV, said peptides comprising an C-terminal sequence selected from the group consisting of:

| Collagen type IV | | |
|---|---|---|
| ...RGPPGP SEQ ID NO148 | ...SVDHGF SEQ ID NO646 | ...PSVDHG SEQ ID NO647 |
| ...VDHGFL SEQ ID NO648 | ...PGQPGY SEQ ID NO649 | ...QPGYTN SEQ ID NO650 |
| ...PGLPGS SEQ ID NO651 | ...GTPSVD SEQ ID NO652 | ...SVGSPG SEQ ID NO653 |
| ...LPGSMG SEQ ID NO654 | ...GPPGVP SEQ ID NO633 | ...PGFPGL SEQ ID NO655 |
| ...PGLPGE SEQ ID NO656 | ...GDPGPP SEQ ID NO373 | ...HQGEMG SEQ ID NO657 |
| ...GPPGLV SEQ ID NO658 | ...PGIPGP SEQ ID NO659 | ...PGFPGT SEQ ID NO660 |
| ...PGLPGP SEQ ID NO58 | ...DGIPGP SEQ ID NO661 | ...SGPKGY SEQ ID NO662 |
| ...GIGPPG SEQ ID NO611 | ...PGPRGE SEQ ID NO663 | ...GQGPPG SEQ ID NO664 |
| ...PGAIGP SEQ ID NO668 | ...KVDMGS SEQ ID NO665 | ...PGIDGV SEQ ID NO666 |
| ...GPKGPP SEQ ID NO671 | ...PPGPPG SEQ ID NO119 | ...KGHMGE SEQ ID NO667 |
| ...GPKGLP SEQ ID NO670 | ...SPGPPG SEQ ID NO672 | ...KGLPGP SEQ ID NO669 |
| ...GSVGYP SEQ ID NO673 | ...PQGPPG SEQ ID NO389 | ...PPGSPG SEQ ID NO674 |
| ...GEPGPP SEQ ID NO675 | ...AGNPGP SEQ ID NO676 | ...FPGPQG SEQ ID NO679 |
| ...PGYTNG SEQ ID NO678 | ...SVDHGF SEQ ID NO646 | ...LSGPPG SEQ ID NO680 |
| ...PGPQGP SEQ ID NO453 | ...GQGPPG SEQ ID NO664 | ...PGAPGL SEQ ID NO467 |
| ...GVMGTP SEQ ID NO681 | ...PGIKGS SEQ ID NO677 | ...PGPPGP SEQ ID NO458 |
| ...GVSGPK SEQ ID NO682 | | |
| HVPGML. SEQ ID NO2228 | LPVPGQ. SEQ ID NO2229 | LGPPGL. SEQ ID NO2230 |
| GVPGQA. SEQ ID NO2231 | VPGQAQ. SEQ ID NO2232 | GPDGFL. SEQ ID NO2233 |
| QEGPLG. SEQ ID NO2234 | LPGEVL. SEQ ID NO2235 | RGIPGF. SEQ ID NO2236 |
| NRGLGF. SEQ ID NO2238 | IPSDTL. SEQ ID NO2239 | PAGEKG. SEQ ID NO2240 |
| GEKGNK. SEQ ID NO2241 | PVGPPG. SEQ ID NO2242 | DIVFRK. SEQ ID NO2243 |
| PPGPKG. SEQ ID NO167 | RGKPGM. SEQ ID NO2244 | PGTRGL. SEQ ID NO2245 |
| GEKGSK. SEQ ID NO2246 | EKGSKG. SEQ ID NO2247 | SGQPGL. SEQ ID NO2248 |
| AGIPQK. SEQ ID NO2237 | | |

10. A method as set out in aspect 1, wherein said immunological binding partner specifically binds a neo-epitope constituted by an N-terminal amino acid sequence present in peptides produced by cleavage of biglycan, decorin, lumican, versican, or perlecan said peptides comprising an N-terminal sequence selected from the group consisting of: biglycan,.

| Biglycan | | |
|---|---|---|
| SVPKEI... SEQ ID NO949 | GLKLNY... SEQ ID NO950 | RISEAK... SEQ ID NO951 |
| NSGFEP... SEQ ID NO952 | LKSVPK... SEQ ID NO953 | AIELED... SEQ ID NO954 |
| LRISEA... SEQ ID NO958 | QCSDLG... SEQ ID NO955 | EAKLTG... SEQ ID NO956 |
| LLDLQN... SEQ ID NO961 | LTGIPK... SEQ ID NO959 | LKAVPK... SEQ ID NO960 |
| IELEDL... SEQ ID NO957 | | |
| | | |

| Decorin | | |
|---|---|---|
| IVIELG... SEQ ID NO962 | DEASGI... SEQ ID NO963 | VNNKIS... SEQ ID NO964 |
| NGLNQM... SEQ ID NO965 | LHLDGN... SEQ ID NO966 | LILVNN... SEQ ID NO967 |
| KITEIK... SEQ ID NO969 | GLPPSL... SEQ ID NO970 | SNPVQY... SEQ ID NO971 |
| SSGIEN... SEQ ID NO968 | | |
| | | |

| Versican | | |
|---|---|---|
| LLASDA... SEQ ID NO972 | LATVGE... SEQ ID NO973 | ETTVLV... SEQ ID NO974 |
| ENQDAR... SEQ ID NO975 | NGFDQC... SEQ ID NO976 | SLTVVK... SEQ ID NO977 |
| | | |

| Lumican | | |
|---|---|---|
| SLEDLQ... SEQ ID NO978 | LKEDAV... SEQ ID NO979 | HLQHNR... SEQ ID NO980 |
| LQHNRL... SEQ ID NO985 | | |
| | | |

| Perlecan | | |
|---|---|---|
| SIEYSP... SEQ ID NO981 | LVNFTR... SEQ ID NO982 | VSEAVV... SEQ ID NO983 |
| EVSEAV... SEQ ID NO984 | | |
| | | |

11. A method as set out in aspect 1, wherein said immunological binding partner specifically binds a neo-epitope constituted by a C-terminal amino acid sequence present in peptides produced by cleavage of biglycan, decorin, lumican, versican, or perlecan, said peptides comprising an C-terminal sequence selected from the group consisting of:

| Biglycan | | |
|---|---|---|
| ...NNDISE SEQ ID NO986 | ...YWEVQP SEQ ID NO987 | ...EDLLRY SEQ ID NO988 |
| ...RISEAK SEQ ID NO951 | ...KIQAIE SEQ ID NO989 | ...PETLNE SEQ ID NO990 |
| ...LRKDDF SEQ ID NO991 | ...LLRYSK SEQ ID NO992 | ...ELRKDD SEQ ID NO993 |
| ...KDLPET SEQ ID NO994 | ...DLLRYS SEQ ID NO995 | ...AFDGLK SEQ ID NO996 |
| ...LNELHL SEQ ID NO997 | | |
| | | |

| Decorin | | |
|---|---|---|
| ...GTNPLK SEQ ID NO998 | ...EVPDDR SEQ ID NO999 | ...GAFTPL SEQ ID NO1000 |
| ...SSGIEN SEQ ID NO968 | ...RVDAAS SEQ ID NO1001 | ...LVKLER SEQ ID 1002 |
| ...GMKKLS SEQ ID NO1003 | ...KDGDFK SEQ ID NO1004 | ...HLDGNK SEQ ID NO1005 |
| ...QPSTFR SEQ ID NO1006 | ...AFQGMK SEQ ID NO1007 | |
| | | |

| Versican | | |
|---|---|---|
| ...CDVMYG SEQ ID NO1008 | ...NGFDQC SEQ ID NO976 | ...QNGINK SEQ ID NO1009 |
| ...IGQDYK SEQ ID NO1010 | | |
| | | |

| Lumican | | |
|---|---|---|
| ...QLTHNK SEQ ID NO1011 | ...VSAAFK 1012 | ...GLKSLE 1013 |
| | | |

| Perlecan | | |
|---|---|---|
| ...EDAGSR SEQ ID NO1014 | ...EFREVS SEQ ID NO1015 | ...VAQQDS SEQ ID NO1016 |
| ...SAKEFR SEQ ID NO1017 | ...LEPEYR SEQ ID NO1018 | |
| | | |

12. A method as set out in aspect 1, wherein said immunological binding partner specifically binds a neo-epitope constituted by an N-terminal amino acid sequence present in peptides produced by cleavage of C-reactive protein, said peptides comprising an N-terminal sequence selected from the group consisting of:

| **CRP** | | |
|---|---|---|
| AFVFPK SEQ ID NO1033 | SFGGNF SEQ ID NO1305 | FGQTDM SEQ ID NO1306 |
| VSLKAP SEQ ID NO1172 | KAFVFP SEQ ID NO1167 | EVFTKP SEQ ID NO1201 |
| TDMSRK SEQ ID NO1307 | MSRKAF SEQ ID NO1308 | SRKAFV SEQ ID NO1309 |
| VFPKES SEQ ID NO1310 | FPKESD SEQ ID NO1311 | KESDTS SEQ ID NO1312 |
| LSSTRG SEQ ID NO1313 | SSTRGY SEQ ID NO1314 | STRGYS SEQ ID NO1080 |
| KRQDNE SEQ ID NO1315 | WSKDIG SEQ ID NO1316 | SKDIGY SEQ ID NO1317 |
| SIILGQ SEQ ID NO1318 | YLGGPF SEQ ID NO1322 | ILGQEQ SEQ ID NO1320 |
| IYLGGP SEQ ID NO1321 | KYEVQG SEQ ID NO1294 | LGGPFS SEQ ID NO1323 |
| ALKYEV SEQ ID NO1133 | YTELSS SEQ ID NO1325 | VQGEVF SEQ ID NO1324 |
| KPQLWP SEQ ID NO1157 | QEQDSF SEQ ID NO1328 | ILIFWS SEQ ID NO1326 |
| LVGDIG SEQ ID NO1327 | QDSFGG SEQ ID NO1331 | RGYSIF SEQ ID NO1329 |
| GAEASI SEQ ID NO1330 | DTSYVS SEQ ID NO1334 | TIYLGG SEQ ID NO1332 |
| EINTIY SEQ ID NO1333 | SPDEIN SEQ ID NO1337 | AEASII SEQ ID NO1335 |
| TSWESA SEQ ID NO1336 | FVLSPD SEQ ID NO1340 | GGPFSP SEQ ID NO1338 |
| YEVQGE SEQ ID NO1339 | IVEFWV SEQ ID NO1343 | LKKGYT SEQ ID NO1341 |
| IILGQE SEQ ID NO1319 | EVQGEV SEQ ID NO1346 | ESDTSY SEQ ID NO1344 |
| RKAFVF SEQ ID NO1342 | SLKAPL SEQ ID NO1222 | FVFPK SEQ ID NO1059 |
| SDTSYV SEQ ID NO1347 | SYATKR SEQ ID NO1349 | LKAPLT SEQ ID NO1173 |
| IFSYAT SEQ ID NO1348 | WVDGKP SEQ ID NO1352 | YATKRQ SEQ ID NO1350 |
| EFWVDG SEQ ID NO1351 | GQEQDS SEQ ID NO1355 | VDGKPR SEQ ID NO1353 |
| LGQEQD SEQ ID NO1354 | LNWRA SEQ ID NO1127 | QSLVGD SEQ ID NO1356 |
| SPNVLN SEQ ID NO1357 | GEVFTK SEQ ID NO1359 | LNWRAL SEQ ID NO1128 |
| QGEVFT SEQ ID NO1358 | VRKSLK SEQ ID NO1205 | VFTKPQ SEQ ID NO1153 |
| IFWSKD SEQ ID NO1181 | ATKRQD SEQ ID NO1362 | KKGYTV SEQ ID NO1360 |
| FSYATK SEQ ID NO1361 | NWRAL SEQ ID NO1253 | FWSKDI SEQ ID NO1363 |
| VLNWRA SEQ ID NO1249 | DFVLSP SEQ ID NO1366 | NWRALK SEQ ID NO1364 |
| GSQSLV SEQ ID NO1365 | INTIYL SEQ ID NO1372 | VLSPDE SEQ ID NO1367 |
| LKYEVQ SEQ ID NO1134 | WRALKY SEQ ID NO1375 | KSLKKG SEQ ID NO1371 |
| GNFEGS SEQ ID NO1369 | TKPQLW SEQ ID NO1345 | RALKYE SEQ ID NO1373 |
| FVFPKE SEQ ID NO1060 | KGYTVG SEQ ID NO1296 | GPFSPN SEQ ID NO1376 |
| FYTELS SEQ ID NO1374 | TKRQDN SEQ ID NO1368 | SLKKGY SEQ ID NO1370 |
| | | |

13. A method as set out in aspect 1, wherein said immunological binding partner specifically binds a neo-epitope constituted by a C-terminal amino acid sequence present in peptides produced by cleavage of C-reactive protein, said peptides comprising an C-terminal sequence selected from the group consisting of:

| **CRP** | | |
|---|---|---|
| AFVFPK SEQ ID NO1033 | KPQLWP SEQ ID NO1157 | PDEINT SEQ ID NO1377 |
| KESDTS SEQ ID NO1312 | DSFGGN SEQ ID NO1378 | VFTKPQ SEQ ID NO1153 |
| LTKPLK SEQ ID NO1379 | SDTSYV SEQ ID NO1347 | DTSYVS SEQ ID NO1334 |
| KDIGYS SEQ ID NO1380 | STRGYS SEQ ID NO1080 | YATKRQ SEQ ID NO1350 |
| GAEASI SEQ ID NO1330 | DGKPRV SEQ ID NO1381 | VDGKPR SEQ ID NO1353 |
| SPNVLN SEQ ID NO1357 | GPFSPN SEQ ID NO1376 | NFEGSQ SEQ ID NO1382 |
| ALKYEV SEQ ID NO1133 | YEVQGE SEQ ID NO1339 | LKYEVQ SEQ ID NO1134 |
| KAFVFP SEQ ID NO1167 | VSLKAP SEQ ID NO1172 | LKAPLT SEQ ID NO1173 |
| LKKGYT SEQ ID NO1341 | LGQEQD SEQ ID NO1354 | NVNMWD SEQ ID NO1383 |
| PRVRKS SEQ ID NO1384 | TVGSEI SEQ ID NO1385 | SRKAFV SEQ ID NO1309 |
| GYSFTV SEQ ID NO1386 | IILGQE SEQ ID NO1319 | EGSQSL SEQ ID NO1387 |
| INTIYL SEQ ID NO1372 | WSKDIG SEQ ID NO1316 | EQDSFG SEQ ID NO1388 |
| NVLNWR SEQ ID NO1389 | ASGIVE SEQ ID NO1390 | NTIYLG SEQ ID NO1391 |
| VGAEAS SEQ ID NO1392 | TDMSRK SEQ ID NO1307 | FVFPKE SEQ ID NO1060 |
| QTDMSR SEQ ID NO1394 | TSYVSL SEQ ID NO1396 | MSRKAF SEQ ID NO1308 |
| PKESDT SEQ ID NO1395 | QDNEIL SEQ ID NO1398 | ESDTSY SEQ ID NO1344 |
| DNEILI SEQ ID NO1397 | TVGAEA SEQ ID NO1401 | NEILIF SEQ ID NO1399 |
| YTVGAE SEQ ID NO1400 | GNFEGS SEQ ID NO1369 | DIGNVN SEQ ID NO1404 |
| FEGSQS SEQ ID NO1403 | LNWRAL SEQ ID NO1128 | NWRALK SEQ ID NO1364 |
| LNWRA SEQ ID NO1127 | EVFTKP SEQ ID NO1201 | VFTKPQ SEQ ID NO1153 |
| KYEVQG SEQ ID NO1294 | RQDNEI SEQ ID NO1405 | IFWSKD SEQ ID NO1181 |
| KRQDNE SEQ ID NO1315 | VRKSLK SEQ ID NO1205 | SYVSLK SEQ ID NO1407 |
| FTKPQL SEQ ID NO1406 | TKPLKA SEQ ID NO1408 | SIFSYA SEQ ID NO1409 |
| SLKAPL SEQ ID NO1222 | GNVNMW SEQ ID NO1410 | SQSLVG SEQ ID NO1411 |
| GSQSLV SEQ ID NO1365 | GGNFEG SEQ ID NO1413 | LVGDIG SEQ ID NO1327 |
| FGGNFE SEQ ID NO1412 | RALKYE SEQ ID NO1373 | KKGYTV SEQ ID NO1360 |
| VQGEVF SEQ ID NO1324 | GKPRVR SEQ ID NO1414 | PFSPNV SEQ ID NO1402 |
| DMSRKA SEQ ID NO1415 | EILIFW SEQ ID NO1416 | QGEVFT SEQ ID NO1358 |
| FPKESD SEQ ID NO1311 | IGYSFT SEQ ID NO1417 | SPDEIN SEQ ID NO1337 |
| APLTKP SEQ ID NO1393 | FWSKDI SEQ ID NO1363 | |
| | | |

14. A method as set out in aspect 1, wherein said immunological binding partner specifically binds a neo-epitope constituted by an N-terminal amino acid sequence present in peptides produced by cleavage of elastin, said peptides comprising an N-terminal sequence selected from the group consisting of:

| **Elastin** | | |
|---|---|---|
| GVPGAI SEQ ID NO1898 | AIPGGV SEQ ID NO1899 | GVPGGV SEQ ID NO1900 |
| ALGGGA SEQ ID NO1901 | LGGGAL SEQ ID NO1902 | GGALGP SEQ ID NO1903 |
| PLKPVP SEQ ID NO1904 | LKPVPG SEQ ID NO1905 | GLAGAG SEQ ID NO1906 |
| GLGAGL SEQ ID NO1907 | LGAGLG SEQ ID NO1908 | AGLGAF SEQ ID NO1909 |
| LVPGGV SEQ ID NO1910 | VADAAA SEQ ID NO1911 | KAAKAG SEQ ID NO1912 |
| PVGYPG SEQ ID NO1913 | ARFPGV SEQ ID NO1914 | RFPGVG SEQ ID NO1915 |
| VGPFGG SEQ ID NO1916 | GPQPGV SEQ ID NO1917 | PQPGVP SEQ ID NO1918 |
| TTGKLP SEQ ID NO1919 | LPYGYG SEQ ID NO1920 | GYGPGG SEQ ID NO1921 |
| FGAGAA SEQ ID NO1922 | GVLPGV SEQ ID NO1923 | VLPGVG SEQ ID NO1924 |
| TPAAAA SEQ ID NO1928 | VPGAIP SEQ ID NO1926 | AIPGIG SEQ ID NO1927 |
| VGVPGA SEQ ID NO1931 | PAAAAA SEQ ID NO1929 | AAAAAA SEQ ID NO1930 |
| GIPVVP SEQ ID NO1934 | AVGPGV SEQ ID NO1932 | GVPGVG SEQ ID NO1933 |
| ARPGVG SEQ ID NO1937 | IPGAAV SEQ ID NO1935 | GAAVPG SEQ ID NO1936 |
| SVGGVP SEQ ID NO1940 | RPGVGV SEQ ID NO1938 | VGGIPT SEQ ID NO1939 |
| VGVAPG SEQ ID NO1943 | VGGVPG SEQ ID NO1941 | GVGTPA SEQ ID NO1942 |
| GVPVAP SEQ ID NO1946 | VAPGVG SEQ ID NO1944 | GVAPGV SEQ ID NO1945 |
| LGAGIP SEQ ID NO1949 | GAGIPG SEQ ID NO1947 | PGGVAA SEQ ID NO1948 |
| PGALAA SEQ ID NO1952 | AKYGAA SEQ ID NO1953 | AGIPGL SEQ ID NO1925 |
| ALGGVG SEQ ID NO1955 | LGGVGI SEQ ID NO1956 | YGAAVP SEQ ID NO1954 |
| AGPAAA SEQ ID NO1958 | GPAAAA SEQ ID NO1959 | GVGIPG SEQ ID NO1957 |
| GLGVPG SEQ ID NO1961 | LGGIPP SEQ ID NO1962 | FGLVGA SEQ ID NO1960 |
| PLGGVA SEQ ID NO1964 | LGGVAA SEQ ID NO1965 | LGGVLG SEQ ID NO1963 |
| GVGLPG SEQ ID NO1967 | VGLPGV SEQ ID NO1968 | GGVAAR SEQ ID NO1966 |
| VPGVPV SEQ ID NO1970 | VPGVGI SEQ ID NO1971 | LPGVYP SEQ ID NO1969 |
| APGVGV SEQ ID NO1973 | VPGGVA SEQ ID NO1974 | VGISPE SEQ ID NO1972 |
| VPGGVF SEQ ID NO1979 | YPTGTG SEQ ID NO1977 | YPGGVL SEQ ID NO1975 |
| LGPGGK SEQ ID NO1982 | GVFYPG SEQ ID NO1980 | VPGAGV SEQ ID NO1978 |
| LAGAGL SEQ ID NO1985 | GPGGKP SEQ ID NO1983 | VFYPGA SEQ ID NO1981 |
| LGAFPA SEQ ID NO1988 | AGAGLG SEQ ID NO1986 | PGGKPL SEQ ID NO1984 |
| LGVSAG SEQ ID NO1991 | AFPAVT SEQ ID NO1989 | GAGLGA SEQ ID NO1987 |
| FPGVGV SEQ ID NO1994 | VPGVGL SEQ ID NO1992 | AVTFPG SEQ ID NO1990 |
| PGVPLG SEQ ID NO1996 | KPGAPG SEQ ID NO1995 | PGVGLP SEQ ID NO1993 |
| YGPGGV SEQ ID NO1999 | GYPIKA SEQ ID NO1997 | PKAPGV SEQ ID NO1493 |
| GAGVPG SEQ ID NO2002 | AGYPTGSEQ ID NO 2000 | PKLPGG SEQ ID NO1998 |
| IPGIGG SEQ ID NO2005 | AGVPGV SEQ ID NO2003 | TGVGPQ SEQ ID NO2001 |
| GPGFGP SEQ ID NO1976 | IGGIAG SEQ ID NO2006 | GVPGVP SEQ ID NO2004 |
| VPGVGV SEQ ID NO2008 | PGFGPG SEQ ID NO1950 | GIAGVG SEQ ID NO2007 |
| AVPGVV SEQ ID NO2011 | VPVGVA SEQ ID NO2009 | PGVVGV SEQ ID NO1951 |
| GVGAGG SEQ ID NO2014 | VPGVVS SEQ ID NO2012 | VPGAGI SEQ ID NO2010 |
| FGLVPG SEQ ID NO2017 | VGAGGF SEQ ID NO2015 | YGARPG SEQ ID NO2013 |
| PGVGLA SEQ ID NO2020 | GLVPGV SEQ ID NO2018 | VGVGGI SEQ ID NO2016 |
| LRAAAG SEQ ID NO2023 | GVGLAP SEQ ID NO2021 | LVPGVG SEQ ID NO2019 |
| GIPGLG SEQ ID NO2026 | LVGAAG SEQ ID NO2024 | VGLAPG SEQ ID NO2022 |
| VGIPGG SEQ ID NO2032 | LGVGVG SEQ ID NO2027 | LVPGGP SEQ ID NO2025 |
| GLVGAA SEQ ID NO2035 | AVPGVL SEQ ID NO2030 | VGVPGL SEQ ID NO2028 |
| GGVLGG SEQ ID NO2038 | IPGGVV SEQ ID NO2033 | VLGGLG SEQ ID NO2031 |
| GVAARP SEQ ID NO2041 | VGAAGL SEQ ID NO2036 | VVGAGP SEQ ID NO2034 |
| GVYPGG SEQ ID NO2044 | GAGQFP SEQ ID NO2039 | LGGLGV SEQ ID NO2037 |
| AAVPGV SEQ ID NO2047 | VAARPG SEQ ID NO2042 | AFQFPL SEQ ID NO2041 |
| VPGVLG SEQ ID NO2050 | AAGLGG SEQ ID NO2048 | RPGFGL SEQ ID NO2043 |
| ISPEAQ SEQ ID NO2053 | GQFPLG SEQ ID NO2051 | FGPGVV SEQ ID NO2046 |
| LGALGG SEQ ID NO2056 | GVLPGA SEQ ID NO2054 | FPGALV SEQ ID NO2049 |
| GKPLKP SEQ ID NO2059 | PQAAAA SEQ ID NO2077 | ALVPGG SEQ ID NO2052 |
| AGLGAG SEQ ID NO2062 | VPGVPG SEQ ID NO2057 | AAAGLG SEQ ID NO2029 |
| VTFPGA SEQ ID NO2065 | IAGVGT SEQ ID NO2060 | VGAGVP SEQ ID NO2055 |
| GLPGVY SEQ ID NO2068 | VVGVPG SEQ ID NO2063 | GGLGAL SEQ ID NO2058 |
| GAFAGI SEQ ID NO2071 | AGIPVV SEQ ID NO2066 | VGAGPA SEQ ID NO2061 |
| YTTGKL SEQ ID NO2074 | GARPGV SEQ ID NO2069 | LGVGGL SEQ ID NO2064 |
| PGVGVA SEQ ID NO2075 | VAGVPS SEQ ID NO2072 | FPLGGV SEQ ID NO2067 |
| LAPGVG SEQ ID NO2078 | VGTPAA SEQ ID NO2076 | PGFGLS SEQ ID NO2070 |
| LPYTTG SEQ ID NO2045 | GPGVVG SEQ ID NO2073 | |

15. A method as set out in aspect 1, wherein said immunological binding partner specifically binds a neo-epitope constituted by a C-terminal amino acid sequence present in peptides produced by cleavage of elastin, said peptides comprising an C-terminal sequence selected from the group consisting of:

| **Elastin** | | |
|---|---|---|
| PGGVPG SEQ ID NO2079 | PGAGLG SEQ ID NO2080 | GAGLGA SEQ ID NO1987 |
| GALGGG SEQ ID NO2081 | LGGGAL SEQ ID NO1902 | GGGALG SEQ ID NO2082 |
| GLGAFP SEQ ID NO2083 | LGAFPA SEQ ID NO1988 | LGAGLG SEQ ID NO1908 |
| VPGGVA SEQ ID NO1974 | ADAAAA SEQ ID NO2084 | VPTGAG SEQ ID NO2087 |
| RFPGVG SEQ ID NO1915 | VGVLPG SEQ ID NO2086 | VPLGYP SEQ ID NO2089 |
| PKAPGV SEQ ID NO1493 | GVGPFG SEQ ID NO2088 | IPGIGG SEQ ID NO2005 |
| LPYGYG SEQ ID NO1920 | AAAAAK SEQ ID NO2090 | IGGIAG SEQ ID NO2006 |
| GIAGVG SEQ ID NO2007 | AIPGIG SEQ ID NO1927 | GVPGVG SEQ ID NO1933 |
| AAAAKA SEQ ID NO2091 | VVGVPG SEQ ID NO2063 | VVSPEA SEQ ID NO2094 |
| GVGVPG SEQ ID NO2092 | PVVPGA SEQ ID NO2093 | GIPTYG SEQ ID NO2096 |
| VGGIPT SEQ ID NO1939 | GGIPTY SEQ ID NO2095 | GVGAGG SEQ ID NO2014 |
| GVGVGG SEQ ID NO2097 | GVGGIP SEQ ID NO2098 | VAPGVG SEQ ID NO1944 |
| GFPGFG SEQ ID NO2099 | FGVGVG SEQ ID NO2100 | APGVGL SEQ ID NO2104 |
| PGVGIS SEQ ID NO2101 | SPEAQA SEQ ID NO2102 | VAPGIG SEQ ID NO2107 |
| PGVGVA SEQ ID NO2075 | GVGVAP SEQ ID NO2103 | AAGLGA SEQ ID NO2109 |
| GIGPGG SEQ ID NO2105 | APGIGP SEQ ID NO2106 | AGVPGL SEQ ID NO2112 |
| RAAAGL SEQ ID NO2108 | AAAGLG SEQ ID NO2029 | GAGPAA SEQ ID NO2113 |
| GVPGLG SEQ ID NO2110 | GVPGFG SEQ ID NO2111 | GLGGLG SEQ ID NO2115 |
| VLGGLG SEQ ID NO2031 | VGIPGG SEQ ID NO2032 | PAAAAK SEQ ID NO2117 |
| PAAAAA SEQ ID NO1929 | VPGVGG SEQ ID NO2114 | AARPGF SEQ ID NO2118 |
| GLGVPG SEQ ID NO1961 | PGVGGL SEQ ID NO2116 | GPGIPG SEQ ID NO2121 |
| RPGFGL SEQ ID NO2043 | GVAARP SEQ ID NO2041 | ALGPGG SEQ ID NO2124 |
| LSPIFP SEQ ID NO2119 | AQAAAA SEQ ID NO2120 | KPVPGG SEQ ID NO2127 |
| GPGGVA SEQ ID NO2122 | LGALGG SEQ ID NO2056 | VTFPGA SEQ ID NO2065 |
| GLGALG SEQ ID NO2123 | PGAIPG SEQ ID NO2146 | VPQPGA SEQ ID NO2130 |
| GALGPG SEQ ID NO2125 | TPAAAA SEQ ID NO1928 | AGVKPK SEQ ID NO2133 |
| AFPAVT SEQ ID NO1989 | AVTFPG SEQ ID NO1990 | YGYGPG SEQ ID NO2136 |
| AAAAYK SEQ ID NO2128 | AAKAGA SEQ ID NO2129 | GVGTPA SEQ ID NO1942 |
| PGVPTG SEQ ID NO2131 | GVPTGA SEQ ID NO2132 | AAAAAA SEQ ID NO1930 |
| PIKAPK SEQ ID NO2134 | KLPGGY SEQ ID NO2135 | GVPGAG SEQ ID NO2140 |
| VGTPAA SEQ ID NO2076 | VPGVPG SEQ ID NO2057 | ARPGVG SEQ ID NO1937 |
| GIGGIA SEQ ID NO2137 | GTPAAA SEQ ID NO2138 | YGVGAG SEQ ID NO2144 |
| IPVVPG SEQ ID NO2139 | VGVPGA SEQ ID NO1931 | VGAGGF SEQ ID NO2015 |
| GAGIPG SEQ ID NO1947 | PEAAAK SEQ ID NO2141 | GISPEA SEQ ID NO2149 |
| IPTYGV SEQ ID NO2145 | TYGVGA SEQ ID NO2143 | VPGAPG SEQ ID NO2150 |
| AGGFPG SEQ ID NO2147 | PTYGVG SEQ ID NO2142 | APGVGV SEQ ID NO1973 |
| VGVAPG SEQ ID NO1943 | GIPGVA SEQ ID NO2148 | PGIGPG SEQ ID NO2152 |
| PGVGLA SEQ ID NO2020 | VGLAPG SEQ ID NO2022 | LGVGVG SEQ ID NO2027 |
| GAGVPG SEQ ID NO2002 | LAPGVG SEQ ID NO2078 | LGGLGA SEQ ID NO2157 |
| PGVGVG SEQ ID NO2169 | GGVAAA SEQ ID NO2151 | GPAAAA SEQ ID NO1959 |
| GLGVGG SEQ ID NO2153 | PGLGVG SEQ ID NO2154 | GVGGLG SEQ ID NO2159 |
| GLGVGA SEQ ID NO2155 | ALAAAK SEQ ID NO2156 | VAARPG SEQ ID NO2042 |
| VGAGPA SEQ ID NO2061 | AGPAAA SEQ ID NO1958 | IFPGGA SEQ ID NO2163 |
| GGLGVG SEQ ID NO2158 | LGVGGL SEQ ID NO2064 | AAKAAK SEQ ID NO2166 |
| AGQFPL SEQ ID NO2160 | GGVAAR SEQ ID NO1966 | PGGVAA SEQ ID NO1948 |
| GFGLSP SEQ ID NO2161 | PIFPGG SEQ ID NO2162 | VGVPGL SEQ ID NO2028 |
| AAKFGA SEQ ID NO2164 | AAKYGA SEQ ID NO2165 | PGVLGG SEQ ID NO2085 |
| AGLGAL SEQ ID NO2167 | RPGVGV SEQ ID NO1938 | GIPGGV SEQ ID NO2168 |
| LKPVPG SEQ ID NO1905 | GGFPGF SEQ ID NO2173 | IPPAAA SEQ ID NO2170 |
| ALVPGG SEQ ID NO2052 | VGGVPG SEQ ID NO1941 | ARPGFG SEQ ID NO2171 |
| VLPGAR SEQ ID NO2172 | GVAPGV SEQ ID NO1945 | GLSPIF SEQ ID NO2174 |
| PTGAGV SEQ ID NO2175 | VAPVGV SEQ ID NO2126 | GIPVVP SEQ ID NO1934 |
| AGAAGK SEQ ID NO2176 | | |
| | | |

16. A method as set out in aspect 1, wherein said immunological binding partner specifically binds a neo-epitope constituted by an N-terminal amino acid sequence present in peptides produced by cleavage of collagen type V, said peptides comprising an N-terminal sequence selected from the group consisting of:

| Collagen type V | | |
|---|---|---|
| GDPGPP SEQ ID NO373 | LRGIPG SEQ ID NO760 | IGPPGI SEQ ID NO761 |
| LQGPPG SEQ ID NO62 | IGSLGH SEQ ID NO762 | IRGPPG SEQ ID NO763 |
| ANGSPG SEQ ID NO764 | LIGTPG SEQ ID NO765 | LPGEPG SEQ ID NO766 |
| IPGRPG SEQ ID NO767 | GPDGPP SEQ ID NO768 | QPGPSG SEQ ID NO769 |
| LKGNEG SEQ ID NO770 | ERGHPG SEQ ID NO771 | GPPGEQ SEQ ID NO772 |
| FPGPKG SEQ ID NO491 | PFRFGG SEQ ID NO773 | ESQAQA SEQ ID NO774 |
| LLGPKG SEQ ID NO775 | QQGNPG SEQ ID NO776 | KEGPPG SEQ ID NO777 |
| IGLIGP SEQ ID NO778 | GPPGPP SEQ ID NO100 | LGPPGE SEQ ID NO779 |
| GPPGPK SEQ ID NO780 | SLGHPG SEQ ID NO781 | KDGIPG SEQ ID NO782 |
| PVGEPG SEQ ID NO783 | LRGIPG SEQ ID NO760 | KDGIPG SEQ ID NO782 |
| ANGSPG SEQ ID NO764 | LIGTPG SEQ ID NO765 | PGEPGP SEQ ID NO784 |
| KDGIP SEQ ID NO813 | VLGPQG SEQ ID NO814 | DGIPGP SEQ ID NO661 |
| QMGPPG SEQ ID NO802 | LLGAPG SEQ ID NO785 | GLPGLE SEQ ID NO786 |
| ALRGPA SEQ ID NO810 | LALRGP SEQ ID NO788 | LTGRPG SEQ ID NO789 |
| ETGFQG SEQ ID NO808 | LRGFPG SEQ ID NO790 | KTGPIG SEQ ID NO791 |
| EAGHPG SEQ ID NO811 | PGPKGN SEQ ID NO527 | EQGLPG SEQ ID NO793 |
| GSKGPM SEQ ID NO809 | AIGGPP SEQ ID NO794 | GPNGDP SEQ ID NO795 |
| EKGHPG SEQ ID NO812 | LLGPRG SEQ ID NO797 | GPDGPP SEQ ID NO768 |
| GPKGSI SEQ ID NO816 | GPKGDP SEQ ID NO799 | GDPGPP SEQ ID NO373 |
| ELGFQG SEQ ID NO817 | LQGPPG SEQ ID NO62 | EKGHIG SEQ ID NO801 |
| LRGPAG SEQ ID NO818 | SRGERG SEQ ID NO803 | EKGKSG SEQ ID NO804 |
| EDGERG SEQ ID NO815 | LPGEPG SEQ ID NO766 | PQGAIG SEQ ID NO806 |
| GIPGEK SEQ ID NO787 | PPGRPG SEQ ID NO792 | PGPPGE SEQ ID NO796 |
| LLGPKG SEQ ID NO775 | GPPGEK SEQ ID NO640 | ERGPNG SEQ ID NO798 |
| GPMGLT SEQ ID NO807 | GPIGEK SEQ ID NO805 | PGIPGE SEQ ID NO800 |
| QMGPPG SEQ ID NO802 | | |

17. A method as set out in aspect 1, wherein said immunological binding partner specifically binds a neo-epitope constituted by a C-terminal amino acid sequence present in peptides produced by cleavage of collagen type V, said peptides comprising an C-terminal sequence selected from the group consisting of:

| Collagen type V | | |
|---|---|---|
| PIGSLG SEQ ID NO819 | PVGEPG SEQ ID NO783 | PGPPGE SEQ ID NO796 |
| PPGPTG SEQ ID NO820 | PKGPPG SEQ ID NO821 | VAGPLG SEQ ID NO822 |
| RPGVTG SEQ ID NO823 | MMPFQF SEQ ID NO824 | PGAAGP SEQ ID NO825 |
| PVGPAG SEQ ID NO826 | HEGPTG SEQ ID NO827 | EPGPRG SEQ ID NO516 |
| GPPGLP SEQ ID NO828 | GQGPPG SEQ ID NO664 | PPGPPG SEQ ID NO119 |
| AGSPGE SEQ ID NO829 | PVGALG SEQ ID NO830 | EQGLPG SEQ ID NO793 |
| YPGPRG SEQ ID NO831 | HPGQRG SEQ ID NO832 | GGGGDA SEQ ID NO833 |
| LIGPPG SEQ ID NO834 | GLPGLK SEQ ID NO835 | PPGPPG SEQ ID NO119 |
| PPGPIG SEQ ID NO174 | KGLPGL SEQ ID NO836 | PGPPGP SEQ ID NO458 |
| QMGPPG SEQ ID NO802 | LLGAPG SEQ ID NO785 | RPGVTG SEQ ID NO823 |
| QQARLA SEQ ID NO837 | PPGHPG SEQ ID NO838 | PQGPRG SEQ ID NO150 |
| PLGPPG SEQ ID NO839 | GEPGLL SEQ ID NO840 | EVGPLG SEQ ID NO841 |
| IMMPFQ SEQ ID NO842 | GLPGLK SEQ ID NO835 | PLGPSG SEQ ID NO843 |
| AAGPSG SEQ ID NO844 | PPGPLG SEQ ID NO845 | PPGSRG SEQ ID NO846 |
| PAGPMG SEQ ID NO847 | PPGSGG SEQ ID NO848 | PPGPPG SEQ ID NO119 |
| GLPGPV SEQ ID NO849 | LPGPVG SEQ ID NO850 | KPGPDG SEQ ID NO851 |
| LPGPQG SEQ ID NO852 | VIQPLP SEQ ID NO853 | RGPNGP SEQ ID NO854 |
| PGPQGS SEQ ID NO855 | EKGPLG SEQ ID NO856 | PLGPPG SEQ ID NO839 |
| PPGPLG SEQ ID NO845 | TGPKGE SEQ ID NO858 | GPKGSI SEQ ID NO816 |
| GPPGAA SEQ ID NO857 | PPGPAG SEQ ID NO52 | PPGPTG SEQ ID NO820 |
| LLGAPG SEQ ID NO785 | PLGPLG SEQ ID NO863 | PPGSRG SEQ ID NO846 |
| PSGLPG SEQ ID NO860 | LPGPPG SEQ ID NO72 | KPGPRG SEQ ID NO862 |
| PPGPAG SEQ ID NO52 | PPGLPG SEQ ID NO861 | PKGPPG SEQ ID NO821 |
| | QGLPGL SEQ ID NO859 | PPGSRG SEQ ID NO846 |
| | | |

18. A method as set out in aspect 1, wherein said immunological binding partner specifically binds a neo-epitope constituted by an N-terminal amino acid sequence present in peptides produced by cleavage of collagen type VI, said peptides comprising an N-terminal sequence selected from the group consisting of:

| Collagen type VI | | |
|---|---|---|
| YRGPEG SEQ ID NO883 | PIGPKG SEQ ID NO865 | GIGIGN SEQ ID NO885 |
| ISGPRG SEQ ID NO886 | PGPAGP SEQ ID NO887 | VAAKPA SEQ ID NO888 |
| GEPGPP SEQ ID NO675 | RGPIGS SEQ ID NO889 | PPPPQP SEQ ID NO890 |
| AQGPAG SEQ ID NO891 | LIGEQG SEQ ID NO892 | PGLIGE SEQ ID NO893 |
| GEPGLN SEQ ID NO894 | IGPKGI SEQ ID NO895 | VAVVQH SEQ ID NO896 |
| FGPSAA SEQ ID NO897 | GPKGET SEQ ID NO898 | LGPMGV SEQ ID NO899 |
| PGEPGP SEQ ID NO784 | | |

19. A method as set out in aspect 1, wherein said immunological binding partner specifically binds a neo-epitope constituted by a C-terminal amino acid sequence present in peptides produced by cleavage of collagen type VI, said peptides comprising an C-terminal sequence selected from the group consisting of:

| Collagen type VI | | |
|---|---|---|
| GDEGPP SEQ ID NO900 | GNADIT SEQ ID NO901 | PAGPPG SEQ ID NO133 |
| DPGLMG SEQ ID NO902 | PEVPRP SEQ ID NO903 | TGPKGI SEQ ID NO904 |
| GDEGGP SEQ ID NO905 | PARSAS SEQ ID NO906 | TPAPPG SEQ ID NO915 |
| ISGPRG SEQ ID NO886 | GISGPR SEQ ID NO907 | GIGNRG SEQ ID NO908 |
| YRGYPG SEQ ID NO909 | KVEFSL SEQ ID NO910 | GVPGRD SEQ ID NO911 |
| PGETGK SEQ ID NO912 | RTGPLG SEQ ID NO913 | APGERG SEQ ID NO914 |

20. A method as set out in aspect 1, wherein said immunological binding partner specifically binds a neo-epitope constituted by a N-terminal amino acid sequence present in peptides produced by cleavage of vimentin, said peptides comprising an N-terminal sequence selected from the group consisting of:

| **Vimentin** | | |
|---|---|---|
| LLQDSV SEQ ID NO2182 | FADLSE SEQ ID NO2183 | ISLPLP SEQ ID NO2184 |

21. A method as set out in aspect 1, wherein said immunological binding partner specifically binds a neo-epitope constituted by a C-terminal amino acid sequence present in peptides produced by cleavage of vimentin, said peptides comprising an C-terminal sequence selected from the group consisting of:

| **Vimentin** | |
|---|---|
| SVPGVR SEQ ID NO2185 | SVPGVL SEQ ID NO2186 |

22. A method as set out in aspect 1, subject to the further proviso that if the neo-epitopes are formed by cleavage of type I collagen, they are not at locations where type I collagen is cleaved by trypsin.
23. A method of immunoassay to measure neo-epitope containing protein fragments naturally present in body fluid sample, wherein said immunoassay is conducted by a method comprising: contacting protein fragments naturally present in said sample with an immunological binding partner reactive with a neo-epitope formed by cleavage of a protein by a proteinase and measuring the extent of binding of peptide fragments to said immunological binding partner to measure therein protein fragments comprising said neo-epitope, and wherein said protein is neurocan, brevican, fibromodulin, serglycin, syndecan, betaglycan, elastin, collagen type I, collagen type IV, collagen type V or collagen type VI, CRP, or vimentin, subject to the proviso that when the neo-epitopes are formed by cleavage of type I collagen, the cleavage is not at a site at which collagen type I is cleaved by cathepsin K.

### Reference List

1. World Health Organization. Reducing Risks, Promoting Healthy Life. Reducing Risks, Promoting Healthy Life, Geneva: WHO, 2002:1-230.
2. Wynn TA. Cellular and molecular mechanisms of fibrosis. J Pathol 2008;214:199-210.
3. Friedman SL. Mechanisms of disease: Mechanisms of hepatic fibrosis and therapeutic implications. Nat Clin Pract Gastroenterol Hepatol 2004;1:98-105.
4. Tomasek JJ, Gabbiani G, Hinz B, Chaponnier C, Brown PA. Myofibroblasts and mechano-regulation of connective tissue remodelling. Nat Rev Mol Cell Biol 2002;3:349-363.
5. Wynn TA. Common and unique mechanisms regulate fibrosis in various fibroproliferative diseases. J Clin Invest 2007;117:524-529.
6. Marcellin P, Asselah T, Boyer N. Fibrosis and disease progression in hepatitis C. Hepatology 2002;36:S47-S56.
7. Gagliano N, Arosio B, Grizzi F, Masson S, Tagliabue J, Dioguardi N, Vergani C, Annoni G. Reduced collagenolytic activity of matrix metalloproteinases and development of liver fibrosis in the aging rat. Mech Ageing Dev 2002;123:413-425.
8. Laurent GJ. Dynamic state of collagen: pathways of collagen degradation in vivo and their possible role in regulation of collagen mass. Am J Physiol 1987;252:C1-C9.
9. Mays PK, McAnulty RJ, Campa JS, Laurent GJ. Age-related changes in collagen synthesis and degradation in rat tissues. Importance of degradation of newly synthesized collagen in regulating collagen production. Biochem J 1991;276 ( Pt 2):307-313.
10. Garrone R, Lethias C, Le Guellec D. Distribution of minor collagens during skin development. Microsc Res Tech 1997;38:407-412.
11. Gelse K, Poschl E, Aigner T. Collagens--structure, function, and biosynthesis. Adv Drug Deliv Rev 2003;55:1531-1546.
12. Phan SH, Thrall RS. Pulmonary Fibrosis. Lung Biology in Health and Disease. 80 ed. New York: Marcel Dekker, Inc., 1995.
13. Martinez-Hernandez A, Amenta PS. The hepatic extracellular matrix. II. Ontogenesis, regeneration and cirrhosis. Virchows Arch A Pathol Anat Histopathol 1993;423:77-84.
14. Gilliam AC. Scleroderma. Curr Dir Autoimmun 2008;10:258-279.
15. Gressner AM, Weiskirchen R. Modern pathogenetic concepts of liver fibrosis suggest stellate cells and TGF-beta as major players and therapeutic targets. J Cell Mol Med 2006;10:76-99.
16. Heinegard D, Oldberg A. Structure and biology of cartilage and bone matrix noncollagenous macromolecules. FASEB J 1989;3:2042-2051.
17. Svensson L, Oldberg A, Heinegard D. Collagen binding proteins. Osteoarthritis and Cartilage 2001;9:S23-S28.
18. Kiani C, Chen L, Wu YJ, Yee AJ, Yang BB. Structure and function of aggrecan. Cell Res 2002;12:19-32.
19. Krusius T, Gehlsen KR, Ruoslahti E. A fibroblast chondroitin sulfate proteoglycan core protein contains lectin-like and growth factor-like sequences. J Biol Chem 1987;262:13120-13125.
20. Yang BL, Zhang Y, Cao L, Yang BB. Cell adhesion and proliferation mediated through the G1 domain of versican. J Cell Biochem 1999;72:210-220.
21. Rauch U, Karthikeyan L, Maurel P, Margolis RU, Margolis RK. Cloning and primary structure of neurocan, a developmentally regulated, aggregating chondroitin sulfate proteoglycan of brain. J Biol Chem 1992;267:19536-19547.
22. Yamada H, Watanabe K, Shimonaka M, Yamaguchi Y. Molecular cloning of brevican, a novel brain proteoglycan of the aggrecan/versican family. J Biol Chem 1994;269:10119-10126.
23. Blochberger TC, Cornuet PK, Hassell JR. Isolation and partial characterization of lumican and decorin from adult chicken corneas. A keratan sulfate-containing isoform of decorin is developmentally regulated. J Biol Chem 1992;267:20613-20619.
24. Fisher LW, Termine JD, Young MF. Deduced protein sequence of bone small proteoglycan I (biglycan) shows homology with proteoglycan II (decorin) and several nonconnective tissue proteins in a variety of species. J Biol Chem 1989;264:4571-4576.
25. Toyama-Sorimachi N, Sorimachi H, Tobita Y, Kitamura F, Yagita H, Suzuki K, Miyasaka M. A novel ligand for CD44 is serglycin, a hematopoietic cell lineage-specific proteoglycan. Possible involvement in lymphoid cell adherence and activation. J Biol Chem 1995;270:7437-7444.
26. Bartlett AH, Hayashida K, Park PW. Molecular and cellular mechanisms of syndecans in tissue injury and inflammation. Mol Cells 2007;24:153-166.
27. Lopez-Casillas F, Wrana JL, Massague J. Betaglycan presents ligand to the TGF beta signaling receptor. Cell 1993;73:1435-1444.
28. Olsen BR. Life without perlecan has its problems. J Cell Biol 1999;147:909-912.
29. Gabay C, Kushner I. Acute-phase proteins and other systemic responses to inflammation. N Engl J Med 1999;340:448-454.
30. Benyon RC, Arthur MJ. Extracellular matrix degradation and the role of hepatic stellate cells. Semin Liver Dis 2001;21:373-384.
31. Guo J, Friedman SL. Hepatic fibrogenesis. Semin Liver Dis 2007;27:413-426.
32. Iredale JP, Benyon RC, Arthur MJ, Ferris WF, Alcolado R, Winwood PJ, Clark N, Murphy G. Tissue inhibitor of metalloproteinase-1 messenger RNA expression is enhanced relative to interstitial collagenase messenger RNA in experimental liver injury and fibrosis. Hepatology 1996;24:176-184.
33. Lee KN, Jackson KW, Christiansen VJ, Lee CS, Chun JG, McKee PA. Antiplasmin-cleaving enzyme is a soluble form of fibroblast activation protein. Blood 2006;107:1397-1404.
34. Acharya PS, Zukas A, Chandan V, Katzenstein AL, Pure E. Fibroblast activation protein: a serine protease expressed at the remodeling interface in idiopathic pulmonary fibrosis. Hum Pathol 2006;37:352-360.
35. Levy MT, McCaughan GW, Marinos G, Gorrell MD. Intrahepatic expression of the hepatic stellate cell marker fibroblast activation protein correlates with the degree of fibrosis in hepatitis C virus infection. Liver 2002;22:93-101.
36. Meyer O. Prognostic markers for systemic sclerosis. Joint Bone Spine 2006;73:490-494.
37. Hummers LK. Microvascular damage in systemic sclerosis: detection and monitoring with biomarkers. Curr Rheumatol Rep 2006;8:131-137.
38. McHugh NJ, Distler O, Giacomelli R, Riemekasten G. Non organ based laboratory markers in systemic sclerosis. Clin Exp Rheumatol 2003;21:S32-S38.
39. Muller-Quernheim J. Serum markers for the staging of disease activity of sarcoidosis and other interstitial lung diseases of unknown etiology. Sarcoidosis Vasc Diffuse Lung Dis 1998;15:22-37.
40. Gressner OA, Weiskirchen R, Gressner AM. Biomarkers of liver fibrosis: clinical translation of molecular pathogenesis or based on liver-dependent malfunction tests. Clin Chim Acta 2007;381:107-113.
41. Gressner OA, Weiskirchen R, Gressner AM. Biomarkers of hepatic fibrosis, fibrogenesis and genetic pre-disposition pending between fiction and reality. J Cell Mol Med 2007;11:1031-1051.
42. Mariat C. [Diagnosis and follow-up of chronic kidney graft dysfunction: from DFG to new biomarkers]. Nephrol Ther 2008;4 Suppl 3:S204-S207.
43. Yoneda M, Mawatari H, Fujita K, Iida H, Yonemitsu K, Kato S, Takahashi H, Kirikoshi H, Inamori M, Nozaki Y, Abe Y, Kubota K, Saito S, Iwasaki T, Terauchi Y, Togo S, Maeyama S, Nakajima A. High-sensitivity C-reactive protein is an independent clinical feature of nonalcoholic steatohepatitis (NASH) and also of the severity of fibrosis in NASH. J Gastroenterol 2007;42:573-582.
44. Wong VS, Hughes V, Trull A, Wight DG, Petrik J, Alexander GJ. Serum hyaluronic acid is a useful marker of liver fibrosis in chronic hepatitis C virus infection. J Viral Hepat 1998;5:187-192.
45. Parise ER, Oliveira AC, Figueiredo-Mendes C, Lanzoni V, Martins J, Nader H, Ferraz ML. Noninvasive serum markers in the diagnosis of structural liver damage in chronic hepatitis C virus infection. Liver Int 2006;26:1095-1099.
46. McHutchison JG, Blatt LM, de Medina M, Craig JR, Conrad A, Schiff ER, Tong MJ. Measurement of serum hyaluronic acid in patients with chronic hepatitis C and its relationship to liver histology. Consensus Interferon Study Group. J Gastroenterol Hepatol 2000;15:945-951.
47. Camacho VR, Silveira TR, Oliveira JR, Barros SG, Cerski CT. Relationship between serum concetrations of type III procollagen, hyluronic acid and histopathological findings in the liver of HCV-positive blood donors. Arq Gastroenterol 2007;44:118-122.
48. Lorenzo-Zuniga V, Bartoli R, Masnou H, Montoliu S, Morillas RM, Planas R. Serum concentrations of insulin-like growth factor-I (igf-I) as a marker of liver fibrosis in patients with chronic hepatitis C. Dig Dis Sci 2007;52:3245-3250.
49. Manolakopoulos S, Bethanis S, Liapi C, Stripeli F, Sklavos P, Margeli A, Christidou A, Katsanika A, Vogiatzakis E, Tzourmakliotis D, Theocharis S. An assessment of serum leptin levels in patients with chronic viral hepatitis: a prospective study. BMC Gastroenterol 2007;7:17.
50. Camacho VR, Silveira TR, Oliveira JR, Barros SG, Cerski CT. Relationship between serum concetrations of type III procollagen, hyluronic acid and histopathological findings in the liver of HCV-positive blood donors. Arq Gastroenterol 2007;44:118-122.
51. Leroy V, Hilleret MN, Sturm N, Trocme C, Renversez JC, Faure P, Morel F, Zarski JP. Prospective comparison of six non-invasive scores for the diagnosis of liver fibrosis in chronic hepatitis C. J Hepatol 2007;46:775-782.
52. Trocme C, Leroy V, Sturm N, Hilleret MN, Bottari S, Morel F, Zarski JP. Longitudinal evaluation of a fibrosis index combining MMP-1 and PIIINP compared with MMP-9, TIMP-1 and hyaluronic acid in patients with chronic hepatitis C treated by interferon-alpha and ribavirin. J Viral Hepat 2006;13:643-651.
53. Zheng M, Cai WM, Weng HL, Liu RH. ROC curves in evaluation of serum fibrosis indices for hepatic fibrosis. World J Gastroenterol 2002;8:1073-1076.
54. Lebensztejn DM, Sobaniec-Lotowska ME, Bauer M, Kaczmarski M, Voelker M, Schuppan D. Serum fibrosis markers as predictors of an antifibrotic effect of interferon alfa in children with chronic hepatitis B. Eur J Gastroenterol Hepatol 2005;17:843-848.
55. Lebensztejn DM, Sobaniec-Lotowska ME, Kaczmarski M, Voelker M, Schuppan D. Matrix-derived serum markers in monitoring liver fibrosis in children with chronic hepatitis B treated with interferon alpha. World J Gastroenterol 2006;12:3338-3343.
56. Tsochatzis E, Papatheodoridis GV, Hadziyannis E, Georgiou A, Kafiri G, Tiniakos DG, Manesis EK, Archimandritis AJ. Serum adipokine levels in chronic liver diseases: association of resistin levels with fibrosis severity. Scand J Gastroenterol 2008;43:1128-1136.
57. Patel K, Gordon SC, Jacobson I, Hezode C, Oh E, Smith KM, Pawlotsky JM, McHutchison JG. Evaluation of a panel of non-invasive serum markers to differentiate mild from moderate-to-advanced liver fibrosis in chronic hepatitis C patients. J Hepatol 2004;41:935-942.
58. Lieber CS, Weiss DG, Paronetto F. Value of fibrosis markers for staging liver fibrosis in patients with precirrhotic alcoholic liver disease. Alcohol Clin Exp Res 2008;32:1031-1039.
59. Forns X, Ampurdanes S, Llovet JM, Aponte J, Quinto L, Martinez-Bauer E, Bruguera M, Sanchez-Tapias JM, Rodes J. Identification of chronic hepatitis C patients without hepatic fibrosis by a simple predictive model. Hepatology 2002;36:986-992.
60. Bourliere M, Penaranda G, Renou C, Botta-Fridlund D, Tran A, Portal I, Lecomte L, Castellani P, Rosenthal-Allieri MA, Gerolami R, Ouzan D, Deydier R, Degott C, Halfon P. Validation and comparison of indexes for fibrosis and cirrhosis prediction in chronic hepatitis C patients: proposal for a pragmatic approach classification without liver biopsies. J Viral Hepat 2006;13:659-670.
61. Cacoub P, Carrat F, Bedossa P, Lambert J, Penaranda G, Perronne C, Pol S, Halfon P. Comparison of non-invasive liver fibrosis biomarkers in HIV/HCV co-infected patients: the fibrovic study--ANRS HC02. J Hepatol 2008;48:765-773.
62. Nunes D, Fleming C, Offner G, O'Brien M, Tumilty S, Fix O, Heeren T, Koziel M, Graham C, Craven DE, Stuver S, Horsburgh CR, Jr. HIV infection does not affect the performance of noninvasive markers of fibrosis for the diagnosis of hepatitis C virus-related liver disease. J Acquir Immune Defic Syndr 2005;40:538-544.
63. Grigorescu M, Rusu M, Neculoiu D, Radu C, Serban A, Catanas M, Grigorescu MD. The FibroTest value in discriminating between insignificant and significant fibrosis in chronic hepatitis C patients. The Romanian experience. J Gastrointestin Liver Dis 2007;16:31-37.
64. Halfon P, Bacq Y, De MA, Penaranda G, Bourliere M, Ouzan D, Tran A, Botta D, Renou C, Brechot MC, Degott C, Paradis V. Comparison of test performance profile for blood tests of liver fibrosis in chronic hepatitis C. J Hepatol 2007;46:395-402.
65. Halfon P, Bourliere M, Deydier R, Botta-Fridlund D, Renou C, Tran A, Portal I, Allemand I, Bertrand JJ, Rosenthal-Allieri A, Rotily M, Sattonet C, Benderitter T, Saint Paul MC, Bonnot HP, Penaranda G, Degott C, Masseyeff MF, Ouzan D. Independent prospective multicenter validation of biochemical markers (fibrotest-actitest) for the prediction of liver fibrosis and activity in patients with chronic hepatitis C: the fibropaca study. Am J Gastroenterol 2006;101:547-555.
66. Leroy V, Halfon P, Bacq Y, Boursier J, Rousselet MC, Bourliere M, De MA, Sturm N, Hunault G, Penaranda G, Brechot MC, Trocme C, Cales P. Diagnostic accuracy, reproducibility and robustness of fibrosis blood tests in chronic hepatitis C: a meta-analysis with individual data. Clin Biochem 2008;41:1368-1376.
67. Ratziu V, Massard J, Charlotte F, Messous D, Imbert-Bismut F, Bonyhay L, Tahiri M, Munteanu M, Thabut D, Cadranel JF, Le BB, de L, V, Poynard T. Diagnostic value of biochemical markers (FibroTest-FibroSURE) for the prediction of liver fibrosis in patients with non-alcoholic fatty liver disease. BMC Gastroenterol 2006;6:6.
68. Poynard T, Imbert-Bismut F, Ratziu V, Chevret S, Jardel C, Moussalli J, Messous D, Degos F. Biochemical markers of liver fibrosis in patients infected by hepatitis C virus: longitudinal validation in a randomized trial. J Viral Hepat 2002;9:128-133.
69. Poynard T, Munteanu M, Imbert-Bismut F, Charlotte F, Thabut D, Le CS, Messous D, Thibault V, Benhamou Y, Moussalli J, Ratziu V. Prospective analysis of discordant results between biochemical markers and biopsy in patients with chronic hepatitis C. Clin Chem 2004;50:1344-1355.
70. Poynard T, Morra R, Halfon P, Castera L, Ratziu V, Imbert-Bismut F, Naveau S, Thabut D, Lebrec D, Zoulim F, Bourliere M, Cacoub P, Messous D, Munteanu M, de L, V. Meta-analyses of FibroTest diagnostic value in chronic liver disease. BMC Gastroenterol 2007;7:40.
71. Ngo Y, Munteanu M, Messous D, Charlotte F, Imbert-Bismut F, Thabut D, Lebray P, Thibault V, Benhamou Y, Moussalli J, Ratziu V, Poynard T. A prospective analysis of the prognostic value of biomarkers (FibroTest) in patients with chronic hepatitis C. Clin Chem 2006;52:1887-1896.
72. Naveau S, Raynard B, Ratziu V, Abella A, Imbert-Bismut F, Messous D, Beuzen F, Capron F, Thabut D, Munteanu M, Chaput JC, Poynard T. Biomarkers for the prediction of liver fibrosis in patients with chronic alcoholic liver disease. Clin Gastroenterol Hepatol 2005;3:167-174.
73. Myers RP, Tainturier MH, Ratziu V, Piton A, Thibault V, Imbert-Bismut F, Messous D, Charlotte F, Di M, V, Benhamou Y, Poynard T. Prediction of liver histological lesions with biochemical markers in patients with chronic hepatitis B. J Hepatol 2003;39:222-230.
74. Jacqueminet S, Lebray P, Morra R, Munteanu M, Devers L, Messous D, Bernard M, Hartemann-Heurtier A, Imbert-Bismut F, Ratziu V, Grimaldi A, Poynard T. Screening for liver fibrosis by using a noninvasive biomarker in patients with diabetes. Clin Gastroenterol Hepatol 2008;6:828-831.
75. Nunes D, Fleming C, Offner G, O'Brien M, Tumilty S, Fix O, Heeren T, Koziel M, Graham C, Craven DE, Stuver S, Horsburgh CR, Jr. HIV infection does not affect the performance of noninvasive markers of fibrosis for the diagnosis of hepatitis C virus-related liver disease. J Acquir Immune Defic Syndr 2005;40:538-544.
76. Poynard T, Zoulim F, Ratziu V, Degos F, Imbert-Bismut F, Deny P, Landais P, El HA, Slama A, Blin P, Thibault V, Parvaz P, Munteanu M, Trepo C. Longitudinal assessment of histology surrogate markers (FibroTest-ActiTest) during lamivudine therapy in patients with chronic hepatitis B infection. Am J Gastroenterol 2005;100:1970-1980.
77. Poynard T, Munteanu M, Imbert-Bismut F, Charlotte F, Thabut D, Le CS, Messous D, Thibault V, Benhamou Y, Moussalli J, Ratziu V. Prospective analysis of discordant results between biochemical markers and biopsy in patients with chronic hepatitis C. Clin Chem 2004;50:1344-1355.
78. Myers RP, Tainturier MH, Ratziu V, Piton A, Thibault V, Imbert-Bismut F, Messous D, Charlotte F, Di M, V, Benhamou Y, Poynard T. Prediction of liver histological lesions with biochemical markers in patients with chronic hepatitis B. J Hepatol 2003;39:222-230.
79. Carvalho-Filho RJ, Schiavon LL, Narciso-Schiavon JL, Sampaio JP, Lanzoni VP, Ferraz ML, Silva AE. Optimized cutoffs improve performance of the aspartate aminotransferase to platelet ratio index for predicting significant liver fibrosis in human immunodeficiency virus/hepatitis C virus co-infection. Liver Int 2008;28:486-493.
80. Al-Mohri H, Cooper C, Murphy T, Klein MB. Validation of a simple model for predicting liver fibrosis in HIV/hepatitis C virus-coinfected patients. HIV Med 2005;6:375-378.
81. Cales P, Laine F, Boursier J, Deugnier Y, Moal V, Oberti F, Hunault G, Rousselet MC, Hubert I, Laafi J, Ducluzeaux PH, Lunel F. Comparison of blood tests for liver fibrosis specific or not to NAFLD. J Hepatol 2008.
82. Paggi S, Colli A, Fraquelli M, Vigano M, Del PP, Facciotto C, Colombo M, Ronchi G, Conte D. A non-invasive algorithm accurately predicts advanced fibrosis in hepatitis C: a comparison using histology with internal-external validation. J Hepatol 2008;49:564-571.
83. Trang T, Petersen JR, Snyder N. Non-invasive markers of hepatic fibrosis in patients co-infected with HCV and HIV: comparison of the APRI and FIB-4 index. Clin Chim Acta 2008;397:51-54.
84. Snyder N, Gajula L, Xiao SY, Grady J, Luxon B, Lau DT, Soloway R, Petersen J. APRI: an easy and validated predictor of hepatic fibrosis in chronic hepatitis C. J Clin Gastroenterol 2006;40:535-542.
85. Snyder N, Nguyen A, Gajula L, Soloway R, Xiao SY, Lau DT, Petersen J. The APRI may be enhanced by the use of the FIBROSpect II in the estimation of fibrosis in chronic hepatitis C. Clin Chim Acta 2007;381:119-123.
86. Hongbo L, Xiaohui L, Hong K, Wei W, Yong Z. Assessing routine and serum markers of liver fibrosis in CHB patients using parallel and serial interpretation. Clin Biochem 2007;40:562-566.
87. Nunes D, Fleming C, Offner G, O'Brien M, Tumilty S, Fix O, Heeren T, Koziel M, Graham C, Craven DE, Stuver S, Horsburgh CR, Jr. HIV infection does not affect the performance of noninvasive markers of fibrosis for the diagnosis of hepatitis C virus-related liver disease. J Acquir Immune Defic Syndr 2005;40:538-544.
88. Adams LA, Bulsara M, Rossi E, DeBoer B, Speers D, George J, Kench J, Farrell G, McCaughan GW, Jeffrey GP. Hepascore: an accurate validated predictor of liver fibrosis in chronic hepatitis C infection. Clin Chem 2005;51:1867-1873.
89. Koda M, Matunaga Y, Kawakami M, Kishimoto Y, Suou T, Murawaki Y. FibroIndex, a practical index for predicting significant fibrosis in patients with chronic hepatitis C. Hepatology 2007;45:297-306.
90. Metwally MA, Zein CO, Zein NN. Predictors and noninvasive identification of severe liver fibrosis in patients with chronic hepatitis C. Dig Dis Sci 2007;52:582-588.
91. Mohamadnejad M, Montazeri G, Fazlollahi A, Zamani F, Nasiri J, Nobakht H, Forouzanfar MH, Abedian S, Tavangar SM, Mohamadkhani A, Ghoujeghi F, Estakhri A, Nouri N, Farzadi Z, Najjari A, Malekzadeh R. Noninvasive markers of liver fibrosis and inflammation in chronic hepatitis B-virus related liver disease. Am J Gastroenterol 2006;101:2537-2545.
92. Zaman A, Rosen HR, Ingram K, Corless CL, Oh E, Smith K. Assessment of FIBROSpect II to detect hepatic fibrosis in chronic hepatitis C patients. Am J Med 2007;120:280-14.
93. Patel K, Nelson DR, Rockey DC, Afdhal NH, Smith KM, Oh E, Hettinger K, Vallee M, Dev A, Smith-Riggs M, McHutchison JG. Correlation of FIBROSpect II with histologic and morphometric evaluation of liver fibrosis in chronic hepatitis C. Clin Gastroenterol Hepatol 2008;6:242-247.
94. Sebastiani G, Vario A, Guido M, Noventa F, Plebani M, Pistis R, Ferrari A, Alberti A. Stepwise combination algorithms of non-invasive markers to diagnose significant fibrosis in chronic hepatitis C. J Hepatol 2006;44:686-693.
95. Imbert-Bismut F, Ratziu V, Pieroni L, Charlotte F, Benhamou Y, Poynard T. Biochemical markers of liver fibrosis in patients with hepatitis C virus infection: a prospective study. Lancet 2001;357:1069-1075.
96. Nunes D, Fleming C, Offner G, O'Brien M, Tumilty S, Fix O, Heeren T, Koziel M, Graham C, Craven DE, Stuver S, Horsburgh CR, Jr. HIV infection does not affect the performance of noninvasive markers of fibrosis for the diagnosis of hepatitis C virus-related liver disease. J Acquir Immune Defic Syndr 2005;40:538-544.
97. Castera L, Vergniol J, Foucher J, Le BB, Chanteloup E, Haaser M, Darriet M, Couzigou P, de L, V. Prospective comparison of transient elastography, Fibrotest, APRI, and liver biopsy for the assessment of fibrosis in chronic hepatitis C. Gastroenterology 2005;128:343-350.
98. Guanabens N, Pares A, Alvarez L, Martinez de Osaba MJ, Monegal A, Peris P, Ballesta AM, Rodes J. Collagen-related markers of bone turnover reflect the severity of liver fibrosis in patients with primary biliary cirrhosis. J Bone Miner Res 1998;13:731-738.
99. Moller S, Hansen M, Hillingso J, Jensen JE, Henriksen JH. Elevated carboxy terminal cross linked telopeptide of type I collagen in alcoholic cirrhosis: relation to liver and kidney function and bone metabolism. Gut 1999;44:417-423.
100. Rosen HN, Parker RA, Greenspan SL, Iloputaife ID, Bookman L, Chapin D, Perlmutter I, Kessel B, Qvist P, Rosenblatt M. Evaluation of ability of biochemical markers of bone turnover to predict a response to increased doses of HRT. Calcif Tissue Int 2004;74:415-423.
101. Lein M, Wirth M, Miller K, Eickenberg HU, Weissbach L, Schmidt K, Haus U, Stephan C, Meissner S, Loening SA, Jung K. Serial Markers of Bone Turnover in Men with Metastatic Prostate Cancer Treated with Zoledronic Acid for Detection of Bone Metastases Progression. Eur Urol 2007.
102. Attallah AM, Toson EA, Shiha GE, Omran MM, bdel-Aziz MM, El-Dosoky I. Evaluation of serum procollagen aminoterminal propeptide III, laminin, and hydroxyproline as predictors of severe fibrosis in patients with chronic hepatitis C. J Immunoassay Immunochem 2007;28:199-211.
103. Ulrich D, Noah EM, von HD, Pallua N. TIMP-1, MMP-2, MMP-9, and PIIINP as serum markers for skin fibrosis in patients following severe burn trauma. Plast Reconstr Surg 2003;111:1423-1431.
104. Farkkila M, Rautiainen H, Karkkainen P, Karvonen AL, Nurmi H, Niemela O. Serological markers for monitoring disease progression in noncirrhotic primary biliary cirrhosis on ursodeoxycholic acid therapy. Liver Int 2008;28:787-797.
105. Guechot J, Poupon RE, Giral P, Balkau B, Giboudeau J, Poupon R. Relationship between procollagen III aminoterminal propeptide and hyaluronan serum levels and histological fibrosis in primary biliary cirrhosis and chronic viral hepatitis C. J Hepatol 1994;20:388-393.
106. Klappacher G, Franzen P, Haab D, Mehrabi M, Binder M, Plesch K, Pacher R, Grimm M, Pribill I, Eichler HG, . Measuring extracellular matrix turnover in the serum of patients with idiopathic or ischemic dilated cardiomyopathy and impact on diagnosis and prognosis. Am J Cardiol 1995;75:913-918.
107. Imbert-Bismut F, Ratziu V, Pieroni L, Charlotte F, Benhamou Y, Poynard T. Biochemical markers of liver fibrosis in patients with hepatitis C virus infection: a prospective study. Lancet 2001;357:1069-1075.
108. Suzuki,K., Enghild,J.J., Morodomi,T., Salvesen,G., and Nagase,H. 1990. Mechanisms of activation of tissue procollagenase by matrix metalloproteinase 3 (stromelysin). Biochemistry 29:10261-10270.
109. Lijnen,H.R. 2001. Plasmin and matrix metalloproteinases in vascular remodeling. Thromb. Haemost. 86:324-333.

## Claims

1. A method of immunoassay to measure neo-epitope containing protein fragments naturally present in a body fluid sample, wherein said immunoassay is conducted by a method comprising: contacting protein fragments naturally present in said sample with an immunological binding partner reactive with an N-terminal neo-epitope formed by cleavage of a protein by a proteinase or a C-terminal neo-epitope formed by cleavage of a protein by a proteinase, which immunological binding partner is non-reactive with the intact protein from which the epitope derives and is not reactive with a prolongated version of the neo-epitope sequence in which the neo-epitope sequence is prolonged past the cleavage site, and measuring the extent of binding of peptide fragments to said immunological binding partner to measure therein protein fragments comprising said neo-epitope, and wherein said protein is collagen type VI.

2. A method as claimed in claim 1, wherein said immunological binding partner specifically binds a neo-epitope constituted by an N-terminal amino acid sequence present in peptides produced by cleavage of collagen type VI, said peptides comprising an N-terminal sequence selected from the group consisting of:
| Collagen type VI | | |
|---|---|---|
| YRGPEG SEQ ID NO883 | PIGPKG SEQ ID NO865 | GIGIGN SEQ ID NO885 |
| ISGPRG SEQ ID NO886 | PGPAGP SEQ ID NO887 | VAAKPA SEQ ID NO888 |
| GEPGPP SEQ ID NO675 | RGPIGS SEQ ID NO889 | PPPPQP SEQ ID NO890 |
| AQGPAG SEQ ID NO891 | LIGEQG SEQ ID NO892 | PGLIGE SEQ ID NO893 |
| GEPGLN SEQ ID NO894 | IGPKGI SEQ ID NO895 | VAVVQH SEQ ID NO896 |
| FGPSAA SEQ ID NO897 | GPKGET SEQ ID NO898 | LGPMGV SEQ ID NO899 |
| PGEPGP SEQ ID NO784 | | |

3. A method as claimed in claim 1, wherein said immunological binding partner specifically binds a neo-epitope constituted by a C-terminal amino acid sequence present in peptides produced by cleavage of collagen type VI, said peptides comprising an C-terminal sequence selected from the group consisting of:
| Collagen type VI | | |
|---|---|---|
| GDEGPP SEQ ID NO900 | GNADIT SEQ ID NO901 | PAGPPG SEQ ID NO133 |
| DPGLMG SEQ ID NO902 | PEVPRP SEQ ID NO903 | TGPKGI SEQ ID NO904 |
| GDEGGP SEQ ID NO905 | PARSAS SEQ ID NO906 | TPAPPG SEQ ID NO915 |
| ISGPRG SEQ ID NO886 | GISGPR SEQ ID NO907 | GIGNRG SEQ ID NO908 |
| YRGYPG SEQ ID NO909 | KVEFSL SEQ ID NO910 | GVPGRD SEQ ID NO911 |
| PGETGK SEQ ID NO912 | RTGPLG SEQ ID NO913 | APGERG SEQ ID NO914 |

4. A method as claimed in any preceding claim, conducted as a method of diagnosis or quantitation of fibrosis and comprising associating an elevation of said measure of neo-epitope containing protein fragments in said body fluid sample above a normal level with the presence or extent of fibrosis.
